# EUROPÄISCHE PATENTANMELDUNG

(11) **EP 4 155 723 A1**
(43) Veröffentlichungstag der Anmeldung: **29.03.2023**
(21) Anmeldenummer: 21198500.7
(22) Anmeldetag: 23.09.2021
(51) Int. Cl.: G01N 27/404, G01N 33/00, H01M 8/00

(54) **ELEKTROCHEMISCHER GASSENSOR UND ELEKTROLYT FÜR EINEN ELEKTROCHEMISCHEN GASSENSOR**

(71) Anmelder: Dräger Safety AG & Co. KGaA, 23560 Lübeck (DE)
(72) Erfinder: Haupt, Stephan, 23558 Lübeck (DE); Eckhardt, Rolf, 23558 Lübeck (DE)
(74) Vertreter: Guthöhrlein, Gerhard

(57) **Zusammenfassung**

Die Erfindung betrifft einen Elektrolyt für einen elektrochemischen Gassensor und elektrochemischer Gassensor. Der Elektrolyt umfasst mindestens eine ringförmige Verbindung auf Basis eines Pyridinium-, Piperidinium-, Pyrrolidinium-, oder Pyrroliumringes, sowie mindestens 5% Wasser.

## Beschreibung

Die vorliegende Erfindung betrifft einen Elektrochemischen Gassensor und einen Elektrolyt für einen elektrochemischen Gassensor.

Elektrochemische Gassensoren sind allgemein bekannt. Sie kommen in den verschiedensten technischen Bereichen zum Einsatz. Waren es vor einigen Jahren vor allem Leck- oder Gasausbruchswarnsysteme, so werden die Sensoren inzwischen auch immer mehr zur Überwachung von Arbeitsplatzkonzentrationen von Schadgasen eingesetzt. Auch eine Überwachung von Produktionsprozessen mit Hilfe solcher Sensoren ist inzwischen denkbar.

Ein elektrochemischer Gassensor ist dabei stets eine elektrochemische Zelle, insbesondere eine Brennstoffzelle, die wenigstens zwei Elektroden aufweist, welche über einen lonenleiter (Elektrolyt) miteinander in Kontakt stehen. Das Zielgas (Analyt) wird an der ersten Elektrode (Messelektrode) elektrochemisch umgesetzt. Dabei ist der durch diese Umsetzung generierte Strom proportional zur anwesenden Gasmenge. Aus diesem Strom generiert sich das Signal, das z.B. von einem Gaswarngerät ausgegeben wird.

Der gebräuchlichste Elektrolyt ist gemeinhin Schwefelsäure. Da sich jedoch nicht alle Gase in sauren Medien umsetzen lassen, wurden weitere Elektrolytsysteme entwickelt, wie z.B. wässrige Lithiumchloridlösung, auf organischen Lösungsmitteln basierende Elektrolyte oder ionische Flüssigkeiten. So sieht beispielsweise DE 42 38 337 C2 einen Gassensor zum Nachweis von Ammoniak vor, dessen Elektrolyt eine wässrige Lösung eines hygroskopischen Salzes eines Alkali- oder Erdalkalimetalles oder einer Mischung daraus ist. Ein Problem bei der Verwendung von wässrigen Elektrolyten ist jedoch, dass diese austrocknen können.

DE 10 2008 044 240 B4 schlägt einen elektrochemischen Gassensor mit einer ionischen Flüssigkeit als Elektrolyt vor, welche mindestens ein Mono-, Di- oder Trialkylammonium-Kation enthält.
GB 2 395 564 B schlägt einen Elektrolyt auf Basis von 1-Ethyl-3-methylimidazolium-tetrafluoroborat oder 1-Ethyl-3-methylimidazolium-chlorid vor. Auch US 7,060,169 B2 schlägt einen Elektrolyten auf Basis einer ionischen Flüssigkeit vor, wobei die ionische Flüssigkeit entweder ein Imidazoliumkation oder ein Pyridiniumkation aufweist. Beispielsweise kann eine solche ionische Flüssigkeit ein alkyl- oder arylsubstituiertes Imidazoliumkation aufweisen.

All diese Lösungen verwenden Edelmetall- oder Kohleelektroden als Messelektroden. Grundsätzlich bietet dies zwar den Vorteil, dass unter idealen Bedingungen damit sehr geringe Gaskonzentrationen nachweisbar sein können. Allerdings setzt dies die Abwesenheit von sämtlichen anderen Gasen voraus, auf die der Sensor ebenfalls empfindlich ist. Bei Kohleelektroden ist zudem die Empfindlichkeit des Sensorsignales gegenüber Änderungen der Umgebungsfeuchte des Sensors problematisch. Dies kann insbesondere durch die Anwesenheit von hydrophoben Anionen im Elektrolyt, insbesondere bei der Verwendung ionischer Flüssigkeiten, noch verstärkt werden. Ausgehend davon ist es unter anderem Ziel der Erfindung, diese und weitere Nachteile des Standes der Technik zu überwinden und einen verbesserten elektrochemischen Gassensor, sowie einen verbesserten Elektrolyten für einen elektrochemischen Gassensor zu schaffen. Dieser soll kostengünstig und einfach herzustellen sein. Beispielsweise soll ein elektrochemischer Gassensor mit einem solchen Elektrolyten eine möglichst kurze Reaktionszeit und hohe Empfindlichkeit aufweisen. Außerdem soll der elektrochemische Gassensor möglichst unabhängig von der Umgebungsfeuchte sein. Weiterhin soll der Gassensor möglichst geringe Querempfindlichkeit aufweisen. Auch eine lange Standzeit ist wünschenswert. Ein solcher Gassensor soll beispielsweise zum Nachweis von SO₂ oder anderen elektrochemisch umsetzbaren Gasen verwendbar sein.

Zur Lösung dieser Aufgabe schlägt die Erfindung einen Elektrolyten für einen elektrochemischen Gassensor entsprechend Anspruch 1, sowie einen elektrochemischen Gassensor entsprechend Anspruch 12 vor und Verwendungen des Elektrolyten und des Gassensors entsprechend der Ansprüche 14 und 15 vor. Ausgestaltungen sind jeweils Gegenstand der abhängigen Ansprüche.

Bei einem Elektrolyt für einen elektrochemischen Gassensor, wobei der Elektrolyt wenigstens eine ringförmige Verbindung und wenigstens 5 Gewichtsprozent Wasser enthält, entspricht die ringförmige Verbindung der Formel (I)

[(A)R¹R²]⁺[Y]⁻

- wobei (A) eine Ringstruktur ist, ausgewählt aus der Gruppe Pyridinium, Piperidinium, Pyrolidinium;
- wobei der Rest R¹ am Stickstoffatom der Ringstruktur A angeordnet ist;
- wobei R¹ ein Kohlenwasserstoffrest ist mit 1, 2, 3, 4, 5, 6, 7, 8, 9, 10, 11 oder 12 C-Atomen;
- wobei R² ein Wasserstoffatom oder ein Kohlenwasserstoffrest mit 1, 2, 3 oder 4 C-Atomen ist;
- und wobei Y ausgewählt ist aus der Gruppe enthaltend Halogenid, Cyanid, Alkylsulfonat, halogeniertes Alkylsulfonat, Acetat.

Es hat sich gezeigt, dass ein elektrochemischer Gassensor, bei dem der Elektrolyt aus einer solchen Mischung einer solchen ringförmigen Verbindung mit wenigstens 5 Gewichtsprozent Wasser besteht, überraschend vorteilhaft in einem sehr breiten Potentialbereich und gleichzeitig in einem sehr breiten Umgebungsfeuchtebereich über einen langen Zeitraum betrieben werden kann. Insbesondere ist es dabei von Vorteil, wenn die ringförmige Verbindung dieser Zusammensetzung, die als Elektrolyt aktive Substanz ist. Es ist mithin denkbar, dass ein solcher Elektrolyt ausschließlich aus der ringförmigen Verbindung und dem Wasser besteht. Die ringförmige Verbindung kann mithin bis zu 95 Gewichtsprozent des Elektrolyten einnehmen.

Unter einem elektrochemischen Gassensor (Gassensor) wird dabei typischerweise eine elektrochemische Zelle mit wenigstens zwei Elektroden, die durch ein ionenleitendes Medium (Elektrolyt) miteinander in leitender Verbindung stehen, verstanden. In einer solchen elektrochemischen Zelle findet in Gegenwart eines nachzuweisenden Gases (Analyt) eine elektrochemische Reaktion statt. Der elektrochemische Gassensor entspricht mithin dem Funktionsprinzip einer Brennstoffzelle. Sinnvoll ist es daher, wenn der Gassensor ein Gehäuse aufweist, in dem die Elektroden und der Elektrolyt angeordnet sind. Das Gehäuse hat typischerweise eine Zutrittsöffnung, durch die der Analyt einströmen kann.

Unter einer ringförmigen Verbindung wird in diesem Zusammenhang eine Verbindung verstanden, die als zentrales Element einen Molekülring aufweist, an dem ein oder mehrere Seitenketten gebunden sein können. Entsprechend der Formel (I) ist der Molekülring die Ringstruktur A. Die Seitenketten sind die Reste R¹ und R². Man erkennt, dass die Ringstruktur bevorzugt ein Heteroaryl-oder Heteroalkyl-Ring mit 5 oder 6 Ringatomen ist, wobei als Fremdatom ein Stickstoffatom im Ring enthalten ist.

Die ringförmige Verbindung liegt bevorzugt als organisches Salz vor. Dabei bildet der Molekülring mit der Ringstruktur A und den Seitenketten R¹ und R² das Kation. Das Kation ist mithin in der Formel (I) durch den Bestandteil [(A)R¹R²]⁺ dargestellt.

Die Reste R¹ und R² können unabhängig voneinander Kohlenwasserstoffreste sein. Unter einem Kohlenwasserstoff wird dabei eine Verbindung verstanden, die ausschließlich aus Kohlenstoff und Wasserstoff besteht. Ein Kohlenwasserstoffrest mit 4 C-Atomen ist insofern Rest, der aus vier C-Atomen und bis zu neun H-Atomen besteht. Dabei ist die Anzahl der H-Atome abhängig von der Art und Weise, wie die C-Atome des Kohlenwasserstoffrestes mit einander verbunden sind.

Das Anion ist in der Formel (I) dargestellt durch den Bestandteil [Y]⁻. Es kann ein Halogenid, ein Cyanid, ein Alkylsulfonat, ein halogeniertes Alkylsulfonat oder ein Acetat sein. Unter einem Halogenid wird dabei das Ion eines Halogens verstanden. Unter einem Cyanid wird das Cyanid-Anion CN⁻ verstanden. Unter Alkylsulfonat werden die Sulfonate aliphatischer Sulfonsäuren verstanden, wobei unter einem Sulfonat das Anion des Salzes einer Sulfonsäure verstanden wird. Unter halogenierten Alkylsulfonaten werden die Sulfonate aliphatischer Sulfonsäuren verstanden, bei denen ein oder mehrere der enthaltenen Wasserstoffatome durch ein Halogen ausgetauscht wurde. Unter Acetat wird das Anion der Salze der Essigsäure verstanden.

Die Angabe Gewichtsprozent wird im Zusammenhang dieser Erfindung stets bezogen auf das Gesamtgewicht des Elektrolyten verstanden, soweit nicht explizit anders angegeben. Die Angabe Gewichtsprozent wird im Folgenden auch abgekürzt als Gew.-%. Weiterhin meint auch die Verwendung von % im Zusammenhang dieser Anmeldung Gewichtsprozent, sofern nicht explizit eine andere Angabe gemacht wird.

Ein so zusammengesetzter Elektrolyt bietet als besonderen Vorteil, dass er sehr austrocknungsresistent ist. Dies kann einen Einsatz bei sehr geringer Umgebungsfeuchte ermöglichen. Ein entsprechender Einsatzort kann zum Beispiel eine Wüste sein. Gleichzeitig handelt es sich bei den erfindungsgemäß vorgeschlagenen ringförmigen Verbindungen des Elektrolyten um organischen Salze, die weitestgehend inert gegenüber nahezu allen denkbaren Elektrodenmaterialien sind. Dies bietet den Vorteil, dass der Gassensor einen sehr niedrigen Nullstrom (Stromantwort des Sensors bei Abwesenheit von Analytgas) aufweist. Mithin ist das Signal zu Rausch-Verhältnis des erfindungsgemäßen Sensors gegenüber Sensoren aus dem Stand der Technik deutlich verbessert.

Überraschenderweise ist ein solcher erfindungsgemäßer Elektrolyt zudem in einem sehr großen Temperaturbereich einsetzbar.

Denkbar ist dabei auch, dass der Elektrolyt wenigstens eine weitere Komponente aufweist. Die Zusammensetzung, die den Elektrolyten bildet, besteht nicht notwendigerweise ausschließlich aus Wasser und wenigstens einer ringförmigen Verbindung, sondern sie kann weitere Substanzen enthalten. Man erkennt dabei, dass die ringförmige Verbindung und alle weiteren Komponenten gemeinsam bis zu 95 Gewichtsprozent des Elektrolyten ausmachen können. Dabei können die ringförmige Verbindung und die weitere Komponente in unterschiedlichen Verhältnissen zueinander vorliegen, bspw. im Verhältnis 1:1 oder auch andere.

Die weitere Komponente kann beispielsweise eine Puffersubstanz, ein Reaktionsvermittler, ein Zuschlag oder ein Gemisch aus zwei oder mehr dieser Komponenten sein.

Unter einer Puffersubstanz (Puffer) wird dabei typischerweise eine Substanz verstanden, die bewirkt, dass sich der pH-Wert eines Stoffgemisches, in welchem sie enthalten ist, bei Zugabe einer Säure oder einer Base wesentlich weniger stark ändert, als dies der Fall wäre, wenn die Puffersubstanz nicht in dem Stoffgemisch enthalten wäre.

Unter einem Reaktionsvermittler (Mediator) wird typischerweise eine Substanz verstanden, die die Reaktion des nachzuweisenden Gases an der Arbeitselektrode vermittelt. Insbesondere wird darunter eine Substanz verstanden, die mit einem gasförmigen Analyten reagiert und anschließend von der Arbeitselektrode in seinen Ausgangszustand zurückverwandelt wird, wobei die Rückverwandlungsreaktion den Sensorstrom liefert. Die Bezeichnungen Reaktionsvermittler und Mediator sind nachfolgend synonym.

Unter einem Zuschlag wird typischerweise eine Substanz verstanden, die zwar in einem Stoffgemisch enthalten ist, an der oder den primären Reaktionen des Stoffgemisches nicht teilnimmt. Die primären Reaktionen des Stoffgemisches sind in diesem Fall die Reaktion des nachzuweisenden Gases mit einem möglicherweise vorhandenen Mediator, die Umsetzung des nachzuweisenden Gases oder des durch die Reaktion mit dem Mediator entstandenen Produktes an der Arbeitselektrode und die zugehörige Rückreaktion an der Gegenelektrode.

Die Puffersubstanz kann beispielsweise ausgewählt sein aus der Gruppe enthaltend Kaliumhydrogenphthalat, Pyridiniumformiat, 3-Morpholinopropansulfonsäure (MOPS), 4-(2-Hydroxiethyl)piperazinyl-1-ethansulfonsäure (HEPES), 2,4,5-Triamino-6-hydroxylpyrimidinsulfat, Bis-(2-hydroxymethyl)aminomethan (Bis-Tris), Tris(hydroxymethyl)aminomethan (TRIS), Triethanolamin (TEA), 2-(Cyclohexylamino)ethansulfonsäure (CHES), N-[Tris-(hydroxymethyl)-methyl]-glycin (TRICIN), Piperazin-1,4-bis(2-ethansulfonsäure) (PIPES), N-(2-Acetamido)iminodiessigsäure (ADA), Ethylendiamintetraacetat (EDTA), Ethylenglycol-tris-aminoethylether-NNNN-tetraacetat (EGTA). Weitere Beispiele sind selbstverständlich vorstellbar. So kann die Puffersubstanz auch ein Carbonat-Puffer sein. Mit anderen Worten es kann vorteilhaft sein, wenn die Puffersubstanz ausgewählt ist aus der Gruppe enthaltend EDTA, EGTA, TEA, Bis-Tris, ADA, Tris, Kaliumhydrogenphthalat, Pyridiniumformiat, MOPS, HEPES, CHES, TRICIN, PIPES, Carbonat.

Der Puffer (Puffersubstanz) kann dabei sowohl ein saurer als auch ein basischer Puffer sein. Mit anderen Worten es ist beispielsweise vorstellbar, dass der Elektrolyt eine erste ringförmige Verbindung, Wasser und einen basischen Puffer enthält. Alternativ ist es beispielsweise auch vorstellbar, dass der Elektrolyt eine erste ringförmigen Verbindung, Wasser und einen sauren Puffer enthält. Unter einem basischen Puffer wird dabei ein Puffer verstanden, der den Elektrolyt im basischen Bereich abpuffert. Unter einem sauren Puffer wird dabei ein Puffer verstanden, der den Elektrolyt im sauren Bereich abpuffert. Dabei zeigt sich, dass ein besonderer Vorteil der vorliegenden Erfindung darin liegt, dass eine basische Pufferung des Elektrolyten möglich ist, oder mit anderen Worten, dass der Elektrolyt ein alkalischer Elektrolyt sein kann.

Der Reaktionsvermittler kann beispielsweise ausgewählt sein aus der Gruppe CuCl₂, CoCl₃, Chinon, Chinonderivate, MnCl₂.

Der Zuschlag kann beispielsweise, aber nicht abschließend, ausgewählt sein aus Methansulfonsäure, Kaliumacetat, Pyridinum-Acetat, BaCl₂, CaCl₂, EuCl₂. Weitere Zuschläge sind denkbar. Man erkennt, dass der Zuschlag ebenfalls eine puffernde Wirkung haben kann, auch wenn dies nicht notwendigerweise der Fall sein muss.

Mit anderen Worten in einer Ausführungsvariante ist es vorstellbar, dass der Elektrolyt eine erste ringförmige Verbindung entsprechend Formel (I) eine Puffersubstanz und Wasser enthält.

In einer weiteren Ausführungsvariante ist es denkbar, dass der Elektrolyt eine erste ringförmige Verbindung entsprechend Formel (I) einen Reaktionsvermittler und Wasser enthält.

In einer weiteren Ausführungsvariante ist es denkbar, dass der Elektrolyt eine erste ringförmige Verbindung entsprechend Formel (I) einen Zuschlag und Wasser enthält.

In einer weiteren Ausführungsvariante ist es denkbar, dass ringförmige Verbindung entsprechend Formel (I), eine zweite ringförmige Verbindung entsprechend Formel (I) und Wasser enthält.

In einer weiteren Ausführungsvariante ist es denkbar, dass der Elektrolyt eine erste ringförmige Verbindung entsprechend Formel (I), eine zweite ringförmige Verbindung entsprechend Formel (I), Wasser und einen Puffer enthält.

In noch einer weiteren Ausführungsvariante ist es denkbar, dass der Elektrolyt eine erste ringförmige Verbindung entsprechend Formel (I), eine zweite ringförmige Verbindung entsprechend Formel (I), Wasser und einen Reaktionsvermittler enthält.

In noch einer weiteren Ausführungsvariante ist es denkbar, dass der Elektrolyt eine erste ringförmige Verbindung entsprechend Formel (I), eine zweite ringförmige Verbindung entsprechend Formel (I), Wasser und einen Zuschlag enthält.

In noch einer weiteren Ausführungsvariante ist es denkbar, dass der Elektrolyt eine erste ringförmige Verbindung entsprechend Formel (I), eine zweite ringförmige Verbindung entsprechend Formel (I), Wasser, einen Puffer und einen Reaktionsvermittler enthält.

In weiteren Ausführungsvarianten ist es auch denkbar, dass der Elektrolyt eine erste ringförmige Verbindung entsprechend Formel (I), eine zweite ringförmige Verbindung entsprechend Formel (I), Wasser, einen Puffer und einen Zuschlag enthält, oder dass der Elektrolyt eine erste ringförmige Verbindung entsprechend Formel (I), eine zweite ringförmige Verbindung entsprechend Formel (I), Wasser, einen Reaktionsvermittler und einen Zuschlag enthält, oder dass der Elektrolyt eine erste ringförmige Verbindung entsprechend Formel (I), eine zweite ringförmige Verbindung entsprechend Formel (I), einen Puffer, einen Reaktionsvermittler, Wasser und einen Zuschlag enthält.

Für jede dieser denkbaren Ausführungsvarianten beträgt der Anteil des Wassers am Elektrolyten stets wenigstens 5 Gewichtsprozent.

Weiterhin ist für jede dieser denkbaren Ausführungsvarianten sowohl vorstellbar, dass der Puffer ein saurer Puffer ist, als auch dass der Puffer ein basischer Puffer ist.

In jedem Fall und unabhängig davon, ob der Elektrolyt nur eine oder mehrere verschiedene ringförmige Verbindungen wie oben definiert aufweist, ist es von Vorteil, wenn die die Ringstruktur jeweils eine Heterocycloalkan-Struktur oder eine Heteroaryl-Struktur ist. Man erkennt insbesondere, dass es günstig ist, wenn die Ringstruktur (A) eine Heterocycloalkan-Struktur oder eine Heteroaryl-Struktur mit genau einem Fremdatom im Ring ist. Dabei ist das Fremdatom wie aus der Formel hervorgeht ein Stickstoffatom. Beispielsweise ist es vorstellbar, dass die Ringstruktur ausgewählt ist aus der Gruppe enthaltend Pyrrolidin-Ring, Pyrrol-Ring, Piperidin-Ring, Pyridin-Ring. Synonym zu den vorgenannten Bezeichnungen werden auch die folgenden Begriffe verwendet und verstanden: Azolidin synonym zu Pyrrolidin, Azol synonym zu Pyrrol, Azinan synonym zu Piperidin, Azin synonym zu Pyridin.

Unter einem Pyrrolidin-Ring wird typischerweise eine Ringstruktur entsprechend der Formel II verstanden, unter einem Pyrrol-Ring eine Ringstruktur entsprechend der Formel III, unter einem Piperidin-Ring eine Ringstruktur entsprechend der Formel IV, unter einem Pyridin-Ring eine Ringstruktur entsprechend der Formel V, wobei das jeweils am Ringstickstoff angeordnete Wasserstoffatom der Formeln II, III, IV und V alternativ in jedem Fall auch durch einen anderen Rest ersetzt sein kann, wie nachfolgend aus der weiteren Beschreibung hervorgeht.

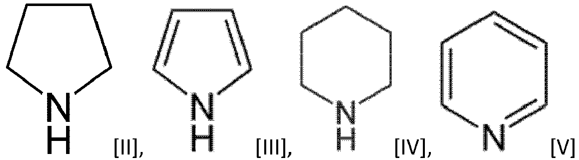

Man erkennt insofern, dass es sich bei den ringförmigen Verbindungen entsprechend Formel (I) insbesondere um Verbindungen handelt, die ausgewählt sind aus der Gruppe enthaltend substituierte Pyrrolidin-Salze, substituierte Pyrrol-Salze, substituierte Piperidin-Salze, substituierte Pyridin-Salze. Bevorzugt ist dabei wenigstens ein Substituent ein Alkyl-Rest, mithin ist es denkbar, dass die Verbindungen ausgewählt sind aus der Gruppe enthaltend alkyl-substituierte Pyrrolidin-Salze, alkyl-substituierte Pyrrol-Salze, alkyl-substituierte Piperidin-Salze, alkyl-substituierte Pyridin-Salze. Dabei ist sowohl denkbar, dass R¹ und R² beide jeweils ein Alkyl-Rest sind, als auch, dass nur R¹ ein Alkyl-Rest ist. Mit anderen Worten, man erkennt, dass es günstig ist, wenn R¹ eine Alkylgruppe ist und/oder wenn R¹ und R² Alkylgruppen sind. In einer weiteren Variante ist auch vorstellbar, dass R¹ und/oder R² substituierte Alkylgruppen sind. Dabei können in jedem Fall R¹ und R² sowohl gleiche als auch unterschiedliche Alkyl-Reste sein. In jedem Fall trägt dabei mindestens das Heteroatom, nämlich das Stickstoffatom, der Ringstruktur einen Alkyl-Rest. Die Begriffe Alkyl-Rest und Alkyl-Substituent werden dabei im Sinne der vorliegenden Anmeldung synonym verwendet. Beide Begriffe bezeichnen dabei einen Austausch eines Wasserstoffatomes gegen eine Alkyl-Gruppe. Mit anderen Worten, die ringförmigen Verbindungen können ausgewählt sein aus der Gruppe enthaltend mono-alkyl-substituierte Pyrrolidin-Salze, mono-alkyl-substituierte Pyrrol-Salze, mono-alkyl-substituierte Piperidin-Salze, mono-alkyl-substituierte Pyridin-Salze, di-alkyl-substituierte Pyrrolidin-Salze, di-alkyl-substituierte Pyrrol-Salze, di-alkyl-substituierte Piperidin-Salze, di-alkyl-substituierte Pyridin-Salze.

Als günstig hat sich dabei herausgestellt, wenn Y das Anion des Salzes bildet und ausgewählt ist aus der Gruppe enthaltend Halogenide, Cyanid, Alkylsulfonat, halogenierte Alkylsulfonat, Acetat, beispielsweise aus der Gruppe enthaltend Cl, F, I, Br, CN, CH₃SO₃, CF₃SO₃, CH₃COO. Im Zuge dieser Anmeldung gilt generell, dass die im Text genannten Anionen ohne zusätzliches Minus-Zeichen angegeben werden, da dieses jeweils in der allgemeinen Formel schon angegeben ist, so dass es beim Einsetzen der jeweiligen Bezeichnungen für den Platzhalter Y klar erkennbar ist, dass es sich um Anionen handelt, d.h. beispielsweise: Das zuvor genannte Cl ist ein Chloridanion, wie sich durch das Einsetzen von Cl für Y in den Formeln ergibt.

Man erkennt insofern, dass die ringförmigen Verbindungen entsprechend Formel (I) in einer ersten Ausführungsvariante der Formel VI 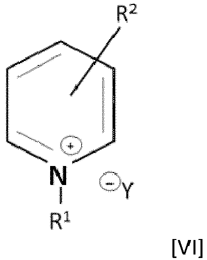 entsprechen, wobei R² ein Wasserstoffatom oder ein Kohlenwasserstoffrest mit 1, 2, 3 oder 4 C-Atomen ist, das Stickstoffatom des Ringes den Rest R¹ trägt, R¹ ein Kohlenwasserstoffrest ist mit 1, 2, 3, 4, 5, 6, 7, 8, 9, 10, 11 oder 12 C-Atomen, und wobei Y ausgewählt ist aus der Gruppe enthaltend Halogenide, Cyanid, Alkylsulfonat, halogeniertes Alkylsulfonat, Acetat, beispielsweise aus der Gruppe enthaltend Cl, F, I, Br, CN, CH₃SO₃, CF₃SO₃, CH₃COO. Man erkennt insofern, dass die Ringstruktur A in diesem Fall eine Ringstruktur mit sechs Atomen ist; d.h. es ist n = 5. Verbindungen entsprechend Formel VI sind mithin Pyridinium-Verbindungen.

In einer weiteren Ausführungsvariante entsprechen die ringförmigen Verbindungen entsprechend Formel (I) der Formel VII 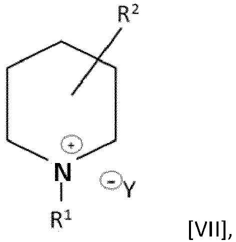 wobei ebenfalls R² ein Wasserstoffatom oder ein Kohlenwasserstoffrest mit 1, 2, 3 oder 4 C-Atomen ist, das Stickstoffatom des Ringes den Rest R¹ trägt, R¹ ein Kohlenwasserstoffrest ist mit 1, 2, 3, 4, 5, 6, 7, 8, 9, 10, 11 oder 12 C-Atomen, und wobei Y ausgewählt ist aus der Gruppe enthaltend Halogenide, Cyanid, Alkylsulfonat, halogeniertes Alkylsulfonat, Acetat, beispielsweise aus der Gruppe enthaltend Cl, F, I, Br, CN, CH₃SO₃, CF₃SO₃, CH₃COO. Man erkennt insofern, dass die Ringstruktur A in diesem Fall eine Ringstruktur mit sechs Atomen ist; d.h. n = 5. Verbindungen der Formel VII sind mithin Piperidinium-Verbindungen.

In einer weiteren Ausführungsvariante entsprechen die ringförmigen Verbindungen entsprechend Formel (I) der Formel VIII 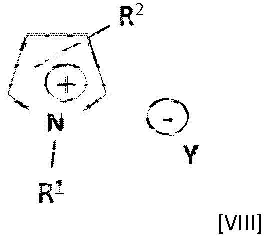 wobei ebenfalls R² ein Wasserstoffatom oder ein Kohlenwasserstoffrest mit 1, 2, 3 oder 4 C-Atomen ist, das Stickstoffatom des Ringes den Rest R¹ trägt, R¹ ein Kohlenwasserstoffrest ist mit 1, 2, 3, 4, 5, 6, 7, 8, 9, 10, 11 oder 12 C-Atomen, und wobei Y ausgewählt ist aus der Gruppe enthaltend Halogenide, Cyanid, Alkylsulfonat, halogeniertes Alkylsulfonat, Acetat. Man erkennt insofern, dass die Ringstruktur A in diesem Fall eine Ringstruktur mit fünf Atomen ist; d.h. n = 4. Verbindungen entsprechend Formel VIII sind mithin Pyrrolidinium-Verbindungen.

In einer weiteren Ausführungsvariante entsprechen die ringförmigen Verbindungen der Formel IX 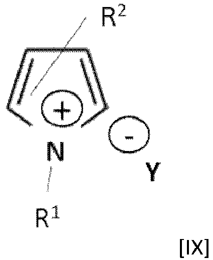 wobei ebenfalls R² ein Wasserstoffatom oder ein Kohlenwasserstoffrest mit 1, 2, 3 oder 4 C-Atomen ist, das Stickstoffatom des Ringes den Rest R¹ trägt, R¹ ein Kohlenwasserstoffrest ist mit 1, 2, 3, 4, 5, 6, 7, 8, 9, 10, 11 oder 12 C-Atomen, und wobei Y ausgewählt ist aus der Gruppe enthaltend Halogenide, Cyanid, Alkylsulfonat, halogeniertes Alkylsulfonat, Acetat. Man erkennt insofern, dass die Ringstruktur A in diesem Fall eine Ringstruktur mit fünf Atomen ist; d.h. n = 4. Verbindungen entsprechend Formel IX sind mithin Pyrrolium-Verbindungen.

In jedem Fall gilt für alle Verbindungen entsprechend einer der Formeln VI, VII, VIII oder IX, dass R² ein Wasserstoffatom oder ein Kohlenwasserstoffrest mit 1, 2, 3 oder 4 C-Atomen ist, X den Rest R¹ trägt, R¹ ein Kohlenwasserstoffrest ist mit 1, 2, 3, 4, 5, 6, 7, 8, 9, 10, 11 oder 12 C-Atomen, und dass Y ausgewählt ist aus der Gruppe enthaltend Halogenide, Cyanid, Alkylsulfonat, halogeniertes Alkylsulfonat, Acetat.

Man erkennt, dass von der Formel IX insbesondere Verbindungen entsprechend den folgenden Formeln (1), (2), (3), (4), (5), (6), (7), (8), (9), (10), (11) oder (12) 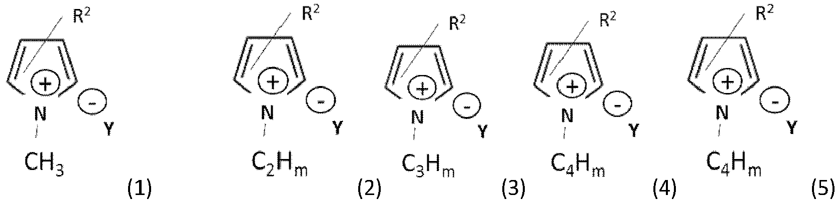 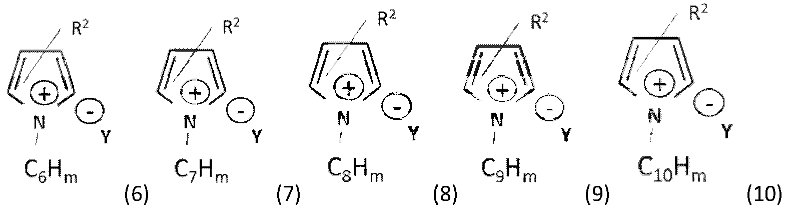 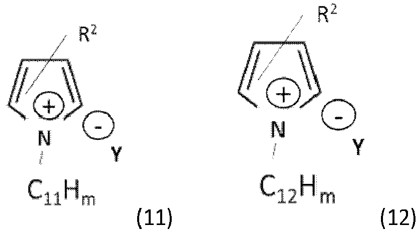 umfasst sind, wobei R² ein Wasserstoffatom oder ein Kohlenwasserstoffrest mit 1, 2, 3 oder 4 C-Atomen ist und Y ausgewählt ist aus der Gruppe enthaltend Halogenide, Cyanid, Alkylsulfonat, halogeniertes Alkylsulfonat, Acetat und wobei für Formel (2) m ausgewählt ist aus 3 oder 5, wobei für Formel (3) m ausgewählt ist aus 3, 5 oder 7, wobei für Formel (4) m ausgewählt ist aus 3, 5, 7, oder 9, wobei für Formel (5) m ausgewählt ist aus 3, 5, 7, 9 oder 11, wobei für Formel (6) m ausgewählt ist aus 3, 5, 7, 9, 11 oder 13, wobei für Formel (7) m ausgewählt ist aus 3, 5, 7, 9, 11, 13 oder 15, wobei für Formel (8) m ausgewählt ist aus 3, 5, 7, 9, 11, 13, 15 oder 17, wobei für Formel (9) m ausgewählt ist aus 3, 5, 7, 9, 11, 13, 15, 17 oder 19, wobei für Formel (10) m ausgewählt ist aus 3, 5, 7, 9, 11, 13, 15, 17, 19 oder 21, wobei für Formel (11) m ausgewählt ist aus 3, 5, 7, 9, 11, 13, 15, 17, 19, 21 oder 23, wobei für Formel (12) m ausgewählt ist aus 3, 5, 7, 9, 11, 13, 15, 17, 19, 21, 23 oder 25. Bevorzugt ist der Rest R¹ entweder gesättigt, einfach ungesättigt oder zweifach ungesättigt, so dass für Formel (2) m ausgewählt ist aus 3 oder 5, für Formel (3) m ausgewählt ist aus 3, 5 oder 7, für Formel (4) m ausgewählt ist aus 5, 7, oder 9, für Formel (5) m ausgewählt ist aus 7, 9 oder 11, für Formel (6) m ausgewählt ist aus 9, 11 oder 13, für Formel (7) m ausgewählt ist aus 11, 13 oder 15, für Formel (8) m ausgewählt ist aus 13, 15 oder 17, für Formel (9) m ausgewählt ist aus 15, 17 oder 19, für Formel (10) m ausgewählt ist aus 17, 19 oder 21, für Formel (11) m ausgewählt ist aus 19, 21 oder 23, und für Formel (12) m ausgewählt ist aus 21, 23 oder 25.

Besonders bevorzugt ist der Rest R¹ ein gesättigter Alkylrest, so dass die Formel IX insbesondere Verbindungen entsprechend Formel (1) sowie entsprechend den folgenden Formeln (13), (14), (15), (16), (17), (18), (19), (20), (21), (22) und (23) 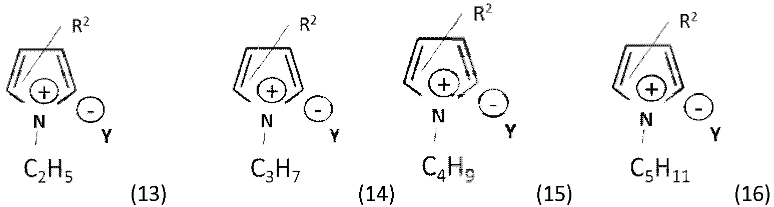 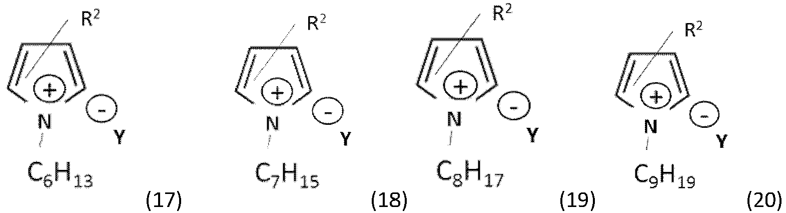 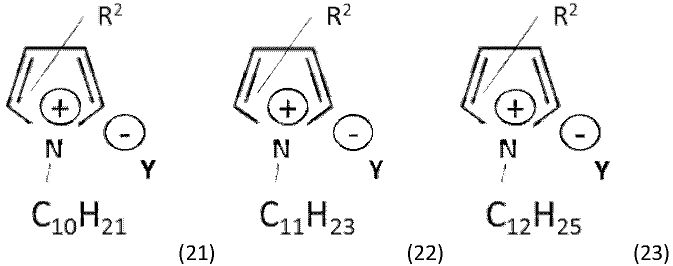 umfasst, wobei R² ein Wasserstoffatom oder ein Kohlenwasserstoffrest mit 1, 2, 3 oder 4 C-Atomen ist und Y ausgewählt ist aus der Gruppe enthaltend Halogenide, Cyanid, Alkylsulfonat, halogeniertes Alkylsulfonat, Acetat.

Man erkennt, dass eine Verbindung entsprechend Formel (1) beispielsweise ausgewählt sein kann aus der Gruppe enthaltend N-Methylpyrroliumchlorid, 1,2-Dimethylpyrroliumchlorid, 1-Methyl-2-ethylpyrroliumchlorid, 1-Methyl-2-propylpyrroliumchlorid, 1-Methyl-2-butylpyrroliumchlorid, 1,3-Dimethylpyrroliumchlorid, 1-Methyl-3-ethylpyrroliumchlorid, 1-Methyl-3-propylpyrroliumchlorid, 1-Methyl-3-butylpyrroliumchlorid, N-Methylpyrroliumfluorid, , 1,2-Dimethylpyrrolium fluorid, 1-Methyl-2-ethylpyrroliumfluorid, 1-Methyl-2-propylpyrroliumfluorid, 1-Methyl-2-butylpyrroliumfluorid, 1,3-Dimethylpyrroliumfluorid, 1-Methyl-3-ethylpyrroliumfluorid, 1-Methyl-3-propylpyrroliumfluorid, 1-Methyl-3-butylpyrroliumfluorid, N-Methylpyrroliumtriflat, 1,2-Dimethylpyrroliumtriflat, 1-Methyl-2-ethylpyrroliumtriflat, 1-Methyl-2-propylpyrroliumtriflat, 1-Methyl-2-butylpyrroliumtriflat, 1,3-Dimethylpyrroliumtriflat, 1-Methyl-3-ethylpyrroliumtriflat, 1-Methyl-3-propylpyrroliumtriflat, 1-Methyl-3-butylpyrroliumtriflat, N-Methylpyrroliummethansulfonat, 1,2-Dimethylpyrroliummethansulfonat, 1-Methyl-2-ethylpyrroliummethansulfonat, 1-Methyl-2-propylpyrroliummethansulfonat, 1-Methyl-2-butylpyrroliummethansulfonat, 1,3-Dimethylpyrroliummethansulfonat, 1-Methyl-3-ethylpyrroliummethansulfonat, 1-Methyl-3-propylpyrroliummethansulfonat, 1-Methyl-3-butylpyrroliummethansulfonat, N-Methylpyrroliumacetat, 1,2-Dimethylpyrroliumacetat, 1-Methyl-2-ethylpyrroliumacetat, 1-Methyl-2-propylpyrroliumacetat, 1-Methyl-2-butylpyrroliumacetat, 1,3-Dimethylpyrroliumacetat, 1-Methyl-3-ethylpyrroliumacetat, 1-Methyl-3-propylpyrroliumacetat, 1-Methyl-3-butylpyrroliumacetat, N-Methylpyrroliumcyanid, 1,2-Dimethylpyrroliumcyanid, 1-Methyl-2-ethylpyrroliumcyanid, 1-Methyl-2-propylpyrroliumcyanid, 1-Methyl-2-butylpyrroliumcyanid, 1,3-Dimethylpyrroliumcyanid, 1-Methyl-3-ethylpyrroliumcyanid, 1-Methyl-3-propylpyrroliumcyanid, 1-Methyl-3-butylpyrroliumcyanid.

Man erkennt, dass eine Verbindung entsprechend Formel (2) beispielsweise ausgewählt sein kann aus der Gruppe enthaltend N-Ethylpyrroliumchlorid, 1-Ethyl-2-methylpyrroliumchlorid, 1,2-Diethylpyrroliumchlorid, 1-Ethyl-2-propylpyrroliumchlorid, 1-Ethyl-2-butylpyrroliumchlorid, 1-Ethyl-3-methylpyrroliumchlorid, 1,3-Diethylpyrroliumchlorid, 1-Ethyl-3-propylpyrroliumchlorid, 1-Ethyl-3-butylpyrroliumchlorid, N-Ethylpyrroliumfluorid, 1-Ethyl-2-methylpyrroliumfluorid, 1,2-Diethylpyrroliumfluorid, 1-Ethyl-2-propylpyrroliumfluorid, 1-Ethyl-2-butylpyrroliumfluorid, 1-Ethyl-3-methylpyrroliumfluorid, 1,3-Diethylpyrroliumfluorid, 1-Ethyl-3-propylpyrroliumfluorid, 1-Ethyl-3-butylpyrroliumfluorid, N-Ethylpyrroliumtriflat, 1-Ethyl-2-methylpyrroliumtriflat, 1,2-Diethylpyrroliumtriflat, 1-Ethyl-2-propylpyrroliumtriflat, 1-Ethyl-2-butylpyrroliumtriflat, 1-Ethyl-3-methylpyrroliumtriflat, 1,3-Diethylpyrroliumtriflat, 1-Ethyl-3-propylpyrroliumtriflat, 1-Ethyl-3-butylpyrroliumtriflat, N-Ethylpyrroliummethansulfonat, 1-Ethyl-2-methylpyrroliummethansulfonat, 1,2-Diethylpyrroliummethansulfonat, 1-Ethyl-2-propylpyrroliummethansulfonat, 1-Ethyl-2-butylpyrroliummethansulfonat, 1-Ethyl-3-methylpyrroliummethansulfonat, 1,3-Diethylpyrroliummethansulfonat, 1-Ethyl-3-propylpyrroliummethansulfonat, 1-Ethyl-3-butylpyrroliummethansulfonat, N-Ethylpyrroliumacetat, 1-Ethyl-2-methylpyrroliumacetat, 1,2-Diethylpyrroliumacetat, 1-Ethyl-2-propylpyrroliumacetat, 1-Ethyl-2-butylpyrroliumacetat, 1-Ethyl-3-methylpyrroliumacetat, 1,3-Diethylpyrroliumacetat, 1-Ethyl-3-propylpyrroliumacetat, 1-Ethyl-3-butylpyrroliumacetat, N-Ethylpyrroliumcyanid, 1-Ethyl-2-methylpyrroliumcyanid, 1,2-Diethylpyrroliumcyanid, 1-Ethyl-2-propylpyrroliumcyanid, 1-Ethyl-2-butylpyrroliumcyanid, 1-Ethyl-3-methylpyrroliumcyanid, 1,3-Diethylpyrroliumcyanid, 1-Ethyl-3-propylpyrroliumcyanid, 1-Ethyl-3-butylpyrroliumcyanid.

Man erkennt, dass eine Verbindung entsprechend Formel (3) beispielsweise ausgewählt sein kann aus der Gruppe enthaltend N-Propylpyrroliumchlorid, 1-Propyl-2-Methylpyrroliumchlorid, 1-Propyl-2-ethylpyrroliumchlorid, 1,2-Dipropylpyrroliumchlorid, 1-Propyl-2-butylpyrroliumchlorid, 1-Propyl-3-Methylpyrroliumchlorid, 1-Propyl-3-ethylpyrroliumchlorid, 1,3-Dipropylpyrroliumchlorid, 1-Propyl-3-butylpyrroliumchlorid, N-Propylpyrroliumfluorid, 1-Propyl-2-Methylpyrroliumfluorid, 1-Propyl-2-ethylpyrroliumfluorid, 1,2-Dipropylpyrroliumfluorid, 1-Propyl-2-butylpyrroliumfluorid, 1-Propyl-3-Methylpyrroliumfluorid, 1-Propyl-3-ethylpyrroliumfluorid, 1,3-Dipropylpyrroliumfluorid, 1-Propyl-3-butylpyrroliumfluorid, N-Propylpyrroliumtriflat, 1-Propyl-2-Methylpyrroliumtriflat, 1-Propyl-2-ethylpyrroliumtriflat, 1,2-Dipropylpyrroliumtriflat, 1-Propyl-2-butylpyrroliumtriflat, 1-Propyl-3-Methylpyrroliumtriflat, 1-Propyl-3-ethylpyrroliumtriflat, 1,3-Dipropylpyrroliumtriflat, 1-Propyl-3-butylpyrroliumtriflat, N-Propylpyrroliummethansulfonat, 1-Propyl-2-Methylpyrroliummethansulfonat, 1-Propyl-2-ethylpyrroliummethansulfonat, 1,2-Dipropylpyrroliummethansulfonat, 1-Propyl-2-butylpyrroliummethansulfonat, 1-Propyl-3-Methylpyrroliummethansulfonat, 1-Propyl-3-ethylpyrroliummethansulfonat, 1,3-Dipropylpyrroliummethansulfonat, 1-Propyl-3-butylpyrroliummethansulfonat, N-Propylpyrroliumacetat, 1-Propyl-2-Methylpyrroliumacetat, 1-Propyl-2-ethylpyrroliumacetat, 1,2-Dipropylpyrroliumacetat, 1-Propyl-2-butylpyrroliumacetat, 1-Propyl-3-Methylpyrroliumacetat, 1-Propyl-3-ethylpyrroliumacetat, 1,3-Dipropylpyrroliumacetat, 1-Propyl-3-butylpyrroliumacetat, N-Propylpyrroliumcyanid, 1-Propyl-2-Methylpyrroliumcyanid, 1-Propyl-2-ethylpyrroliumcyanid, 1,2-Dipropylpyrroliumcyanid, 1-Propyl-2-butylpyrroliumcyanid, 1-Propyl-3-Methylpyrroliumcyanid, 1-Propyl-3-ethylpyrroliumcyanid, 1,3-Dipropylpyrroliumcyanid, 1-Propyl-3-butylpyrroliumcyanid.

Man erkennt, dass eine Verbindung entsprechend Formel (4) beispielsweise ausgewählt sein kann aus der Gruppe enthaltend N-Butylpyrroliumchlorid, 1-Butyl-2-Methylpyrroliumchlorid, 1-Butyl-2-ethylpyrroliumchlorid, 1-Butyl-2-propylpyrroliumchlorid, 1,2-Dibutylpyrroliumchlorid, 1-Butyl-3-Methylpyrroliumchlorid, 1-Butyl-3-ethylpyrroliumchlorid, 1-Butyl-3-propylpyrroliumchlorid, 1,3-Dibutylpyrroliumchlorid, N-Butylpyrroliumfluorid, 1-Butyl-2-Methylpyrroliumfluorid, 1-Butyl-2-ethylpyrroliumfluorid, 1-Butyl-2-propylpyrroliumfluorid, 1,2-Dibutylpyrroliumfluorid, 1-Butyl-3-Methylpyrroliumfluorid, 1-Butyl-3-ethylpyrroliumfluorid, 1-Butyl-3-propylpyrroliumfluorid, 1,3-Dibutylpyrroliumfluorid, N-Butylpyrroliumtriflat, 1-Butyl-2-Methylpyrroliumtriflat, 1-Butyl-2-ethylpyrroliumtriflat, 1-Butyl-2-propylpyrroliumtriflat, 1,2-Dibutylpyrroliumtriflat, 1-Butyl-3-Methylpyrroliumtriflat, 1-Butyl-3-ethylpyrroliumtriflat, 1-Butyl-3-propylpyrroliumtriflat, 1,3-Dibutylpyrroliumtriflat, N-Butylpyrroliummethansulfonat, 1-Butyl-2-Methylpyrroliummethansulfonat, 1-Butyl-2-ethylpyrroliummethansulfonat, 1-Butyl-2-propylpyrroliummethansulfonat, 1,2-Dibutylpyrroliummethansulfonat, 1-Butyl-3-Methylpyrroliummethansulfonat, 1-Butyl-3-ethylpyrroliummethansulfonat, 1-Butyl-3-propylpyrroliummethansulfonat, 1,3-Dibutylpyrroliummethansulfonat, N-Butylpyrroliumacetat, 1-Butyl-2-Methylpyrroliumacetat, 1-Butyl-2-ethylpyrroliumacetat, 1-Butyl-2-propylpyrroliumacetat, 1,2-Dibutylpyrroliumacetat, 1-Butyl-3-Methylpyrroliumacetat, 1-Butyl-3-ethylpyrroliumacetat, 1-Butyl-3-propylpyrroliumacetat, 1,3-Dibutylpyrroliumacetat, N-Butylpyrroliumcyanid, 1-Butyl-2-Methylpyrroliumcyanid, 1-Butyl-2-ethylpyrroliumcyanid, 1-Butyl-2-propylpyrroliumcyanid, 1,2-Dibutylpyrroliumcyanid, 1-Butyl-3-Methylpyrroliumcyanid, 1-Butyl-3-ethylpyrroliumcyanid, 1-Butyl-3-propylpyrroliumcyanid, 1,3-Dibutylpyrroliumcyanid.

Man erkennt, dass eine Verbindung entsprechend Formel (5) beispielsweise ausgewählt sein kann aus der Gruppe enthaltend N-Pentylpyrroliumchlorid, 1-Pentyl-2-Methylpyrroliumchlorid, 1-Pentyl-2-ethylpyrroliumchlorid, 1-Pentyl-2-propylpyrroliumchlorid, 1-Pentyl-2-butylpyrroliumchlorid, 1-Pentyl-3-Methylpyrroliumchlorid, 1-Pentyl-3-ethylpyrroliumchlorid, 1-Pentyl-3-propylpyrroliumchlorid, 1-Pentyl-3-butylpyrroliumchlorid, N-Pentylpyrroliumfluorid, 1-Pentyl-2-Methylpyrroliumfluorid, 1-Pentyl-2-ethylpyrroliumfluorid, 1-Pentyl-2-propylpyrroliumfluorid, 1-Pentyl-2-butylpyrroliumfluorid, 1-Pentyl-3-Methylpyrroliumfluorid, 1-Pentyl-3-ethylpyrroliumfluorid, 1-Pentyl-3-propylpyrroliumfluorid, 1-Pentyl-3-butylpyrroliumfluorid, N-Pentylpyrroliumtriflat, 1-Pentyl-2-Methylpyrroliumtriflat, 1-Pentyl-2-ethylpyrroliumtriflat, 1-Pentyl-2-propylpyrroliumtriflat, 1-Pentyl-2-butylpyrroliumtriflat, 1-Pentyl-3-Methylpyrroliumtriflat, 1-Pentyl-3-ethylpyrroliumtriflat, 1-Pentyl-3-propylpyrroliumtriflat, 1-Pentyl-3-butylpyrroliumtriflat, N-Pentylpyrroliummethansulfonat, 1-Pentyl-2-Methylpyrroliummethansulfonat, 1-Pentyl-2-ethylpyrroliummethansulfonat, 1-Pentyl-2-propylpyrroliummethansulfonat, 1-Pentyl-2-butylpyrroliummethansulfonat, 1-Pentyl-3-Methylpyrroliummethansulfonat, 1-Pentyl-3-ethylpyrroliummethansulfonat, 1-Pentyl-3-propylpyrroliummethansulfonat, 1-Pentyl-3-butylpyrroliummethansulfonat, N-Pentylpyrroliumacetat, 1-Pentyl-2-Methylpyrroliumacetat, 1-Pentyl-2-ethylpyrroliumacetat, 1-Pentyl-2-propylpyrroliumacetat, 1-Pentyl-2-butylpyrroliumacetat, 1-Pentyl-3-Methylpyrroliumacetat, 1-Pentyl-3-ethylpyrroliumacetat, 1-Pentyl-3-propylpyrroliumacetat, 1-Pentyl-3-butylpyrroliumacetat, N-Pentylpyrroliumcyanid, 1-Pentyl-2-Methylpyrroliumcyanid, 1-Pentyl-2-ethylpyrroliumcyanid, 1-Pentyl-2-propylpyrroliumcyanid, 1-Pentyl-2-butylpyrroliumcyanid, 1-Pentyl-3-Methylpyrroliumcyanid, 1-Pentyl-3-ethylpyrroliumcyanid, 1-Pentyl-3-propylpyrroliumcyanid, 1-Pentyl-3-butylpyrroliumcyanid.

Man erkennt weiter, dass eine Verbindung entsprechend Formel (6) ausgewählt sein kann aus der Gruppe enthaltend N-Hexylpyrroliumchlorid, 1-Hexyl-2-Methylpyrroliumchlorid, 1-Hexyl-2-ethylpyrroliumchlorid, 1-Hexyl-2-propylpyrroliumchlorid, 1-Hexyl-2-butylpyrroliumchlorid, 1-Hexyl-3-Methylpyrroliumchlorid, 1-Hexyl-3-ethylpyrroliumchlorid, 1-Hexyl-3-propylpyrroliumchlorid, 1-Hexyl-3-butylpyrroliumchlorid, N-Hexylpyrroliumfluorid, 1-Hexyl-2-Methylpyrroliumfluorid, 1-Hexyl-2-ethylpyrroliumfluorid, 1-Hexyl-2-propylpyrroliumfluorid, 1-Hexyl-2-butylpyrroliumfluorid, 1-Hexyl-3-Methylpyrroliumfluorid, 1-Hexyl-3-ethylpyrroliumfluorid, 1-Hexyl-3-propylpyrroliumfluorid, 1-Hexyl-3-butylpyrroliumfluorid, N-Hexylpyrroliumtriflat, 1-Hexyl-2-Methylpyrroliumtriflat, 1-Hexyl-2-ethylpyrroliumtriflat, 1-Hexyl-2-propylpyrroliumtriflat, 1-Hexyl-2-butylpyrroliumtriflat, 1-Hexyl-3-Methylpyrroliumtriflat, 1-Hexyl-3-ethylpyrroliumtriflat, 1-Hexyl-3-propylpyrroliumtriflat, 1-Hexyl-3-butylpyrroliumtriflat, N-Hexylpyrroliummethansulfonat, 1-Hexyl-2-Methylpyrroliummethansulfonat, 1-Hexyl-2-ethylpyrroliummethansulfonat, 1-Hexyl-2-propylpyrroliummethansulfonat, 1-Hexyl-2-butylpyrroliummethansulfonat, 1-Hexyl-3-Methylpyrroliummethansulfonat, 1-Hexyl-3-ethylpyrroliummethansulfonat, 1-Hexyl-3-propylpyrroliummethansulfonat, 1-Hexyl-3-butylpyrroliummethansulfonat, N-Hexylpyrroliumacetat, 1-Hexyl-2-Methylpyrroliumacetat, 1-Hexyl-2-ethylpyrroliumacetat, 1-Hexyl-2-propylpyrroliumacetat, 1-Hexyl-2-butylpyrroliumacetat, 1-Hexyl-3-Methylpyrroliumacetat, 1-Hexyl-3-ethylpyrroliumacetat, 1-Hexyl-3-propylpyrroliumacetat, 1-Hexyl-3-butylpyrroliumacetat, N-Hexylpyrroliumcyanid, 1-Hexyl-2-Methylpyrroliumcyanid, 1-Hexyl-2-ethylpyrroliumcyanid, 1-Hexyl-2-propylpyrroliumcyanid, 1-Hexyl-2-butylpyrroliumcyanid, 1-Hexyl-3-Methylpyrroliumcyanid, 1-Hexyl-3-ethylpyrroliumcyanid, 1-Hexyl-3-propylpyrroliumcyanid, 1-Hexyl-3-butylpyrroliumcyanid.

Man erkennt weiter, dass eine Verbindung entsprechend Formel (7) ausgewählt sein kann aus der Gruppe enthaltend N-Heptylpyrroliumchlorid, 1-Heptyl-2-Methylpyrroliumchlorid, 1-Heptyl-2-ethylpyrroliumchlorid, 1-Heptyl-2-propylpyrroliumchlorid, 1-Heptyl-2-butylpyrroliumchlorid, 1-Heptyl-3-Methylpyrroliumchlorid, 1-Heptyl-3-ethylpyrroliumchlorid, 1-Heptyl-3-propylpyrroliumchlorid, 1-Heptyl-3-butylpyrroliumchlorid, N-Heptylpyrroliumfluorid, 1-Heptyl-2-Methylpyrroliumfluorid, 1-Heptyl-2-ethylpyrroliumfluorid, 1-Heptyl-2-propylpyrroliumfluorid, 1-Heptyl-2-butylpyrroliumfluorid, 1-Heptyl-3-Methylpyrroliumfluorid, 1-Heptyl-3-ethylpyrroliumfluorid, 1-Heptyl-3-propylpyrroliumfluorid, 1-Heptyl-3-butylpyrroliumfluorid, N-Heptylpyrroliumtriflat, 1-Heptyl-2-Methylpyrroliumtriflat, 1-Heptyl-2-ethylpyrroliumtriflat, 1-Heptyl-2-propylpyrroliumtriflat, 1-Heptyl-2-butylpyrroliumtriflat, 1-Heptyl-3-Methylpyrroliumtriflat, 1-Heptyl-3-ethylpyrroliumtriflat, 1-Heptyl-3-propylpyrroliumtriflat, 1-Heptyl-3-butylpyrroliumtriflat, N-Heptylpyrroliummethansulfonat, 1-Heptyl-2-Methylpyrroliummethansulfonat, 1-Heptyl-2-ethylpyrroliummethansulfonat, 1-Heptyl-2-propylpyrroliummethansulfonat, 1-Heptyl-2-butylpyrroliummethansulfonat, 1-Heptyl-3-Methylpyrroliummethansulfonat, 1-Heptyl-3-ethylpyrroliummethansulfonat, 1-Heptyl-3-propylpyrroliummethansulfonat, 1-Heptyl-3-butylpyrroliummethansulfonat, N-Heptylpyrroliumacetat, 1-Heptyl-2-Methylpyrroliumacetat, 1-Heptyl-2-ethylpyrroliumacetat, 1-Heptyl-2-propylpyrroliumacetat, 1-Heptyl-2-butylpyrroliumacetat, 1-Heptyl-3-Methylpyrroliumacetat, 1-Heptyl-3-ethylpyrroliumacetat, 1-Heptyl-3-propylpyrroliumacetat, 1-Heptyl-3-butylpyrroliumacetat, N-Heptylpyrroliumcyanid, 1-Heptyl-1-methylpyrroliumcyanid, 1-Heptyl-2-Methylpyrroliumcyanid, 1-Heptyl-2-ethylpyrroliumcyanid, 1-Heptyl-2-propylpyrroliumcyanid, 1-Heptyl-2-butylpyrroliumcyanid, 1-Heptyl-3-Methylpyrroliumcyanid, 1-Heptyl-3-ethylpyrroliumcyanid, 1-Heptyl-3-propylpyrroliumcyanid, 1-Heptyl-3-butylpyrroliumcyanid.

Man erkennt weiter, dass eine Verbindung entsprechend Formel (8) ausgewählt sein kann aus der Gruppe enthaltend N-Octylpyrroliumchlorid, 1-Octyl-2-Methylpyrroliumchlorid, 1-Octyl-2-ethylpyrroliumchlorid, 1-Octyl-2-propylpyrroliumchlorid, 1-Octyl-2-butylpyrroliumchlorid, 1-Octyl-3-Methylpyrroliumchlorid, 1-Octyl-3-ethylpyrroliumchlorid, 1-Octyl-3-propylpyrroliumchlorid, 1-Octyl-3-butylpyrroliumchlorid, N-Octylpyrroliumfluorid, 1-Octyl-2-Methylpyrroliumfluorid, 1-Octyl-2-ethylpyrroliumfluorid, 1-Octyl-2-propylpyrroliumfluorid, 1-Octyl-2-butylpyrroliumfluorid, 1-Octyl-3-Methylpyrroliumfluorid, 1-Octyl-3-ethylpyrroliumfluorid, 1-Octyl-3-propylpyrroliumfluorid, 1-Octyl-3-butylpyrroliumfluorid, N-Octylpyrroliumtriflat, 1-Octyl-2-Methylpyrroliumtriflat, 1-Octyl-2-ethylpyrroliumtriflat, 1-Octyl-2-propylpyrroliumtriflat, 1-Octyl-2-butylpyrroliumtriflat, 1-Octyl-3-Methylpyrroliumtriflat, 1-Octyl-3-ethylpyrroliumtriflat, 1-Octyl-3-propylpyrroliumtriflat, 1-Octyl-3-butylpyrroliumtriflat, N-Octylpyrroliummethansulfonat, 1-Octyl-2-Methylpyrroliummethansulfonat, 1-Octyl-2-ethylpyrroliummethansulfonat, 1-Octyl-2-propylpyrroliummethansulfonat, 1-Octyl-2-butylpyrroliummethansulfonat, 1-Octyl-3-Methylpyrroliummethansulfonat, 1-Octyl-3-ethylpyrroliummethansulfonat, 1-Octyl-3-propylpyrroliummethansulfonat, 1-Octyl-3-butylpyrroliummethansulfonat, N-Octylpyrroliumacetat, 1-Octyl-2-Methylpyrroliumacetat, 1-Octyl-2-ethylpyrroliumacetat, 1-Octyl-2-propylpyrroliumacetat, 1-Octyl-2-butylpyrroliumacetat, 1-Octyl-3-Methylpyrroliumacetat, 1-Octyl-3-ethylpyrroliumacetat, 1-Octyl-3-propylpyrroliumacetat, 1-Octyl-3-butylpyrroliumacetat, N-Octylpyrroliumcyanid, 1-Octyl-2-Methylpyrroliumcyanid, 1-Octyl-2-ethylpyrroliumcyanid, 1-Octyl-2-propylpyrroliumcyanid, 1-Octyl-2-butylpyrroliumcyanid, 1-Octyl-3-Methylpyrroliumcyanid, 1-Octyl-3-ethylpyrroliumcyanid, 1-Octyl-3-propylpyrroliumcyanid, 1-Octyl-3-butylpyrroliumcyanid.

Man erkennt weiter, dass eine Verbindung entsprechend Formel (9) ausgewählt sein kann aus der Gruppe enthaltend N-Nonylpyrroliumchlorid, 1-Nonyl-2-Methylpyrroliumchlorid, 1-Nonyl-2-ethylpyrroliumchlorid, 1-Nonyl-2-propylpyrroliumchlorid, 1-Nonyl-2-butylpyrroliumchlorid, 1-Nonyl-3-Methylpyrroliumchlorid, 1-Nonyl-3-ethylpyrroliumchlorid, 1-Nonyl-3-propylpyrroliumchlorid, 1-Nonyl-3-butylpyrroliumchlorid, N-Nonylpyrroliumfluorid, 1-Nonyl-2-Methylpyrroliumfluorid, 1-Nonyl-2-ethylpyrroliumfluorid, 1-Nonyl-2-propylpyrroliumfluorid, 1-Nonyl-2-butylpyrroliumfluorid, 1-Nonyl-3-Methylpyrroliumfluorid, 1-Nonyl-3-ethylpyrroliumfluorid, 1-Nonyl-3-propylpyrroliumfluorid, 1-Nonyl-3-butylpyrroliumfluorid, N-Nonylpyrroliumtriflat, 1-Nonyl-2-Methylpyrroliumtriflat, 1-Nonyl-2-ethylpyrroliumtriflat, 1-Nonyl-2-propylpyrroliumtriflat, 1-Nonyl-2-butylpyrroliumtriflat, 1-Nonyl-3-Methylpyrroliumtriflat, 1-Nonyl-3-ethylpyrroliumtriflat, 1-Nonyl-3-propylpyrroliumtriflat, 1-Nonyl-3-butylpyrroliumtriflat, N-Nonylpyrroliummethansulfonat, 1-Nonyl-2-Methylpyrroliummethansulfonat, 1-Nonyl-2-ethylpyrroliummethansulfonat, 1-Nonyl-2-propylpyrroliummethansulfonat, 1-Nonyl-2-butylpyrroliummethansulfonat, 1-Nonyl-3-Methylpyrroliummethansulfonat, 1-Nonyl-3-ethylpyrroliummethansulfonat, 1-Nonyl-3-propylpyrroliummethansulfonat, 1-Nonyl-3-butylpyrroliummethansulfonat, N-Nonylpyrroliumacetat, 1-Nonyl-2-Methylpyrroliumacetat, 1-Nonyl-2-ethylpyrroliumacetat, 1-Nonyl-2-propylpyrroliumacetat, 1-Nonyl-2-butylpyrroliumacetat, 1-Nonyl-3-Methylpyrroliumacetat, 1-Nonyl-3-ethylpyrroliumacetat, 1-Nonyl-3-propylpyrroliumacetat, 1-Nonyl-3-butylpyrroliumacetat, N-Nonylpyrroliumcyanid, 1-Nonyl-2-Methylpyrroliumcyanid, 1-Nonyl-2-ethylpyrroliumcyanid, 1-Nonyl-2-propylpyrroliumcyanid, 1-Nonyl-2-butylpyrroliumcyanid, 1-Nonyl-3-Methylpyrroliumcyanid, 1-Nonyl-3-ethylpyrroliumcyanid, 1-Nonyl-3-propylpyrroliumcyanid, 1-Nonyl-3-butylpyrroliumcyanid.

Man erkennt weiter, dass eine Verbindung entsprechend Formel (10) ausgewählt sein kann aus der Gruppe enthaltend N-Decylpyrroliumchlorid, 1-Decyl-2-Methylpyrroliumchlorid, 1-Decyl-2-ethylpyrroliumchlorid, 1-Decyl-2-propylpyrroliumchlorid, 1-Decyl-2-butylpyrroliumchlorid, 1-Decyl-3-Methylpyrroliumchlorid, 1-Decyl-3-ethylpyrroliumchlorid, 1-Decyl-3-propylpyrroliumchlorid, 1-Decyl-3-butylpyrroliumchlorid, N-Decylpyrroliumfluorid, 1-Decyl-l-butylpyrroliumfluorid, 1-Decyl-2-Methylpyrroliumfluorid, 1-Decyl-2-ethylpyrroliumfluorid, 1-Decyl-2-propylpyrroliumfluorid, 1-Decyl-2-butylpyrroliumfluorid, 1-Decyl-3-Methylpyrroliumfluorid, 1-Decyl-3-ethylpyrroliumfluorid, 1-Decyl-3-propylpyrroliumfluorid, 1-Decyl-3-butylpyrroliumfluorid, N-Decylpyrroliumtriflat, 1-Decyl-2-Methylpyrroliumtriflat, 1-Decyl-2-ethylpyrroliumtriflat, 1-Decyl-2-propylpyrroliumtriflat, 1-Decyl-2-butylpyrroliumtriflat, 1-Decyl-3-Methylpyrroliumtriflat, 1-Decyl-3-ethylpyrroliumtriflat, 1-Decyl-3-propylpyrroliumtriflat, 1-Decyl-3-butylpyrroliumtriflat, N-Decylpyrroliummethansulfonat, 1-Decyl-2-Methylpyrroliummethansulfonat, 1-Decyl-2-ethylpyrroliummethansulfonat, 1-Decyl-2-propylpyrroliummethansulfonat, 1-Decyl-2-butylpyrroliummethansulfonat, 1-Decyl-3-Methylpyrroliummethansulfonat, 1-Decyl-3-ethylpyrroliummethansulfonat, 1-Decyl-3-propylpyrroliummethansulfonat, 1-Decyl-3-butylpyrroliummethansulfonat, N-Decylpyrroliumacetat, 1-Decyl-2-Methylpyrroliumacetat, 1-Decyl-2-ethylpyrroliumacetat, 1-Decyl-2-propylpyrroliumacetat, 1-Decyl-2-butylpyrroliumacetat, 1-Decyl-3-Methylpyrroliumacetat, 1-Decyl-3-ethylpyrroliumacetat, 1-Decyl-3-propylpyrroliumacetat, 1-Decyl-3-butylpyrroliumacetat, N-Decylpyrroliumcyanid, 1-Decyl-2-Methylpyrroliumcyanid, 1-Decyl-2-ethylpyrroliumcyanid, 1-Decyl-2-propylpyrroliumcyanid, 1-Decyl-2-butylpyrroliumcyanid, 1-Decyl-3-Methylpyrroliumcyanid, 1-Decyl-3-ethylpyrroliumcyanid, 1-Decyl-3-propylpyrroliumcyanid, 1-Decyl-3-butylpyrroliumcyanid.

Man erkennt weiter, dass ein Verbindung entsprechend Formel (11) ausgewählt sein kann aus der Gruppe enthaltend N-Undecylpyrroliumchlorid, 1-Undecyl-2-Methylpyrroliumchlorid, 1-Undecyl-2-ethylpyrroliumchlorid, 1-Undecyl-2-propylpyrroliumchlorid, 1-Undecyl-2-butylpyrroliumchlorid, 1-Undecyl-3-Methylpyrroliumchlorid, 1-Undecyl-3-ethylpyrroliumchlorid, 1-Undecyl-3-propylpyrroliumchlorid, 1-Undecyl-3-butylpyrroliumchlorid, N-Undecylpyrroliumfluorid, 1-Undecyl-2-Methylpyrroliumfluorid, 1-Undecyl-2-ethylpyrroliumfluorid, 1-Undecyl-2-propylpyrroliumfluorid, 1-Undecyl-2-butylpyrroliumfluorid, 1-Undecyl-3-Methylpyrroliumfluorid, 1-Undecyl-3-ethylpyrroliumfluorid, 1-Undecyl-3-propylpyrroliumfluorid, 1-Undecyl-3-butylpyrroliumfluorid, N-Undecylpyrroliumtriflat, 1-Undecyl-2-Methylpyrroliumtriflat, 1-Undecyl-2-ethylpyrroliumtriflat, 1-Undecyl-2-propylpyrroliumtriflat, 1-Undecyl-2-butylpyrroliumtriflat, 1-Undecyl-3-Methylpyrroliumtriflat, 1-Undecyl-3-ethylpyrroliumtriflat, 1-Undecyl-3-propylpyrroliumtriflat, 1-Undecyl-3-butylpyrroliumtriflat, N-Undecylpyrroliummethansulfonat, 1-Undecyl-2-ethylpyrroliummethansulfonat, 1-Undecyl-2-propylpyrroliummethansulfonat, 1-Undecyl-2-butylpyrroliummethansulfonat, 1-Undecyl-3-Methylpyrroliummethansulfonat, 1-Undecyl-3-ethylpyrroliummethansulfonat, 1-Undecyl-3-propylpyrroliummethansulfonat, 1-Undecyl-3-butylpyrroliummethansulfonat, N-Undecylpyrroliumacetat, 1-Undecyl-2-Methylpyrroliumacetat, 1-Undecyl-2-ethylpyrroliumacetat, 1-Undecyl-2-propylpyrroliumacetat, 1-Undecyl-2-butylpyrroliumacetat, 1-Undecyl-3-Methylpyrroliumacetat, 1-Undecyl-3-ethylpyrroliumacetat, 1-Undecyl-3-propylpyrroliumacetat, 1-Undecyl-3-butylpyrroliumacetat, N-Undecylpyrroliumcyanid, 1-Undecyl-2-Methylpyrroliumcyanid, 1-Undecyl-2-ethylpyrroliumcyanid, 1-Undecyl-2-propylpyrroliumcyanid, 1-Undecyl-2-butylpyrroliumcyanid, 1-Undecyl-3-Methylpyrroliumcyanid, 1-Undecyl-3-ethylpyrroliumcyanid, 1-Undecyl-3-propylpyrroliumcyanid, 1-Undecyl-3-butylpyrroliumcyanid.

Man erkennt weiter, dass eine Verbindung entsprechend Formel (12) ausgewählt sein kann aus der Gruppe enthaltend N-Dodecylpyrroliumchlorid, 1-Dodecyl-2-Methylpyrroliumchlorid, 1-Dodecyl-2-ethylpyrroliumchlorid, 1-Dodecyl-2-propylpyrroliumchlorid, 1-Dodecyl-2-butylpyrroliumchlorid, 1-Dodecyl-3-Methylpyrroliumchlorid, 1-Dodecyl-3-ethylpyrroliumchlorid, 1-Dodecyl-3-propylpyrroliumchlorid, 1-Dodecyl-3-butylpyrroliumchlorid, N-Dodecylpyrroliumfluorid, 1-Dodecyl-2-Methylpyrroliumfluorid, 1-Dodecyl-2-ethylpyrroliumfluorid, 1-Dodecyl-2-propylpyrroliumfluorid, 1-Dodecyl-2-butylpyrroliumfluorid, 1-Dodecyl-3-Methylpyrroliumfluorid, 1-Dodecyl-3-ethylpyrroliumfluorid, 1-Dodecyl-3-propylpyrroliumfluorid, 1-Dodecyl-3-butylpyrroliumfluorid, N-Dodecylpyrroliumtriflat, 1-Dodecyl-2-Methylpyrroliumtriflat, 1-Dodecyl-2-ethylpyrroliumtriflat, 1-Dodecyl-2-propylpyrroliumtriflat, 1-Dodecyl-2-butylpyrroliumtriflat, 1-Dodecyl-3-Methylpyrroliumtriflat, 1-Dodecyl-3-ethylpyrroliumtriflat, 1-Dodecyl-3-propylpyrroliumtriflat, 1-Dodecyl-3-butylpyrroliumtriflat, N-Dodecylpyrroliummethansulfonat, 1-Dodecyl-2-Methylpyrroliummethansulfonat, 1-Dodecyl-2-ethylpyrroliummethansulfonat, 1-Dodecyl-2-propylpyrroliummethansulfonat, 1-Dodecyl-2-butylpyrroliummethansulfonat, 1-Dodecyl-3-Methylpyrroliummethansulfonat, 1-Dodecyl-3-ethylpyrroliummethansulfonat, 1-Dodecyl-3-propylpyrroliummethansulfonat, 1-Dodecyl-3-butylpyrroliummethansulfonat, N-Dodecylpyrroliumacetat, 1-Dodecyl-2-Methylpyrroliumacetat, 1-Dodecyl-2-ethylpyrroliumacetat, 1-Dodecyl-2-propylpyrroliumacetat, 1-Dodecyl-2-butylpyrroliumacetat, 1-Dodecyl-3-Methylpyrroliumacetat, 1-Dodecyl-3-ethylpyrroliumacetat, 1-Dodecyl-3-propylpyrroliumacetat, 1-Dodecyl-3-butylpyrroliumacetat, N-Dodecylpyrroliumcyanid, 1-Dodecyl-2-Methylpyrroliumcyanid, 1-Dodecyl-2-ethylpyrroliumcyanid, 1-Dodecyl-2-propylpyrroliumcyanid, 1-Dodecyl-2-butylpyrroliumcyanid, 1-Dodecyl-3-Methylpyrroliumcyanid, 1-Dodecyl-3-ethylpyrroliumcyanid, 1-Dodecyl-3-propylpyrroliumcyanid, 1-Dodecyl-3-butylpyrroliumcyanid.

Man erkennt weiterhin, dass von der Formel VIII insbesondere Verbindungen entsprechend den folgenden Formeln (24), (25), (26), (27), (28), (29), (30), (31), (32), (33), (34) oder (35) 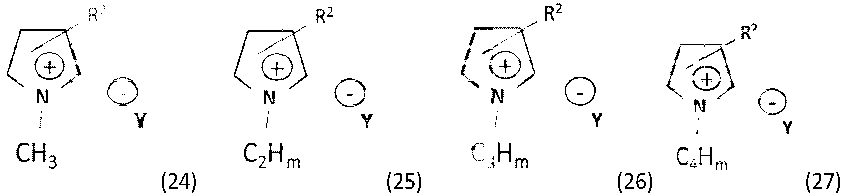 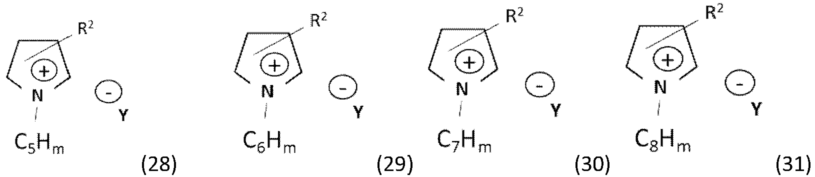 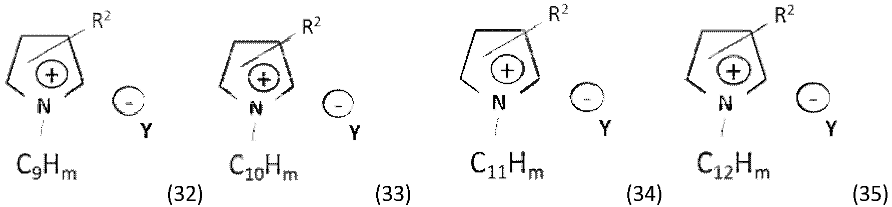 umfasst sind, wobei R² ein Wasserstoffatom oder ein Kohlenwasserstoffrest mit 1, 2, 3 oder 4 C-Atomen ist und Y ausgewählt ist aus der Gruppe enthaltend Halogenide, Cyanid, Alkylsulfonat, halogeniertes Alkylsulfonat, Acetat und wobei für Formel (25) m ausgewählt ist aus 3 oder 5, wobei für Formel (26) m ausgewählt ist aus 3, 5 oder 7, wobei für Formel (27) m ausgewählt ist aus 3, 5, 7, oder 9, wobei für Formel (28) m ausgewählt ist aus 3, 5, 7, 9 oder 11, wobei für Formel (29) m ausgewählt ist aus 3, 5, 7, 9, 11 oder 13, wobei für Formel (30) m ausgewählt ist aus 3, 5, 7, 9, 11, 13 oder 15, wobei für Formel (31) m ausgewählt ist aus 3, 5, 7, 9, 11, 13, 15 oder 17, wobei für Formel (32) m ausgewählt ist aus 3, 5, 7, 9, 11, 13, 15, 17 oder 19, wobei für Formel (33) m ausgewählt ist aus 3, 5, 7, 9, 11, 13, 15, 17, 19 oder 21, wobei für Formel (34) m ausgewählt ist aus 3, 5, 7, 9, 11, 13, 15, 17, 19, 21 oder 23, wobei für Formel (35) m ausgewählt ist aus 3, 5, 7, 9, 11, 13, 15, 17, 19, 21, 23 oder 25. Bevorzugt ist der Rest R¹ entweder gesättigt, einfach ungesättigt oder zweifach ungesättigt, so dass für Formel (25) m ausgewählt ist aus 3 oder 5, für Formel (26) m ausgewählt ist aus 3, 5 oder 7, für Formel (27) m ausgewählt ist aus 5, 7, oder 9, für Formel (28) m ausgewählt ist aus 7, 9 oder 11, für Formel (29) m ausgewählt ist aus 9, 11 oder 13, für Formel (30) m ausgewählt ist aus 11, 13 oder 15, für Formel (31) m ausgewählt ist aus 13, 15 oder 17, für Formel (32) m ausgewählt ist aus 15, 17 oder 19, für Formel (33) m ausgewählt ist aus 17, 19 oder 21, für Formel (34) m ausgewählt ist aus 19, 21 oder 23, und für Formel (35) m ausgewählt ist aus 21, 23 oder 25.

Besonders bevorzugt ist der Rest R¹ ein gesättigter Alkylrest, so dass die Formel VII insbesondere Verbindungen entsprechend Formel (25) sowie entsprechend den folgenden Formeln (36), (37), (38), (39), (40), (41), (42), (43), (44), (45) und (46) 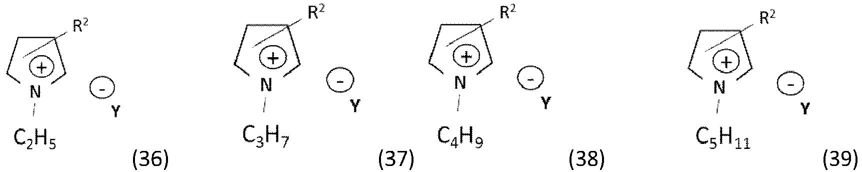 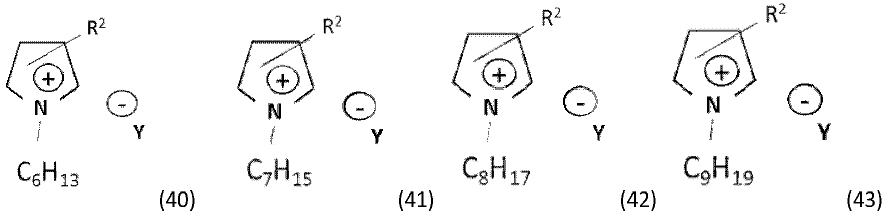 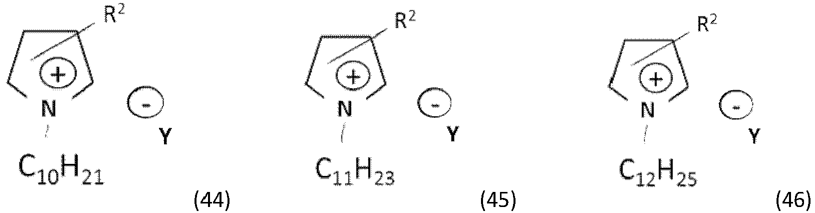 umfasst, wobei R² ein Wasserstoffatom oder ein Kohlenwasserstoffrest mit 1, 2, 3 oder 4 C-Atomen ist und Y ausgewählt ist aus der Gruppe enthaltend Halogenide, Cyanid, Alkylsulfonat, halogeniertes Alkylsulfonat, Acetat.

Man erkennt, dass eine Verbindung entsprechend Formel (24) beispielsweise ausgewählt sein kann aus der Gruppe enthaltend N-Methylpyrrolidiniumchlorid, 1,1-Dimethylpyrrolidiniumchlorid, 1-Methyl-1-ethylpyrrolidiniumchlorid, 1-Methyl-1-propylpyrrolidiniumchlorid, 1-Methyl-1-butylpyrrolidiniumchlorid, 1,2-Dimethylpyrrolidiniumchlorid, 1-Methyl-2-ethylpyrrolidiniumchlorid, 1-Methyl-2-propylpyrrolidiniumchlorid, 1-Methyl-2-butylpyrrolidiniumchlorid, 1,3-Dimethylpyrrolidiniumchlorid, 1-Methyl-3-ethylpyrrolidiniumchlorid, 1-Methyl-3-propylpyrrolidiniumchlorid, 1-Methyl-3-butylpyrrolidiniumchlorid, N-Methylpyrrolidiniumfluorid, 1,1-Dimethylpyrrolidiniumfluorid, 1-Methyl-1-ethylpyrrolidiniumfluorid, 1-Methyl-1-propylpyrrolidiniumfluorid, 1-Methyl-1-butylpyrrolidiniumfluorid, 1,2-Dimethylpyrrolidinium fluorid, 1-Methyl-2-ethylpyrrolidiniumfluorid, 1-Methyl-2-propylpyrrolidiniumfluorid, 1-Methyl-2-butylpyrrolidiniumfluorid, 1,3-Dimethylpyrrolidiniumfluorid, 1-Methyl-3-ethylpyrrolidiniumfluorid, 1-Methyl-3-propylpyrrolidiniumfluorid, 1-Methyl-3-butylpyrrolidiniumfluorid, N-Methylpyrrolidiniumtriflat, 1,1-Dimethylpyrrolidiniumtriflat, 1-Methyl-1-ethylpyrrolidiniumtriflat, 1-Methyl-1-propylpyrrolidiniumtriflat, 1-Methyl-1-butylpyrrolidiniumtriflat, 1,2-Dimethylpyrrolidiniumtriflat, 1-Methyl-2-ethylpyrrolidiniumtriflat, 1-Methyl-2-propylpyrrolidiniumtriflat, 1-Methyl-2-butylpyrrolidiniumtriflat, 1,3-Dimethylpyrrolidiniumtriflat, 1-Methyl-3-ethylpyrrolidiniumtriflat, 1-Methyl-3-propylpyrrolidiniumtriflat, 1-Methyl-3-butylpyrrolidiniumtriflat, N-Methylpyrrolidiniummethansulfonat, 1,1-Dimethylpyrrolidiniummethansulfonat, 1-Methyl-1-ethylpyrrolidiniummethansulfonat, 1-Methyl-1-propylpyrrolidiniummethansulfonat, 1-Methyl-1-butylpyrrolidiniummethansulfonat, 1,2-Dimethylpyrrolidiniummethansulfonat, 1-Methyl-2-ethylpyrrolidiniummethansulfonat, 1-Methyl-2-propylpyrrolidiniummethansulfonat, 1-Methyl-2-butylpyrrolidiniummethansulfonat, 1,3-Dimethylpyrrolidiniummethansulfonat, 1-Methyl-3-ethylpyrrolidiniummethansulfonat, 1-Methyl-3-propylpyrrolidiniummethansulfonat, 1-Methyl-3-butylpyrrolidiniummethansulfonat, N-Methylpyrrolidiniumacetat, 1,1-Dimethylpyrrolidiniumacetat, 1-Methyl-1-ethylpyrrolidiniumacetat, 1-Methyl-1-propylpyrrolidiniumacetat, 1-Methyl-1-butylpyrrolidiniumacetat, 1,2-Dimethylpyrrolidiniumacetat, 1-Methyl-2-ethylpyrrolidiniumacetat, 1-Methyl-2-propylpyrrolidiniumacetat, 1-Methyl-2-butylpyrrolidiniumacetat, 1,3-Dimethylpyrrolidiniumacetat, 1-Methyl-3-ethylpyrrolidiniumacetat, 1-Methyl-3-propylpyrrolidiniumacetat, 1-Methyl-3-butylpyrrolidiniumacetat, N-Methylpyrrolidiniumcyanid, 1,1-Dimethylpyrrolidiniumcyanid, 1-Methyl-1-ethylpyrrolidiniumcyanid, 1-Methyl-1-propylpyrrolidiniumcyanid, 1-Methyl-1-butylpyrrolidiniumcyanid, 1,2-Dimethylpyrrolidiniumcyanid, 1-Methyl-2-ethylpyrrolidiniumcyanid, 1-Methyl-2-propylpyrrolidiniumcyanid, 1-Methyl-2-butylpyrrolidiniumcyanid, 1,3-Dimethylpyrrolidiniumcyanid, 1-Methyl-3-ethylpyrrolidiniumcyanid, 1-Methyl-3-propylpyrrolidiniumcyanid, 1-Methyl-3-butylpyrrolidiniumcyanid.

Man erkennt, dass eine Verbindung entsprechend Formel (25) beispielsweise ausgewählt sein kann aus der Gruppe enthaltend N-Ethylpyrrolidiniumchlorid, 1,1-Diethylpyrrolidiniumchlorid, 1-Ethyl-1-Methylpyrrolidiniumchlorid, 1-Ethyl-1-propylpyrrolidiniumchlorid, 1-Ethyl-1-butylpyrrolidiniumchlorid, 1-Ethyl-2-methylpyrrolidiniumchlorid, 1,2-Diethylpyrrolidiniumchlorid, 1-Ethyl-2-propylpyrrolidiniumchlorid, 1-Ethyl-2-butylpyrrolidiniumchlorid, 1-Ethyl-3-methylpyrrolidiniumchlorid, 1,3-Diethylpyrrolidiniumchlorid, 1-Ethyl-3-propylpyrrolidiniumchlorid, 1-Ethyl-3-butylpyrrolidiniumchlorid, N-Ethylpyrrolidiniumfluorid, 1,1-Diethylpyrrolidiniumfluorid, 1-Ethyl-1-Methylpyrrolidiniumfluorid, 1-Ethyl-1-propylpyrrolidiniumfluorid, 1-Ethyl-1-butylpyrrolidiniumfluorid, 1-Ethyl-2-methylpyrrolidiniumfluorid, 1,2-Diethylpyrrolidiniumfluorid, 1-Ethyl-2-propylpyrrolidiniumfluorid, 1-Ethyl-2-butylpyrrolidiniumfluorid, 1-Ethyl-3-methylpyrrolidiniumfluorid, 1,3-Diethylpyrrolidiniumfluorid, 1-Ethyl-3-propylpyrrolidiniumfluorid, 1-Ethyl-3-butylpyrrolidiniumfluorid, N-Ethylpyrrolidiniumtriflat, 1,1-Diethylpyrrolidiniumtriflat, 1-Ethyl-1-Methylpyrrolidiniumtriflat, 1-Ethyl-1-propylpyrrolidiniumtriflat, 1-Ethyl-1-butylpyrrolidiniumtriflat, 1-Ethyl-2-methylpyrrolidiniumtriflat, 1,2-Diethylpyrrolidiniumtriflat, 1-Ethyl-2-propylpyrrolidiniumtriflat, 1-Ethyl-2-butylpyrrolidiniumtriflat, 1-Ethyl-3-methylpyrrolidiniumtriflat, 1,3-Diethylpyrrolidiniumtriflat, 1-Ethyl-3-propylpyrrolidiniumtriflat, 1-Ethyl-3-butylpyrrolidiniumtriflat, N-Ethylpyrrolidiniummethansulfonat, 1,1-Diethylpyrrolidiniummethansulfonat, 1-Ethyl-1-Methylpyrrolidiniummethansulfonat, 1-Ethyl-1-propylpyrrolidiniummethansulfonat, 1-Ethyl-1-butylpyrrolidiniummethansulfonat, 1-Ethyl-2-methylpyrrolidiniummethansulfonat, 1,2-Diethylpyrrolidiniummethansulfonat, 1-Ethyl-2-propylpyrrolidiniummethansulfonat, 1-Ethyl-2-butylpyrrolidiniummethansulfonat, 1-Ethyl-3-methylpyrrolidiniummethansulfonat, 1,3-Diethylpyrrolidiniummethansulfonat, 1-Ethyl-3-propylpyrrolidiniummethansulfonat, 1-Ethyl-3-butylpyrrolidiniummethansulfonat, N-Ethylpyrrolidiniumacetat, 1,1-Diethylpyrrolidiniumacetat, 1-Ethyl-1-Methylpyrrolidiniumacetat, 1-Ethyl-1-propylpyrrolidiniumacetat, 1-Ethyl-1-butylpyrrolidiniumacetat, 1-Ethyl-2-methylpyrrolidiniumacetat, 1,2-Diethylpyrrolidiniumacetat, 1-Ethyl-2-propylpyrrolidiniumacetat, 1-Ethyl-2-butylpyrrolidiniumacetat, 1-Ethyl-3-methylpyrrolidiniumacetat, 1,3-Diethylpyrrolidiniumacetat, 1-Ethyl-3-propylpyrrolidiniumacetat, 1-Ethyl-3-butylpyrrolidiniumacetat, N-Ethylpyrrolidiniumcyanid, 1,1-Diethylpyrrolidiniumcyanid, 1-Ethyl-1-Methylpyrrolidiniumcyanid, 1-Ethyl-1-propylpyrrolidiniumcyanid, 1-Ethyl-1-butylpyrrolidiniumcyanid, 1-Ethyl-2-methylpyrrolidiniumcyanid, 1,2-Diethylpyrrolidiniumcyanid, 1-Ethyl-2-propylpyrrolidiniumcyanid, 1-Ethyl-2-butylpyrrolidiniumcyanid, 1-Ethyl-3-methylpyrrolidiniumcyanid, 1,3-Diethylpyrrolidiniumcyanid, 1-Ethyl-3-propylpyrrolidiniumcyanid, 1-Ethyl-3-butylpyrrolidiniumcyanid.

Man erkennt, dass eine Verbindung entsprechend Formel (26) beispielsweise ausgewählt sein kann aus der Gruppe enthaltend N-Propylpyrrolidiniumchlorid, 1,1-Dipropylpyrrolidiniumchlorid, 1-Propyl-1-Methylpyrrolidiniumchlorid, 1-Propyl-1-ethylpyrrolidiniumchlorid, 1-Propyl-1-butylpyrrolidiniumchlorid, 1-Propyl-2-Methylpyrrolidiniumchlorid, 1-Propyl-2-ethylpyrrolidiniumchlorid, 1,2-Dipropylpyrrolidiniumchlorid, 1-Propyl-2-butylpyrrolidiniumchlorid, 1-Propyl-3-Methylpyrrolidiniumchlorid, 1-Propyl-3-ethylpyrrolidiniumchlorid, 1,3-Dipropylpyrrolidiniumchlorid, 1-Propyl-3-butylpyrrolidiniumchlorid, N-Propylpyrrolidiniumfluorid, 1,1-Dipropylpyrrolidiniumfluorid, 1-Propyl-1-Methylpyrrolidiniumfluorid, 1-Propyl-1-ethylpyrrolidiniumfluorid, 1-Propyl-1-butylpyrrolidiniumfluorid, 1-Propyl-2-Methylpyrrolidiniumfluorid, 1-Propyl-2-ethylpyrrolidiniumfluorid, 1,2-Dipropylpyrrolidiniumfluorid, 1-Propyl-2-butylpyrrolidiniumfluorid, 1-Propyl-3-Methylpyrrolidiniumfluorid, 1-Propyl-3-ethylpyrrolidiniumfluorid, 1,3-Dipropylpyrrolidiniumfluorid, 1-Propyl-3-butylpyrrolidiniumfluorid, N-Propylpyrrolidiniumtriflat, 1,1-Dipropylpyrrolidiniumtriflat, 1-Propyl-1-Methylpyrrolidiniumtriflat, 1-Propyl-1-ethylpyrrolidiniumtriflat, 1-Propyl-1-butylpyrrolidiniumtriflat, 1-Propyl-2-Methylpyrrolidiniumtriflat, 1-Propyl-2-ethylpyrrolidiniumtriflat, 1,2-Dipropylpyrrolidiniumtriflat, 1-Propyl-2-butylpyrrolidiniumtriflat, 1-Propyl-3-Methylpyrrolidiniumtriflat, 1-Propyl-3-ethylpyrrolidiniumtriflat, 1,3-Dipropylpyrrolidiniumtriflat, 1-Propyl-3-butylpyrrolidiniumtriflat, N-Propylpyrrolidiniummethansulfonat, 1,1-Dipropylpyrrolidiniummethansulfonat, 1-Propyl-1-Methylpyrrolidiniummethansulfonat, 1-Propyl-1-ethylpyrrolidiniummethansulfonat, 1-Propyl-1-butylpyrrolidiniummethansulfonat, 1-Propyl-2-Methylpyrrolidiniummethansulfonat, 1-Propyl-2-ethylpyrrolidiniummethansulfonat, 1,2-Dipropylpyrrolidiniummethansulfonat, 1-Propyl-2-butylpyrrolidiniummethansulfonat, 1-Propyl-3-Methylpyrrolidiniummethansulfonat, 1-Propyl-3-ethylpyrrolidiniummethansulfonat, 1,3-Dipropylpyrrolidiniummethansulfonat, 1-Propyl-3-butylpyrrolidiniummethansulfonat, N-Propylpyrrolidiniumacetat, 1,1-Dipropylpyrrolidiniumacetat, 1-Propyl-1-Methylpyrrolidiniumacetat, 1-Propyl-1-ethylpyrrolidiniumacetat, 1-Propyl-1-butylpyrrolidiniumacetat, 1-Propyl-2-Methylpyrrolidiniumacetat, 1-Propyl-2-ethylpyrrolidiniumacetat, 1,2-Dipropylpyrrolidiniumacetat, 1-Propyl-2-butylpyrrolidiniumacetat, 1-Propyl-3-Methylpyrrolidiniumacetat, 1-Propyl-3-ethylpyrrolidiniumacetat, 1,3-Dipropylpyrrolidiniumacetat, 1-Propyl-3-butylpyrrolidiniumacetat, N-Propylpyrrolidiniumcyanid, 1,1-Dipropylpyrrolidiniumcyanid, 1-Propyl-1-Methylpyrrolidiniumcyanid, 1-Propyl-1-ethylpyrrolidiniumcyanid, 1-Propyl-1-butylpyrrolidiniumcyanid, 1-Propyl-2-Methylpyrrolidiniumcyanid, 1-Propyl-2-ethylpyrrolidiniumcyanid, 1,2-Dipropylpyrrolidiniumcyanid, 1-Propyl-2-butylpyrrolidiniumcyanid, 1-Propyl-3-Methylpyrrolidiniumcyanid, 1-Propyl-3-ethylpyrrolidiniumcyanid, 1,3-Dipropylpyrrolidiniumcyanid, 1-Propyl-3-butylpyrrolidiniumcyanid.

Man erkennt, dass eine Verbindung entsprechend Formel (27) beispielsweise ausgewählt sein kann aus der Gruppe enthaltend N-Butylpyrrolidiniumchlorid, 1-Butyl-1-methylpyrrolidiniumchlorid, 1-Butyl-1-ethylpyrrolidiniumchlorid, 1-Butyl-1-propylpyrrolidiniumchlorid, 1,1-Dibutylpyrrolidiniumchlorid, 1-Butyl-2-Methylpyrrolidiniumchlorid, 1-Butyl-2-ethylpyrrolidiniumchlorid, 1-Butyl-2-propylpyrrolidiniumchlorid, 1,2-Dibutylpyrrolidiniumchlorid, 1-Butyl-3-Methylpyrrolidiniumchlorid, 1-Butyl-3-ethylpyrrolidiniumchlorid, 1-Butyl-3-propylpyrrolidiniumchlorid, 1,3-Dibutylpyrrolidiniumchlorid, N-Butylpyrrolidiniumfluorid, 1-Butyl-1-methylpyrrolidiniumfluorid, 1-Butyl-1-ethylpyrrolidiniumfluorid, 1-Butyl-1-propylpyrrolidiniumfluorid, 1,1-Dibutylpyrrolidiniumfluorid, 1-Butyl-2-Methylpyrrolidiniumfluorid, 1-Butyl-2-ethylpyrrolidiniumfluorid, 1-Butyl-2-propylpyrrolidiniumfluorid, 1,2-Dibutylpyrrolidiniumfluorid, 1-Butyl-3-Methylpyrrolidiniumfluorid, 1-Butyl-3-ethylpyrrolidiniumfluorid, 1-Butyl-3-propylpyrrolidiniumfluorid, 1,3-Dibutylpyrrolidiniumfluorid, N-Butylpyrrolidiniumtriflat, 1-Butyl-1-methylpyrrolidiniumtriflat, 1-Butyl-1-ethylpyrrolidiniumtriflat, 1-Butyl-1-propylpyrrolidiniumtriflat, 1,1-Dibutylpyrrolidiniumtriflat, 1-Butyl-2-Methylpyrrolidiniumtriflat, 1-Butyl-2-ethylpyrrolidiniumtriflat, 1-Butyl-2-propylpyrrolidiniumtriflat, 1,2-Dibutylpyrrolidiniumtriflat, 1-Butyl-3-Methylpyrrolidiniumtriflat, 1-Butyl-3-ethylpyrrolidiniumtriflat, 1-Butyl-3-propylpyrrolidiniumtriflat, 1,3-Dibutylpyrrolidiniumtriflat, N-Butylpyrrolidiniummethansulfonat, 1-Butyl-1-methylpyrrolidiniummethansulfonat, 1-Butyl-1-ethylpyrrolidiniummethansulfonat, 1-Butyl-1-propylpyrrolidiniummethansulfonat, 1,1-Dibutylpyrrolidiniummethansulfonat, 1-Butyl-2-Methylpyrrolidiniummethansulfonat, 1-Butyl-2-ethylpyrrolidiniummethansulfonat, 1-Butyl-2-propylpyrrolidiniummethansulfonat, 1,2-Dibutylpyrrolidiniummethansulfonat, 1-Butyl-3-Methylpyrrolidiniummethansulfonat, 1-Butyl-3-ethylpyrrolidiniummethansulfonat, 1-Butyl-3-propylpyrrolidiniummethansulfonat, 1,3-Dibutylpyrrolidiniummethansulfonat, N-Butylpyrrolidiniumacetat, 1-Butyl-1-methylpyrrolidiniumacetat, 1-Butyl-1-ethylpyrrolidiniumacetat, 1-Butyl-1-propylpyrrolidiniumacetat, 1,1-Dibutylpyrrolidiniumacetat, 1-Butyl-2-Methylpyrrolidiniumacetat, 1-Butyl-2-ethylpyrrolidiniumacetat, 1-Butyl-2-propylpyrrolidiniumacetat, 1,2-Dibutylpyrrolidiniumacetat, 1-Butyl-3-Methylpyrrolidiniumacetat, 1-Butyl-3-ethylpyrrolidiniumacetat, 1-Butyl-3-propylpyrrolidiniumacetat, 1,3-Dibutylpyrrolidiniumacetat, N-Butylpyrrolidiniumcyanid, 1-Butyl-1-methylpyrrolidiniumcyanid, 1-Butyl-1-ethylpyrrolidiniumcyanid, 1-Butyl-1-propylpyrrolidiniumcyanid, 1,1-Dibutylpyrrolidiniumcyanid, 1-Butyl-2-Methylpyrrolidiniumcyanid, 1-Butyl-2-ethylpyrrolidiniumcyanid, 1-Butyl-2-propylpyrrolidiniumcyanid, 1,2-Dibutylpyrrolidiniumcyanid, 1-Butyl-3-Methylpyrrolidiniumcyanid, 1-Butyl-3-ethylpyrrolidiniumcyanid, 1-Butyl-3-propylpyrrolidiniumcyanid, 1,3-Dibutylpyrrolidiniumcyanid.

Man erkennt, dass eine Verbindung entsprechend Formel (28) beispielsweise ausgewählt sein kann aus der Gruppe enthaltend N-Pentylpyrrolidiniumchlorid, 1-Pentyl-1-methylpyrrolidiniumchlorid, 1-Pentyl-1-ethylpyrrolidiniumchlorid, 1-Pentyl-1-propylpyrrolidiniumchlorid, 1-Pentyl-1-butylpyrrolidiniumchlorid, 1-Pentyl-2-Methylpyrrolidiniumchlorid, 1-Pentyl-2-ethylpyrrolidiniumchlorid, 1-Pentyl-2-propylpyrrolidiniumchlorid, 1-Pentyl-2-butylpyrrolidiniumchlorid, 1-Pentyl-3-Methylpyrrolidiniumchlorid, 1-Pentyl-3-ethylpyrrolidiniumchlorid, 1-Pentyl-3-propylpyrrolidiniumchlorid, 1-Pentyl-3-butylpyrrolidiniumchlorid, N-Pentylpyrrolidiniumfluorid, 1-Pentyl-1-methylpyrrolidiniumfluorid, 1-Pentyl-1-ethylpyrrolidiniumfluorid, 1-Pentyl-1-propylpyrrolidiniumfluorid, 1-Pentyl-1-butylpyrrolidiniumfluorid, 1-Pentyl-2-Methylpyrrolidiniumfluorid, 1-Pentyl-2-ethylpyrrolidiniumfluorid, 1-Pentyl-2-propylpyrrolidiniumfluorid, 1-Pentyl-2-butylpyrrolidiniumfluorid, 1-Pentyl-3-Methylpyrrolidiniumfluorid, 1-Pentyl-3-ethylpyrrolidiniumfluorid, 1-Pentyl-3-propylpyrrolidiniumfluorid, 1-Pentyl-3-butylpyrrolidiniumfluorid, N-Pentylpyrrolidiniumtriflat, 1-Pentyl-1-methylpyrrolidiniumtriflat, 1-Pentyl-1-ethylpyrrolidiniumtriflat, 1-Pentyl-1-propylpyrrolidiniumtriflat, 1-Pentyl-1-butylpyrrolidiniumtriflat, 1-Pentyl-2-Methylpyrrolidiniumtriflat, 1-Pentyl-2-ethylpyrrolidiniumtriflat, 1-Pentyl-2-propylpyrrolidiniumtriflat, 1-Pentyl-2-butylpyrrolidiniumtriflat, 1-Pentyl-3-Methylpyrrolidiniumtriflat, 1-Pentyl-3-ethylpyrrolidiniumtriflat, 1-Pentyl-3-propylpyrrolidiniumtriflat, 1-Pentyl-3-butylpyrrolidiniumtriflat, N-Pentylpyrrolidiniummethansulfonat, 1-Pentyl-1-methylpyrrolidiniummethansulfonat, 1-Pentyl-1-ethylpyrrolidiniummethansulfonat, 1-Pentyl-1-propylpyrrolidiniummethansulfonat, 1-Pentyl-1-butylpyrrolidiniummethansulfonat, 1-Pentyl-2-Methylpyrrolidiniummethansulfonat, 1-Pentyl-2-ethylpyrrolidiniummethansulfonat, 1-Pentyl-2-propylpyrrolidiniummethansulfonat, 1-Pentyl-2-butylpyrrolidiniummethansulfonat, 1-Pentyl-3-Methylpyrrolidiniummethansulfonat, 1-Pentyl-3-ethylpyrrolidiniummethansulfonat, 1-Pentyl-3-propylpyrrolidiniummethansulfonat, 1-Pentyl-3-butylpyrrolidiniummethansulfonat, N-Pentylpyrrolidiniumacetat, 1-Pentyl-1-methylpyrrolidiniumacetat, 1-Pentyl-1-ethylpyrrolidiniumacetat, 1-Pentyl-1-propylpyrrolidiniumacetat, 1-Pentyl-1-butylpyrrolidiniumacetat, 1-Pentyl-2-Methylpyrrolidiniumacetat, 1-Pentyl-2-ethylpyrrolidiniumacetat, 1-Pentyl-2-propylpyrrolidiniumacetat, 1-Pentyl-2-butylpyrrolidiniumacetat, 1-Pentyl-3-Methylpyrrolidiniumacetat, 1-Pentyl-3-ethylpyrrolidiniumacetat, 1-Pentyl-3-propylpyrrolidiniumacetat, 1-Pentyl-3-butylpyrrolidiniumacetat, N-Pentylpyrrolidiniumcyanid, 1-Pentyl-1-methylpyrrolidiniumcyanid, 1-Pentyl-1-ethylpyrrolidiniumcyanid, 1-Pentyl-1-propylpyrrolidiniumcyanid, 1-Pentyl-1-butylpyrrolidiniumcyanid, 1-Pentyl-2-Methylpyrrolidiniumcyanid, 1-Pentyl-2-ethylpyrrolidiniumcyanid, 1-Pentyl-2-propylpyrrolidiniumcyanid, 1-Pentyl-2-butylpyrrolidiniumcyanid, 1-Pentyl-3-Methylpyrrolidiniumcyanid, 1-Pentyl-3-ethylpyrrolidiniumcyanid, 1-Pentyl-3-propylpyrrolidiniumcyanid, 1-Pentyl-3-butylpyrrolidiniumcyanid.

Man erkennt weiter, dass eine Verbindung entsprechend Formel (29) ausgewählt sein kann aus der Gruppe enthaltend N-Hexylpyrrolidiniumchlorid, 1-Hexyl-1-methylpyrrolidiniumchlorid, 1-Hexyl-1-ethylpyrrolidiniumchlorid, 1-Hexyl-1-propylpyrrolidiniumchlorid, 1-Hexyl-1-butylpyrrolidiniumchlorid, 1-Hexyl-2-Methylpyrrolidiniumchlorid, 1-Hexyl-2-ethylpyrrolidiniumchlorid, 1-Hexyl-2-propylpyrrolidiniumchlorid, 1-Hexyl-2-butylpyrrolidiniumchlorid, 1-Hexyl-3-Methylpyrrolidiniumchlorid, 1-Hexyl-3-ethylpyrrolidiniumchlorid, 1-Hexyl-3-propylpyrrolidiniumchlorid, 1-Hexyl-3-butylpyrrolidiniumchlorid, N-Hexylpyrrolidiniumfluorid, 1-Hexyl-1-methylpyrrolidiniumfluorid, 1-Hexyl-1-ethylpyrrolidiniumfluorid, 1-Hexyl-1-propylpyrrolidiniumfluorid, 1-Hexyl-1-butylpyrrolidiniumfluorid, 1-Hexyl-2-Methylpyrrolidiniumfluorid, 1-Hexyl-2-ethylpyrrolidiniumfluorid, 1-Hexyl-2-propylpyrrolidiniumfluorid, 1-Hexyl-2-butylpyrrolidiniumfluorid, 1-Hexyl-3-Methylpyrrolidiniumfluorid, 1-Hexyl-3-ethylpyrrolidiniumfluorid, 1-Hexyl-3-propylpyrrolidiniumfluorid, 1-Hexyl-3-butylpyrrolidiniumfluorid, N-Hexylpyrrolidiniumtriflat, 1-Hexyl-1-methylpyrrolidiniumtriflat, 1-Hexyl-1-ethylpyrrolidiniumtriflat, 1-Hexyl-1-propylpyrrolidiniumtriflat, 1-Hexyl-1-butylpyrrolidiniumtriflat, 1-Hexyl-2-Methylpyrrolidiniumtriflat, 1-Hexyl-2-ethylpyrrolidiniumtriflat, 1-Hexyl-2-propylpyrrolidiniumtriflat, 1-Hexyl-2-butylpyrrolidiniumtriflat, 1-Hexyl-3-Methylpyrrolidiniumtriflat, 1-Hexyl-3-ethylpyrrolidiniumtriflat, 1-Hexyl-3-propylpyrrolidiniumtriflat, 1-Hexyl-3-butylpyrrolidiniumtriflat, N-Hexylpyrrolidiniummethansulfonat, 1-Hexyl-1-methylpyrrolidiniummethansulfonat, 1-Hexyl-1-ethylpyrrolidiniummethansulfonat, 1-Hexyl-1-propylpyrrolidiniummethansulfonat, 1-Hexyl-1-butylpyrrolidiniummethansulfonat, 1-Hexyl-2-Methylpyrrolidiniummethansulfonat, 1-Hexyl-2-ethylpyrrolidiniummethansulfonat, 1-Hexyl-2-propylpyrrolidiniummethansulfonat, 1-Hexyl-2-butylpyrrolidiniummethansulfonat, 1-Hexyl-3-Methylpyrrolidiniummethansulfonat, 1-Hexyl-3-ethylpyrrolidiniummethansulfonat, 1-Hexyl-3-propylpyrrolidiniummethansulfonat, 1-Hexyl-3-butylpyrrolidiniummethansulfonat, N-Hexylpyrrolidiniumacetat, 1-Hexyl-1-methylpyrrolidiniumacetat, 1-Hexyl-1-ethylpyrrolidiniumacetat, 1-Hexyl-1-propylpyrrolidiniumacetat, 1-Hexyl-1-butylpyrrolidiniumacetat, 1-Hexyl-2-Methylpyrrolidiniumacetat, 1-Hexyl-2-ethylpyrrolidiniumacetat, 1-Hexyl-2-propylpyrrolidiniumacetat, 1-Hexyl-2-butylpyrrolidiniumacetat, 1-Hexyl-3-Methylpyrrolidiniumacetat, 1-Hexyl-3-ethylpyrrolidiniumacetat, 1-Hexyl-3-propylpyrrolidiniumacetat, 1-Hexyl-3-butylpyrrolidiniumacetat, N-Hexylpyrrolidiniumcyanid, 1-Hexyl-1-methylpyrrolidiniumcyanid, 1-Hexyl-1-ethylpyrrolidiniumcyanid, 1-Hexyl-1-propylpyrrolidiniumcyanid, 1-Hexyl-1-butylpyrrolidiniumcyanid, 1-Hexyl-2-Methylpyrrolidiniumcyanid, 1-Hexyl-2-ethylpyrrolidiniumcyanid, 1-Hexyl-2-propylpyrrolidiniumcyanid, 1-Hexyl-2-butylpyrrolidiniumcyanid, 1-Hexyl-3-Methylpyrrolidiniumcyanid, 1-Hexyl-3-ethylpyrrolidiniumcyanid, 1-Hexyl-3-propylpyrrolidiniumcyanid, 1-Hexyl-3-butylpyrrolidiniumcyanid.

Man erkennt weiter, dass eine Verbindung entsprechend Formel (30) ausgewählt sein kann aus der Gruppe enthaltend N-Heptylpyrrolidiniumchlorid, 1-Heptyl-1-methylpyrrolidiniumchlorid, 1-Heptyl-1-ethylpyrrolidiniumchlorid, 1-Heptyl-1-propylpyrrolidiniumchlorid, 1-Heptyl-1-butylpyrrolidiniumchlorid, 1-Heptyl-2-Methylpyrrolidiniumchlorid, 1-Heptyl-2-ethylpyrrolidiniumchlorid, 1-Heptyl-2-propylpyrrolidiniumchlorid, 1-Heptyl-2-butylpyrrolidiniumchlorid, 1-Heptyl-3-Methylpyrrolidiniumchlorid, 1-Heptyl-3-ethylpyrrolidiniumchlorid, 1-Heptyl-3-propylpyrrolidiniumchlorid, 1-Heptyl-3-butylpyrrolidiniumchlorid, N-Heptylpyrrolidiniumfluorid, 1-Heptyl-1-methylpyrrolidiniumfluorid, 1-Heptyl-1-ethylpyrrolidiniumfluorid, 1-Heptyl-1-propylpyrrolidiniumfluorid, 1-Heptyl-1-butylpyrrolidiniumfluorid, 1-Heptyl-2-Methylpyrrolidiniumfluorid, 1-Heptyl-2-ethylpyrrolidiniumfluorid, 1-Heptyl-2-propylpyrrolidiniumfluorid, 1-Heptyl-2-butylpyrrolidiniumfluorid, 1-Heptyl-3-Methylpyrrolidiniumfluorid, 1-Heptyl-3-ethylpyrrolidiniumfluorid, 1-Heptyl-3-propylpyrrolidiniumfluorid, 1-Heptyl-3-butylpyrrolidiniumfluorid, N-Heptylpyrrolidiniumtriflat, 1-Heptyl-1-methylpyrrolidiniumtriflat, 1-Heptyl-1-ethylpyrrolidiniumtriflat, 1-Heptyl-1-propylpyrrolidiniumtriflat, 1-Heptyl-1-butylpyrrolidiniumtriflat, 1-Heptyl-2-Methylpyrrolidiniumtriflat, 1-Heptyl-2-ethylpyrrolidiniumtriflat, 1-Heptyl-2-propylpyrrolidiniumtriflat, 1-Heptyl-2-butylpyrrolidiniumtriflat, 1-Heptyl-3-Methylpyrrolidiniumtriflat, 1-Heptyl-3-ethylpyrrolidiniumtriflat, 1-Heptyl-3-propylpyrrolidiniumtriflat, 1-Heptyl-3-butylpyrrolidiniumtriflat, N-Heptylpyrrolidiniummethansulfonat, 1-Heptyl-1-methylpyrrolidiniummethansulfonat, 1-Heptyl-1-ethylpyrrolidiniummethansulfonat, 1-Heptyl-1-propylpyrrolidiniummethansulfonat, 1-Heptyl-1-butylpyrrolidiniummethansulfonat, 1-Heptyl-2-Methylpyrrolidiniummethansulfonat, 1-Heptyl-2-ethylpyrrolidiniummethansulfonat, 1-Heptyl-2-propylpyrrolidiniummethansulfonat, 1-Heptyl-2-butylpyrrolidiniummethansulfonat, 1-Heptyl-3-Methylpyrrolidiniummethansulfonat, 1-Heptyl-3-ethylpyrrolidiniummethansulfonat, 1-Heptyl-3-propylpyrrolidiniummethansulfonat, 1-Heptyl-3-butylpyrrolidiniummethansulfonat, N-Heptylpyrrolidiniumacetat, 1-Heptyl-1-methylpyrrolidiniumacetat, 1-Heptyl-1-ethylpyrrolidiniumacetat, 1-Heptyl-1-propylpyrrolidiniumacetat, 1-Heptyl-1-butylpyrrolidiniumacetat, 1-Heptyl-2-Methylpyrrolidiniumacetat, 1-Heptyl-2-ethylpyrrolidiniumacetat, 1-Heptyl-2-propylpyrrolidiniumacetat, 1-Heptyl-2-butylpyrrolidiniumacetat, 1-Heptyl-3-Methylpyrrolidiniumacetat, 1-Heptyl-3-ethylpyrrolidiniumacetat, 1-Heptyl-3-propylpyrrolidiniumacetat, 1-Heptyl-3-butylpyrrolidiniumacetat, N-Heptylpyrrolidiniumcyanid, 1-Heptyl-1-methylpyrrolidiniumcyanid, 1-Heptyl-1-ethylpyrrolidiniumcyanid, 1-Heptyl-1-propylpyrrolidiniumcyanid, 1-Heptyl-1-butylpyrrolidiniumcyanid, 1-Heptyl-2-Methylpyrrolidiniumcyanid, 1-Heptyl-2-ethylpyrrolidiniumcyanid, 1-Heptyl-2-propylpyrrolidiniumcyanid, 1-Heptyl-2-butylpyrrolidiniumcyanid, 1-Heptyl-3-Methylpyrrolidiniumcyanid, 1-Heptyl-3-ethylpyrrolidiniumcyanid, 1-Heptyl-3-propylpyrrolidiniumcyanid, 1-Heptyl-3-butylpyrrolidiniumcyanid.

Man erkennt weiter, dass eine Verbindung entsprechend Formel (31) ausgewählt sein kann aus der Gruppe enthaltend N-Octylpyrrolidiniumchlorid, 1-Octyl-1-methylpyrrolidiniumchlorid, 1-Octyl-1-ethylpyrrolidiniumchlorid, 1-Octyl-1-propylpyrrolidiniumchlorid, 1-Octyl-1-butylpyrrolidiniumchlorid, 1-Octyl-2-Methylpyrrolidiniumchlorid, 1-Octyl-2-ethylpyrrolidiniumchlorid, 1-Octyl-2-propylpyrrolidiniumchlorid, 1-Octyl-2-butylpyrrolidiniumchlorid, 1-Octyl-3-Methylpyrrolidiniumchlorid, 1-Octyl-3-ethylpyrrolidiniumchlorid, 1-Octyl-3-propylpyrrolidiniumchlorid, 1-Octyl-3-butylpyrrolidiniumchlorid, N-Octylpyrrolidiniumfluorid, 1-Octyl-1-methylpyrrolidiniumfluorid, 1-Octyl-1-ethylpyrrolidiniumfluorid, 1-Octyl-1-propylpyrrolidiniumfluorid, 1-Octyl-1-butylpyrrolidiniumfluorid, 1-Octyl-2-Methylpyrrolidiniumfluorid, 1-Octyl-2-ethylpyrrolidiniumfluorid, 1-Octyl-2-propylpyrrolidiniumfluorid, 1-Octyl-2-butylpyrrolidiniumfluorid, 1-Octyl-3-Methylpyrrolidiniumfluorid, 1-Octyl-3-ethylpyrrolidiniumfluorid, 1-Octyl-3-propylpyrrolidiniumfluorid, 1-Octyl-3-butylpyrrolidiniumfluorid, N-Octylpyrrolidiniumtriflat, 1-Octyl-1-methylpyrrolidiniumtriflat, 1-Octyl-1-ethylpyrrolidiniumtriflat, 1-Octyl-1-propylpyrrolidiniumtriflat, 1-Octyl-1-butylpyrrolidiniumtriflat, 1-Octyl-2-Methylpyrrolidiniumtriflat, 1-Octyl-2-ethylpyrrolidiniumtriflat, 1-Octyl-2-propylpyrrolidiniumtriflat, 1-Octyl-2-butylpyrrolidiniumtriflat, 1-Octyl-3-Methylpyrrolidiniumtriflat, 1-Octyl-3-ethylpyrrolidiniumtriflat, 1-Octyl-3-propylpyrrolidiniumtriflat, 1-Octyl-3-butylpyrrolidiniumtriflat, N-Octylpyrrolidiniummethansulfonat, 1-Octyl-1-methylpyrrolidiniummethansulfonat, 1-Octyl-1-ethylpyrrolidiniummethansulfonat, 1-Octyl-1-propylpyrrolidiniummethansulfonat, 1-Octyl-1-butylpyrrolidiniummethansulfonat, 1-Octyl-2-Methylpyrrolidiniummethansulfonat, 1-Octyl-2-ethylpyrrolidiniummethansulfonat, 1-Octyl-2-propylpyrrolidiniummethansulfonat, 1-Octyl-2-butylpyrrolidiniummethansulfonat, 1-Octyl-3-Methylpyrrolidiniummethansulfonat, 1-Octyl-3-ethylpyrrolidiniummethansulfonat, 1-Octyl-3-propylpyrrolidiniummethansulfonat, 1-Octyl-3-butylpyrrolidiniummethansulfonat, N-Octylpyrrolidiniumacetat, 1-Octyl-1-methylpyrrolidiniumacetat, 1-Octyl-1-ethylpyrrolidiniumacetat, 1-Octyl-1-propylpyrrolidiniumacetat, 1-Octyl-1-butylpyrrolidiniumacetat, 1-Octyl-2-Methylpyrrolidiniumacetat, 1-Octyl-2-ethylpyrrolidiniumacetat, 1-Octyl-2-propylpyrrolidiniumacetat, 1-Octyl-2-butylpyrrolidiniumacetat, 1-Octyl-3-Methylpyrrolidiniumacetat, 1-Octyl-3-ethylpyrrolidiniumacetat, 1-Octyl-3-propylpyrrolidiniumacetat, 1-Octyl-3-butylpyrrolidiniumacetat, N-Octylpyrrolidiniumcyanid, 1-Octyl-1-methylpyrrolidiniumcyanid, 1-Octyl-1-ethylpyrrolidiniumcyanid, 1-Octyl-1-propylpyrrolidiniumcyanid, 1-Octyl-1-butylpyrrolidiniumcyanid, 1-Octyl-2-Methylpyrrolidiniumcyanid, 1-Octyl-2-ethylpyrrolidiniumcyanid, 1-Octyl-2-propylpyrrolidiniumcyanid, 1-Octyl-2-butylpyrrolidiniumcyanid, 1-Octyl-3-Methylpyrrolidiniumcyanid, 1-Octyl-3-ethylpyrrolidiniumcyanid, 1-Octyl-3-propylpyrrolidiniumcyanid, 1-Octyl-3-butylpyrrolidiniumcyanid.

Man erkennt weiter, dass eine Verbindung entsprechend Formel (32) ausgewählt sein kann aus der Gruppe enthaltend N-Nonylpyrrolidiniumchlorid, 1-Nonyl-1-methylpyrrolidiniumchlorid, 1-Nonyl-1-ethylpyrrolidiniumchlorid, 1-Nonyl-1-propylpyrrolidiniumchlorid, 1-Nonyl-1-butylpyrrolidiniumchlorid, 1-Nonyl-2-Methylpyrrolidiniumchlorid, 1-Nonyl-2-ethylpyrrolidiniumchlorid, 1-Nonyl-2-propylpyrrolidiniumchlorid, 1-Nonyl-2-butylpyrrolidiniumchlorid, 1-Nonyl-3-Methylpyrrolidiniumchlorid, 1-Nonyl-3-ethylpyrrolidiniumchlorid, 1-Nonyl-3-propylpyrrolidiniumchlorid, 1-Nonyl-3-butylpyrrolidiniumchlorid, N-Nonylpyrrolidiniumfluorid, 1-Nonyl-1-methylpyrrolidiniumfluorid, 1-Nonyl-1-ethylpyrrolidiniumfluorid, 1-Nonyl-1-propylpyrrolidiniumfluorid, 1-Nonyl-1-butylpyrrolidiniumfluorid, 1-Nonyl-2-Methylpyrrolidiniumfluorid, 1-Nonyl-2-ethylpyrrolidiniumfluorid, 1-Nonyl-2-propylpyrrolidiniumfluorid, 1-Nonyl-2-butylpyrrolidiniumfluorid, 1-Nonyl-3-Methylpyrrolidiniumfluorid, 1-Nonyl-3-ethylpyrrolidiniumfluorid, 1-Nonyl-3-propylpyrrolidiniumfluorid, 1-Nonyl-3-butylpyrrolidiniumfluorid, N-Nonylpyrrolidiniumtriflat, 1-Nonyl-1-methylpyrrolidiniumtriflat, 1-Nonyl-1-ethylpyrrolidiniumtriflat, 1-Nonyl-1-propylpyrrolidiniumtriflat, 1-Nonyl-1-butylpyrrolidiniumtriflat, 1-Nonyl-2-Methylpyrrolidiniumtriflat, 1-Nonyl-2-ethylpyrrolidiniumtriflat, 1-Nonyl-2-propylpyrrolidiniumtriflat, 1-Nonyl-2-butylpyrrolidiniumtriflat, 1-Nonyl-3-Methylpyrrolidiniumtriflat, 1-Nonyl-3-ethylpyrrolidiniumtriflat, 1-Nonyl-3-propylpyrrolidiniumtriflat, 1-Nonyl-3-butylpyrrolidiniumtriflat, N-Nonylpyrrolidiniummethansulfonat, 1-Nonyl-1-methylpyrrolidiniummethansulfonat, 1-Nonyl-1-ethylpyrrolidiniummethansulfonat, 1-Nonyl-1-propylpyrrolidiniummethansulfonat, 1-Nonyl-1-butylpyrrolidiniummethansulfonat, 1-Nonyl-2-Methylpyrrolidiniummethansulfonat, 1-Nonyl-2-ethylpyrrolidiniummethansulfonat, 1-Nonyl-2-propylpyrrolidiniummethansulfonat, 1-Nonyl-2-butylpyrrolidiniummethansulfonat, 1-Nonyl-3-Methylpyrrolidiniummethansulfonat, 1-Nonyl-3-ethylpyrrolidiniummethansulfonat, 1-Nonyl-3-propylpyrrolidiniummethansulfonat, 1-Nonyl-3-butylpyrrolidiniummethansulfonat, N-Nonylpyrrolidiniumacetat, 1-Nonyl-1-methylpyrrolidiniumacetat, 1-Nonyl-1-ethylpyrrolidiniumacetat, 1-Nonyl-1-propylpyrrolidiniumacetat, 1-Nonyl-1-butylpyrrolidiniumacetat, 1-Nonyl-2-Methylpyrrolidiniumacetat, 1-Nonyl-2-ethylpyrrolidiniumacetat, 1-Nonyl-2-propylpyrrolidiniumacetat, 1-Nonyl-2-butylpyrrolidiniumacetat, 1-Nonyl-3-Methylpyrrolidiniumacetat, 1-Nonyl-3-ethylpyrrolidiniumacetat, 1-Nonyl-3-propylpyrrolidiniumacetat, 1-Nonyl-3-butylpyrrolidiniumacetat, N-Nonylpyrrolidiniumcyanid, 1-Nonyl-1-methylpyrrolidiniumcyanid, 1-Nonyl-1-ethylpyrrolidiniumcyanid, 1-Nonyl-1-propylpyrrolidiniumcyanid, 1-Nonyl-1-butylpyrrolidiniumcyanid, 1-Nonyl-2-Methylpyrrolidiniumcyanid, 1-Nonyl-2-ethylpyrrolidiniumcyanid, 1-Nonyl-2-propylpyrrolidiniumcyanid, 1-Nonyl-2-butylpyrrolidiniumcyanid, 1-Nonyl-3-Methylpyrrolidiniumcyanid, 1-Nonyl-3-ethylpyrrolidiniumcyanid, 1-Nonyl-3-propylpyrrolidiniumcyanid, 1-Nonyl-3-butylpyrrolidiniumcyanid.

Man erkennt weiter, dass eine Verbindung entsprechend Formel (33) ausgewählt sein kann aus der Gruppe enthaltend N-Decylpyrrolidiniumchlorid, 1-Decyl-1-methylpyrrolidiniumchlorid, 1-Decyl-1-ethylpyrrolidiniumchlorid, 1-Decyl-1-propylpyrrolidiniumchlorid, 1-Decyl-1-butylpyrrolidiniumchlorid, 1-Decyl-2-Methylpyrrolidiniumchlorid, 1-Decyl-2-ethylpyrrolidiniumchlorid, 1-Decyl-2-propylpyrrolidiniumchlorid, 1-Decyl-2-butylpyrrolidiniumchlorid, 1-Decyl-3-Methylpyrrolidiniumchlorid, 1-Decyl-3-ethylpyrrolidiniumchlorid, 1-Decyl-3-propylpyrrolidiniumchlorid, 1-Decyl-3-butylpyrrolidiniumchlorid, N-Decylpyrrolidiniumfluorid, 1-Decyl-1-methylpyrrolidiniumfluorid, 1-Decyl-1-ethylpyrrolidiniumfluorid, 1-Decyl-1-propylpyrrolidiniumfluorid, 1-Decyl-1-butylpyrrolidiniumfluorid, 1-Decyl-2-Methylpyrrolidiniumfluorid, 1-Decyl-2-ethylpyrrolidiniumfluorid, 1-Decyl-2-propylpyrrolidiniumfluorid, 1-Decyl-2-butylpyrrolidiniumfluorid, 1-Decyl-3-Methylpyrrolidiniumfluorid, 1-Decyl-3-ethylpyrrolidiniumfluorid, 1-Decyl-3-propylpyrrolidiniumfluorid, 1-Decyl-3-butylpyrrolidiniumfluorid, N-Decylpyrrolidiniumtriflat, 1-Decyl-1-methylpyrrolidiniumtriflat, 1-Decyl-1-ethylpyrrolidiniumtriflat, 1-Decyl-1-propylpyrrolidiniumtriflat, 1-Decyl-1-butylpyrrolidiniumtriflat, 1-Decyl-2-Methylpyrrolidiniumtriflat, 1-Decyl-2-ethylpyrrolidiniumtriflat, 1-Decyl-2-propylpyrrolidiniumtriflat, 1-Decyl-2-butylpyrrolidiniumtriflat, 1-Decyl-3-Methylpyrrolidiniumtriflat, 1-Decyl-3-ethylpyrrolidiniumtriflat, 1-Decyl-3-propylpyrrolidiniumtriflat, 1-Decyl-3-butylpyrrolidiniumtriflat, N-Decylpyrrolidiniummethansulfonat, 1-Decyl-1-methylpyrrolidiniummethansulfonat, 1-Decyl-1-ethylpyrrolidiniummethansulfonat, 1-Decyl-1-propylpyrrolidiniummethansulfonat, 1-Decyl-1-butylpyrrolidiniummethansulfonat, 1-Decyl-2-Methylpyrrolidiniummethansulfonat, 1-Decyl-2-ethylpyrrolidiniummethansulfonat, 1-Decyl-2-propylpyrrolidiniummethansulfonat, 1-Decyl-2-butylpyrrolidiniummethansulfonat, 1-Decyl-3-Methylpyrrolidiniummethansulfonat, 1-Decyl-3-ethylpyrrolidiniummethansulfonat, 1-Decyl-3-propylpyrrolidiniummethansulfonat, 1-Decyl-3-butylpyrrolidiniummethansulfonat, N-Decylpyrrolidiniumacetat, 1-Decyl-1-methylpyrrolidiniumacetat, 1-Decyl-1-ethylpyrrolidiniumacetat, 1-Decyl-1-propylpyrrolidiniumacetat, 1-Decyl-1-butylpyrrolidiniumacetat, 1-Decyl-2-Methylpyrrolidiniumacetat, 1-Decyl-2-ethylpyrrolidiniumacetat, 1-Decyl-2-propylpyrrolidiniumacetat, 1-Decyl-2-butylpyrrolidiniumacetat, 1-Decyl-3-Methylpyrrolidiniumacetat, 1-Decyl-3-ethylpyrrolidiniumacetat, 1-Decyl-3-propylpyrrolidiniumacetat, 1-Decyl-3-butylpyrrolidiniumacetat, N-Decylpyrrolidiniumcyanid, 1-Decyl-1-methylpyrrolidiniumcyanid, 1-Decyl-1-ethylpyrrolidiniumcyanid, 1-Decyl-1-propylpyrrolidiniumcyanid, 1-Decyl-1-butylpyrrolidiniumcyanid, 1-Decyl-2-Methylpyrrolidiniumcyanid, 1-Decyl-2-ethylpyrrolidiniumcyanid, 1-Decyl-2-propylpyrrolidiniumcyanid, 1-Decyl-2-butylpyrrolidiniumcyanid, 1-Decyl-3-Methylpyrrolidiniumcyanid, 1-Decyl-3-ethylpyrrolidiniumcyanid, 1-Decyl-3-propylpyrrolidiniumcyanid, 1-Decyl-3-butylpyrrolidiniumcyanid.

Man erkennt weiter, dass ein Verbindung entsprechend Formel (34) ausgewählt sein kann aus der Gruppe enthaltend N-Undecylpyrrolidiniumchlorid, 1-Undecyl-1-methylpyrrolidiniumchlorid, 1-Undecyl-1-ethylpyrrolidiniumchlorid, 1-Undecyl-1-propylpyrrolidiniumchlorid, 1-Undecyl-1-butylpyrrolidiniumchlorid, 1-Undecyl-2-Methylpyrrolidiniumchlorid, 1-Undecyl-2-ethylpyrrolidiniumchlorid, 1-Undecyl-2-propylpyrrolidiniumchlorid, 1-Undecyl-2-butylpyrrolidiniumchlorid, 1-Undecyl-3-Methylpyrrolidiniumchlorid, 1-Undecyl-3-ethylpyrrolidiniumchlorid, 1-Undecyl-3-propylpyrrolidiniumchlorid, 1-Undecyl-3-butylpyrrolidiniumchlorid, N-Undecylpyrrolidiniumfluorid, 1-Undecyl-1-methylpyrrolidiniumfluorid, 1-Undecyl-1-ethylpyrrolidiniumfluorid, 1-Undecyl-1-propylpyrrolidiniumfluorid, 1-Undecyl-1-butylpyrrolidiniumfluorid, 1-Undecyl-2-Methylpyrrolidiniumfluorid, 1-Undecyl-2-ethylpyrrolidiniumfluorid, 1-Undecyl-2-propylpyrrolidiniumfluorid, 1-Undecyl-2-butylpyrrolidiniumfluorid, 1-Undecyl-3-Methylpyrrolidiniumfluorid, 1-Undecyl-3-ethylpyrrolidiniumfluorid, 1-Undecyl-3-propylpyrrolidiniumfluorid, 1-Undecyl-3-butylpyrrolidiniumfluorid, N-Undecylpyrrolidiniumtriflat, 1-Undecyl-1-methylpyrrolidiniumtriflat, 1-Undecyl-1-ethylpyrrolidiniumtriflat, 1-Undecyl-1-propylpyrrolidiniumtriflat, 1-Undecyl-1-butylpyrrolidiniumtriflat, 1-Undecyl-2-Methylpyrrolidiniumtriflat, 1-Undecyl-2-ethylpyrrolidiniumtriflat, 1-Undecyl-2-propylpyrrolidiniumtriflat, 1-Undecyl-2-butylpyrrolidiniumtriflat, 1-Undecyl-3-Methylpyrrolidiniumtriflat, 1-Undecyl-3-ethylpyrrolidiniumtriflat, 1-Undecyl-3-propylpyrrolidiniumtriflat, 1-Undecyl-3-butylpyrrolidiniumtriflat, N-Undecylpyrrolidiniummethansulfonat, 1-Undecyl-1-methylpyrrolidiniummethansulfonat, 1-Undecyl-1-ethylpyrrolidiniummethansulfonat, 1-Undecyl-1-propylpyrrolidiniummethansulfonat, 1-Undecyl-1-butylpyrrolidiniummethansulfonat, 1-Undecyl-2-Methylpyrrolidiniummethansulfonat, 1-Undecyl-2-ethylpyrrolidiniummethansulfonat, 1-Undecyl-2-propylpyrrolidiniummethansulfonat, 1-Undecyl-2-butylpyrrolidiniummethansulfonat, 1-Undecyl-3-Methylpyrrolidiniummethansulfonat, 1-Undecyl-3-ethylpyrrolidiniummethansulfonat, 1-Undecyl-3-propylpyrrolidiniummethansulfonat, 1-Undecyl-3-butylpyrrolidiniummethansulfonat, N-Undecylpyrrolidiniumacetat, 1-Undecyl-1-methylpyrrolidiniumacetat, 1-Undecyl-1-ethylpyrrolidiniumacetat, 1-Undecyl-1-propylpyrrolidiniumacetat, 1-Undecyl-1-butylpyrrolidiniumacetat, 1-Undecyl-2-Methylpyrrolidiniumacetat, 1-Undecyl-2-ethylpyrrolidiniumacetat, 1-Undecyl-2-propylpyrrolidiniumacetat, 1-Undecyl-2-butylpyrrolidiniumacetat, 1-Undecyl-3-Methylpyrrolidiniumacetat, 1-Undecyl-3-ethylpyrrolidiniumacetat, 1-Undecyl-3-propylpyrrolidiniumacetat, 1-Undecyl-3-butylpyrrolidiniumacetat, N-Undecylpyrrolidiniumcyanid, 1-Undecyl-1-methylpyrrolidiniumcyanid, 1-Undecyl-1-ethylpyrrolidiniumcyanid, 1-Undecyl-1-propylpyrrolidiniumcyanid, 1-Undecyl-1-butylpyrrolidiniumcyanid, 1-Undecyl-2-Methylpyrrolidiniumcyanid, 1-Undecyl-2-ethylpyrrolidiniumcyanid, 1-Undecyl-2-propylpyrrolidiniumcyanid, 1-Undecyl-2-butylpyrrolidiniumcyanid, 1-Undecyl-3-Methylpyrrolidiniumcyanid, 1-Undecyl-3-ethylpyrrolidiniumcyanid, 1-Undecyl-3-propylpyrrolidiniumcyanid, 1-Undecyl-3-butylpyrrolidiniumcyanid.

Man erkennt weiter, dass eine Verbindung entsprechend Formel (35) ausgewählt sein kann aus der Gruppe enthaltend N-Dodecylpyrrolidiniumchlorid, 1-Dodecyl-1-methylpyrrolidiniumchlorid, 1-Dodecyl-1-ethylpyrrolidiniumchlorid, 1-Dodecyl-1-propylpyrrolidiniumchlorid, 1-Dodecyl-1-butylpyrrolidiniumchlorid, 1-Dodecyl-2-Methylpyrrolidiniumchlorid, 1-Dodecyl-2-ethylpyrrolidiniumchlorid, 1-Dodecyl-2-propylpyrrolidiniumchlorid, 1-Dodecyl-2-butylpyrrolidiniumchlorid, 1-Dodecyl-3-Methylpyrrolidiniumchlorid, 1-Dodecyl-3-ethylpyrrolidiniumchlorid, 1-Dodecyl-3-propylpyrrolidiniumchlorid, 1-Dodecyl-3-butylpyrrolidiniumchlorid, N-Dodecylpyrrolidiniumfluorid, 1-Dodecyl-1-methylpyrrolidiniumfluorid, 1-Dodecyl-1-ethylpyrrolidiniumfluorid, 1-Dodecyl-1-propylpyrrolidiniumfluorid, 1-Dodecyl-1-butylpyrrolidiniumfluorid, 1-Dodecyl-2-Methylpyrrolidiniumfluorid, 1-Dodecyl-2-ethylpyrrolidiniumfluorid, 1-Dodecyl-2-propylpyrrolidiniumfluorid, 1-Dodecyl-2-butylpyrrolidiniumfluorid, 1-Dodecyl-3-Methylpyrrolidiniumfluorid, 1-Dodecyl-3-ethylpyrrolidiniumfluorid, 1-Dodecyl-3-propylpyrrolidiniumfluorid, 1-Dodecyl-3-butylpyrrolidiniumfluorid, N-Dodecylpyrrolidiniumtriflat, 1-Dodecyl-1-methylpyrrolidiniumtriflat, 1-Dodecyl-1-ethylpyrrolidiniumtriflat, 1-Dodecyl-1-propylpyrrolidiniumtriflat, 1-Dodecyl-1-butylpyrrolidiniumtriflat, 1-Dodecyl-2-Methylpyrrolidiniumtriflat, 1-Dodecyl-2-ethylpyrrolidiniumtriflat, 1-Dodecyl-2-propylpyrrolidiniumtriflat, 1-Dodecyl-2-butylpyrrolidiniumtriflat, 1-Dodecyl-3-Methylpyrrolidiniumtriflat, 1-Dodecyl-3-ethylpyrrolidiniumtriflat, 1-Dodecyl-3-propylpyrrolidiniumtriflat, 1-Dodecyl-3-butylpyrrolidiniumtriflat, N-Dodecylpyrrolidiniummethansulfonat, 1-Dodecyl-1-methylpyrrolidiniummethansulfonat, 1-Dodecyl-1-ethylpyrrolidiniummethansulfonat, 1-Dodecyl-1-propylpyrrolidiniummethansulfonat, 1-Dodecyl-1-butylpyrrolidiniummethansulfonat, 1-Dodecyl-2-Methylpyrrolidiniummethansulfonat, 1-Dodecyl-2-ethylpyrrolidiniummethansulfonat, 1-Dodecyl-2-propylpyrrolidiniummethansulfonat, 1-Dodecyl-2-butylpyrrolidiniummethansulfonat, 1-Dodecyl-3-Methylpyrrolidiniummethansulfonat, 1-Dodecyl-3-ethylpyrrolidiniummethansulfonat, 1-Dodecyl-3-propylpyrrolidiniummethansulfonat, 1-Dodecyl-3-butylpyrrolidiniummethansulfonat, N-Dodecylpyrrolidiniumacetat, 1-Dodecyl-1-methylpyrrolidiniumacetat, 1-Dodecyl-1-ethylpyrrolidiniumacetat, 1-Dodecyl-1-propylpyrrolidiniumacetat, 1-Dodecyl-1-butylpyrrolidiniumacetat, 1-Dodecyl-2-Methylpyrrolidiniumacetat, 1-Dodecyl-2-ethylpyrrolidiniumacetat, 1-Dodecyl-2-propylpyrrolidiniumacetat, 1-Dodecyl-2-butylpyrrolidiniumacetat, 1-Dodecyl-3-Methylpyrrolidiniumacetat, 1-Dodecyl-3-ethylpyrrolidiniumacetat, 1-Dodecyl-3-propylpyrrolidiniumacetat, 1-Dodecyl-3-butylpyrrolidiniumacetat, N-Dodecylpyrrolidiniumcyanid, 1-Dodecyl-1-methylpyrrolidiniumcyanid, 1-Dodecyl-1-ethylpyrrolidiniumcyanid, 1-Dodecyl-1-propylpyrrolidiniumcyanid, 1-Dodecyl-1-butylpyrrolidiniumcyanid, 1-Dodecyl-2-Methylpyrrolidiniumcyanid, 1-Dodecyl-2-ethylpyrrolidiniumcyanid, 1-Dodecyl-2-propylpyrrolidiniumcyanid, 1-Dodecyl-2-butylpyrrolidiniumcyanid, 1-Dodecyl-3-Methylpyrrolidiniumcyanid, 1-Dodecyl-3-ethylpyrrolidiniumcyanid, 1-Dodecyl-3-propylpyrrolidiniumcyanid, 1-Dodecyl-3-butylpyrrolidiniumcyanid.

Man erkennt insofern, dass von der Formel VII insbesondere Verbindungen entsprechend den folgenden Formeln (47), (48), (49), (50), (51), (52), (53), (54), (55), (56), (57) oder (58) 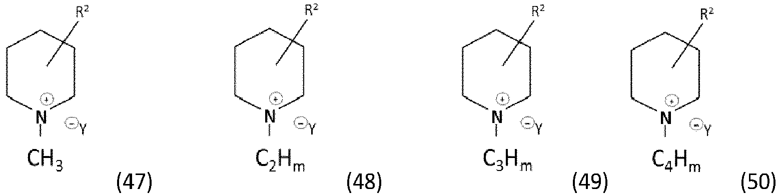 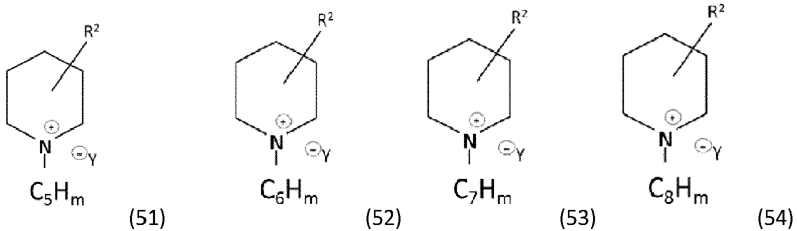 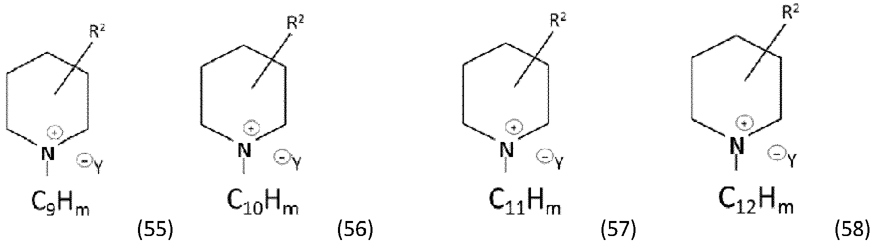 umfasst sind, wobei R² ein Wasserstoffatom oder ein Kohlenwasserstoffrest mit 1, 2, 3 oder 4 C-Atomen ist und Y ausgewählt ist aus der Gruppe enthaltend Halogenide, Cyanid, Alkylsulfonat, halogeniertes Alkylsulfonat, Acetat und wobei für Formel (48) m ausgewählt ist aus 3 oder 5, wobei für Formel (49) m ausgewählt ist aus 3, 5 oder 7, wobei für Formel (50) m ausgewählt ist aus 3, 5, 7, oder 9, wobei für Formel (51) m ausgewählt ist aus 3, 5, 7, 9 oder 11, wobei für Formel (52) m ausgewählt ist aus 3, 5, 7, 9, 11 oder 13, wobei für Formel (53) m ausgewählt ist aus 3, 5, 7, 9, 11, 13 oder 15, wobei für Formel (54) m ausgewählt ist aus 3, 5, 7, 9, 11, 13, 15 oder 17, wobei für Formel (55) m ausgewählt ist aus 3, 5, 7, 9, 11, 13, 15, 17 oder 19, wobei für Formel (56) m ausgewählt ist aus 3, 5, 7, 9, 11, 13, 15, 17, 19 oder 21, wobei für Formel (57) m ausgewählt ist aus 3, 5, 7, 9, 11, 13, 15, 17, 19, 21 oder 23, wobei für Formel (58) m ausgewählt ist aus 3, 5, 7, 9, 11, 13, 15, 17, 19, 21, 23 oder 25. Bevorzugt ist der Rest R¹ entweder gesättigt, einfach ungesättigt oder zweifach ungesättigt, so dass für Formel (48) m ausgewählt ist aus 3 oder 5, für Formel (49) m ausgewählt ist aus 3, 5 oder 7, für Formel (50) m ausgewählt ist aus 5, 7, oder 9, für Formel (51) m ausgewählt ist aus 7, 9 oder 11, für Formel (52) m ausgewählt ist aus 9, 11 oder 13, für Formel (53) m ausgewählt ist aus 11, 13 oder 15, für Formel (54) m ausgewählt ist aus 13, 15 oder 17, für Formel (55) m ausgewählt ist aus 15, 17 oder 19, für Formel (56) m ausgewählt ist aus 17, 19 oder 21, für Formel (57) m ausgewählt ist aus 19, 21 oder 23, und für Formel (58) m ausgewählt ist aus 21, 23 oder 25.

Besonders bevorzugt ist der Rest R¹ ein gesättigter Alkylrest, so dass die Formel VII insbesondere Verbindungen entsprechend Formel (47) sowie entsprechend den folgenden Formeln (59), (60), (61), (62), (63), (64), (65), (66), (67), (68) und (69) 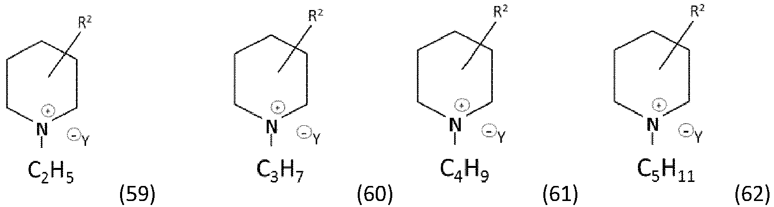 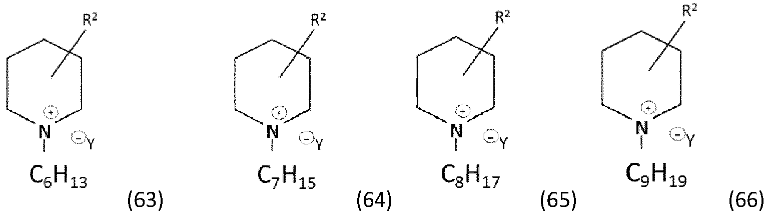 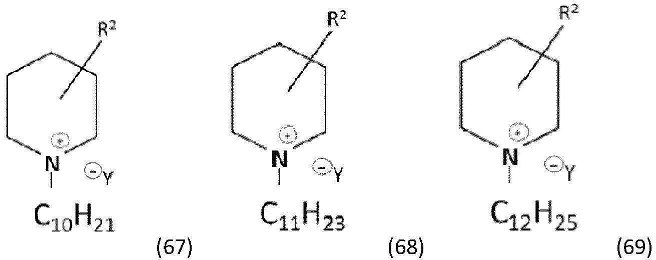 umfasst, wobei R² ein Wasserstoffatom oder ein Kohlenwasserstoffrest mit 1, 2, 3 oder 4 C-Atomen ist und Y ausgewählt ist aus der Gruppe enthaltend Halogenide, Cyanid, Alkylsulfonat, halogeniertes Alkylsulfonat, Acetat.

Man erkennt, dass eine Verbindung entsprechend Formel (47) beispielsweise ausgewählt sein kann aus der Gruppe enthaltend N-Methylpiperidiniumchlorid, 1,1-Dimethylpiperidiniumchlorid, 1-Methyl-1-ethylpiperidiniumchlorid, 1-Methyl-1-propylpiperidiniumchlorid, 1-Methyl-1-butylpiperidiniumchlorid, 1,2-Dimethylpiperidiniumchlorid, 1-Methyl-2-ethylpiperidiniumchlorid, 1-Methyl-2-propylpiperidiniumchlorid, 1-Methyl-2-butylpiperidiniumchlorid, 1,3-Dimethylpiperidiniumchlorid, 1-Methyl-3-ethylpiperidiniumchlorid, 1-Methyl-3-propylpiperidiniumchlorid, 1-Methyl-3-butylpiperidiniumchlorid, 1,4-Dimethylpiperidiniumchlorid, 1-Methyl-4-ethylpiperidiniumchlorid, 1-Methyl-4-propylpiperidiniumchlorid, 1-Methyl-4-butylpiperidiniumchlorid, N-Methylpiperidiniumfluorid, 1,1-Dimethylpiperidiniumfluorid, 1-Methyl-1-ethylpiperidiniumfluorid, 1-Methyl-1-propylpiperidiniumfluorid, 1-Methyl-1-butylpiperidiniumfluorid, 1,2-Dimethylpiperidinium fluorid, 1-Methyl-2-ethylpiperidiniumfluorid, 1-Methyl-2-propylpiperidiniumfluorid, 1-Methyl-2-butylpiperidiniumfluorid, 1,3-Dimethylpiperidiniumfluorid, 1-Methyl-3-ethylpiperidiniumfluorid, 1-Methyl-3-propylpiperidiniumfluorid, 1-Methyl-3-butylpiperidiniumfluorid, 1,4-Dimethylpiperidiniumfluorid, 1-Methyl-4-ethylpiperidiniumfluorid, 1-Methyl-4-propylpiperidiniumfluorid, 1-Methyl-4-butylpiperidiniumfluorid, N-Methylpiperidiniumtriflat, 1,1-Dimethylpiperidiniumtriflat, 1-Methyl-1-ethylpiperidiniumtriflat, 1-Methyl-1-propylpiperidiniumtriflat, 1-Methyl-1-butylpiperidiniumtriflat, 1,2-Dimethylpiperidiniumtriflat, 1-Methyl-2-ethylpiperidiniumtriflat, 1-Methyl-2-propylpiperidiniumtriflat, 1-Methyl-2-butylpiperidiniumtriflat, 1,3-Dimethylpiperidiniumtriflat, 1-Methyl-3-ethylpiperidiniumtriflat, 1-Methyl-3-propylpiperidiniumtriflat, 1-Methyl-3-butylpiperidiniumtriflat, 1,4-Dimethylpiperidiniumtriflat, 1-Methyl-4-ethylpiperidiniumtriflat, 1-Methyl-4-propylpiperidiniumtriflat, 1-Methyl-4-butylpiperidiniumtriflat, N-Methylpiperidiniummethansulfonat, 1,1-Dimethylpiperidiniummethansulfonat, 1-Methyl-1-ethylpiperidiniummethansulfonat, 1-Methyl-1-propylpiperidiniummethansulfonat, 1-Methyl-1-butylpiperidiniummethansulfonat, 1,2-Dimethylpiperidiniummethansulfonat, 1-Methyl-2-ethylpiperidiniummethansulfonat, 1-Methyl-2-propylpiperidiniummethansulfonat, 1-Methyl-2-butylpiperidiniummethansulfonat, 1,3-Dimethylpiperidiniummethansulfonat, 1-Methyl-3-ethylpiperidiniummethansulfonat, 1-Methyl-3-propylpiperidiniummethansulfonat, 1-Methyl-3-butylpiperidiniummethansulfonat, 1,4-Dimethylpiperidiniummethansulfonat, 1-Methyl-4-ethylpiperidiniummethansulfonat, 1-Methyl-4-propylpiperidiniummethansulfonat, 1-Methyl-4-butylpiperidiniummethansulfonat, N-Methylpiperidiniumacetat, 1,1-Dimethylpiperidiniumacetat, 1-Methyl-1-ethylpiperidiniumacetat, 1-Methyl-1-propylpiperidiniumacetat, 1-Methyl-1-butylpiperidiniumacetat, 1,2-Dimethylpiperidiniumacetat, 1-Methyl-2-ethylpiperidiniumacetat, 1-Methyl-2-propylpiperidiniumacetat, 1-Methyl-2-butylpiperidiniumacetat, 1,3-Dimethylpiperidiniumacetat, 1-Methyl-3-ethylpiperidiniumacetat, 1-Methyl-3-propylpiperidiniumacetat, 1-Methyl-3-butylpiperidiniumacetat, 1,4-Dimethylpiperidiniumacetat, 1-Methyl-4-ethylpiperidiniumacetat, 1-Methyl-4-propylpiperidiniumacetat, 1-Methyl-4-butylpiperidiniumacetat, N-Methylpiperidiniumcyanid, 1,1-Dimethylpiperidiniumcyanid, 1-Methyl-1-ethylpiperidiniumcyanid, 1-Methyl-1-propylpiperidiniumcyanid, 1-Methyl-1-butylpiperidiniumcyanid, 1,2-Dimethylpiperidiniumcyanid, 1-Methyl-2-ethylpiperidiniumcyanid, 1-Methyl-2-propylpiperidiniumcyanid, 1-Methyl-2-butylpiperidiniumcyanid, 1,3-Dimethylpiperidiniumcyanid, 1-Methyl-3-ethylpiperidiniumcyanid, 1-Methyl-3-propylpiperidiniumcyanid, 1-Methyl-3-butylpiperidiniumcyanid, 1,4-Dimethylpiperidiniumcyanid, 1-Methyl-4-ethylpiperidiniumcyanid, 1-Methyl-4-propylpiperidiniumcyanid, 1-Methyl-4-butylpiperidiniumcyanid.

Man erkennt, dass eine Verbindung entsprechend Formel (48) beispielsweise ausgewählt sein kann aus der Gruppe enthaltend N-Ethylpiperidiniumchlorid, 1,1-Diethylpiperidiniumchlorid, 1-Ethyl-1-Methylpiperidiniumchlorid, 1-Ethyl-1-propylpiperidiniumchlorid, 1-Ethyl-1-butylpiperidiniumchlorid, 1-Ethyl-2-methylpiperidiniumchlorid, 1,2-Diethylpiperidiniumchlorid, 1-Ethyl-2-propylpiperidiniumchlorid, 1-Ethyl-2-butylpiperidiniumchlorid, 1-Ethyl-3-methylpiperidiniumchlorid, 1,3-Diethylpiperidiniumchlorid, 1-Ethyl-3-propylpiperidiniumchlorid, 1-Ethyl-3-butylpiperidiniumchlorid, 1-Ethyl-4-methylpiperidiniumchlorid, 1,4-Diethylpiperidiniumchlorid, 1-Ethyl-4-propylpiperidiniumchlorid, 1-Ethyl-4-butylpiperidiniumchlorid, N-Ethylpiperidiniumfluorid, 1,1-Diethylpiperidiniumfluorid, 1-Ethyl-1-Methylpiperidiniumfluorid, 1-Ethyl-1-propylpiperidiniumfluorid, 1-Ethyl-1-butylpiperidiniumfluorid, 1-Ethyl-2-methylpiperidiniumfluorid, 1,2-Diethylpiperidiniumfluorid, 1-Ethyl-2-propylpiperidiniumfluorid, 1-Ethyl-2-butylpiperidiniumfluorid, 1-Ethyl-3-methylpiperidiniumfluorid, 1,3-Diethylpiperidiniumfluorid, 1-Ethyl-3-propylpiperidiniumfluorid, 1-Ethyl-3-butylpiperidiniumfluorid, 1-Ethyl-4-methylpiperidiniumfluorid, 1,4-Diethylpiperidiniumfluorid, 1-Ethyl-4-propylpiperidiniumfluorid, 1-Ethyl-4-butylpiperidiniumfluorid, N-Ethylpiperidiniumtriflat, 1,1-Diethylpiperidiniumtriflat, 1-Ethyl-1-Methylpiperidiniumtriflat, 1-Ethyl-1-propylpiperidiniumtriflat, 1-Ethyl-1-butylpiperidiniumtriflat, 1-Ethyl-2-methylpiperidiniumtriflat, 1,2-Diethylpiperidiniumtriflat, 1-Ethyl-2-propylpiperidiniumtriflat, 1-Ethyl-2-butylpiperidiniumtriflat, 1-Ethyl-3-methylpiperidiniumtriflat, 1,3-Diethylpiperidiniumtriflat, 1-Ethyl-3-propylpiperidiniumtriflat, 1-Ethyl-3-butylpiperidiniumtriflat, 1-Ethyl-4-methylpiperidiniumtriflat, 1,4-Diethylpiperidiniumtriflat, 1-Ethyl-4-propylpiperidiniumtriflat, 1-Ethyl-4-butylpiperidiniumtriflat, N-Ethylpiperidiniummethansulfonat, 1,1-Diethylpiperidiniummethansulfonat, 1-Ethyl-1-Methylpiperidiniummethansulfonat, 1-Ethyl-1-propylpiperidiniummethansulfonat, 1-Ethyl-1-butylpiperidiniummethansulfonat, 1-Ethyl-2-methylpiperidiniummethansulfonat, 1,2-Diethylpiperidiniummethansulfonat, 1-Ethyl-2-propylpiperidiniummethansulfonat, 1-Ethyl-2-butylpiperidiniummethansulfonat, 1-Ethyl-3-methylpiperidiniummethansulfonat, 1,3-Diethylpiperidiniummethansulfonat, 1-Ethyl-3-propylpiperidiniummethansulfonat, 1-Ethyl-3-butylpiperidiniummethansulfonat, 1-Ethyl-4-methylpiperidiniummethansulfonat, 1,4-Diethylpiperidiniummethansulfonat, 1-Ethyl-4-propylpiperidiniummethansulfonat, 1-Ethyl-4-butylpiperidiniummethansulfonat, N-Ethylpiperidiniumacetat, 1,1-Diethylpiperidiniumacetat, 1-Ethyl-1-Methylpiperidiniumacetat, 1-Ethyl-1-propylpiperidiniumacetat, 1-Ethyl-1-butylpiperidiniumacetat, 1-Ethyl-2-methylpiperidiniumacetat, 1,2-Diethylpiperidiniumacetat, 1-Ethyl-2-propylpiperidiniumacetat, 1-Ethyl-2-butylpiperidiniumacetat, 1-Ethyl-3-methylpiperidiniumacetat, 1,3-Diethylpiperidiniumacetat, 1-Ethyl-3-propylpiperidiniumacetat, 1-Ethyl-3-butylpiperidiniumacetat, 1-Ethyl-4-methylpiperidiniumacetat, 1,4-Diethylpiperidiniumacetat, 1-Ethyl-4-propylpiperidiniumacetat, 1-Ethyl-4-butylpiperidiniumacetat, N-Ethylpiperidiniumcyanid, 1,1-Diethylpiperidiniumcyanid, 1-Ethyl-1-Methylpiperidiniumcyanid, 1-Ethyl-1-propylpiperidiniumcyanid, 1-Ethyl-1-butylpiperidiniumcyanid, 1-Ethyl-2-methylpiperidiniumcyanid, 1,2-Diethylpiperidiniumcyanid, 1-Ethyl-2-propylpiperidiniumcyanid, 1-Ethyl-2-butylpiperidiniumcyanid, 1-Ethyl-3-methylpiperidiniumcyanid, 1,3-Diethylpiperidiniumcyanid, 1-Ethyl-3-propylpiperidiniumcyanid, 1-Ethyl-3-butylpiperidiniumcyanid, 1-Ethyl-4-methylpiperidiniumcyanid, 1,4-Diethylpiperidiniumcyanid, 1-Ethyl-4-propylpiperidiniumcyanid, 1-Ethyl-4-butylpiperidiniumcyanid.

Man erkennt, dass eine Verbindung entsprechend Formel (49) beispielsweise ausgewählt sein kann aus der Gruppe enthaltend N-Propylpiperidiniumchlorid, 1,1-Dipropylpiperidiniumchlorid, 1-Propyl-1-Methylpiperidiniumchlorid, 1-Propyl-1-ethylpiperidiniumchlorid, 1-Propyl-1-butylpiperidiniumchlorid, 1-Propyl-2-Methylpiperidiniumchlorid, 1-Propyl-2-ethylpiperidiniumchlorid, 1,2-Dipropylpiperidiniumchlorid, 1-Propyl-2-butylpiperidiniumchlorid, 1-Propyl-3-Methylpiperidiniumchlorid, 1-Propyl-3-ethylpiperidiniumchlorid, 1,3-Dipropylpiperidiniumchlorid, 1-Propyl-3-butylpiperidiniumchlorid, 1-Propyl-4-Methylpiperidiniumchlorid, 1-Propyl-4-ethylpiperidiniumchlorid, 1,4-Dipropylpiperidiniumchlorid, 1-Propyl-4-butylpiperidiniumchlorid, N-Propylpiperidiniumfluorid, 1,1-Dipropylpiperidiniumfluorid, 1-Propyl-1-Methylpiperidiniumfluorid, 1-Propyl-1-ethylpiperidiniumfluorid, 1-Propyl-1-butylpiperidiniumfluorid, 1-Propyl-2-Methylpiperidiniumfluorid, 1-Propyl-2-ethylpiperidiniumfluorid, 1,2-Dipropylpiperidiniumfluorid, 1-Propyl-2-butylpiperidiniumfluorid, 1-Propyl-3-Methylpiperidiniumfluorid, 1-Propyl-3-ethylpiperidiniumfluorid, 1,3-Dipropylpiperidiniumfluorid, 1-Propyl-3-butylpiperidiniumfluorid, 1-Propyl-4-Methylpiperidiniumfluorid, 1-Propyl-4-ethylpiperidiniumfluorid, 1,4-Dipropylpiperidiniumfluorid, 1-Propyl-4-butylpiperidiniumfluorid, N-Propylpiperidiniumtriflat, 1,1-Dipropylpiperidiniumtriflat, 1-Propyl-1-Methylpiperidiniumtriflat, 1-Propyl-1-ethylpiperidiniumtriflat, 1-Propyl-1-butylpiperidiniumtriflat, 1-Propyl-2-Methylpiperidiniumtriflat, 1-Propyl-2-ethylpiperidiniumtriflat, 1,2-Dipropylpiperidiniumtriflat, 1-Propyl-2-butylpiperidiniumtriflat, 1-Propyl-3-Methylpiperidiniumtriflat, 1-Propyl-3-ethylpiperidiniumtriflat, 1,3-Dipropylpiperidiniumtriflat, 1-Propyl-3-butylpiperidiniumtriflat, 1-Propyl-4-Methylpiperidiniumtriflat, 1-Propyl-4-ethylpiperidiniumtriflat, 1,4-Dipropylpiperidiniumtriflat, 1-Propyl-4-butylpiperidiniumtriflat, N-Propylpiperidiniummethansulfonat, 1,1-Dipropylpiperidiniummethansulfonat, 1-Propyl-1-Methylpiperidiniummethansulfonat, 1-Propyl-1-ethylpiperidiniummethansulfonat, 1-Propyl-1-butylpiperidiniummethansulfonat, 1-Propyl-2-Methylpiperidiniummethansulfonat, 1-Propyl-2-ethylpiperidiniummethansulfonat, 1,2-Dipropylpiperidiniummethansulfonat, 1-Propyl-2-butylpiperidiniummethansulfonat, 1-Propyl-3-Methylpiperidiniummethansulfonat, 1-Propyl-3-ethylpiperidiniummethansulfonat, 1,3-Dipropylpiperidiniummethansulfonat, 1-Propyl-3-butylpiperidiniummethansulfonat, 1-Propyl-4-Methylpiperidiniummethansulfonat, 1-Propyl-4-ethylpiperidiniummethansulfonat, 1,4-Dipropylpiperidiniummethansulfonat, 1-Propyl-4-butylpiperidiniummethansulfonat, N-Propylpiperidiniumacetat, 1,1-Dipropylpiperidiniumacetat, 1-Propyl-1-Methylpiperidiniumacetat, 1-Propyl-1-ethylpiperidiniumacetat, 1-Propyl-1-butylpiperidiniumacetat, 1-Propyl-2-Methylpiperidiniumacetat, 1-Propyl-2-ethylpiperidiniumacetat, 1,2-Dipropylpiperidiniumacetat, 1-Propyl-2-butylpiperidiniumacetat, 1-Propyl-3-Methylpiperidiniumacetat, 1-Propyl-3-ethylpiperidiniumacetat, 1,3-Dipropylpiperidiniumacetat, 1-Propyl-3-butylpiperidiniumacetat, 1-Propyl-4-Methylpiperidiniumacetat, 1-Propyl-4-ethylpiperidiniumacetat, 1,4-Dipropylpiperidiniumacetat, 1-Propyl-4-butylpiperidiniumacetat, N-Propylpiperidiniumcyanid, 1,1-Dipropylpiperidiniumcyanid, 1-Propyl-1-Methylpiperidiniumcyanid, 1-Propyl-1-ethylpiperidiniumcyanid, 1-Propyl-1-butylpiperidiniumcyanid, 1-Propyl-2-Methylpiperidiniumcyanid, 1-Propyl-2-ethylpiperidiniumcyanid, 1,2-Dipropylpiperidiniumcyanid, 1-Propyl-2-butylpiperidiniumcyanid, 1-Propyl-3-Methylpiperidiniumcyanid, 1-Propyl-3-ethylpiperidiniumcyanid, 1,3-Dipropylpiperidiniumcyanid, 1-Propyl-3-butylpiperidiniumcyanid, 1-Propyl-4-Methylpiperidiniumcyanid, 1-Propyl-4-ethylpiperidiniumcyanid, 1,4-Dipropylpiperidiniumcyanid, 1-Propyl-4-butylpiperidiniumcyanid.

Man erkennt, dass eine Verbindung entsprechend Formel (50) beispielsweise ausgewählt sein kann aus der Gruppe enthaltend N-Butylpiperidiniumchlorid, 1-Butyl-1-methylpiperidiniumchlorid, 1-Butyl-1-ethylpiperidiniumchlorid, 1-Butyl-1-propylpiperidiniumchlorid, 1,1-Dibutylpiperidiniumchlorid, 1-Butyl-2-Methylpiperidiniumchlorid, 1-Butyl-2-ethylpiperidiniumchlorid, 1-Butyl-2-propylpiperidiniumchlorid, 1,2-Dibutylpiperidiniumchlorid, 1-Butyl-3-Methylpiperidiniumchlorid, 1-Butyl-3-ethylpiperidiniumchlorid, 1-Butyl-3-propylpiperidiniumchlorid, 1,3-Dibutylpiperidiniumchlorid, 1-Butyl-4-Methylpiperidiniumchlorid, 1-Butyl-4-ethylpiperidiniumchlorid, 1-Butyl-4-propylpiperidiniumchlorid, 1,4-Dibutylpiperidiniumchlorid, N-Butylpiperidiniumfluorid, 1-Butyl-1-methylpiperidiniumfluorid, 1-Butyl-1-ethylpiperidiniumfluorid, 1-Butyl-1-propylpiperidiniumfluorid, 1,1-Dibutylpiperidiniumfluorid, 1-Butyl-2-Methylpiperidiniumfluorid, 1-Butyl-2-ethylpiperidiniumfluorid, 1-Butyl-2-propylpiperidiniumfluorid, 1,2-Dibutylpiperidiniumfluorid, 1-Butyl-3-Methylpiperidiniumfluorid, 1-Butyl-3-ethylpiperidiniumfluorid, 1-Butyl-3-propylpiperidiniumfluorid, 1,3-Dibutylpiperidiniumfluorid, 1-Butyl-4-Methylpiperidiniumfluorid, 1-Butyl-4-ethylpiperidiniumfluorid, 1-Butyl-4-propylpiperidiniumfluorid, 1,4-Dibutylpiperidiniumfluorid, N-Butylpiperidiniumtriflat, 1-Butyl-1-methylpiperidiniumtriflat, 1-Butyl-1-ethylpiperidiniumtriflat, 1-Butyl-1-propylpiperidiniumtriflat, 1,1-Dibutylpiperidiniumtriflat, 1-Butyl-2-Methylpiperidiniumtriflat, 1-Butyl-2-ethylpiperidiniumtriflat, 1-Butyl-2-propylpiperidiniumtriflat, 1,2-Dibutylpiperidiniumtriflat, 1-Butyl-3-Methylpiperidiniumtriflat, 1-Butyl-3-ethylpiperidiniumtriflat, 1-Butyl-3-propylpiperidiniumtriflat, 1,3-Dibutylpiperidiniumtriflat, 1-Butyl-4-Methylpiperidiniumtriflat, 1-Butyl-4-ethylpiperidiniumtriflat, 1-Butyl-4-propylpiperidiniumtriflat, 1,4-Dibutylpiperidiniumtriflat, N-Butylpiperidiniummethansulfonat, 1-Butyl-1-methylpiperidiniummethansulfonat, 1-Butyl-1-ethylpiperidiniummethansulfonat, 1-Butyl-1-propylpiperidiniummethansulfonat, 1,1-Dibutylpiperidiniummethansulfonat, 1-Butyl-2-Methylpiperidiniummethansulfonat, 1-Butyl-2-ethylpiperidiniummethansulfonat, 1-Butyl-2-propylpiperidiniummethansulfonat, 1,2-Dibutylpiperidiniummethansulfonat, 1-Butyl-3-Methylpiperidiniummethansulfonat, 1-Butyl-3-ethylpiperidiniummethansulfonat, 1-Butyl-3-propylpiperidiniummethansulfonat, 1,3-Dibutylpiperidiniummethansulfonat, 1-Butyl-4-Methylpiperidiniummethansulfonat, 1-Butyl-4-ethylpiperidiniummethansulfonat, 1-Butyl-4-propylpiperidiniummethansulfonat, 1,4-Dibutylpiperidiniummethansulfonat, N-Butylpiperidiniumacetat, 1-Butyl-1-methylpiperidiniumacetat, 1-Butyl-1-ethylpiperidiniumacetat, 1-Butyl-1-propylpiperidiniumacetat, 1,1-Dibutylpiperidiniumacetat, 1-Butyl-2-Methylpiperidiniumacetat, 1-Butyl-2-ethylpiperidiniumacetat, 1-Butyl-2-propylpiperidiniumacetat, 1,2-Dibutylpiperidiniumacetat, 1-Butyl-3-Methylpiperidiniumacetat, 1-Butyl-3-ethylpiperidiniumacetat, 1-Butyl-3-propylpiperidiniumacetat, 1,3-Dibutylpiperidiniumacetat, 1-Butyl-4-Methylpiperidiniumacetat, 1-Butyl-4-ethylpiperidiniumacetat, 1-Butyl-4-propylpiperidiniumacetat, 1,4-Dibutylpiperidiniumacetat, N-Butylpiperidiniumcyanid, 1-Butyl-1-methylpiperidiniumcyanid, 1-Butyl-1-ethylpiperidiniumcyanid, 1-Butyl-1-propylpiperidiniumcyanid, 1,1-Dibutylpiperidiniumcyanid, 1-Butyl-2-Methylpiperidiniumcyanid, 1-Butyl-2-ethylpiperidiniumcyanid, 1-Butyl-2-propylpiperidiniumcyanid, 1,2-Dibutylpiperidiniumcyanid, 1-Butyl-3-Methylpiperidiniumcyanid, 1-Butyl-3-ethylpiperidiniumcyanid, 1-Butyl-3-propylpiperidiniumcyanid, 1,3-Dibutylpiperidiniumcyanid, 1-Butyl-4-Methylpiperidiniumcyanid, 1-Butyl-4-ethylpiperidiniumcyanid, 1-Butyl-4-propylpiperidiniumcyanid, 1,4-Dibutylpiperidiniumcyanid.

Man erkennt, dass eine Verbindung entsprechend Formel (51) beispielsweise ausgewählt sein kann aus der Gruppe enthaltend N-Pentylpiperidiniumchlorid, 1-Pentyl-1-methylpiperidiniumchlorid, 1-Pentyl-1-ethylpiperidiniumchlorid, 1-Pentyl-1-propylpiperidiniumchlorid, 1-Pentyl-1-butylpiperidiniumchlorid, 1-Pentyl-2-Methylpiperidiniumchlorid, 1-Pentyl-2-ethylpiperidiniumchlorid, 1-Pentyl-2-propylpiperidiniumchlorid, 1-Pentyl-2-butylpiperidiniumchlorid, 1-Pentyl-3-Methylpiperidiniumchlorid, 1-Pentyl-3-ethylpiperidiniumchlorid, 1-Pentyl-3-propylpiperidiniumchlorid, 1-Pentyl-3-butylpiperidiniumchlorid, 1-Pentyl-4-Methylpiperidiniumchlorid, 1-Pentyl-4-ethylpiperidiniumchlorid, 1-Pentyl-4-propylpiperidiniumchlorid, 1-Pentyl-4-butylpiperidiniumchlorid, N-Pentylpiperidiniumfluorid, 1-Pentyl-1-methylpiperidiniumfluorid, 1-Pentyl-1-ethylpiperidiniumfluorid, 1-Pentyl-1-propylpiperidiniumfluorid, 1-Pentyl-1-butylpiperidiniumfluorid, 1-Pentyl-2-Methylpiperidiniumfluorid, 1-Pentyl-2-ethylpiperidiniumfluorid, 1-Pentyl-2-propylpiperidiniumfluorid, 1-Pentyl-2-butylpiperidiniumfluorid, 1-Pentyl-3-Methylpiperidiniumfluorid, 1-Pentyl-3-ethylpiperidiniumfluorid, 1-Pentyl-3-propylpiperidiniumfluorid, 1-Pentyl-3-butylpiperidiniumfluorid, 1-Pentyl-4-Methylpiperidiniumfluorid, 1-Pentyl-4-ethylpiperidiniumfluorid, 1-Pentyl-4-propylpiperidiniumfluorid, 1-Pentyl-4-butylpiperidiniumfluorid, N-Pentylpiperidiniumtriflat, 1-Pentyl-1-methylpiperidiniumtriflat, 1-Pentyl-1-ethylpiperidiniumtriflat, 1-Pentyl-1-propylpiperidiniumtriflat, 1-Pentyl-1-butylpiperidiniumtriflat, 1-Pentyl-2-Methylpiperidiniumtriflat, 1-Pentyl-2-ethylpiperidiniumtriflat, 1-Pentyl-2-propylpiperidiniumtriflat, 1-Pentyl-2-butylpiperidiniumtriflat, 1-Pentyl-3-Methylpiperidiniumtriflat, 1-Pentyl-3-ethylpiperidiniumtriflat, 1-Pentyl-3-propylpiperidiniumtriflat, 1-Pentyl-3-butylpiperidiniumtriflat, 1-Pentyl-4-Methylpiperidiniumtriflat, 1-Pentyl-4-ethylpiperidiniumtriflat, 1-Pentyl-4-propylpiperidiniumtriflat, 1-Pentyl-4-butylpiperidiniumtriflat, N-Pentylpiperidiniummethansulfonat, 1-Pentyl-1-methylpiperidiniummethansulfonat, 1-Pentyl-1-ethylpiperidiniummethansulfonat, 1-Pentyl-1-propylpiperidiniummethansulfonat, 1-Pentyl-1-butylpiperidiniummethansulfonat, 1-Pentyl-2-Methylpiperidiniummethansulfonat, 1-Pentyl-2-ethylpiperidiniummethansulfonat, 1-Pentyl-2-propylpiperidiniummethansulfonat, 1-Pentyl-2-butylpiperidiniummethansulfonat, 1-Pentyl-3-Methylpiperidiniummethansulfonat, 1-Pentyl-3-ethylpiperidiniummethansulfonat, 1-Pentyl-3-propylpiperidiniummethansulfonat, 1-Pentyl-3-butylpiperidiniummethansulfonat, 1-Pentyl-4-Methylpiperidiniummethansulfonat, 1-Pentyl-4-ethylpiperidiniummethansulfonat, 1-Pentyl-4-propylpiperidiniummethansulfonat, 1-Pentyl-4-butylpiperidiniummethansulfonat, N-Pentylpiperidiniumacetat, 1-Pentyl-1-methylpiperidiniumacetat, 1-Pentyl-1-ethylpiperidiniumacetat, 1-Pentyl-1-propylpiperidiniumacetat, 1-Pentyl-1-butylpiperidiniumacetat, 1-Pentyl-2-Methylpiperidiniumacetat, 1-Pentyl-2-ethylpiperidiniumacetat, 1-Pentyl-2-propylpiperidiniumacetat, 1-Pentyl-2-butylpiperidiniumacetat, 1-Pentyl-3-Methylpiperidiniumacetat, 1-Pentyl-3-ethylpiperidiniumacetat, 1-Pentyl-3-propylpiperidiniumacetat, 1-Pentyl-3-butylpiperidiniumacetat, 1-Pentyl-4-Methylpiperidiniumacetat, 1-Pentyl-4-ethylpiperidiniumacetat, 1-Pentyl-4-propylpiperidiniumacetat, 1-Pentyl-4-butylpiperidiniumacetat, N-Pentylpiperidiniumcyanid, 1-Pentyl-1-methylpiperidiniumcyanid, 1-Pentyl-1-ethylpiperidiniumcyanid, 1-Pentyl-1-propylpiperidiniumcyanid, 1-Pentyl-1-butylpiperidiniumcyanid, 1-Pentyl-2-Methylpiperidiniumcyanid, 1-Pentyl-2-ethylpiperidiniumcyanid, 1-Pentyl-2-propylpiperidiniumcyanid, 1-Pentyl-2-butylpiperidiniumcyanid, 1-Pentyl-3-Methylpiperidiniumcyanid, 1-Pentyl-3-ethylpiperidiniumcyanid, 1-Pentyl-3-propylpiperidiniumcyanid, 1-Pentyl-3-butylpiperidiniumcyanid, 1-Pentyl-4-Methylpiperidiniumcyanid, 1-Pentyl-4-ethylpiperidiniumcyanid, 1-Pentyl-4-propylpiperidiniumcyanid, 1-Pentyl-4-butylpiperidiniumcyanid.

Man erkennt weiter, dass eine Verbindung entsprechend Formel (52) ausgewählt sein kann aus der Gruppe enthaltend N-Hexylpiperidiniumchlorid, 1-Hexyl-1-methylpiperidiniumchlorid, 1-Hexyl-1-ethylpiperidiniumchlorid, 1-Hexyl-1-propylpiperidiniumchlorid, 1-Hexyl-1-butylpiperidiniumchlorid, 1-Hexyl-2-Methylpiperidiniumchlorid, 1-Hexyl-2-ethylpiperidiniumchlorid, 1-Hexyl-2-propylpiperidiniumchlorid, 1-Hexyl-2-butylpiperidiniumchlorid, 1-Hexyl-3-Methylpiperidiniumchlorid, 1-Hexyl-3-ethylpiperidiniumchlorid, 1-Hexyl-3-propylpiperidiniumchlorid, 1-Hexyl-3-butylpiperidiniumchlorid, 1-Hexyl-4-Methylpiperidiniumchlorid, 1-Hexyl-4-ethylpiperidiniumchlorid, 1-Hexyl-4-propylpiperidiniumchlorid, 1-Hexyl-4-butylpiperidiniumchlorid, N-Hexylpiperidiniumfluorid, 1-Hexyl-1-methylpiperidiniumfluorid, 1-Hexyl-1-ethylpiperidiniumfluorid, 1-Hexyl-1-propylpiperidiniumfluorid, 1-Hexyl-1-butylpiperidiniumfluorid, 1-Hexyl-2-Methylpiperidiniumfluorid, 1-Hexyl-2-ethylpiperidiniumfluorid, 1-Hexyl-2-propylpiperidiniumfluorid, 1-Hexyl-2-butylpiperidiniumfluorid, 1-Hexyl-3-Methylpiperidiniumfluorid, 1-Hexyl-3-ethylpiperidiniumfluorid, 1-Hexyl-3-propylpiperidiniumfluorid, 1-Hexyl-3-butylpiperidiniumfluorid, 1-Hexyl-4-Methylpiperidiniumfluorid, 1-Hexyl-4-ethylpiperidiniumfluorid, 1-Hexyl-4-propylpiperidiniumfluorid, 1-Hexyl-4-butylpiperidiniumfluorid, N-Hexylpiperidiniumtriflat, 1-Hexyl-1-methylpiperidiniumtriflat, 1-Hexyl-1-ethylpiperidiniumtriflat, 1-Hexyl-1-propylpiperidiniumtriflat, 1-Hexyl-1-butylpiperidiniumtriflat, 1-Hexyl-2-Methylpiperidiniumtriflat, 1-Hexyl-2-ethylpiperidiniumtriflat, 1-Hexyl-2-propylpiperidiniumtriflat, 1-Hexyl-2-butylpiperidiniumtriflat, 1-Hexyl-3-Methylpiperidiniumtriflat, 1-Hexyl-3-ethylpiperidiniumtriflat, 1-Hexyl-3-propylpiperidiniumtriflat, 1-Hexyl-3-butylpiperidiniumtriflat, 1-Hexyl-4-Methylpiperidiniumtriflat, 1-Hexyl-4-ethylpiperidiniumtriflat, 1-Hexyl-4-propylpiperidiniumtriflat, 1-Hexyl-4-butylpiperidiniumtriflat, N-Hexylpiperidiniummethansulfonat, 1-Hexyl-1-methylpiperidiniummethansulfonat, 1-Hexyl-1-ethylpiperidiniummethansulfonat, 1-Hexyl-1-propylpiperidiniummethansulfonat, 1-Hexyl-1-butylpiperidiniummethansulfonat, 1-Hexyl-2-Methylpiperidiniummethansulfonat, 1-Hexyl-2-ethylpiperidiniummethansulfonat, 1-Hexyl-2-propylpiperidiniummethansulfonat, 1-Hexyl-2-butylpiperidiniummethansulfonat, 1-Hexyl-3-Methylpiperidiniummethansulfonat, 1-Hexyl-3-ethylpiperidiniummethansulfonat, 1-Hexyl-3-propylpiperidiniummethansulfonat, 1-Hexyl-3-butylpiperidiniummethansulfonat, 1-Hexyl-4-Methylpiperidiniummethansulfonat, 1-Hexyl-4-ethylpiperidiniummethansulfonat, 1-Hexyl-4-propylpiperidiniummethansulfonat, 1-Hexyl-4-butylpiperidiniummethansulfonat, N-Hexylpiperidiniumacetat, 1-Hexyl-1-methylpiperidiniumacetat, 1-Hexyl-1-ethylpiperidiniumacetat, 1-Hexyl-1-propylpiperidiniumacetat, 1-Hexyl-1-butylpiperidiniumacetat, 1-Hexyl-2-Methylpiperidiniumacetat, 1-Hexyl-2-ethylpiperidiniumacetat, 1-Hexyl-2-propylpiperidiniumacetat, 1-Hexyl-2-butylpiperidiniumacetat, 1-Hexyl-3-Methylpiperidiniumacetat, 1-Hexyl-3-ethylpiperidiniumacetat, 1-Hexyl-3-propylpiperidiniumacetat, 1-Hexyl-3-butylpiperidiniumacetat, 1-Hexyl-4-Methylpiperidiniumacetat, 1-Hexyl-4-ethylpiperidiniumacetat, 1-Hexyl-4-propylpiperidiniumacetat, 1-Hexyl-4-butylpiperidiniumacetat, N-Hexylpiperidiniumcyanid, 1-Hexyl-1-methylpiperidiniumcyanid, 1-Hexyl-1-ethylpiperidiniumcyanid, 1-Hexyl-1-propylpiperidiniumcyanid, 1-Hexyl-1-butylpiperidiniumcyanid, 1-Hexyl-2-Methylpiperidiniumcyanid, 1-Hexyl-2-ethylpiperidiniumcyanid, 1-Hexyl-2-propylpiperidiniumcyanid, 1-Hexyl-2-butylpiperidiniumcyanid, 1-Hexyl-3-Methylpiperidiniumcyanid, 1-Hexyl-3-ethylpiperidiniumcyanid, 1-Hexyl-3-propylpiperidiniumcyanid, 1-Hexyl-3-butylpiperidiniumcyanid, 1-Hexyl-4-Methylpiperidiniumcyanid, 1-Hexyl-4-ethylpiperidiniumcyanid, 1-Hexyl-4-propylpiperidiniumcyanid, 1-Hexyl-4-butylpiperidiniumcyanid.

Man erkennt weiter, dass eine Verbindung entsprechend Formel (53) ausgewählt sein kann aus der Gruppe enthaltend N-Heptylpiperidiniumchlorid, 1-Heptyl-1-methylpiperidiniumchlorid, 1-Heptyl-1-ethylpiperidiniumchlorid, 1-Heptyl-1-propylpiperidiniumchlorid, 1-Heptyl-1-butylpiperidiniumchlorid, 1-Heptyl-2-Methylpiperidiniumchlorid, 1-Heptyl-2-ethylpiperidiniumchlorid, 1-Heptyl-2-propylpiperidiniumchlorid, 1-Heptyl-2-butylpiperidiniumchlorid, 1-Heptyl-3-Methylpiperidiniumchlorid, 1-Heptyl-3-ethylpiperidiniumchlorid, 1-Heptyl-3-propylpiperidiniumchlorid, 1-Heptyl-3-butylpiperidiniumchlorid, 1-Heptyl-4-Methylpiperidiniumchlorid, 1-Heptyl-4-ethylpiperidiniumchlorid, 1-Heptyl-4-propylpiperidiniumchlorid, 1-Heptyl-4-butylpiperidiniumchlorid, N-Heptylpiperidiniumfluorid, 1-Heptyl-1-methylpiperidiniumfluorid, 1-Heptyl-1-ethylpiperidiniumfluorid, 1-Heptyl-1-propylpiperidiniumfluorid, 1-Heptyl-1-butylpiperidiniumfluorid, 1-Heptyl-2-Methylpiperidiniumfluorid, 1-Heptyl-2-ethylpiperidiniumfluorid, 1-Heptyl-2-propylpiperidiniumfluorid, 1-Heptyl-2-butylpiperidiniumfluorid, 1-Heptyl-3-Methylpiperidiniumfluorid, 1-Heptyl-3-ethylpiperidiniumfluorid, 1-Heptyl-3-propylpiperidiniumfluorid, 1-Heptyl-3-butylpiperidiniumfluorid, 1-Heptyl-4-Methylpiperidiniumfluorid, 1-Heptyl-4-ethylpiperidiniumfluorid, 1-Heptyl-4-propylpiperidiniumfluorid, 1-Heptyl-4-butylpiperidiniumfluorid, N-Heptylpiperidiniumtriflat, 1-Heptyl-1-methylpiperidiniumtriflat, 1-Heptyl-1-ethylpiperidiniumtriflat, 1-Heptyl-1-propylpiperidiniumtriflat, 1-Heptyl-1-butylpiperidiniumtriflat, 1-Heptyl-2-Methylpiperidiniumtriflat, 1-Heptyl-2-ethylpiperidiniumtriflat, 1-Heptyl-2-propylpiperidiniumtriflat, 1-Heptyl-2-butylpiperidiniumtriflat, 1-Heptyl-3-Methylpiperidiniumtriflat, 1-Heptyl-3-ethylpiperidiniumtriflat, 1-Heptyl-3-propylpiperidiniumtriflat, 1-Heptyl-3-butylpiperidiniumtriflat, 1-Heptyl-4-Methylpiperidiniumtriflat, 1-Heptyl-4-ethylpiperidiniumtriflat, 1-Heptyl-4-propylpiperidiniumtriflat, 1-Heptyl-4-butylpiperidiniumtriflat, N-Heptylpiperidiniummethansulfonat, 1-Heptyl-1-methylpiperidiniummethansulfonat, 1-Heptyl-1-ethylpiperidiniummethansulfonat, 1-Heptyl-1-propylpiperidiniummethansulfonat, 1-Heptyl-1-butylpiperidiniummethansulfonat, 1-Heptyl-2-Methylpiperidiniummethansulfonat, 1-Heptyl-2-ethylpiperidiniummethansulfonat, 1-Heptyl-2-propylpiperidiniummethansulfonat, 1-Heptyl-2-butylpiperidiniummethansulfonat, 1-Heptyl-3-Methylpiperidiniummethansulfonat, 1-Heptyl-3-ethylpiperidiniummethansulfonat, 1-Heptyl-3-propylpiperidiniummethansulfonat, 1-Heptyl-3-butylpiperidiniummethansulfonat, 1-Heptyl-4-Methylpiperidiniummethansulfonat, 1-Heptyl-4-ethylpiperidiniummethansulfonat, 1-Heptyl-4-propylpiperidiniummethansulfonat, 1-Heptyl-4-butylpiperidiniummethansulfonat, N-Heptylpiperidiniumacetat, 1-Heptyl-1-methylpiperidiniumacetat, 1-Heptyl-1-ethylpiperidiniumacetat, 1-Heptyl-1-propylpiperidiniumacetat, 1-Heptyl-1-butylpiperidiniumacetat, 1-Heptyl-2-Methylpiperidiniumacetat, 1-Heptyl-2-ethylpiperidiniumacetat, 1-Heptyl-2-propylpiperidiniumacetat, 1-Heptyl-2-butylpiperidiniumacetat, 1-Heptyl-3-Methylpiperidiniumacetat, 1-Heptyl-3-ethylpiperidiniumacetat, 1-Heptyl-3-propylpiperidiniumacetat, 1-Heptyl-3-butylpiperidiniumacetat, 1-Heptyl-4-Methylpiperidiniumacetat, 1-Heptyl-4-ethylpiperidiniumacetat, 1-Heptyl-4-propylpiperidiniumacetat, 1-Heptyl-4-butylpiperidiniumacetat, N-Heptylpiperidiniumcyanid, 1-Heptyl-1-methylpiperidiniumcyanid, 1-Heptyl-1-ethylpiperidiniumcyanid, 1-Heptyl-1-propylpiperidiniumcyanid, 1-Heptyl-1-butylpiperidiniumcyanid, 1-Heptyl-2-Methylpiperidiniumcyanid, 1-Heptyl-2-ethylpiperidiniumcyanid, 1-Heptyl-2-propylpiperidiniumcyanid, 1-Heptyl-2-butylpiperidiniumcyanid, 1-Heptyl-3-Methylpiperidiniumcyanid, 1-Heptyl-3-ethylpiperidiniumcyanid, 1-Heptyl-3-propylpiperidiniumcyanid, 1-Heptyl-3-butylpiperidiniumcyanid, 1-Heptyl-4-Methylpiperidiniumcyanid, 1-Heptyl-4-ethylpiperidiniumcyanid, 1-Heptyl-4-propylpiperidiniumcyanid, 1-Heptyl-4-butylpiperidiniumcyanid.

Man erkennt weiter, dass eine Verbindung entsprechend Formel (54) ausgewählt sein kann aus der Gruppe enthaltend N-Octylpiperidiniumchlorid, 1-Octyl-1-methylpiperidiniumchlorid, 1-Octyl-1-ethylpiperidiniumchlorid, 1-Octyl-1-propylpiperidiniumchlorid, 1-Octyl-1-butylpiperidiniumchlorid, 1-Octyl-2-Methylpiperidiniumchlorid, 1-Octyl-2-ethylpiperidiniumchlorid, 1-Octyl-2-propylpiperidiniumchlorid, 1-Octyl-2-butylpiperidiniumchlorid, 1-Octyl-3-Methylpiperidiniumchlorid, 1-Octyl-3-ethylpiperidiniumchlorid, 1-Octyl-3-propylpiperidiniumchlorid, 1-Octyl-3-butylpiperidiniumchlorid, 1-Octyl-4-Methylpiperidiniumchlorid, 1-Octyl-4-ethylpiperidiniumchlorid, 1-Octyl-4-propylpiperidiniumchlorid, 1-Octyl-4-butylpiperidiniumchlorid, N-Octylpiperidiniumfluorid, 1-Octyl-1-methylpiperidiniumfluorid, 1-Octyl-1-ethylpiperidiniumfluorid, 1-Octyl-1-propylpiperidiniumfluorid, 1-Octyl-1-butylpiperidiniumfluorid, 1-Octyl-2-Methylpiperidiniumfluorid, 1-Octyl-2-ethylpiperidiniumfluorid, 1-Octyl-2-propylpiperidiniumfluorid, 1-Octyl-2-butylpiperidiniumfluorid, 1-Octyl-3-Methylpiperidiniumfluorid, 1-Octyl-3-ethylpiperidiniumfluorid, 1-Octyl-3-propylpiperidiniumfluorid, 1-Octyl-3-butylpiperidiniumfluorid, 1-Octyl-4-Methylpiperidiniumfluorid, 1-Octyl-4-ethylpiperidiniumfluorid, 1-Octyl-4-propylpiperidiniumfluorid, 1-Octyl-4-butylpiperidiniumfluorid, N-Octylpiperidiniumtriflat, 1-Octyl-1-methylpiperidiniumtriflat, 1-Octyl-1-ethylpiperidiniumtriflat, 1-Octyl-1-propylpiperidiniumtriflat, 1-Octyl-1-butylpiperidiniumtriflat, 1-Octyl-2-Methylpiperidiniumtriflat, 1-Octyl-2-ethylpiperidiniumtriflat, 1-Octyl-2-propylpiperidiniumtriflat, 1-Octyl-2-butylpiperidiniumtriflat, 1-Octyl-3-Methylpiperidiniumtriflat, 1-Octyl-3-ethylpiperidiniumtriflat, 1-Octyl-3-propylpiperidiniumtriflat, 1-Octyl-3-butylpiperidiniumtriflat, 1-Octyl-4-Methylpiperidiniumtriflat, 1-Octyl-4-ethylpiperidiniumtriflat, 1-Octyl-4-propylpiperidiniumtriflat, 1-Octyl-4-butylpiperidiniumtriflat, N-Octylpiperidiniummethansulfonat, 1-Octyl-1-methylpiperidiniummethansulfonat, 1-Octyl-1-ethylpiperidiniummethansulfonat, 1-Octyl-1-propylpiperidiniummethansulfonat, 1-Octyl-1-butylpiperidiniummethansulfonat, 1-Octyl-2-Methylpiperidiniummethansulfonat, 1-Octyl-2-ethylpiperidiniummethansulfonat, 1-Octyl-2-propylpiperidiniummethansulfonat, 1-Octyl-2-butylpiperidiniummethansulfonat, 1-Octyl-3-Methylpiperidiniummethansulfonat, 1-Octyl-3-ethylpiperidiniummethansulfonat, 1-Octyl-3-propylpiperidiniummethansulfonat, 1-Octyl-3-butylpiperidiniummethansulfonat, 1-Octyl-4-Methylpiperidiniummethansulfonat, 1-Octyl-4-ethylpiperidiniummethansulfonat, 1-Octyl-4-propylpiperidiniummethansulfonat, 1-Octyl-4-butylpiperidiniummethansulfonat, N-Octylpiperidiniumacetat, 1-Octyl-1-methylpiperidiniumacetat, 1-Octyl-1-ethylpiperidiniumacetat, 1-Octyl-1-propylpiperidiniumacetat, 1-Octyl-1-butylpiperidiniumacetat, 1-Octyl-2-Methylpiperidiniumacetat, 1-Octyl-2-ethylpiperidiniumacetat, 1-Octyl-2-propylpiperidiniumacetat, 1-Octyl-2-butylpiperidiniumacetat, 1-Octyl-3-Methylpiperidiniumacetat, 1-Octyl-3-ethylpiperidiniumacetat, 1-Octyl-3-propylpiperidiniumacetat, 1-Octyl-3-butylpiperidiniumacetat, 1-Octyl-4-Methylpiperidiniumacetat, 1-Octyl-4-ethylpiperidiniumacetat, 1-Octyl-4-propylpiperidiniumacetat, 1-Octyl-4-butylpiperidiniumacetat, N-Octylpiperidiniumcyanid, 1-Octyl-1-methylpiperidiniumcyanid, 1-Octyl-1-ethylpiperidiniumcyanid, 1-Octyl-1-propylpiperidiniumcyanid, 1-Octyl-1-butylpiperidiniumcyanid, 1-Octyl-2-Methylpiperidiniumcyanid, 1-Octyl-2-ethylpiperidiniumcyanid, 1-Octyl-2-propylpiperidiniumcyanid, 1-Octyl-2-butylpiperidiniumcyanid, 1-Octyl-3-Methylpiperidiniumcyanid, 1-Octyl-3-ethylpiperidiniumcyanid, 1-Octyl-3-propylpiperidiniumcyanid, 1-Octyl-3-butylpiperidiniumcyanid, 1-Octyl-4-Methylpiperidiniumcyanid, 1-Octyl-4-ethylpiperidiniumcyanid, 1-Octyl-4-propylpiperidiniumcyanid, 1-Octyl-4-butylpiperidiniumcyanid.

Man erkennt weiter, dass eine Verbindung entsprechend Formel (55) ausgewählt sein kann aus der Gruppe enthaltend N-Nonylpiperidiniumchlorid, 1-Nonyl-1-methylpiperidiniumchlorid, 1-Nonyl-1-ethylpiperidiniumchlorid, 1-Nonyl-1-propylpiperidiniumchlorid, 1-Nonyl-1-butylpiperidiniumchlorid, 1-Nonyl-2-Methylpiperidiniumchlorid, 1-Nonyl-2-ethylpiperidiniumchlorid, 1-Nonyl-2-propylpiperidiniumchlorid, 1-Nonyl-2-butylpiperidiniumchlorid, 1-Nonyl-3-Methylpiperidiniumchlorid, 1-Nonyl-3-ethylpiperidiniumchlorid, 1-Nonyl-3-propylpiperidiniumchlorid, 1-Nonyl-3-butylpiperidiniumchlorid, 1-Nonyl-4-Methylpiperidiniumchlorid, 1-Nonyl-4-ethylpiperidiniumchlorid, 1-Nonyl-4-propylpiperidiniumchlorid, 1-Nonyl-4-butylpiperidiniumchlorid, N-Nonylpiperidiniumfluorid, 1-Nonyl-1-methylpiperidiniumfluorid, 1-Nonyl-1-ethylpiperidiniumfluorid, 1-Nonyl-1-propylpiperidiniumfluorid, 1-Nonyl-1-butylpiperidiniumfluorid, 1-Nonyl-2-Methylpiperidiniumfluorid, 1-Nonyl-2-ethylpiperidiniumfluorid, 1-Nonyl-2-propylpiperidiniumfluorid, 1-Nonyl-2-butylpiperidiniumfluorid, 1-Nonyl-3-Methylpiperidiniumfluorid, 1-Nonyl-3-ethylpiperidiniumfluorid, 1-Nonyl-3-propylpiperidiniumfluorid, 1-Nonyl-3-butylpiperidiniumfluorid, 1-Nonyl-4-Methylpiperidiniumfluorid, 1-Nonyl-4-ethylpiperidiniumfluorid, 1-Nonyl-4-propylpiperidiniumfluorid, 1-Nonyl-4-butylpiperidiniumfluorid, N-Nonylpiperidiniumtriflat, 1-Nonyl-1-methylpiperidiniumtriflat, 1-Nonyl-1-ethylpiperidiniumtriflat, 1-Nonyl-1-propylpiperidiniumtriflat, 1-Nonyl-1-butylpiperidiniumtriflat, 1-Nonyl-2-Methylpiperidiniumtriflat, 1-Nonyl-2-ethylpiperidiniumtriflat, 1-Nonyl-2-propylpiperidiniumtriflat, 1-Nonyl-2-butylpiperidiniumtriflat, 1-Nonyl-3-Methylpiperidiniumtriflat, 1-Nonyl-3-ethylpiperidiniumtriflat, 1-Nonyl-3-propylpiperidiniumtriflat, 1-Nonyl-3-butylpiperidiniumtriflat, 1-Nonyl-4-Methylpiperidiniumtriflat, 1-Nonyl-4-ethylpiperidiniumtriflat, 1-Nonyl-4-propylpiperidiniumtriflat, 1-Nonyl-4-butylpiperidiniumtriflat, N-Nonylpiperidiniummethansulfonat, 1-Nonyl-1-methylpiperidiniummethansulfonat, 1-Nonyl-1-ethylpiperidiniummethansulfonat, 1-Nonyl-1-propylpiperidiniummethansulfonat, 1-Nonyl-1-butylpiperidiniummethansulfonat, 1-Nonyl-2-Methylpiperidiniummethansulfonat, 1-Nonyl-2-ethylpiperidiniummethansulfonat, 1-Nonyl-2-propylpiperidiniummethansulfonat, 1-Nonyl-2-butylpiperidiniummethansulfonat, 1-Nonyl-3-Methylpiperidiniummethansulfonat, 1-Nonyl-3-ethylpiperidiniummethansulfonat, 1-Nonyl-3-propylpiperidiniummethansulfonat, 1-Nonyl-3-butylpiperidiniummethansulfonat, 1-Nonyl-4-Methylpiperidiniummethansulfonat, 1-Nonyl-4-ethylpiperidiniummethansulfonat, 1-Nonyl-4-propylpiperidiniummethansulfonat, 1-Nonyl-4-butylpiperidiniummethansulfonat, N-Nonylpiperidiniumacetat, 1-Nonyl-1-methylpiperidiniumacetat, 1-Nonyl-1-ethylpiperidiniumacetat, 1-Nonyl-1-propylpiperidiniumacetat, 1-Nonyl-1-butylpiperidiniumacetat, 1-Nonyl-2-Methylpiperidiniumacetat, 1-Nonyl-2-ethylpiperidiniumacetat, 1-Nonyl-2-propylpiperidiniumacetat, 1-Nonyl-2-butylpiperidiniumacetat, 1-Nonyl-3-Methylpiperidiniumacetat, 1-Nonyl-3-ethylpiperidiniumacetat, 1-Nonyl-3-propylpiperidiniumacetat, 1-Nonyl-3-butylpiperidiniumacetat, 1-Nonyl-4-Methylpiperidiniumacetat, 1-Nonyl-4-ethylpiperidiniumacetat, 1-Nonyl-4-propylpiperidiniumacetat, 1-Nonyl-4-butylpiperidiniumacetat, N-Nonylpiperidiniumcyanid, 1-Nonyl-1-methylpiperidiniumcyanid, 1-Nonyl-1-ethylpiperidiniumcyanid, 1-Nonyl-1-propylpiperidiniumcyanid, 1-Nonyl-1-butylpiperidiniumcyanid, 1-Nonyl-2-Methylpiperidiniumcyanid, 1-Nonyl-2-ethylpiperidiniumcyanid, 1-Nonyl-2-propylpiperidiniumcyanid, 1-Nonyl-2-butylpiperidiniumcyanid, 1-Nonyl-3-Methylpiperidiniumcyanid, 1-Nonyl-3-ethylpiperidiniumcyanid, 1-Nonyl-3-propylpiperidiniumcyanid, 1-Nonyl-3-butylpiperidiniumcyanid, 1-Nonyl-4-Methylpiperidiniumcyanid, 1-Nonyl-4-ethylpiperidiniumcyanid, 1-Nonyl-4-propylpiperidiniumcyanid, 1-Nonyl-4-butylpiperidiniumcyanid.

Man erkennt weiter, dass eine Verbindung entsprechend Formel (56) ausgewählt sein kann aus der Gruppe enthaltend N-Decylpiperidiniumchlorid, 1-Decyl-1-methylpiperidiniumchlorid, 1-Decyl-1-ethylpiperidiniumchlorid, 1-Decyl-1-propylpiperidiniumchlorid, 1-Decyl-1-butylpiperidiniumchlorid, 1-Decyl-2-Methylpiperidiniumchlorid, 1-Decyl-2-ethylpiperidiniumchlorid, 1-Decyl-2-propylpiperidiniumchlorid, 1-Decyl-2-butylpiperidiniumchlorid, 1-Decyl-3-Methylpiperidiniumchlorid, 1-Decyl-3-ethylpiperidiniumchlorid, 1-Decyl-3-propylpiperidiniumchlorid, 1-Decyl-3-butylpiperidiniumchlorid, 1-Decyl-4-Methylpiperidiniumchlorid, 1-Decyl-4-ethylpiperidiniumchlorid, 1-Decyl-4-propylpiperidiniumchlorid, 1-Decyl-4-butylpiperidiniumchlorid, N-Decylpiperidiniumfluorid, 1-Decyl-1-methylpiperidiniumfluorid, 1-Decyl-1-ethylpiperidiniumfluorid, 1-Decyl-1-propylpiperidiniumfluorid, 1-Decyl-1-butylpiperidiniumfluorid, 1-Decyl-2-Methylpiperidiniumfluorid, 1-Decyl-2-ethylpiperidiniumfluorid, 1-Decyl-2-propylpiperidiniumfluorid, 1-Decyl-2-butylpiperidiniumfluorid, 1-Decyl-3-Methylpiperidiniumfluorid, 1-Decyl-3-ethylpiperidiniumfluorid, 1-Decyl-3-propylpiperidiniumfluorid, 1-Decyl-3-butylpiperidiniumfluorid, 1-Decyl-4-Methylpiperidiniumfluorid, 1-Decyl-4-ethylpiperidiniumfluorid, 1-Decyl-4-propylpiperidiniumfluorid, 1-Decyl-4-butylpiperidiniumfluorid, N-Decylpiperidiniumtriflat, 1-Decyl-1-methylpiperidiniumtriflat, 1-Decyl-1-ethylpiperidiniumtriflat, 1-Decyl-1-propylpiperidiniumtriflat, 1-Decyl-1-butylpiperidiniumtriflat, 1-Decyl-2-Methylpiperidiniumtriflat, 1-Decyl-2-ethylpiperidiniumtriflat, 1-Decyl-2-propylpiperidiniumtriflat, 1-Decyl-2-butylpiperidiniumtriflat, 1-Decyl-3-Methylpiperidiniumtriflat, 1-Decyl-3-ethylpiperidiniumtriflat, 1-Decyl-3-propylpiperidiniumtriflat, 1-Decyl-3-butylpiperidiniumtriflat, 1-Decyl-4-Methylpiperidiniumtriflat, 1-Decyl-4-ethylpiperidiniumtriflat, 1-Decyl-4-propylpiperidiniumtriflat, 1-Decyl-4-butylpiperidiniumtriflat, N-Decylpiperidiniummethansulfonat, 1-Decyl-1-methylpiperidiniummethansulfonat, 1-Decyl-1-ethylpiperidiniummethansulfonat, 1-Decyl-1-propylpiperidiniummethansulfonat, 1-Decyl-1-butylpiperidiniummethansulfonat, 1-Decyl-2-Methylpiperidiniummethansulfonat, 1-Decyl-2-ethylpiperidiniummethansulfonat, 1-Decyl-2-propylpiperidiniummethansulfonat, 1-Decyl-2-butylpiperidiniummethansulfonat, 1-Decyl-3-Methylpiperidiniummethansulfonat, 1-Decyl-3-ethylpiperidiniummethansulfonat, 1-Decyl-3-propylpiperidiniummethansulfonat, 1-Decyl-3-butylpiperidiniummethansulfonat, 1-Decyl-4-Methylpiperidiniummethansulfonat, 1-Decyl-4-ethylpiperidiniummethansulfonat, 1-Decyl-4-propylpiperidiniummethansulfonat, 1-Decyl-4-butylpiperidiniummethansulfonat, N-Decylpiperidiniumacetat, 1-Decyl-1-methylpiperidiniumacetat, 1-Decyl-1-ethylpiperidiniumacetat, 1-Decyl-1-propylpiperidiniumacetat, 1-Decyl-1-butylpiperidiniumacetat, 1-Decyl-2-Methylpiperidiniumacetat, 1-Decyl-2-ethylpiperidiniumacetat, 1-Decyl-2-propylpiperidiniumacetat, 1-Decyl-2-butylpiperidiniumacetat, 1-Decyl-3-Methylpiperidiniumacetat, 1-Decyl-3-ethylpiperidiniumacetat, 1-Decyl-3-propylpiperidiniumacetat, 1-Decyl-3-butylpiperidiniumacetat, 1-Decyl-4-Methylpiperidiniumacetat, 1-Decyl-4-ethylpiperidiniumacetat, 1-Decyl-4-propylpiperidiniumacetat, 1-Decyl-4-butylpiperidiniumacetat, N-Decylpiperidiniumcyanid, 1-Decyl-1-methylpiperidiniumcyanid, 1-Decyl-1-ethylpiperidiniumcyanid, 1-Decyl-1-propylpiperidiniumcyanid, 1-Decyl-1-butylpiperidiniumcyanid, 1-Decyl-2-Methylpiperidiniumcyanid, 1-Decyl-2-ethylpiperidiniumcyanid, 1-Decyl-2-propylpiperidiniumcyanid, 1-Decyl-2-butylpiperidiniumcyanid, 1-Decyl-3-Methylpiperidiniumcyanid, 1-Decyl-3-ethylpiperidiniumcyanid, 1-Decyl-3-propylpiperidiniumcyanid, 1-Decyl-3-butylpiperidiniumcyanid, 1-Decyl-4-Methylpiperidiniumcyanid, 1-Decyl-4-ethylpiperidiniumcyanid, 1-Decyl-4-propylpiperidiniumcyanid, 1-Decyl-4-butylpiperidiniumcyanid.

Man erkennt weiter, dass ein Verbindung entsprechend Formel (57) ausgewählt sein kann aus der Gruppe enthaltend N-Undecylpiperidiniumchlorid, 1-Undecyl-1-methylpiperidiniumchlorid, 1-Undecyl-1-ethylpiperidiniumchlorid, 1-Undecyl-1-propylpiperidiniumchlorid, 1-Undecyl-1-butylpiperidiniumchlorid, 1-Undecyl-2-Methylpiperidiniumchlorid, 1-Undecyl-2-ethylpiperidiniumchlorid, 1-Undecyl-2-propylpiperidiniumchlorid, 1-Undecyl-2-butylpiperidiniumchlorid, 1-Undecyl-3-Methylpiperidiniumchlorid, 1-Undecyl-3-ethylpiperidiniumchlorid, 1-Undecyl-3-propylpiperidiniumchlorid, 1-Undecyl-3-butylpiperidiniumchlorid, 1-Undecyl-4-Methylpiperidiniumchlorid, 1-Undecyl-4-ethylpiperidiniumchlorid, 1-Undecyl-4-propylpiperidiniumchlorid, 1-Undecyl-4-butylpiperidiniumchlorid, N-Undecylpiperidiniumfluorid, 1-Undecyl-1-methylpiperidiniumfluorid, 1-Undecyl-1-ethylpiperidiniumfluorid, 1-Undecyl-1-propylpiperidiniumfluorid, 1-Undecyl-1-butylpiperidiniumfluorid, 1-Undecyl-2-Methylpiperidiniumfluorid, 1-Undecyl-2-ethylpiperidiniumfluorid, 1-Undecyl-2-propylpiperidiniumfluorid, 1-Undecyl-2-butylpiperidiniumfluorid, 1-Undecyl-3-Methylpiperidiniumfluorid, 1-Undecyl-3-ethylpiperidiniumfluorid, 1-Undecyl-3-propylpiperidiniumfluorid, 1-Undecyl-3-butylpiperidiniumfluorid, 1-Undecyl-4-Methylpiperidiniumfluorid, 1-Undecyl-4-ethylpiperidiniumfluorid, 1-Undecyl-4-propylpiperidiniumfluorid, 1-Undecyl-4-butylpiperidiniumfluorid, N-Undecylpiperidiniumtriflat, 1-Undecyl-1-methylpiperidiniumtriflat, 1-Undecyl-1-ethylpiperidiniumtriflat, 1-Undecyl-1-propylpiperidiniumtriflat, 1-Undecyl-1-butylpiperidiniumtriflat, 1-Undecyl-2-Methylpiperidiniumtriflat, 1-Undecyl-2-ethylpiperidiniumtriflat, 1-Undecyl-2-propylpiperidiniumtriflat, 1-Undecyl-2-butylpiperidiniumtriflat, 1-Undecyl-3-Methylpiperidiniumtriflat, 1-Undecyl-3-ethylpiperidiniumtriflat, 1-Undecyl-3-propylpiperidiniumtriflat, 1-Undecyl-3-butylpiperidiniumtriflat, 1-Undecyl-4-Methylpiperidiniumtriflat, 1-Undecyl-4-ethylpiperidiniumtriflat, 1-Undecyl-4-propylpiperidiniumtriflat, 1-Undecyl-4-butylpiperidiniumtriflat, N-Undecylpiperidiniummethansulfonat, 1-Undecyl-1-methylpiperidiniummethansulfonat, 1-Undecyl-1-ethylpiperidiniummethansulfonat, 1-Undecyl-1-propylpiperidiniummethansulfonat, 1-Undecyl-1-butylpiperidiniummethansulfonat, 1-Undecyl-2-Methylpiperidiniummethansulfonat, 1-Undecyl-2-ethylpiperidiniummethansulfonat, 1-Undecyl-2-propylpiperidiniummethansulfonat, 1-Undecyl-2-butylpiperidiniummethansulfonat, 1-Undecyl-3-Methylpiperidiniummethansulfonat, 1-Undecyl-3-ethylpiperidiniummethansulfonat, 1-Undecyl-3-propylpiperidiniummethansulfonat, 1-Undecyl-3-butylpiperidiniummethansulfonat, 1-Undecyl-4-Methylpiperidiniummethansulfonat, 1-Undecyl-4-ethylpiperidiniummethansulfonat, 1-Undecyl-4-propylpiperidiniummethansulfonat, 1-Undecyl-4-butylpiperidiniummethansulfonat, N-Undecylpiperidiniumacetat, 1-Undecyl-1-methylpiperidiniumacetat, 1-Undecyl-1-ethylpiperidiniumacetat, 1-Undecyl-1-propylpiperidiniumacetat, 1-Undecyl-1-butylpiperidiniumacetat, 1-Undecyl-2-Methylpiperidiniumacetat, 1-Undecyl-2-ethylpiperidiniumacetat, 1-Undecyl-2-propylpiperidiniumacetat, 1-Undecyl-2-butylpiperidiniumacetat, 1-Undecyl-3-Methylpiperidiniumacetat, 1-Undecyl-3-ethylpiperidiniumacetat, 1-Undecyl-3-propylpiperidiniumacetat, 1-Undecyl-3-butylpiperidiniumacetat, 1-Undecyl-4-Methylpiperidiniumacetat, 1-Undecyl-4-ethylpiperidiniumacetat, 1-Undecyl-4-propylpiperidiniumacetat, 1-Undecyl-4-butylpiperidiniumacetat, N-Undecylpiperidiniumcyanid, 1-Undecyl-1-methylpiperidiniumcyanid, 1-Undecyl-1-ethylpiperidiniumcyanid, 1-Undecyl-1-propylpiperidiniumcyanid, 1-Undecyl-1-butylpiperidiniumcyanid, 1-Undecyl-2-Methylpiperidiniumcyanid, 1-Undecyl-2-ethylpiperidiniumcyanid, 1-Undecyl-2-propylpiperidiniumcyanid, 1-Undecyl-2-butylpiperidiniumcyanid, 1-Undecyl-3-Methylpiperidiniumcyanid, 1-Undecyl-3-ethylpiperidiniumcyanid, 1-Undecyl-3-propylpiperidiniumcyanid, 1-Undecyl-3-butylpiperidiniumcyanid, 1-Undecyl-4-Methylpiperidiniumcyanid, 1-Undecyl-4-ethylpiperidiniumcyanid, 1-Undecyl-4-propylpiperidiniumcyanid, 1-Undecyl-4-butylpiperidiniumcyanid.

Man erkennt weiter, dass eine Verbindung entsprechend Formel (58) ausgewählt sein kann aus der Gruppe enthaltend N-Dodecylpiperidiniumchlorid, 1-Dodecyl-1-methylpiperidiniumchlorid, 1-Dodecyl-1-ethylpiperidiniumchlorid, 1-Dodecyl-1-propylpiperidiniumchlorid, 1-Dodecyl-1-butylpiperidiniumchlorid, 1-Dodecyl-2-Methylpiperidiniumchlorid, 1-Dodecyl-2-ethylpiperidiniumchlorid, 1-Dodecyl-2-propylpiperidiniumchlorid, 1-Dodecyl-2-butylpiperidiniumchlorid, 1-Dodecyl-3-Methylpiperidiniumchlorid, 1-Dodecyl-3-ethylpiperidiniumchlorid, 1-Dodecyl-3-propylpiperidiniumchlorid, 1-Dodecyl-3-butylpiperidiniumchlorid, 1-Dodecyl-4-Methylpiperidiniumchlorid, 1-Dodecyl-4-ethylpiperidiniumchlorid, 1-Dodecyl-4-propylpiperidiniumchlorid, 1-Dodecyl-4-butylpiperidiniumchlorid, N-Dodecylpiperidiniumfluorid, 1-Dodecyl-1-methylpiperidiniumfluorid, 1-Dodecyl-1-ethylpiperidiniumfluorid, 1-Dodecyl-1-propylpiperidiniumfluorid, 1-Dodecyl-1-butylpiperidiniumfluorid, 1-Dodecyl-2-Methylpiperidiniumfluorid, 1-Dodecyl-2-ethylpiperidiniumfluorid, 1-Dodecyl-2-propylpiperidiniumfluorid, 1-Dodecyl-2-butylpiperidiniumfluorid, 1-Dodecyl-3-Methylpiperidiniumfluorid, 1-Dodecyl-3-ethylpiperidiniumfluorid, 1-Dodecyl-3-propylpiperidiniumfluorid, 1-Dodecyl-3-butylpiperidiniumfluorid, 1-Dodecyl-4-Methylpiperidiniumfluorid, 1-Dodecyl-4-ethylpiperidiniumfluorid, 1-Dodecyl-4-propylpiperidiniumfluorid, 1-Dodecyl-4-butylpiperidiniumfluorid, N-Dodecylpiperidiniumtriflat, 1-Dodecyl-1-methylpiperidiniumtriflat, 1-Dodecyl-1-ethylpiperidiniumtriflat, 1-Dodecyl-1-propylpiperidiniumtriflat, 1-Dodecyl-1-butylpiperidiniumtriflat, 1-Dodecyl-2-Methylpiperidiniumtriflat, 1-Dodecyl-2-ethylpiperidiniumtriflat, 1-Dodecyl-2-propylpiperidiniumtriflat, 1-Dodecyl-2-butylpiperidiniumtriflat, 1-Dodecyl-3-Methylpiperidiniumtriflat, 1-Dodecyl-3-ethylpiperidiniumtriflat, 1-Dodecyl-3-propylpiperidiniumtriflat, 1-Dodecyl-3-butylpiperidiniumtriflat, 1-Dodecyl-4-Methylpiperidiniumtriflat, 1-Dodecyl-4-ethylpiperidiniumtriflat, 1-Dodecyl-4-propylpiperidiniumtriflat, 1-Dodecyl-4-butylpiperidiniumtriflat, N-Dodecylpiperidiniummethansulfonat, 1-Dodecyl-1-methylpiperidiniummethansulfonat, 1-Dodecyl-1-ethylpiperidiniummethansulfonat, 1-Dodecyl-1-propylpiperidiniummethansulfonat, 1-Dodecyl-1-butylpiperidiniummethansulfonat, 1-Dodecyl-2-Methylpiperidiniummethansulfonat, 1-Dodecyl-2-ethylpiperidiniummethansulfonat, 1-Dodecyl-2-propylpiperidiniummethansulfonat, 1-Dodecyl-2-butylpiperidiniummethansulfonat, 1-Dodecyl-3-Methylpiperidiniummethansulfonat, 1-Dodecyl-3-ethylpiperidiniummethansulfonat, 1-Dodecyl-3-propylpiperidiniummethansulfonat, 1-Dodecyl-3-butylpiperidiniummethansulfonat, 1-Dodecyl-4-Methylpiperidiniummethansulfonat, 1-Dodecyl-4-ethylpiperidiniummethansulfonat, 1-Dodecyl-4-propylpiperidiniummethansulfonat, 1-Dodecyl-4-butylpiperidiniummethansulfonat, N-Dodecylpiperidiniumacetat, 1-Dodecyl-1-methylpiperidiniumacetat, 1-Dodecyl-1-ethylpiperidiniumacetat, 1-Dodecyl-1-propylpiperidiniumacetat, 1-Dodecyl-1-butylpiperidiniumacetat, 1-Dodecyl-2-Methylpiperidiniumacetat, 1-Dodecyl-2-ethylpiperidiniumacetat, 1-Dodecyl-2-propylpiperidiniumacetat, 1-Dodecyl-2-butylpiperidiniumacetat, 1-Dodecyl-3-Methylpiperidiniumacetat, 1-Dodecyl-3-ethylpiperidiniumacetat, 1-Dodecyl-3-propylpiperidiniumacetat, 1-Dodecyl-3-butylpiperidiniumacetat, 1-Dodecyl-4-Methylpiperidiniumacetat, 1-Dodecyl-4-ethylpiperidiniumacetat, 1-Dodecyl-4-propylpiperidiniumacetat, 1-Dodecyl-4-butylpiperidiniumacetat, N-Dodecylpiperidiniumcyanid, 1-Dodecyl-1-methylpiperidiniumcyanid, 1-Dodecyl-1-ethylpiperidiniumcyanid, 1-Dodecyl-1-propylpiperidiniumcyanid, 1-Dodecyl-1-butylpiperidiniumcyanid, 1-Dodecyl-2-Methylpiperidiniumcyanid, 1-Dodecyl-2-ethylpiperidiniumcyanid, 1-Dodecyl-2-propylpiperidiniumcyanid, 1-Dodecyl-2-butylpiperidiniumcyanid, 1-Dodecyl-3-Methylpiperidiniumcyanid, 1-Dodecyl-3-ethylpiperidiniumcyanid, 1-Dodecyl-3-propylpiperidiniumcyanid, 1-Dodecyl-3-butylpiperidiniumcyanid, 1-Dodecyl-4-Methylpiperidiniumcyanid, 1-Dodecyl-4-ethylpiperidiniumcyanid, 1-Dodecyl-4-propylpiperidiniumcyanid, 1-Dodecyl-4-butylpiperidiniumcyanid.

Man erkennt insofern, dass von der Formel VI insbesondere Verbindungen entsprechend den folgenden Formeln (70), (71), (72), (73), (74), (75), (76), (77), (78), (79), (80) oder (81) 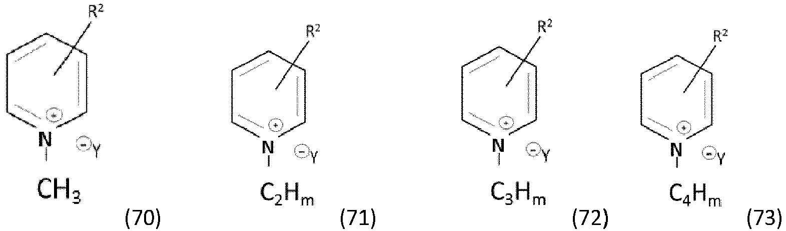 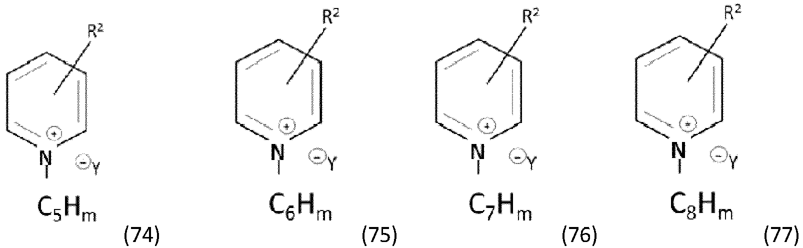 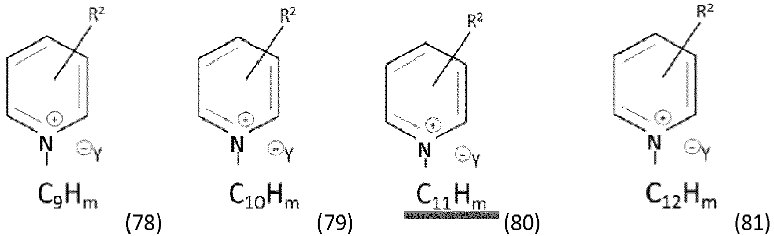 umfasst sind, wobei R² ein Wasserstoffatom oder ein Kohlenwasserstoffrest mit 1, 2, 3 oder 4 C-Atomen ist und Y ausgewählt ist aus der Gruppe enthaltend Halogenide, Cyanid, Alkylsulfonat, halogeniertes Alkylsulfonat, Acetat und wobei für Formel (71) m ausgewählt ist aus 3 oder 5, wobei für Formel (72) m ausgewählt ist aus 3, 5 oder 7, wobei für Formel (73) m ausgewählt ist aus 3, 5, 7, oder 9, wobei für Formel (74) m ausgewählt ist aus 3, 5, 7, 9 oder 11, wobei für Formel (75) m ausgewählt ist aus 3, 5, 7, 9, 11 oder 13, wobei für Formel (76) m ausgewählt ist aus 3, 5, 7, 9, 11, 13 oder 15, wobei für Formel (77) m ausgewählt ist aus 3, 5, 7, 9, 11, 13, 15 oder 17, wobei für Formel (78) m ausgewählt ist aus 3, 5, 7, 9, 11, 13, 15, 17 oder 19, wobei für Formel (79) m ausgewählt ist aus 3, 5, 7, 9, 11, 13, 15, 17, 19 oder 21, wobei für Formel (80) m ausgewählt ist aus 3, 5, 7, 9, 11, 13, 15, 17, 19, 21 oder 23, wobei für Formel (81) m ausgewählt ist aus 3, 5, 7, 9, 11, 13, 15, 17, 19, 21, 23 oder 25. Bevorzugt ist der Rest R¹ entweder gesättigt, einfach ungesättigt oder zweifach ungesättigt, so dass für Formel (71) m ausgewählt ist aus 3 oder 5, für Formel (72) m ausgewählt ist aus 3, 5 oder 7, für Formel (73) m ausgewählt ist aus 5, 7, oder 9, für Formel (74) m ausgewählt ist aus 7, 9 oder 11, für Formel (75) m ausgewählt ist aus 9, 11 oder 13, für Formel (76) m ausgewählt ist aus 11, 13 oder 15, für Formel (77) m ausgewählt ist aus 13, 15 oder 17, für Formel (78) m ausgewählt ist aus 15, 17 oder 19, für Formel (79) m ausgewählt ist aus 17, 19 oder 21, für Formel (80) m ausgewählt ist aus 19, 21 oder 23, und für Formel (81) m ausgewählt ist aus 21, 23 oder 25.

Besonders bevorzugt ist der Rest R¹ ein gesättigter Alkylrest, so dass die Formel VI insbesondere Verbindungen entsprechend Formel (70) sowie entsprechend den folgenden Formeln (82), (83), (84), (85), (86), (87), (88), (89), (90), (91) und (92) 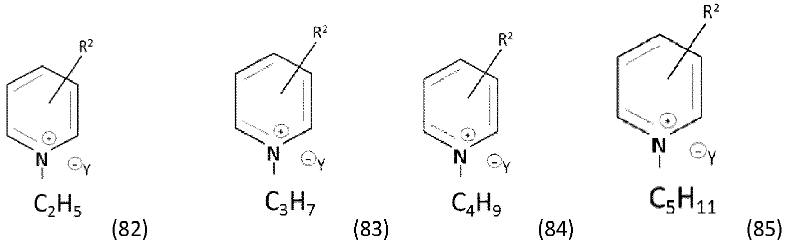 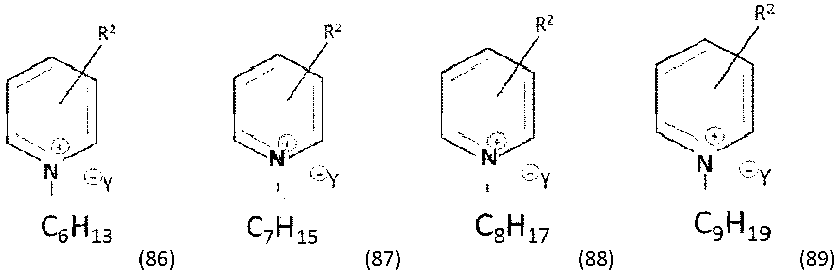 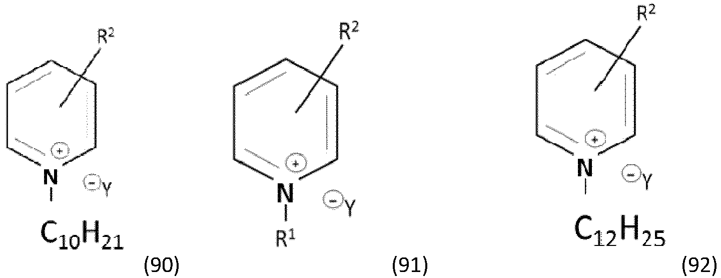 umfasst, wobei R² ein Wasserstoffatom oder ein Kohlenwasserstoffrest mit 1, 2, 3 oder 4 C-Atomen ist und Y ausgewählt ist aus der Gruppe enthaltend Halogenide, Cyanid, Alkylsulfonat, halogeniertes Alkylsulfonat, Acetat.

Man erkennt, dass eine Verbindung entsprechend Formel (70) beispielsweise ausgewählt sein kann aus der Gruppe enthaltend N-Methylpyridiniumchlorid, 1,2-Dimethylpyridiniumchlorid, 1-Methyl-2-ethylpyridiniumchlorid, 1-Methyl-2-propylpyridiniumchlorid, 1-Methyl-2-butylpyridiniumchlorid, 1,3-Dimethylpyridiniumchlorid, 1-Methyl-3-ethylpyridiniumchlorid, 1-Methyl-3-propylpyridiniumchlorid, 1-Methyl-3-butylpyridiniumchlorid, 1,4-Dimethylpyridiniumchlorid, 1-Methyl-4-ethylpyridiniumchlorid, 1-Methyl-4-propylpyridiniumchlorid, 1-Methyl-4-butylpyridiniumchlorid, N-Methylpyridiniumfluorid, 1,2-Dimethylpyridiniumfluorid, 1-Methyl-2-ethylpyridiniumfluorid, 1-Methyl-2-propylpyridiniumfluorid, 1-Methyl-2-butylpyridiniumfluorid, 1,3-Dimethylpyridiniumfluorid, 1-Methyl-3-ethylpyridiniumfluorid, 1-Methyl-3-propylpyridiniumfluorid, 1-Methyl-3-butylpyridiniumfluorid, 1,4-Dimethylpyridiniumfluorid, 1-Methyl-4-ethylpyridiniumfluorid, 1-Methyl-4-propylpyridiniumfluorid, 1-Methyl-4-butylpyridiniumfluorid, N-Methylpyridiniumtriflat, 1,2-Dimethylpyridiniumtriflat, 1-Methyl-2-ethylpyridiniumtriflat, 1-Methyl-2-propylpyridiniumtriflat, 1-Methyl-2-butylpyridiniumtriflat, 1,3-Dimethylpyridiniumtriflat, 1-Methyl-3-ethylpyridiniumtriflat, 1-Methyl-3-propylpyridiniumtriflat, 1-Methyl-3-butylpyridiniumtriflat, 1,4-Dimethylpyridiniumtriflat, 1-Methyl-4-ethylpyridiniumtriflat, 1-Methyl-4-propylpyridiniumtriflat, 1-Methyl-4-butylpyridiniumtriflat, N-Methylpyridiniummethansulfonat, 1,2-Dimethylpyridiniummethansulfonat, 1-Methyl-2-ethylpyridiniummethansulfonat, 1-Methyl-2-propylpyridiniummethansulfonat, 1-Methyl-2-butylpyridiniummethansulfonat, 1,3-Dimethylpyridiniummethansulfonat, 1-Methyl-3-ethylpyridiniummethansulfonat, 1-Methyl-3-propylpyridiniummethansulfonat, 1-Methyl-3-butylpyridiniummethansulfonat, 1,4-Dimethylpyridiniummethansulfonat, 1-Methyl-4-ethylpyridiniummethansulfonat, 1-Methyl-4-propylpyridiniummethansulfonat, 1-Methyl-4-butylpyridiniummethansulfonat, N-Methylpyridiniumacetat, 1,2-Dimethylpyridiniumacetat, 1-Methyl-2-ethylpyridiniumacetat, 1-Methyl-2-propylpyridiniumacetat, 1-Methyl-2-butylpyridiniumacetat, 1,3-Dimethylpyridiniumacetat, 1-Methyl-3-ethylpyridiniumacetat, 1-Methyl-3-propylpyridiniumacetat, 1-Methyl-3-butylpyridiniumacetat, 1,4-Dimethylpyridiniumacetat, 1-Methyl-4-ethylpyridiniumacetat, 1-Methyl-4-propylpyridiniumacetat, 1-Methyl-4-butylpyridiniumacetat, N-Methylpyridiniumcyanid, 1,2-Dimethylpyridiniumcyanid, 1-Methyl-2-ethylpyridiniumcyanid, 1-Methyl-2-propylpyridiniumcyanid, 1-Methyl-2-butylpyridiniumcyanid, 1,3-Dimethylpyridiniumcyanid, 1-Methyl-3-ethylpyridiniumcyanid, 1-Methyl-3-propylpyridiniumcyanid, 1-Methyl-3-butylpyridiniumcyanid, 1,4-Dimethylpyridiniumcyanid, 1-Methyl-4-ethylpyridiniumcyanid, 1-Methyl-4-propylpyridiniumcyanid, 1-Methyl-4-butylpyridiniumcyanid.

Man erkennt, dass eine Verbindung entsprechend Formel (71) beispielsweise ausgewählt sein kann aus der Gruppe enthaltend N-Ethylpyridiniumchlorid, 1-Ethyl-2-methylpyridiniumchlorid, 1,2-Diethylpyridiniumchlorid, 1-Ethyl-2-propylpyridiniumchlorid, 1-Ethyl-2-butylpyridiniumchlorid, 1-Ethyl-3-methylpyridiniumchlorid, 1,3-Diethylpyridiniumchlorid, 1-Ethyl-3-propylpyridiniumchlorid, 1-Ethyl-3-butylpyridiniumchlorid, 1-Ethyl-4-methylpyridiniumchlorid, 1,4-Diethylpyridiniumchlorid, 1-Ethyl-4-propylpyridiniumchlorid, 1-Ethyl-4-butylpyridiniumchlorid, N-Ethylpyridiniumfluorid, 1-Ethyl-2-methylpyridiniumfluorid, 1,2-Diethylpyridiniumfluorid, 1-Ethyl-2-propylpyridiniumfluorid, 1-Ethyl-2-butylpyridiniumfluorid, 1-Ethyl-3-methylpyridiniumfluorid, 1,3-Diethylpyridiniumfluorid, 1-Ethyl-3-propylpyridiniumfluorid, 1-Ethyl-3-butylpyridiniumfluorid, 1-Ethyl-4-methylpyridiniumfluorid, 1,4-Diethylpyridiniumfluorid, 1-Ethyl-4-propylpyridiniumfluorid, 1-Ethyl-4-butylpyridiniumfluorid, N-Ethylpyridiniumtriflat, 1-Ethyl-2-methylpyridiniumtriflat, 1,2-Diethylpyridiniumtriflat, 1-Ethyl-2-propylpyridiniumtriflat, 1-Ethyl-2-butylpyridiniumtriflat, 1-Ethyl-3-methylpyridiniumtriflat, 1,3-Diethylpyridiniumtriflat, 1-Ethyl-3-propylpyridiniumtriflat, 1-Ethyl-3-butylpyridiniumtriflat, 1-Ethyl-4-methylpyridiniumtriflat, 1,4-Diethylpyridiniumtriflat, 1-Ethyl-4-propylpyridiniumtriflat, 1-Ethyl-4-butylpyridiniumtriflat, N-Ethylpyridiniummethansulfonat, 1-Ethyl-2-methylpyridiniummethansulfonat, 1,2-Diethylpyridiniummethansulfonat, 1-Ethyl-2-propylpyridiniummethansulfonat, 1-Ethyl-2-butylpyridiniummethansulfonat, 1-Ethyl-3-methylpyridiniummethansulfonat, 1,3-Diethylpyridiniummethansulfonat, 1-Ethyl-3-propylpyridiniummethansulfonat, 1-Ethyl-3-butylpyridiniummethansulfonat, 1-Ethyl-4-methylpyridiniummethansulfonat, 1,4-Diethylpyridiniummethansulfonat, 1-Ethyl-4-propylpyridiniummethansulfonat, 1-Ethyl-4-butylpyridiniummethansulfonat, N-Ethylpyridiniumacetat, 1-Ethyl-2-methylpyridiniumacetat, 1,2-Diethylpyridiniumacetat, 1-Ethyl-2-propylpyridiniumacetat, 1-Ethyl-2-butylpyridiniumacetat, 1-Ethyl-3-methylpyridiniumacetat, 1,3-Diethylpyridiniumacetat, 1-Ethyl-3-propylpyridiniumacetat, 1-Ethyl-3-butylpyridiniumacetat, 1-Ethyl-4-methylpyridiniumacetat, 1,4-Diethylpyridiniumacetat, 1-Ethyl-4-propylpyridiniumacetat, 1-Ethyl-4-butylpyridiniumacetat, N-Ethylpyridiniumcyanid, 1-Ethyl-2-methylpyridiniumcyanid, 1,2-Diethylpyridiniumcyanid, 1-Ethyl-2-propylpyridiniumcyanid, 1-Ethyl-2-butylpyridiniumcyanid, 1-Ethyl-3-methylpyridiniumcyanid, 1,3-Diethylpyridiniumcyanid, 1-Ethyl-3-propylpyridiniumcyanid, 1-Ethyl-3-butylpyridiniumcyanid, 1-Ethyl-4-methylpyridiniumcyanid, 1,4-Diethylpyridiniumcyanid, 1-Ethyl-4-propylpyridiniumcyanid, 1-Ethyl-4-butylpyridiniumcyanid.

Man erkennt, dass eine Verbindung entsprechend Formel (72) beispielsweise ausgewählt sein kann aus der Gruppe enthaltend N-Propylpyridiniumchlorid, 1-Propyl-2-Methylpyridiniumchlorid, 1-Propyl-2-ethylpyridiniumchlorid, 1,2-Dipropylpyridiniumchlorid, 1-Propyl-2-butylpyridiniumchlorid, 1-Propyl-3-Methylpyridiniumchlorid, 1-Propyl-3-ethylpyridiniumchlorid, 1,3-Dipropylpyridiniumchlorid, 1-Propyl-3-butylpyridiniumchlorid, 1-Propyl-4-Methylpyridiniumchlorid, 1-Propyl-4-ethylpyridiniumchlorid, 1,4-Dipropylpyridiniumchlorid, 1-Propyl-4-butylpyridiniumchlorid, N-Propylpyridiniumfluorid, 1-Propyl-2-Methylpyridiniumfluorid, 1-Propyl-2-ethylpyridiniumfluorid, 1,2-Dipropylpyridiniumfluorid, 1-Propyl-2-butylpyridiniumfluorid, 1-Propyl-3-Methylpyridiniumfluorid, 1-Propyl-3-ethylpyridiniumfluorid, 1,3-Dipropylpyridiniumfluorid, 1-Propyl-3-butylpyridiniumfluorid, 1-Propyl-4-Methylpyridiniumfluorid, 1-Propyl-4-ethylpyridiniumfluorid, 1,4-Dipropylpyridiniumfluorid, 1-Propyl-4-butylpyridiniumfluorid, N-Propylpyridiniumtriflat, 1-Propyl-2-Methylpyridiniumtriflat, 1-Propyl-2-ethylpyridiniumtriflat, 1,2-Dipropylpyridiniumtriflat, 1-Propyl-2-butylpyridiniumtriflat, 1-Propyl-3-Methylpyridiniumtriflat, 1-Propyl-3-ethylpyridiniumtriflat, 1,3-Dipropylpyridiniumtriflat, 1-Propyl-3-butylpyridiniumtriflat, 1-Propyl-4-Methylpyridiniumtriflat, 1-Propyl-4-ethylpyridiniumtriflat, 1,4-Dipropylpyridiniumtriflat, 1-Propyl-4-butylpyridiniumtriflat, N-Propylpyridiniummethansulfonat, 1-Propyl-2-Methylpyridiniummethansulfonat, 1-Propyl-2-ethylpyridiniummethansulfonat, 1,2-Dipropylpyridiniummethansulfonat, 1-Propyl-2-butylpyridiniummethansulfonat, 1-Propyl-3-Methylpyridiniummethansulfonat, 1-Propyl-3-ethylpyridiniummethansulfonat, 1,3-Dipropylpyridiniummethansulfonat, 1-Propyl-3-butylpyridiniummethansulfonat, 1-Propyl-4-Methylpyridiniummethansulfonat, 1-Propyl-4-ethylpyridiniummethansulfonat, 1,4-Dipropylpyridiniummethansulfonat, 1-Propyl-4-butylpyridiniummethansulfonat, N-Propylpyridiniumacetat, 1-Propyl-2-Methylpyridiniumacetat, 1-Propyl-2-ethylpyridiniumacetat, 1,2-Dipropylpyridiniumacetat, 1-Propyl-2-butylpyridiniumacetat, 1-Propyl-3-Methylpyridiniumacetat, 1-Propyl-3-ethylpyridiniumacetat, 1,3-Dipropylpyridiniumacetat, 1-Propyl-3-butylpyridiniumacetat, 1-Propyl-4-Methylpyridiniumacetat, 1-Propyl-4-ethylpyridiniumacetat, 1,4-Dipropylpyridiniumacetat, 1-Propyl-4-butylpyridiniumacetat, N-Propylpyridiniumcyanid, 1-Propyl-2-Methylpyridiniumcyanid, 1-Propyl-2-ethylpyridiniumcyanid, 1,2-Dipropylpyridiniumcyanid, 1-Propyl-2-butylpyridiniumcyanid, 1-Propyl-3-Methylpyridiniumcyanid, 1-Propyl-3-ethylpyridiniumcyanid, 1,3-Dipropylpyridiniumcyanid, 1-Propyl-3-butylpyridiniumcyanid, 1-Propyl-4-Methylpyridiniumcyanid, 1-Propyl-4-ethylpyridiniumcyanid, 1,4-Dipropylpyridiniumcyanid, 1-Propyl-4-butylpyridiniumcyanid.

Man erkennt, dass eine Verbindung entsprechend Formel (73) beispielsweise ausgewählt sein kann aus der Gruppe enthaltend N-Butylpyridiniumchlorid, 1-Butyl-2-Methylpyridiniumchlorid, 1-Butyl-2-ethylpyridiniumchlorid, 1-Butyl-2-propylpyridiniumchlorid, 1,2-Dibutylpyridiniumchlorid, 1-Butyl-3-Methylpyridiniumchlorid, 1-Butyl-3-ethylpyridiniumchlorid, 1-Butyl-3-propylpyridiniumchlorid, 1,3-Dibutylpyridiniumchlorid, 1-Butyl-4-Methylpyridiniumchlorid, 1-Butyl-4-ethylpyridiniumchlorid, 1-Butyl-4-propylpyridiniumchlorid, 1,4-Dibutylpyridiniumchlorid, N-Butylpyridiniumfluorid, 1-Butyl-2-Methylpyridiniumfluorid, 1-Butyl-2-ethylpyridiniumfluorid, 1-Butyl-2-propylpyridiniumfluorid, 1,2-Dibutylpyridiniumfluorid, 1-Butyl-3-Methylpyridiniumfluorid, 1-Butyl-3-ethylpyridiniumfluorid, 1-Butyl-3-propylpyridiniumfluorid, 1,3-Dibutylpyridiniumfluorid, 1-Butyl-4-Methylpyridiniumfluorid, 1-Butyl-4-ethylpyridiniumfluorid, 1-Butyl-4-propylpyridiniumfluorid, 1,4-Dibutylpyridiniumfluorid, N-Butylpyridiniumtriflat, 1-Butyl-2-Methylpyridiniumtriflat, 1-Butyl-2-ethylpyridiniumtriflat, 1-Butyl-2-propylpyridiniumtriflat, 1,2-Dibutylpyridiniumtriflat, 1-Butyl-3-Methylpyridiniumtriflat, 1-Butyl-3-ethylpyridiniumtriflat, 1-Butyl-3-propylpyridiniumtriflat, 1,3-Dibutylpyridiniumtriflat, 1-Butyl-4-Methylpyridiniumtriflat, 1-Butyl-4-ethylpyridiniumtriflat, 1-Butyl-4-propylpyridiniumtriflat, 1,4-Dibutylpyridiniumtriflat, N-Butylpyridiniummethansulfonat, 1-Butyl-2-Methylpyridiniummethansulfonat, 1-Butyl-2-ethylpyridiniummethansulfonat, 1-Butyl-2-propylpyridiniummethansulfonat, 1,2-Dibutylpyridiniummethansulfonat, 1-Butyl-3-Methylpyridiniummethansulfonat, 1-Butyl-3-ethylpyridiniummethansulfonat, 1-Butyl-3-propylpyridiniummethansulfonat, 1,3-Dibutylpyridiniummethansulfonat, 1-Butyl-4-Methylpyridiniummethansulfonat, 1-Butyl-4-ethylpyridiniummethansulfonat, 1-Butyl-4-propylpyridiniummethansulfonat, 1,4-Dibutylpyridiniummethansulfonat, N-Butylpyridiniumacetat, 1-Butyl-2-Methylpyridiniumacetat, 1-Butyl-2-ethylpyridiniumacetat, 1-Butyl-2-propylpyridiniumacetat, 1,2-Dibutylpyridiniumacetat, 1-Butyl-3-Methylpyridiniumacetat, 1-Butyl-3-ethylpyridiniumacetat, 1-Butyl-3-propylpyridiniumacetat, 1,3-Dibutylpyridiniumacetat, 1-Butyl-4-Methylpyridiniumacetat, 1-Butyl-4-ethylpyridiniumacetat, 1-Butyl-4-propylpyridiniumacetat, 1,4-Dibutylpyridiniumacetat, N-Butylpyridiniumcyanid, 1-Butyl-2-Methylpyridiniumcyanid, 1-Butyl-2-ethylpyridiniumcyanid, 1-Butyl-2-propylpyridiniumcyanid, 1,2-Dibutylpyridiniumcyanid, 1-Butyl-3-Methylpyridiniumcyanid, 1-Butyl-3-ethylpyridiniumcyanid, 1-Butyl-3-propylpyridiniumcyanid, 1,3-Dibutylpyridiniumcyanid, 1-Butyl-4-Methylpyridiniumcyanid, 1-Butyl-4-ethylpyridiniumcyanid, 1-Butyl-4-propylpyridiniumcyanid, 1,4-Dibutylpyridiniumcyanid.

Man erkennt, dass eine Verbindung entsprechend Formel (74) beispielsweise ausgewählt sein kann aus der Gruppe enthaltend N-Pentylpyridiniumchlorid, 1-Pentyl-2-Methylpyridiniumchlorid, 1-Pentyl-2-ethylpyridiniumchlorid, 1-Pentyl-2-propylpyridiniumchlorid, 1-Pentyl-2-butylpyridiniumchlorid, 1-Pentyl-3-Methylpyridiniumchlorid, 1-Pentyl-3-ethylpyridiniumchlorid, 1-Pentyl-3-propylpyridiniumchlorid, 1-Pentyl-3-butylpyridiniumchlorid, 1-Pentyl-4-Methylpyridiniumchlorid, 1-Pentyl-4-ethylpyridiniumchlorid, 1-Pentyl-4-propylpyridiniumchlorid, 1-Pentyl-4-butylpyridiniumchlorid, N-Pentylpyridiniumfluorid, 1-Pentyl-2-Methylpyridiniumfluorid, 1-Pentyl-2-ethylpyridiniumfluorid, 1-Pentyl-2-propylpyridiniumfluorid, 1-Pentyl-2-butylpyridiniumfluorid, 1-Pentyl-3-Methylpyridiniumfluorid, 1-Pentyl-3-ethylpyridiniumfluorid, 1-Pentyl-3-propylpyridiniumfluorid, 1-Pentyl-3-butylpyridiniumfluorid, 1-Pentyl-4-Methylpyridiniumfluorid, 1-Pentyl-4-ethylpyridiniumfluorid, 1-Pentyl-4-propylpyridiniumfluorid, 1-Pentyl-4-butylpyridiniumfluorid, N-Pentylpyridiniumtriflat, 1-Pentyl-2-Methylpyridiniumtriflat, 1-Pentyl-2-ethylpyridiniumtriflat, 1-Pentyl-2-propylpyridiniumtriflat, 1-Pentyl-2-butylpyridiniumtriflat, 1-Pentyl-3-Methylpyridiniumtriflat, 1-Pentyl-3-ethylpyridiniumtriflat, 1-Pentyl-3-propylpyridiniumtriflat, 1-Pentyl-3-butylpyridiniumtriflat, 1-Pentyl-4-Methylpyridiniumtriflat, 1-Pentyl-4-ethylpyridiniumtriflat, 1-Pentyl-4-propylpyridiniumtriflat, 1-Pentyl-4-butylpyridiniumtriflat, N-Pentylpyridiniummethansulfonat, 1-Pentyl-2-Methylpyridiniummethansulfonat, 1-Pentyl-2-ethylpyridiniummethansulfonat, 1-Pentyl-2-propylpyridiniummethansulfonat, 1-Pentyl-2-butylpyridiniummethansulfonat, 1-Pentyl-3-Methylpyridiniummethansulfonat, 1-Pentyl-3-ethylpyridiniummethansulfonat, 1-Pentyl-3-propylpyridiniummethansulfonat, 1-Pentyl-3-butylpyridiniummethansulfonat, 1-Pentyl-4-Methylpyridiniummethansulfonat, 1-Pentyl-4-ethylpyridiniummethansulfonat, 1-Pentyl-4-propylpyridiniummethansulfonat, 1-Pentyl-4-butylpyridiniummethansulfonat, N-Pentylpyridiniumacetat, 1-Pentyl-2-Methylpyridiniumacetat, 1-Pentyl-2-ethylpyridiniumacetat, 1-Pentyl-2-propylpyridiniumacetat, 1-Pentyl-2-butylpyridiniumacetat, 1-Pentyl-3-Methylpyridiniumacetat, 1-Pentyl-3-ethylpyridiniumacetat, 1-Pentyl-3-propylpyridiniumacetat, 1-Pentyl-3-butylpyridiniumacetat, 1-Pentyl-4-Methylpyridiniumacetat, 1-Pentyl-4-ethylpyridiniumacetat, 1-Pentyl-4-propylpyridiniumacetat, 1-Pentyl-4-butylpyridiniumacetat, N-Pentylpyridiniumcyanid, 1-Pentyl-2-Methylpyridiniumcyanid, 1-Pentyl-2-ethylpyridiniumcyanid, 1-Pentyl-2-propylpyridiniumcyanid, 1-Pentyl-2-butylpyridiniumcyanid, 1-Pentyl-3-Methylpyridiniumcyanid, 1-Pentyl-3-ethylpyridiniumcyanid, 1-Pentyl-3-propylpyridiniumcyanid, 1-Pentyl-3-butylpyridiniumcyanid, 1-Pentyl-4-Methylpyridiniumcyanid, 1-Pentyl-4-ethylpyridiniumcyanid, 1-Pentyl-4-propylpyridiniumcyanid, 1-Pentyl-4-butylpyridiniumcyanid.

Man erkennt weiter, dass eine Verbindung entsprechend Formel (75) ausgewählt sein kann aus der Gruppe enthaltend N-Hexylpyridiniumchlorid, 1-Hexyl-2-Methylpyridiniumchlorid, 1-Hexyl-2-ethylpyridiniumchlorid, 1-Hexyl-2-propylpyridiniumchlorid, 1-Hexyl-2-butylpyridiniumchlorid, 1-Hexyl-3-Methylpyridiniumchlorid, 1-Hexyl-3-ethylpyridiniumchlorid, 1-Hexyl-3-propylpyridiniumchlorid, 1-Hexyl-3-butylpyridiniumchlorid, 1-Hexyl-4-Methylpyridiniumchlorid, 1-Hexyl-4-ethylpyridiniumchlorid, 1-Hexyl-4-propylpyridiniumchlorid, 1-Hexyl-4-butylpyridiniumchlorid, N-Hexylpyridiniumfluorid, 1-Hexyl-2-Methylpyridiniumfluorid, 1-Hexyl-2-ethylpyridiniumfluorid, 1-Hexyl-2-propylpyridiniumfluorid, 1-Hexyl-2-butylpyridiniumfluorid, 1-Hexyl-3-Methylpyridiniumfluorid, 1-Hexyl-3-ethylpyridiniumfluorid, 1-Hexyl-3-propylpyridiniumfluorid, 1-Hexyl-3-butylpyridiniumfluorid, 1-Hexyl-4-Methylpyridiniumfluorid, 1-Hexyl-4-ethylpyridiniumfluorid, 1-Hexyl-4-propylpyridiniumfluorid, 1-Hexyl-4-butylpyridiniumfluorid, N-Hexylpyridiniumtriflat, 1-Hexyl-2-Methylpyridiniumtriflat, 1-Hexyl-2-ethylpyridiniumtriflat, 1-Hexyl-2-propylpyridiniumtriflat, 1-Hexyl-2-butylpyridiniumtriflat, 1-Hexyl-3-Methylpyridiniumtriflat, 1-Hexyl-3-ethylpyridiniumtriflat, 1-Hexyl-3-propylpyridiniumtriflat, 1-Hexyl-3-butylpyridiniumtriflat, 1-Hexyl-4-Methylpyridiniumtriflat, 1-Hexyl-4-ethylpyridiniumtriflat, 1-Hexyl-4-propylpyridiniumtriflat, 1-Hexyl-4-butylpyridiniumtriflat, N-Hexylpyridiniummethansulfonat, 1-Hexyl-2-Methylpyridiniummethansulfonat, 1-Hexyl-2-ethylpyridiniummethansulfonat, 1-Hexyl-2-propylpyridiniummethansulfonat, 1-Hexyl-2-butylpyridiniummethansulfonat, 1-Hexyl-3-Methylpyridiniummethansulfonat, 1-Hexyl-3-ethylpyridiniummethansulfonat, 1-Hexyl-3-propylpyridiniummethansulfonat, 1-Hexyl-3-butylpyridiniummethansulfonat, 1-Hexyl-4-Methylpyridiniummethansulfonat, 1-Hexyl-4-ethylpyridiniummethansulfonat, 1-Hexyl-4-propylpyridiniummethansulfonat, 1-Hexyl-4-butylpyridiniummethansulfonat, N-Hexylpyridiniumacetat, 1-Hexyl-2-Methylpyridiniumacetat, 1-Hexyl-2-ethylpyridiniumacetat, 1-Hexyl-2-propylpyridiniumacetat, 1-Hexyl-2-butylpyridiniumacetat, 1-Hexyl-3-Methylpyridiniumacetat, 1-Hexyl-3-ethylpyridiniumacetat, 1-Hexyl-3-propylpyridiniumacetat, 1-Hexyl-3-butylpyridiniumacetat, 1-Hexyl-4-Methylpyridiniumacetat, 1-Hexyl-4-ethylpyridiniumacetat, 1-Hexyl-4-propylpyridiniumacetat, 1-Hexyl-4-butylpyridiniumacetat, N-Hexylpyridiniumcyanid, 1-Hexyl-2-Methylpyridiniumcyanid, 1-Hexyl-2-ethylpyridiniumcyanid, 1-Hexyl-2-propylpyridiniumcyanid, 1-Hexyl-2-butylpyridiniumcyanid, 1-Hexyl-3-Methylpyridiniumcyanid, 1-Hexyl-3-ethylpyridiniumcyanid, 1-Hexyl-3-propylpyridiniumcyanid, 1-Hexyl-3-butylpyridiniumcyanid, 1-Hexyl-4-Methylpyridiniumcyanid, 1-Hexyl-4-ethylpyridiniumcyanid, 1-Hexyl-4-propylpyridiniumcyanid, 1-Hexyl-4-butylpyridiniumcyanid.

Man erkennt weiter, dass eine Verbindung entsprechend Formel (76) ausgewählt sein kann aus der Gruppe enthaltend N-Heptylpyridiniumchlorid, 1-Heptyl-2-Methylpyridiniumchlorid, 1-Heptyl-2-ethylpyridiniumchlorid, 1-Heptyl-2-propylpyridiniumchlorid, 1-Heptyl-2-butylpyridiniumchlorid, 1-Heptyl-3-Methylpyridiniumchlorid, 1-Heptyl-3-ethylpyridiniumchlorid, 1-Heptyl-3-propylpyridiniumchlorid, 1-Heptyl-3-butylpyridiniumchlorid, 1-Heptyl-4-Methylpyridiniumchlorid, 1-Heptyl-4-ethylpyridiniumchlorid, 1-Heptyl-4-propylpyridiniumchlorid, 1-Heptyl-4-butylpyridiniumchlorid, N-Heptylpyridiniumfluorid, 1-Heptyl-2-Methylpyridiniumfluorid, 1-Heptyl-2-ethylpyridiniumfluorid, 1-Heptyl-2-propylpyridiniumfluorid, 1-Heptyl-2-butylpyridiniumfluorid, 1-Heptyl-3-Methylpyridiniumfluorid, 1-Heptyl-3-ethylpyridiniumfluorid, 1-Heptyl-3-propylpyridiniumfluorid, 1-Heptyl-3-butylpyridiniumfluorid, 1-Heptyl-4-Methylpyridiniumfluorid, 1-Heptyl-4-ethylpyridiniumfluorid, 1-Heptyl-4-propylpyridiniumfluorid, 1-Heptyl-4-butylpyridiniumfluorid, N-Heptylpyridiniumtriflat, 1-Heptyl-2-Methylpyridiniumtriflat, 1-Heptyl-2-ethylpyridiniumtriflat, 1-Heptyl-2-propylpyridiniumtriflat, 1-Heptyl-2-butylpyridiniumtriflat, 1-Heptyl-3-Methylpyridiniumtriflat, 1-Heptyl-3-ethylpyridiniumtriflat, 1-Heptyl-3-propylpyridiniumtriflat, 1-Heptyl-3-butylpyridiniumtriflat, 1-Heptyl-4-Methylpyridiniumtriflat, 1-Heptyl-4-ethylpyridiniumtriflat, 1-Heptyl-4-propylpyridiniumtriflat, 1-Heptyl-4-butylpyridiniumtriflat, N-Heptylpyridiniummethansulfonat, 1-Heptyl-2-Methylpyridiniummethansulfonat, 1-Heptyl-2-ethylpyridiniummethansulfonat, 1-Heptyl-2-propylpyridiniummethansulfonat, 1-Heptyl-2-butylpyridiniummethansulfonat, 1-Heptyl-3-Methylpyridiniummethansulfonat, 1-Heptyl-3-ethylpyridiniummethansulfonat, 1-Heptyl-3-propylpyridiniummethansulfonat, 1-Heptyl-3-butylpyridiniummethansulfonat, 1-Heptyl-4-Methylpyridiniummethansulfonat, 1-Heptyl-4-ethylpyridiniummethansulfonat, 1-Heptyl-4-propylpyridiniummethansulfonat, 1-Heptyl-4-butylpyridiniummethansulfonat, N-Heptylpyridiniumacetat, 1-Heptyl-2-Methylpyridiniumacetat, 1-Heptyl-2-ethylpyridiniumacetat, 1-Heptyl-2-propylpyridiniumacetat, 1-Heptyl-2-butylpyridiniumacetat, 1-Heptyl-3-Methylpyridiniumacetat, 1-Heptyl-3-ethylpyridiniumacetat, 1-Heptyl-3-propylpyridiniumacetat, 1-Heptyl-3-butylpyridiniumacetat, 1-Heptyl-4-Methylpyridiniumacetat, 1-Heptyl-4-ethylpyridiniumacetat, 1-Heptyl-4-propylpyridiniumacetat, 1-Heptyl-4-butylpyridiniumacetat, N-Heptylpyridiniumcyanid, 1-Heptyl-2-Methylpyridiniumcyanid, 1-Heptyl-2-ethylpyridiniumcyanid, 1-Heptyl-2-propylpyridiniumcyanid, 1-Heptyl-2-butylpyridiniumcyanid, 1-Heptyl-3-Methylpyridiniumcyanid, 1-Heptyl-3-ethylpyridiniumcyanid, 1-Heptyl-3-propylpyridiniumcyanid, 1-Heptyl-3-butylpyridiniumcyanid, 1-Heptyl-4-Methylpyridiniumcyanid, 1-Heptyl-4-ethylpyridiniumcyanid, 1-Heptyl-4-propylpyridiniumcyanid, 1-Heptyl-4-butylpyridiniumcyanid.

Man erkennt weiter, dass eine Verbindung entsprechend Formel (77) ausgewählt sein kann aus der Gruppe enthaltend N-Octylpyridiniumchlorid, 1-Octyl-2-Methylpyridiniumchlorid, 1-Octyl-2-ethylpyridiniumchlorid, 1-Octyl-2-propylpyridiniumchlorid, 1-Octyl-2-butylpyridiniumchlorid, 1-Octyl-3-Methylpyridiniumchlorid, 1-Octyl-3-ethylpyridiniumchlorid, 1-Octyl-3-propylpyridiniumchlorid, 1-Octyl-3-butylpyridiniumchlorid, 1-Octyl-4-Methylpyridiniumchlorid, 1-Octyl-4-ethylpyridiniumchlorid, 1-Octyl-4-propylpyridiniumchlorid, 1-Octyl-4-butylpyridiniumchlorid, N-Octylpyridiniumfluorid, 1-Octyl-2-Methylpyridiniumfluorid, 1-Octyl-2-ethylpyridiniumfluorid, 1-Octyl-2-propylpyridiniumfluorid, 1-Octyl-2-butylpyridiniumfluorid, 1-Octyl-3-Methylpyridiniumfluorid, 1-Octyl-3-ethylpyridiniumfluorid, 1-Octyl-3-propylpyridiniumfluorid, 1-Octyl-3-butylpyridiniumfluorid, 1-Octyl-4-Methylpyridiniumfluorid, 1-Octyl-4-ethylpyridiniumfluorid, 1-Octyl-4-propylpyridiniumfluorid, 1-Octyl-4-butylpyridiniumfluorid, N-Octylpyridiniumtriflat, 1-Octyl-2-Methylpyridiniumtriflat, 1-Octyl-2-ethylpyridiniumtriflat, 1-Octyl-2-propylpyridiniumtriflat, 1-Octyl-2-butylpyridiniumtriflat, 1-Octyl-3-Methylpyridiniumtriflat, 1-Octyl-3-ethylpyridiniumtriflat, 1-Octyl-3-propylpyridiniumtriflat, 1-Octyl-3-butylpyridiniumtriflat, 1-Octyl-4-Methylpyridiniumtriflat, 1-Octyl-4-ethylpyridiniumtriflat, 1-Octyl-4-propylpyridiniumtriflat, 1-Octyl-4-butylpyridiniumtriflat, N-Octylpyridiniummethansulfonat, 1-Octyl-2-Methylpyridiniummethansulfonat, 1-Octyl-2-ethylpyridiniummethansulfonat, 1-Octyl-2-propylpyridiniummethansulfonat, 1-Octyl-2-butylpyridiniummethansulfonat, 1-Octyl-3-Methylpyridiniummethansulfonat, 1-Octyl-3-ethylpyridiniummethansulfonat, 1-Octyl-3-propylpyridiniummethansulfonat, 1-Octyl-3-butylpyridiniummethansulfonat, 1-Octyl-4-Methylpyridiniummethansulfonat, 1-Octyl-4-ethylpyridiniummethansulfonat, 1-Octyl-4-propylpyridiniummethansulfonat, 1-Octyl-4-butylpyridiniummethansulfonat, N-Octylpyridiniumacetat, 1-Octyl-2-Methylpyridiniumacetat, 1-Octyl-2-ethylpyridiniumacetat, 1-Octyl-2-propylpyridiniumacetat, 1-Octyl-2-butylpyridiniumacetat, 1-Octyl-3-Methylpyridiniumacetat, 1-Octyl-3-ethylpyridiniumacetat, 1-Octyl-3-propylpyridiniumacetat, 1-Octyl-3-butylpyridiniumacetat, 1-Octyl-4-Methylpyridiniumacetat, 1-Octyl-4-ethylpyridiniumacetat, 1-Octyl-4-propylpyridiniumacetat, 1-Octyl-4-butylpyridiniumacetat, N-Octylpyridiniumcyanid, 1-Octyl-2-Methylpyridiniumcyanid, 1-Octyl-2-ethylpyridiniumcyanid, 1-Octyl-2-propylpyridiniumcyanid, 1-Octyl-2-butylpyridiniumcyanid, 1-Octyl-3-Methylpyridiniumcyanid, 1-Octyl-3-ethylpyridiniumcyanid, 1-Octyl-3-propylpyridiniumcyanid, 1-Octyl-3-butylpyridiniumcyanid, 1-Octyl-4-Methylpyridiniumcyanid, 1-Octyl-4-ethylpyridiniumcyanid, 1-Octyl-4-propylpyridiniumcyanid, 1-Octyl-4-butylpyridiniumcyanid.

Man erkennt weiter, dass eine Verbindung entsprechend Formel (78) ausgewählt sein kann aus der Gruppe enthaltend N-Nonylpyridiniumchlorid, 1-Nonyl-2-Methylpyridiniumchlorid, 1-Nonyl-2-ethylpyridiniumchlorid, 1-Nonyl-2-propylpyridiniumchlorid, 1-Nonyl-2-butylpyridiniumchlorid, 1-Nonyl-3-Methylpyridiniumchlorid, 1-Nonyl-3-ethylpyridiniumchlorid, 1-Nonyl-3-propylpyridiniumchlorid, 1-Nonyl-3-butylpyridiniumchlorid, 1-Nonyl-4-Methylpyridiniumchlorid, 1-Nonyl-4-ethylpyridiniumchlorid, 1-Nonyl-4-propylpyridiniumchlorid, 1-Nonyl-4-butylpyridiniumchlorid, N-Nonylpyridiniumfluorid, 1-Nonyl-2-Methylpyridiniumfluorid, 1-Nonyl-2-ethylpyridiniumfluorid, 1-Nonyl-2-propylpyridiniumfluorid, 1-Nonyl-2-butylpyridiniumfluorid, 1-Nonyl-3-Methylpyridiniumfluorid, 1-Nonyl-3-ethylpyridiniumfluorid, 1-Nonyl-3-propylpyridiniumfluorid, 1-Nonyl-3-butylpyridiniumfluorid, 1-Nonyl-4-Methylpyridiniumfluorid, 1-Nonyl-4-ethylpyridiniumfluorid, 1-Nonyl-4-propylpyridiniumfluorid, 1-Nonyl-4-butylpyridiniumfluorid, N-Nonylpyridiniumtriflat, 1-Nonyl-2-Methylpyridiniumtriflat, 1-Nonyl-2-ethylpyridiniumtriflat, 1-Nonyl-2-propylpyridiniumtriflat, 1-Nonyl-2-butylpyridiniumtriflat, 1-Nonyl-3-Methylpyridiniumtriflat, 1-Nonyl-3-ethylpyridiniumtriflat, 1-Nonyl-3-propylpyridiniumtriflat, 1-Nonyl-3-butylpyridiniumtriflat, 1-Nonyl-4-Methylpyridiniumtriflat, 1-Nonyl-4-ethylpyridiniumtriflat, 1-Nonyl-4-propylpyridiniumtriflat, 1-Nonyl-4-butylpyridiniumtriflat, N-Nonylpyridiniummethansulfonat, 1-Nonyl-2-Methylpyridiniummethansulfonat, 1-Nonyl-2-ethylpyridiniummethansulfonat, 1-Nonyl-2-propylpyridiniummethansulfonat, 1-Nonyl-2-butylpyridiniummethansulfonat, 1-Nonyl-3-Methylpyridiniummethansulfonat, 1-Nonyl-3-ethylpyridiniummethansulfonat, 1-Nonyl-3-propylpyridiniummethansulfonat, 1-Nonyl-3-butylpyridiniummethansulfonat, 1-Nonyl-4-Methylpyridiniummethansulfonat, 1-Nonyl-4-ethylpyridiniummethansulfonat, 1-Nonyl-4-propylpyridiniummethansulfonat, 1-Nonyl-4-butylpyridiniummethansulfonat, N-Nonylpyridiniumacetat, 1-Nonyl-2-Methylpyridiniumacetat, 1-Nonyl-2-ethylpyridiniumacetat, 1-Nonyl-2-propylpyridiniumacetat, 1-Nonyl-2-butylpyridiniumacetat, 1-Nonyl-3-Methylpyridiniumacetat, 1-Nonyl-3-ethylpyridiniumacetat, 1-Nonyl-3-propylpyridiniumacetat, 1-Nonyl-3-butylpyridiniumacetat, 1-Nonyl-4-Methylpyridiniumacetat, 1-Nonyl-4-ethylpyridiniumacetat, 1-Nonyl-4-propylpyridiniumacetat, 1-Nonyl-4-butylpyridiniumacetat, N-Nonylpyridiniumcyanid, 1-Nonyl-2-Methylpyridiniumcyanid, 1-Nonyl-2-ethylpyridiniumcyanid, 1-Nonyl-2-propylpyridiniumcyanid, 1-Nonyl-2-butylpyridiniumcyanid, 1-Nonyl-3-Methylpyridiniumcyanid, 1-Nonyl-3-ethylpyridiniumcyanid, 1-Nonyl-3-propylpyridiniumcyanid, 1-Nonyl-3-butylpyridiniumcyanid, 1-Nonyl-4-Methylpyridiniumcyanid, 1-Nonyl-4-ethylpyridiniumcyanid, 1-Nonyl-4-propylpyridiniumcyanid, 1-Nonyl-4-butylpyridiniumcyanid.

Man erkennt weiter, dass eine Verbindung entsprechend Formel (79) ausgewählt sein kann aus der Gruppe enthaltend N-Decylpyridiniumchlorid, 1-Decyl-2-Methylpyridiniumchlorid, 1-Decyl-2-ethylpyridiniumchlorid, 1-Decyl-2-propylpyridiniumchlorid, 1-Decyl-2-butylpyridiniumchlorid, 1-Decyl-3-Methylpyridiniumchlorid, 1-Decyl-3-ethylpyridiniumchlorid, 1-Decyl-3-propylpyridiniumchlorid, 1-Decyl-3-butylpyridiniumchlorid, 1-Decyl-4-Methylpyridiniumchlorid, 1-Decyl-4-ethylpyridiniumchlorid, 1-Decyl-4-propylpyridiniumchlorid, 1-Decyl-4-butylpyridiniumchlorid, N-Decylpyridiniumfluorid, 1-Decyl-2-Methylpyridiniumfluorid, 1-Decyl-2-ethylpyridiniumfluorid, 1-Decyl-2-propylpyridiniumfluorid, 1-Decyl-2-butylpyridiniumfluorid, 1-Decyl-3-Methylpyridiniumfluorid, 1-Decyl-3-ethylpyridiniumfluorid, 1-Decyl-3-propylpyridiniumfluorid, 1-Decyl-3-butylpyridiniumfluorid, 1-Decyl-4-Methylpyridiniumfluorid, 1-Decyl-4-ethylpyridiniumfluorid, 1-Decyl-4-propylpyridiniumfluorid, 1-Decyl-4-butylpyridiniumfluorid, N-Decylpyridiniumtriflat, 1-Decyl-2-Methylpyridiniumtriflat, 1-Decyl-2-ethylpyridiniumtriflat, 1-Decyl-2-propylpyridiniumtriflat, 1-Decyl-2-butylpyridiniumtriflat, 1-Decyl-3-Methylpyridiniumtriflat, 1-Decyl-3-ethylpyridiniumtriflat, 1-Decyl-3-propylpyridiniumtriflat, 1-Decyl-3-butylpyridiniumtriflat, 1-Decyl-4-Methylpyridiniumtriflat, 1-Decyl-4-ethylpyridiniumtriflat, 1-Decyl-4-propylpyridiniumtriflat, 1-Decyl-4-butylpyridiniumtriflat, N-Decylpyridiniummethansulfonat, 1-Decyl-2-Methylpyridiniummethansulfonat, 1-Decyl-2-ethylpyridiniummethansulfonat, 1-Decyl-2-propylpyridiniummethansulfonat, 1-Decyl-2-butylpyridiniummethansulfonat, 1-Decyl-3-Methylpyridiniummethansulfonat, 1-Decyl-3-ethylpyridiniummethansulfonat, 1-Decyl-3-propylpyridiniummethansulfonat, 1-Decyl-3-butylpyridiniummethansulfonat, 1-Decyl-4-Methylpyridiniummethansulfonat, 1-Decyl-4-ethylpyridiniummethansulfonat, 1-Decyl-4-propylpyridiniummethansulfonat, 1-Decyl-4-butylpyridiniummethansulfonat, N-Decylpyridiniumacetat, 1-Decyl-2-Methylpyridiniumacetat, 1-Decyl-2-ethylpyridiniumacetat, 1-Decyl-2-propylpyridiniumacetat, 1-Decyl-2-butylpyridiniumacetat, 1-Decyl-3-Methylpyridiniumacetat, 1-Decyl-3-ethylpyridiniumacetat, 1-Decyl-3-propylpyridiniumacetat, 1-Decyl-3-butylpyridiniumacetat, 1-Decyl-4-Methylpyridiniumacetat, 1-Decyl-4-ethylpyridiniumacetat, 1-Decyl-4-propylpyridiniumacetat, 1-Decyl-4-butylpyridiniumacetat, N-Decylpyridiniumcyanid, 1-Decyl-2-Methylpyridiniumcyanid, 1-Decyl-2-ethylpyridiniumcyanid, 1-Decyl-2-propylpyridiniumcyanid, 1-Decyl-2-butylpyridiniumcyanid, 1-Decyl-3-Methylpyridiniumcyanid, 1-Decyl-3-ethylpyridiniumcyanid, 1-Decyl-3-propylpyridiniumcyanid, 1-Decyl-3-butylpyridiniumcyanid, 1-Decyl-4-Methylpyridiniumcyanid, 1-Decyl-4-ethylpyridiniumcyanid, 1-Decyl-4-propylpyridiniumcyanid, 1-Decyl-4-butylpyridiniumcyanid.

Man erkennt weiter, dass ein Verbindung entsprechend Formel (80) ausgewählt sein kann aus der Gruppe enthaltend N-Undecylpyridiniumchlorid, 1-Undecyl-2-Methylpyridiniumchlorid, 1-Undecyl-2-ethylpyridiniumchlorid, 1-Undecyl-2-propylpyridiniumchlorid, 1-Undecyl-2-butylpyridiniumchlorid, 1-Undecyl-3-Methylpyridiniumchlorid, 1-Undecyl-3-ethylpyridiniumchlorid, 1-Undecyl-3-propylpyridiniumchlorid, 1-Undecyl-3-butylpyridiniumchlorid, 1-Undecyl-4-Methylpyridiniumchlorid, 1-Undecyl-4-ethylpyridiniumchlorid, 1-Undecyl-4-propylpyridiniumchlorid, 1-Undecyl-4-butylpyridiniumchlorid, N-Undecylpyridiniumfluorid, 1-Undecyl-2-Methylpyridiniumfluorid, 1-Undecyl-2-ethylpyridiniumfluorid, 1-Undecyl-2-propylpyridiniumfluorid, 1-Undecyl-2-butylpyridiniumfluorid, 1-Undecyl-3-Methylpyridiniumfluorid, 1-Undecyl-3-ethylpyridiniumfluorid, 1-Undecyl-3-propylpyridiniumfluorid, 1-Undecyl-3-butylpyridiniumfluorid, 1-Undecyl-4-Methylpyridiniumfluorid, 1-Undecyl-4-ethylpyridiniumfluorid, 1-Undecyl-4-propylpyridiniumfluorid, 1-Undecyl-4-butylpyridiniumfluorid, N-Undecylpyridiniumtriflat, 1-Undecyl-2-Methylpyridiniumtriflat, 1-Undecyl-2-ethylpyridiniumtriflat, 1-Undecyl-2-propylpyridiniumtriflat, 1-Undecyl-2-butylpyridiniumtriflat, 1-Undecyl-3-Methylpyridiniumtriflat, 1-Undecyl-3-ethylpyridiniumtriflat, 1-Undecyl-3-propylpyridiniumtriflat, 1-Undecyl-3-butylpyridiniumtriflat, 1-Undecyl-4-Methylpyridiniumtriflat, 1-Undecyl-4-ethylpyridiniumtriflat, 1-Undecyl-4-propylpyridiniumtriflat, 1-Undecyl-4-butylpyridiniumtriflat, N-Undecylpyridiniummethansulfonat, 1-Undecyl-2-Methylpyridiniummethansulfonat, 1-Undecyl-2-ethylpyridiniummethansulfonat, 1-Undecyl-2-propylpyridiniummethansulfonat, 1-Undecyl-2-butylpyridiniummethansulfonat, 1-Undecyl-3-Methylpyridiniummethansulfonat, 1-Undecyl-3-ethylpyridiniummethansulfonat, 1-Undecyl-3-propylpyridiniummethansulfonat, 1-Undecyl-3-butylpyridiniummethansulfonat, 1-Undecyl-4-Methylpyridiniummethansulfonat, 1-Undecyl-4-ethylpyridiniummethansulfonat, 1-Undecyl-4-propylpyridiniummethansulfonat, 1-Undecyl-4-butylpyridiniummethansulfonat, N-Undecylpyridiniumacetat, 1-Undecyl-2-Methylpyridiniumacetat, 1-Undecyl-2-ethylpyridiniumacetat, 1-Undecyl-2-propylpyridiniumacetat, 1-Undecyl-2-butylpyridiniumacetat, 1-Undecyl-3-Methylpyridiniumacetat, 1-Undecyl-3-ethylpyridiniumacetat, 1-Undecyl-3-propylpyridiniumacetat, 1-Undecyl-3-butylpyridiniumacetat, 1-Undecyl-4-Methylpyridiniumacetat, 1-Undecyl-4-ethylpyridiniumacetat, 1-Undecyl-4-propylpyridiniumacetat, 1-Undecyl-4-butylpyridiniumacetat, N-Undecylpyridiniumcyanid, 1-Undecyl-2-Methylpyridiniumcyanid, 1-Undecyl-2-ethylpyridiniumcyanid, 1-Undecyl-2-propylpyridiniumcyanid, 1-Undecyl-2-butylpyridiniumcyanid, 1-Undecyl-3-Methylpyridiniumcyanid, 1-Undecyl-3-ethylpyridiniumcyanid, 1-Undecyl-3-propylpyridiniumcyanid, 1-Undecyl-3-butylpyridiniumcyanid, 1-Undecyl-4-Methylpyridiniumcyanid, 1-Undecyl-4-ethylpyridiniumcyanid, 1-Undecyl-4-propylpyridiniumcyanid, 1-Undecyl-4-butylpyridiniumcyanid.

Man erkennt weiter, dass eine Verbindung entsprechend Formel (81) ausgewählt sein kann aus der Gruppe enthaltend N-Dodecylpyridiniumchlorid, 1-Dodecyl-2-Methylpyridiniumchlorid, 1-Dodecyl-2-ethylpyridiniumchlorid, 1-Dodecyl-2-propylpyridiniumchlorid, 1-Dodecyl-2-butylpyridiniumchlorid, 1-Dodecyl-3-Methylpyridiniumchlorid, 1-Dodecyl-3-ethylpyridiniumchlorid, 1-Dodecyl-3-propylpyridiniumchlorid, 1-Dodecyl-3-butylpyridiniumchlorid, 1-Dodecyl-4-Methylpyridiniumchlorid, 1-Dodecyl-4-ethylpyridiniumchlorid, 1-Dodecyl-4-propylpyridiniumchlorid, 1-Dodecyl-4-butylpyridiniumchlorid, N-Dodecylpyridiniumfluorid, 1-Dodecyl-2-Methylpyridiniumfluorid, 1-Dodecyl-2-ethylpyridiniumfluorid, 1-Dodecyl-2-propylpyridiniumfluorid, 1-Dodecyl-2-butylpyridiniumfluorid, 1-Dodecyl-3-Methylpyridiniumfluorid, 1-Dodecyl-3-ethylpyridiniumfluorid, 1-Dodecyl-3-propylpyridiniumfluorid, 1-Dodecyl-3-butylpyridiniumfluorid, 1-Dodecyl-4-Methylpyridiniumfluorid, 1-Dodecyl-4-ethylpyridiniumfluorid, 1-Dodecyl-4-propylpyridiniumfluorid, 1-Dodecyl-4-butylpyridiniumfluorid, N-Dodecylpyridiniumtriflat, 1-Dodecyl-2-Methylpyridiniumtriflat, 1-Dodecyl-2-ethylpyridiniumtriflat, 1-Dodecyl-2-propylpyridiniumtriflat, 1-Dodecyl-2-butylpyridiniumtriflat, 1-Dodecyl-3-Methylpyridiniumtriflat, 1-Dodecyl-3-ethylpyridiniumtriflat, 1-Dodecyl-3-propylpyridiniumtriflat, 1-Dodecyl-3-butylpyridiniumtriflat, 1-Dodecyl-4-Methylpyridiniumtriflat, 1-Dodecyl-4-ethylpyridiniumtriflat, 1-Dodecyl-4-propylpyridiniumtriflat, 1-Dodecyl-4-butylpyridiniumtriflat, N-Dodecylpyridiniummethansulfonat, 1-Dodecyl-2-Methylpyridiniummethansulfonat, 1-Dodecyl-2-ethylpyridiniummethansulfonat, 1-Dodecyl-2-propylpyridiniummethansulfonat, 1-Dodecyl-2-butylpyridiniummethansulfonat, 1-Dodecyl-3-Methylpyridiniummethansulfonat, 1-Dodecyl-3-ethylpyridiniummethansulfonat, 1-Dodecyl-3-propylpyridiniummethansulfonat, 1-Dodecyl-3-butylpyridiniummethansulfonat, 1-Dodecyl-4-Methylpyridiniummethansulfonat, 1-Dodecyl-4-ethylpyridiniummethansulfonat, 1-Dodecyl-4-propylpyridiniummethansulfonat, 1-Dodecyl-4-butylpyridiniummethansulfonat, N-Dodecylpyridiniumacetat, 1-Dodecyl-2-Methylpyridiniumacetat, 1-Dodecyl-2-ethylpyridiniumacetat, 1-Dodecyl-2-propylpyridiniumacetat, 1-Dodecyl-2-butylpyridiniumacetat, 1-Dodecyl-3-Methylpyridiniumacetat, 1-Dodecyl-3-ethylpyridiniumacetat, 1-Dodecyl-3-propylpyridiniumacetat, 1-Dodecyl-3-butylpyridiniumacetat, 1-Dodecyl-4-Methylpyridiniumacetat, 1-Dodecyl-4-ethylpyridiniumacetat, 1-Dodecyl-4-propylpyridiniumacetat, 1-Dodecyl-4-butylpyridiniumacetat, N-Dodecylpyridiniumcyanid, 1-Dodecyl-2-Methylpyridiniumcyanid, 1-Dodecyl-2-ethylpyridiniumcyanid, 1-Dodecyl-2-propylpyridiniumcyanid, 1-Dodecyl-2-butylpyridiniumcyanid, 1-Dodecyl-3-Methylpyridiniumcyanid, 1-Dodecyl-3-ethylpyridiniumcyanid, 1-Dodecyl-3-propylpyridiniumcyanid, 1-Dodecyl-3-butylpyridiniumcyanid, 1-Dodecyl-4-Methylpyridiniumcyanid, 1-Dodecyl-4-ethylpyridiniumcyanid, 1-Dodecyl-4-propylpyridiniumcyanid, 1-Dodecyl-4-butylpyridiniumcyanid.

Sofern R² ein Alkyl-Rest ist, kann R² in allen angeführten Beispielen in ortho-Stellung, in meta-Stellung oder in para-Stellung zu R¹ angeordnet sein. In einer Ausführungsvariante ist beispielsweise vorgesehen, dass für wenigstens eine der ringförmigen Verbindungen R² ein Kohlenwasserstoffrest ist, der in meta Stellung zu R¹ angeordnet ist. In einer weiteren Ausführungsvariante kann vorgesehen sein, dass für wenigstens eine der ringförmigen Verbindungen R² ein Kohlenwasserstoffrest ist, der in ortho Stellung zu R¹ angeordnet ist. In noch einer weiteren Ausführungsvariante kann vorgesehen sein, dass für wenigstens eine der ringförmigen Verbindungen R² ein Kohlenwasserstoffrest ist, der in para Stellung zu R¹ angeordnet ist.

In einer besonders bevorzugten Ausführungsform weist der Elektrolyt neben der ersten ringförmigen Verbindung noch eine zweite ringförmige Verbindung auf, wobei auch die zweite ringförmige Verbindung der Formel (I)

[(A)R¹R²]⁺[Y]⁻

entspricht;
- wobei (A) eine Ringstruktur ist, ausgewählt aus der Gruppe Pyridinium, Piperidinium, Pyrolidinium;
- wobei der Rest R¹ am Stickstoffatom der Ringstruktur A angeordnet ist;
- R¹ ein Kohlenwasserstoffrest ist mit 1, 2, 3, 4, 5, 6, 7, 8, 9, 10, 11 oder 12 C-Atomen;
- wobei R² ein Wasserstoffatom oder ein Kohlenwasserstoffrest mit 1, 2, 3 oder 4 C-Atomen ist;
- und wobei Y ausgewählt ist aus der Gruppe enthaltend Halogenid, Cyanid, Alkylsulfonat, halogeniertes Alkylsulfonat, Acetat;
und wobei die zweite ringförmige Verbindung eine andere Verbindung ist als die erste ringförmige Verbindung.

Dabei ist es selbstverständlich zweckmäßig, wenn die erste ringförmige Verbindung und die zweite ringförmige Verbindung unterschiedliche Verbindungen sind.

Man erkennt in jedem Fall, dass es günstig ist, wenn der Alkylrest R¹ ein gesättigter Akylrest ist. Mit anderen Worten ist es günstig wenn R¹ ausgewählt ist aus CH₃, C₂H₅, C₃H₇, C₄H₉, C₅H₁₁, C₆H₁₃, C₇H₁₅, C₈H₁₇, C₉H₁₉, C₁₀H₂₁, C₁₁H₂₃, C₁₂H₂₅. Dabei ist es besonders bevorzugt, wenn R¹ ausgewählt ist aus C₄H₉, C₆H₁₃, C₁₂H₂₅.

Weiterhin ist es in jedem Fall denkbar, dass die erste ringförmige Verbindung und die zweite ringförmige Verbindung den gleichen Rest R¹ aufweisen. Alternativ ist auch vorstellbar, dass sie unterschiedliche Reste R¹ aufweisen. In jedem Fall ist jedoch die erste ringförmige Verbindung von der zweiten ringförmigen Verbindung verschieden. Wenn die erste und die zweite ringförmige Verbindung den gleichen Rest R¹ aufweisen, ist es insofern denkbar, dass entweder die Reste R² unterschiedliche Stellungen am Ring haben, dass die Verbindungen unterschiedliche Reste R² aufweisen oder dass die Verbindungen unterschiedliche Reste R² aufweisen, die unterschiedliche Stellungen am Ring haben.

Insofern ist es zweckmäßig, wenn R² entweder ein Wasserstoffatom oder ein gesättigter Alkylrest ist. Mit anderen Worten, es ist günstig wenn R² ausgewählt ist aus H, CH₃, C₂H₅, C₃H₇, C₄H₉. Dabei ist es besonders bevorzugt, wenn R² ausgewählt ist aus H, CH₃.

In einer Variante ist es vorgesehen, dass die ringförmigen Verbindungen Pyridinium-Verbindungen und/oder Piperidinium-Verbindungen sind. Dabei können entweder beide ringförmigen Verbindungen Pyridinium-Verbindungen sein, oder es können beide ringförmige Verbindungen Pipieridinium-Verbindungen sein, oder es kann die erste ringförmige Verbindung eine Pyridinum-Verbindung sein und die zweite eine Piperidinium-Verbindung. Mit anderen Worten die Ringstruktur ist für diese Ausführungsvarianten ein Ring mit sechs Ringatomen.

Mit anderen Worten, bei einem Elektrolyt, der eine erste ringförmige Verbindung entsprechend Formel (I), eine zweite ringförmige Verbindung entsprechend Formel (I), wenigstens 5 Gewichtsprozent Wasser und optional eine oder mehrere weitere Komponenten wie oben beschrieben aufweist, kann die erste ringförmige Verbindung eine Pyridin-Verbindung entsprechend Formel (VI) sein und die zweite ringförmige Verbindung eine Piperidin-Verbindung entsprechend Formel (VII) sein.

In einer weiteren Ausführungsvariante ist folgendes denkbar: Bei einem Elektrolyt, der eine erste ringförmige Verbindung entsprechend Formel (I), eine zweite ringförmige Verbindung entsprechend Formel (I), wenigstens 5 Gewichtsprozent Wasser und optional eine oder mehrere weitere Komponenten wie oben beschrieben aufweist, kann sowohl die erste ringförmige Verbindung, als auch die zweite ringförmige Verbindung eine Pyridin-Verbindung entsprechend Formel (VI) sein.

In einer weiteren Ausführungsvariante ist folgendes denkbar: Bei einem Elektrolyt, der eine erste ringförmige Verbindung entsprechend Formel (I), eine zweite ringförmige Verbindung entsprechend Formel (I), wenigstens 5 Gewichtsprozent Wasser und optional eine oder mehrere weitere Komponenten wie oben beschrieben aufweist, kann sowohl die erste ringförmige Verbindung, als auch die zweite ringförmige Verbindung eine Piperidin-Verbindung entsprechend Formel (VII) sein.

Sind beide ringförmige Verbindungen Pyridin-Verbindungen und haben die ringförmigen Verbindungen unterschiedliche Reste R¹, so sind beispielsweise, aber nicht abschließend, folgende Ausführungsvarianten denkbar.

In einer ersten Ausführungsvariante ist es denkbar, dass die erste ringförmige Verbindung eine Verbindung entsprechend Formel (70) ist, insbesondere eine der oben aufgeführten Verbindungen entsprechend Formel (70), und dass die zweite ringförmige Verbindung ausgewählt ist aus der Gruppe enthaltend Verbindungen entsprechend einer der Formeln (71), (72), (73), (74), (75), (76), (77), (78), (79), (80), (81), insbesondere eine der oben aufgeführten Verbindungen.

In einer weiteren Ausführungsvariante ist es denkbar, dass die erste ringförmige Verbindung eine Verbindung entsprechend Formel (71) ist, insbesondere eine der oben aufgeführten Verbindungen, und dass die zweite ringförmige Verbindung ausgewählt ist aus der Gruppe enthaltend Verbindungen entsprechend einer der Formeln (70), (72), (73), (74), (75), (76), (77), (78), (79), (80), (81), insbesondere eine der oben aufgeführten Verbindungen. Dabei ist es insbesondere vorstellbar, dass die erste ringförmige Verbindung eine Verbindung entsprechend Formel (82) ist und dass die zweite ringförmige Verbindung eine Verbindung entsprechend einer der Formeln (83), (84), (85), (86), (87), (88), (89), (90), (91) oder (92) ist.

In einer weiteren Ausführungsvariante ist es denkbar, dass die erste ringförmige Verbindung eine Verbindung entsprechend Formel (72) ist, insbesondere eine der oben aufgeführten Verbindungen, und dass die zweite ringförmige Verbindung ausgewählt ist aus der Gruppe enthaltend Verbindungen entsprechend einer der Formeln (70), (71), (73), (74), (75), (76), (77), (78), (79), (80), (81), insbesondere eine der oben aufgeführten Verbindungen. Dabei ist es insbesondere vorstellbar, dass die erste ringförmige Verbindung eine Verbindung entsprechend Formel (83) ist und dass die zweite ringförmige Verbindung eine Verbindung entsprechend einer der Formeln (70), (82), (84), (85), (86), (87), (88), (89), (90), (91) oder (92) ist.

In einer weiteren Ausführungsvariante ist es denkbar, dass die erste ringförmige Verbindung eine Verbindung entsprechend Formel (73) ist, insbesondere eine der oben aufgeführten Verbindungen, und dass die zweite ringförmige Verbindung ausgewählt ist aus der Gruppe enthaltend Verbindungen entsprechend einer der Formeln (70), (71), (72), (74), (75), (76), (77), (78), (79), (80), (81), insbesondere eine der oben aufgeführten Verbindungen. Dabei ist es insbesondere vorstellbar, dass die erste ringförmige Verbindung eine Verbindung entsprechend Formel (84) ist und dass die zweite ringförmige Verbindung eine Verbindung entsprechend einer der Formeln (70), (82), (83), (85), (86), (87), (88), (89), (90), (91) oder (92) ist.

In einer weiteren Ausführungsvariante ist es denkbar, dass die erste ringförmige Verbindung eine Verbindung entsprechend Formel (74) ist, insbesondere eine der oben aufgeführten Verbindungen, und dass die zweite ringförmige Verbindung ausgewählt ist aus der Gruppe enthaltend Verbindungen entsprechend einer der Formeln (70), (71), (72), (73), (75), (76), (77), (78), (79), (80), (81), insbesondere eine der oben aufgeführten Verbindungen. Dabei ist es insbesondere vorstellbar, dass die erste ringförmige Verbindung eine Verbindung entsprechend Formel (85) ist und dass die zweite ringförmige Verbindung eine Verbindung entsprechend einer der Formeln (70), (82), (83), (84), (86), (87), (88), (89), (90), (91) oder (92) ist.

In einer weiteren Ausführungsvariante ist es denkbar, dass die erste ringförmige Verbindung eine Verbindung entsprechend Formel (75) ist, insbesondere eine der oben aufgeführten Verbindungen, und dass die zweite ringförmige Verbindung ausgewählt ist aus der Gruppe enthaltend Verbindungen entsprechend einer der Formeln (70), (71), (72), (73), (74), (76), (77), (78), (79), (80), (81), insbesondere eine der oben aufgeführten Verbindungen. Dabei ist es insbesondere vorstellbar, dass die erste ringförmige Verbindung eine Verbindung entsprechend Formel (86) ist und dass die zweite ringförmige Verbindung eine Verbindung entsprechend einer der Formeln (70), (82), (83), (84), (85), (87), (88), (89), (90), (91) oder (92) ist.

In einer weiteren Ausführungsvariante ist es denkbar, dass die erste ringförmige Verbindung eine Verbindung entsprechend Formel (76) ist, insbesondere eine der oben aufgeführten Verbindungen, und dass die zweite ringförmige Verbindung ausgewählt ist aus der Gruppe enthaltend Verbindungen entsprechend einer der Formeln (70), (71), (72), (73), (74), (75), (77), (78), (79), (80), (81), insbesondere eine der oben aufgeführten Verbindungen. Dabei ist es insbesondere vorstellbar, dass die erste ringförmige Verbindung eine Verbindung entsprechend Formel (87) ist und dass die zweite ringförmige Verbindung eine Verbindung entsprechend einer der Formeln (70), (82), (83), (84), (85), (86), (88), (89), (90), (91) oder (92) ist.

In einer weiteren Ausführungsvariante ist es denkbar, dass die erste ringförmige Verbindung eine Verbindung entsprechend Formel (77) ist, insbesondere eine der oben aufgeführten Verbindungen, und dass die zweite ringförmige Verbindung ausgewählt ist aus der Gruppe enthaltend Verbindungen entsprechend einer der Formeln (70), (71), (72), (73), (74), (75), (76), (78), (79), (80), (81), insbesondere eine der oben aufgeführten Verbindungen. Dabei ist es insbesondere vorstellbar, dass die erste ringförmige Verbindung eine Verbindung entsprechend Formel (88) ist und dass die zweite ringförmige Verbindung eine Verbindung entsprechend einer der Formeln (70), (82), (83), (84), (85), (86), (87), (89), (90), (91) oder (92) ist.

In einer weiteren Ausführungsvariante ist es denkbar, dass die erste ringförmige Verbindung eine Verbindung entsprechend Formel (78) ist, insbesondere eine der oben aufgeführten Verbindungen, und dass die zweite ringförmige Verbindung ausgewählt ist aus der Gruppe enthaltend Verbindungen entsprechend einer der Formeln (70), (71), (72), (73), (74), (75), (76), (77), (79), (80), (81), insbesondere eine der oben aufgeführten Verbindungen. Dabei ist es insbesondere vorstellbar, dass die erste ringförmige Verbindung eine Verbindung entsprechend Formel (89) ist und dass die zweite ringförmige Verbindung eine Verbindung entsprechend einer der Formeln (70), (82), (83), (84), (85), (86), (87), (88), (90), (91) oder (92) ist.

In einer weiteren Ausführungsvariante ist es denkbar, dass die erste ringförmige Verbindung eine Verbindung entsprechend Formel (79) ist, insbesondere eine der oben aufgeführten Verbindungen, und dass die zweite ringförmige Verbindung ausgewählt ist aus der Gruppe enthaltend Verbindungen entsprechend einer der Formeln (70), (71), (72), (73), (74), (75), (76), (77), (78), (80), (81), insbesondere eine der oben aufgeführten Verbindungen. Dabei ist es insbesondere vorstellbar, dass die erste ringförmige Verbindung eine Verbindung entsprechend Formel (90) ist und dass die zweite ringförmige Verbindung eine Verbindung entsprechend einer der Formeln (70), (82), (83), (84), (85), (86), (87), (88), (89), (91) oder (92) ist.

In einer weiteren Ausführungsvariante ist es denkbar, dass die erste ringförmige Verbindung eine Verbindung entsprechend Formel (80) ist, insbesondere eine der oben aufgeführten Verbindungen, und dass die zweite ringförmige Verbindung ausgewählt ist aus der Gruppe enthaltend Verbindungen entsprechend einer der Formeln (70), (71), (72), (73), (74), (75), (76), (77), (78), (79), (81), insbesondere eine der oben aufgeführten Verbindungen. Dabei ist es insbesondere vorstellbar, dass die erste ringförmige Verbindung eine Verbindung entsprechend Formel (91) ist und dass die zweite ringförmige Verbindung eine Verbindung entsprechend einer der Formeln (70), (82), (83), (84), (85), (86), (87), (88), (89), (90) oder (92) ist.

In einer weiteren Ausführungsvariante ist es denkbar, dass die erste ringförmige Verbindung eine Verbindung entsprechend Formel (81) ist, insbesondere eine der oben aufgeführten Verbindungen, und dass die zweite ringförmige Verbindung ausgewählt ist aus der Gruppe enthaltend Verbindungen entsprechend einer der Formeln (70), (71), (72), (73), (74), (75), (76), (77), (78), (79), (80), insbesondere eine der oben aufgeführten Verbindungen. Dabei ist es insbesondere vorstellbar, dass die erste ringförmige Verbindung eine Verbindung entsprechend Formel (92) ist und dass die zweite ringförmige Verbindung eine Verbindung entsprechend einer der Formeln (70), (82), (83), (84), (85), (86), (87), (88), (89), (90) oder (91) ist.

Sind beide ringförmige Verbindungen Piperidin-Verbindungen und haben die ringförmigen Verbindungen unterschiedliche Reste R¹, so sind beispielsweise, aber nicht abschließend, folgende Ausführungsvarianten denkbar.

In einer Ausführungsvariante ist es dabei denkbar, dass die erste ringförmige Verbindung eine Verbindung entsprechend Formel (47) ist, insbesondere eine der oben aufgeführten Verbindungen entsprechend Formel (47), und dass die zweite ringförmige Verbindung ausgewählt ist aus der Gruppe enthaltend Verbindungen entsprechend einer der Formeln (48), (49), (50), (51), (52), (53), (54), (55), (56), (57), (58), insbesondere eine der oben aufgeführten Verbindungen.

In einer weiteren Ausführungsvariante ist es denkbar, dass die erste ringförmige Verbindung eine Verbindung entsprechend Formel (48) ist, insbesondere eine der oben aufgeführten Verbindungen, und dass die zweite ringförmige Verbindung ausgewählt ist aus der Gruppe enthaltend Verbindungen entsprechend einer der Formeln (47), (49), (50), (51), (52), (53), (54), (55), (56), (57), (58), insbesondere eine der oben aufgeführten Verbindungen. Dabei ist es insbesondere vorstellbar, dass die erste ringförmige Verbindung eine Verbindung entsprechend Formel (59) ist und dass die zweite ringförmige Verbindung eine Verbindung entsprechend einer der Formeln (47), (60), (61), (62), (63), (64), (65), (66), (67), (68) oder (69) ist.

In einer weiteren Ausführungsvariante ist es denkbar, dass die erste ringförmige Verbindung eine Verbindung entsprechend Formel (49) ist, insbesondere eine der oben aufgeführten Verbindungen, und dass die zweite ringförmige Verbindung ausgewählt ist aus der Gruppe enthaltend Verbindungen entsprechend einer der Formeln (47), (48), (50), (51), (52), (53), (54), (55), (56), (57), (58), insbesondere eine der oben aufgeführten Verbindungen. Dabei ist es insbesondere vorstellbar, dass die erste ringförmige Verbindung eine Verbindung entsprechend Formel (60) ist und dass die zweite ringförmige Verbindung eine Verbindung entsprechend einer der Formeln (47), (59), (61), (62), (63), (64), (65), (66), (67), (68) oder (69) ist.

In einer weiteren Ausführungsvariante ist es denkbar, dass die erste ringförmige Verbindung eine Verbindung entsprechend Formel (50) ist, insbesondere eine der oben aufgeführten Verbindungen, und dass die zweite ringförmige Verbindung ausgewählt ist aus der Gruppe enthaltend Verbindungen entsprechend einer der Formeln (47), (48), (49), (51), (52), (53), (54), (55), (56), (57), (58), insbesondere eine der oben aufgeführten Verbindungen. Dabei ist es insbesondere vorstellbar, dass die erste ringförmige Verbindung eine Verbindung entsprechend Formel (61) ist und dass die zweite ringförmige Verbindung eine Verbindung entsprechend einer der Formeln (47), (59), (60), (62), (63), (64), (65), (66), (67), (68) oder (69) ist.

In einer weiteren Ausführungsvariante ist es denkbar, dass die erste ringförmige Verbindung eine Verbindung entsprechend Formel (51) ist, insbesondere eine der oben aufgeführten Verbindungen, und dass die zweite ringförmige Verbindung ausgewählt ist aus der Gruppe enthaltend Verbindungen entsprechend einer der Formeln (47), (48), (49), (50), (52), (53), (54), (55), (56), (57), (58), insbesondere eine der oben aufgeführten Verbindungen. Dabei ist es insbesondere vorstellbar, dass die erste ringförmige Verbindung eine Verbindung entsprechend Formel (62) ist und dass die zweite ringförmige Verbindung eine Verbindung entsprechend einer der Formeln (47), (59), (60), (61), (63), (64), (65), (66), (67), (68) oder (69) ist.

In einer weiteren Ausführungsvariante ist es denkbar, dass die erste ringförmige Verbindung eine Verbindung entsprechend Formel (52) ist, insbesondere eine der oben aufgeführten Verbindungen, und dass die zweite ringförmige Verbindung ausgewählt ist aus der Gruppe enthaltend Verbindungen entsprechend einer der Formeln (47), (48), (49), (50), (51), (53), (54), (55), (56), (57), (58), insbesondere eine der oben aufgeführten Verbindungen. Dabei ist es insbesondere vorstellbar, dass die erste ringförmige Verbindung eine Verbindung entsprechend Formel (63) ist und dass die zweite ringförmige Verbindung eine Verbindung entsprechend einer der Formeln (47), (59), (60), (61), (62), (64), (65), (66), (67), (68) oder (69) ist.

In einer weiteren Ausführungsvariante ist es denkbar, dass die erste ringförmige Verbindung eine Verbindung entsprechend Formel (53) ist, insbesondere eine der oben aufgeführten Verbindungen, und dass die zweite ringförmige Verbindung ausgewählt ist aus der Gruppe enthaltend Verbindungen entsprechend einer der Formeln (47), (48), (49), (50), (51), (52), (54), (55), (56), (57), (58), insbesondere eine der oben aufgeführten Verbindungen. Dabei ist es insbesondere vorstellbar, dass die erste ringförmige Verbindung eine Verbindung entsprechend Formel (64) ist und dass die zweite ringförmige Verbindung eine Verbindung entsprechend einer der Formeln (47), (59), (60), (61), (62), (63), (65), (66), (67), (68) oder (69) ist.

In einer weiteren Ausführungsvariante ist es denkbar, dass die erste ringförmige Verbindung eine Verbindung entsprechend Formel (54) ist, insbesondere eine der oben aufgeführten Verbindungen, und dass die zweite ringförmige Verbindung ausgewählt ist aus der Gruppe enthaltend Verbindungen entsprechend einer der Formeln (47), (48), (49), (50), (51), (52), (53), (55), (56), (57), (58), insbesondere eine der oben aufgeführten Verbindungen. Dabei ist es insbesondere vorstellbar, dass die erste ringförmige Verbindung eine Verbindung entsprechend Formel (65) ist und dass die zweite ringförmige Verbindung eine Verbindung entsprechend einer der Formeln (47), (59), (60), (61), (62), (63), (64), (66), (67), (68) oder (69) ist.

In einer weiteren Ausführungsvariante ist es denkbar, dass die erste ringförmige Verbindung eine Verbindung entsprechend Formel (55) ist, insbesondere eine der oben aufgeführten Verbindungen, und dass die zweite ringförmige Verbindung ausgewählt ist aus der Gruppe enthaltend Verbindungen entsprechend einer der Formeln (47), (48), (49), (50), (51), (52), (53), (54), (56), (57), (58), insbesondere eine der oben aufgeführten Verbindungen. Dabei ist es insbesondere vorstellbar, dass die erste ringförmige Verbindung eine Verbindung entsprechend Formel (66) ist und dass die zweite ringförmige Verbindung eine Verbindung entsprechend einer der Formeln (47), (59), (60), (61), (62), (63), (64), (65), (67), (68) oder (69) ist.

In einer weiteren Ausführungsvariante ist es denkbar, dass die erste ringförmige Verbindung eine Verbindung entsprechend Formel (56) ist, insbesondere eine der oben aufgeführten Verbindungen, und dass die zweite ringförmige Verbindung ausgewählt ist aus der Gruppe enthaltend Verbindungen entsprechend einer der Formeln (47), (48), (49), (50), (51), (52), (53), (54), (55), (57), (58), insbesondere eine der oben aufgeführten Verbindungen. Dabei ist es insbesondere vorstellbar, dass die erste ringförmige Verbindung eine Verbindung entsprechend Formel (67) ist und dass die zweite ringförmige Verbindung eine Verbindung entsprechend einer der Formeln (47), (59), (60), (61), (62), (63), (64), (65), (66), (68) oder (69) ist.

In einer weiteren Ausführungsvariante ist es denkbar, dass die erste ringförmige Verbindung eine Verbindung entsprechend Formel (57) ist, insbesondere eine der oben aufgeführten Verbindungen, und dass die zweite ringförmige Verbindung ausgewählt ist aus der Gruppe enthaltend Verbindungen entsprechend einer der Formeln (47), (48), (49), (50), (51), (52), (53), (54), (55), (56), (58), insbesondere eine der oben aufgeführten Verbindungen. Dabei ist es insbesondere vorstellbar, dass die erste ringförmige Verbindung eine Verbindung entsprechend Formel (68) ist und dass die zweite ringförmige Verbindung eine Verbindung entsprechend einer der Formeln (47), (59), (60), (61), (62), (63), (64), (65), (66), (67) oder (69) ist.

In einer weiteren Ausführungsvariante ist es denkbar, dass die erste ringförmige Verbindung eine Verbindung entsprechend Formel (58) ist, insbesondere eine der oben aufgeführten Verbindungen, und dass die zweite ringförmige Verbindung ausgewählt ist aus der Gruppe enthaltend Verbindungen entsprechend einer der Formeln (47), (48), (49), (50), (51), (52), (53), (54), (55), (56), (57), insbesondere eine der oben aufgeführten Verbindungen. Dabei ist es insbesondere vorstellbar, dass die erste ringförmige Verbindung eine Verbindung entsprechend Formel (69) ist und dass die zweite ringförmige Verbindung eine Verbindung entsprechend einer der Formeln (47), (59), (60), (61), (62), (63), (64), (65), (66), (67) oder (68) ist.

Ist die erste ringförmige Verbindung eine Pyridin-Verbindung und die zweite ringförmige Verbindung eine Piperidin-Verbindung oder umgekehrt, so sind beispielsweise, aber nicht abschließend, folgende Ausführungsvarianten denkbar.

In einer ersten Ausführungsvariante ist es denkbar, dass die erste ringförmige Verbindung eine Verbindung entsprechend Formel (70) ist, insbesondere eine der oben aufgeführten Verbindungen entsprechend Formel (70), und dass die zweite ringförmige Verbindung ausgewählt ist aus der Gruppe enthaltend Verbindungen entsprechend einer der Formeln (47), (48), (49), (50), (51), (52), (53), (54), (55), (56), (57), (58) insbesondere eine der oben aufgeführten Verbindungen.

In einer weiteren Ausführungsvariante ist es denkbar, dass die erste ringförmige Verbindung eine Verbindung entsprechend Formel (71) ist, insbesondere eine der oben aufgeführten Verbindungen entsprechend Formel (71), und dass die zweite ringförmige Verbindung ausgewählt ist aus der Gruppe enthaltend Verbindungen entsprechend einer der Formeln (47), (48), (49), (50), (51), (52), (53), (54), (55), (56), (57), (58) insbesondere eine der oben aufgeführten Verbindungen.

In einer weiteren Ausführungsvariante ist es denkbar, dass die erste ringförmige Verbindung eine Verbindung entsprechend Formel (72) ist, insbesondere eine der oben aufgeführten Verbindungen entsprechend Formel (72), und dass die zweite ringförmige Verbindung ausgewählt ist aus der Gruppe enthaltend Verbindungen entsprechend einer der Formeln (47), (48), (49), (50), (51), (52), (53), (54), (55), (56), (57), (58) insbesondere eine der oben aufgeführten Verbindungen.

In einer weiteren Ausführungsvariante ist es denkbar, dass die erste ringförmige Verbindung eine Verbindung entsprechend Formel (73) ist, insbesondere eine der oben aufgeführten Verbindungen entsprechend Formel (73), und dass die zweite ringförmige Verbindung ausgewählt ist aus der Gruppe enthaltend Verbindungen entsprechend einer der Formeln (47), (48), (49), (50), (51), (52), (53), (54), (55), (56), (57), (58) insbesondere eine der oben aufgeführten Verbindungen.

In einer weiteren Ausführungsvariante ist es denkbar, dass die erste ringförmige Verbindung eine Verbindung entsprechend Formel (74) ist, insbesondere eine der oben aufgeführten Verbindungen entsprechend Formel (74), und dass die zweite ringförmige Verbindung ausgewählt ist aus der Gruppe enthaltend Verbindungen entsprechend einer der Formeln (47), (48), (49), (50), (51), (52), (53), (54), (55), (56), (57), (58) insbesondere eine der oben aufgeführten Verbindungen.

In einer weiteren Ausführungsvariante ist es denkbar, dass die erste ringförmige Verbindung eine Verbindung entsprechend Formel (75) ist, insbesondere eine der oben aufgeführten Verbindungen entsprechend Formel (75), und dass die zweite ringförmige Verbindung ausgewählt ist aus der Gruppe enthaltend Verbindungen entsprechend einer der Formeln (47), (48), (49), (50), (51), (52), (53), (54), (55), (56), (57), (58) insbesondere eine der oben aufgeführten Verbindungen.

In einer weiteren Ausführungsvariante ist es denkbar, dass die erste ringförmige Verbindung eine Verbindung entsprechend Formel (76) ist, insbesondere eine der oben aufgeführten Verbindungen entsprechend Formel (76), und dass die zweite ringförmige Verbindung ausgewählt ist aus der Gruppe enthaltend Verbindungen entsprechend einer der Formeln (47), (48), (49), (50), (51), (52), (53), (54), (55), (56), (57), (58) insbesondere eine der oben aufgeführten Verbindungen.

In einer weiteren Ausführungsvariante ist es denkbar, dass die erste ringförmige Verbindung eine Verbindung entsprechend Formel (77) ist, insbesondere eine der oben aufgeführten Verbindungen entsprechend Formel (77), und dass die zweite ringförmige Verbindung ausgewählt ist aus der Gruppe enthaltend Verbindungen entsprechend einer der Formeln (47), (48), (49), (50), (51), (52), (53), (54), (55), (56), (57), (58) insbesondere eine der oben aufgeführten Verbindungen.

In einer weiteren Ausführungsvariante ist es denkbar, dass die erste ringförmige Verbindung eine Verbindung entsprechend Formel (78) ist, insbesondere eine der oben aufgeführten Verbindungen entsprechend Formel (78), und dass die zweite ringförmige Verbindung ausgewählt ist aus der Gruppe enthaltend Verbindungen entsprechend einer der Formeln (47), (48), (49), (50), (51), (52), (53), (54), (55), (56), (57), (58) insbesondere eine der oben aufgeführten Verbindungen.

In einer weiteren Ausführungsvariante ist es denkbar, dass die erste ringförmige Verbindung eine Verbindung entsprechend Formel (79) ist, insbesondere eine der oben aufgeführten Verbindungen entsprechend Formel (79), und dass die zweite ringförmige Verbindung ausgewählt ist aus der Gruppe enthaltend Verbindungen entsprechend einer der Formeln (47), (48), (49), (50), (51), (52), (53), (54), (55), (56), (57), (58) insbesondere eine der oben aufgeführten Verbindungen.

In einer weiteren Ausführungsvariante ist es denkbar, dass die erste ringförmige Verbindung eine Verbindung entsprechend Formel (80) ist, insbesondere eine der oben aufgeführten Verbindungen entsprechend Formel (80), und dass die zweite ringförmige Verbindung ausgewählt ist aus der Gruppe enthaltend Verbindungen entsprechend einer der Formeln (47), (48), (49), (50), (51), (52), (53), (54), (55), (56), (57), (58) insbesondere eine der oben aufgeführten Verbindungen.

In einer weiteren Ausführungsvariante ist es denkbar, dass die erste ringförmige Verbindung eine Verbindung entsprechend Formel (81) ist, insbesondere eine der oben aufgeführten Verbindungen entsprechend Formel (81), und dass die zweite ringförmige Verbindung ausgewählt ist aus der Gruppe enthaltend Verbindungen entsprechend einer der Formeln (47), (48), (49), (50), (51), (52), (53), (54), (55), (56), (57), (58) insbesondere eine der oben aufgeführten Verbindungen.

In einer weiteren Ausführungsvariante ist es denkbar, dass die erste ringförmige Verbindung eine Verbindung entsprechend Formel (47) ist, insbesondere eine der oben aufgeführten Verbindungen entsprechend Formel (47), und dass die zweite ringförmige Verbindung ausgewählt ist aus der Gruppe enthaltend Verbindungen entsprechend einer der Formeln (70), (71), (72), (73), (74), (75), (76), (77), (78), (79), (80), (81) insbesondere eine der oben aufgeführten Verbindungen.

In einer weiteren Ausführungsvariante ist es denkbar, dass die erste ringförmige Verbindung eine Verbindung entsprechend Formel (48) ist, insbesondere eine der oben aufgeführten Verbindungen entsprechend Formel (48), und dass die zweite ringförmige Verbindung ausgewählt ist aus der Gruppe enthaltend Verbindungen entsprechend einer der Formeln (70), (71), (72), (73), (74), (75), (76), (77), (78), (79), (80), (81) insbesondere eine der oben aufgeführten Verbindungen.

In einer weiteren Ausführungsvariante ist es denkbar, dass die erste ringförmige Verbindung eine Verbindung entsprechend Formel (49) ist, insbesondere eine der oben aufgeführten Verbindungen entsprechend Formel (49), und dass die zweite ringförmige Verbindung ausgewählt ist aus der Gruppe enthaltend Verbindungen entsprechend einer der Formeln (70), (71), (72), (73), (74), (75), (76), (77), (78), (79), (80), (81) insbesondere eine der oben aufgeführten Verbindungen.

In einer weiteren Ausführungsvariante ist es denkbar, dass die erste ringförmige Verbindung eine Verbindung entsprechend Formel (50) ist, insbesondere eine der oben aufgeführten Verbindungen entsprechend Formel (50), und dass die zweite ringförmige Verbindung ausgewählt ist aus der Gruppe enthaltend Verbindungen entsprechend einer der Formeln (70), (71), (72), (73), (74), (75), (76), (77), (78), (79), (80), (81) insbesondere eine der oben aufgeführten Verbindungen.

In einer weiteren Ausführungsvariante ist es denkbar, dass die erste ringförmige Verbindung eine Verbindung entsprechend Formel (51) ist, insbesondere eine der oben aufgeführten Verbindungen entsprechend Formel (51), und dass die zweite ringförmige Verbindung ausgewählt ist aus der Gruppe enthaltend Verbindungen entsprechend einer der Formeln (70), (71), (72), (73), (74), (75), (76), (77), (78), (79), (80), (81) insbesondere eine der oben aufgeführten Verbindungen.

In einer weiteren Ausführungsvariante ist es denkbar, dass die erste ringförmige Verbindung eine Verbindung entsprechend Formel (52) ist, insbesondere eine der oben aufgeführten Verbindungen entsprechend Formel (52), und dass die zweite ringförmige Verbindung ausgewählt ist aus der Gruppe enthaltend Verbindungen entsprechend einer der Formeln (70), (71), (72), (73), (74), (75), (76), (77), (78), (79), (80), (81) insbesondere eine der oben aufgeführten Verbindungen.

In einer weiteren Ausführungsvariante ist es denkbar, dass die erste ringförmige Verbindung eine Verbindung entsprechend Formel (53) ist, insbesondere eine der oben aufgeführten Verbindungen entsprechend Formel (53), und dass die zweite ringförmige Verbindung ausgewählt ist aus der Gruppe enthaltend Verbindungen entsprechend einer der Formeln (70), (71), (72), (73), (74), (75), (76), (77), (78), (79), (80), (81) insbesondere eine der oben aufgeführten Verbindungen.

In einer weiteren Ausführungsvariante ist es denkbar, dass die erste ringförmige Verbindung eine Verbindung entsprechend Formel (54) ist, insbesondere eine der oben aufgeführten Verbindungen entsprechend Formel (54), und dass die zweite ringförmige Verbindung ausgewählt ist aus der Gruppe enthaltend Verbindungen entsprechend einer der Formeln (70), (71), (72), (73), (74), (75), (76), (77), (78), (79), (80), (81) insbesondere eine der oben aufgeführten Verbindungen.

In einer weiteren Ausführungsvariante ist es denkbar, dass die erste ringförmige Verbindung eine Verbindung entsprechend Formel (55) ist, insbesondere eine der oben aufgeführten Verbindungen entsprechend Formel (55), und dass die zweite ringförmige Verbindung ausgewählt ist aus der Gruppe enthaltend Verbindungen entsprechend einer der Formeln (70), (71), (72), (73), (74), (75), (76), (77), (78), (79), (80), (81) insbesondere eine der oben aufgeführten Verbindungen.

In einer weiteren Ausführungsvariante ist es denkbar, dass die erste ringförmige Verbindung eine Verbindung entsprechend Formel (56) ist, insbesondere eine der oben aufgeführten Verbindungen entsprechend Formel (56), und dass die zweite ringförmige Verbindung ausgewählt ist aus der Gruppe enthaltend Verbindungen entsprechend einer der Formeln (70), (71), (72), (73), (74), (75), (76), (77), (78), (79), (80), (81) insbesondere eine der oben aufgeführten Verbindungen.

In einer weiteren Ausführungsvariante ist es denkbar, dass die erste ringförmige Verbindung eine Verbindung entsprechend Formel (57) ist, insbesondere eine der oben aufgeführten Verbindungen entsprechend Formel (57), und dass die zweite ringförmige Verbindung ausgewählt ist aus der Gruppe enthaltend Verbindungen entsprechend einer der Formeln (70), (71), (72), (73), (74), (75), (76), (77), (78), (79), (80), (81) insbesondere eine der oben aufgeführten Verbindungen.

In einer weiteren Ausführungsvariante ist es denkbar, dass die erste ringförmige Verbindung eine Verbindung entsprechend Formel (58) ist, insbesondere eine der oben aufgeführten Verbindungen entsprechend Formel (58), und dass die zweite ringförmige Verbindung ausgewählt ist aus der Gruppe enthaltend Verbindungen entsprechend einer der Formeln (70), (71), (72), (73), (74), (75), (76), (77), (78), (79), (80), (81) insbesondere eine der oben aufgeführten Verbindungen.

In einer weiteren Variante ist es vorgesehen, dass die ringförmigen Verbindungen Pyrrolidinium-Verbindungen und/oder Pyrrolium-Verbindungen sind. Dabei können entweder beide ringförmigen Verbindungen Pyrrolidinium-Verbindungen sein, oder es können beide ringförmige Verbindungen Pyrrolium-Verbindungen sein, oder es kann die erste ringförmige Verbindung eine Pyrrolidinium-Verbindung sein und die zweite eine Pyrrolium-Verbindung. Mit anderen Worten die Ringstruktur ist für diese Ausführungsvarianten ein Ring mit fünf Ringatomen.

Mit anderen Worten, bei einem Elektrolyt, der eine erste ringförmige Verbindung entsprechend Formel (I), eine zweite ringförmige Verbindung entsprechend Formel (I), wenigstens 5 Gewichtsprozent Wasser und optional eine oder mehrere weitere Komponenten wie oben beschrieben aufweist, kann die erste ringförmige Verbindung eine Pyrrolidin-Verbindung entsprechend Formel (VIII) sein und die zweite ringförmige Verbindung eine Pyrrol-Verbindung entsprechend Formel (IX) sein.

In einer weiteren Ausführungsvariante ist folgendes denkbar: Bei einem Elektrolyt, der eine erste ringförmige Verbindung entsprechend Formel (I), eine zweite ringförmige Verbindung entsprechend Formel (I), wenigstens 5 Gewichtsprozent Wasser und optional eine oder mehrere weitere Komponenten wie oben beschrieben aufweist, kann sowohl die erste ringförmige Verbindung, als auch die zweite ringförmige Verbindung eine Pyrolidin-Verbindung entsprechend Formel (VIII) sein.

In einer weiteren Ausführungsvariante ist folgendes denkbar: Bei einem Elektrolyt, der eine erste ringförmige Verbindung entsprechend Formel (I), eine zweite ringförmige Verbindung entsprechend Formel (I), wenigstens 5 Gewichtsprozent Wasser und optional eine oder mehrere weitere Komponenten wie oben beschrieben aufweist, kann sowohl die erste ringförmige Verbindung, als auch die zweite ringförmige Verbindung eine Pyrrol-Verbindung entsprechend Formel (IX) sein.

Sind beide ringförmige Verbindungen Pyrrolidin-Verbindungen und haben die ringförmigen Verbindungen unterschiedliche Reste R¹, so sind beispielsweise, aber nicht abschließend, folgende Ausführungsvarianten denkbar.

In einer Ausführungsvariante ist es denkbar, dass die erste ringförmige Verbindung eine Verbindung entsprechend Formel (24) ist, insbesondere eine der oben aufgeführten Verbindungen entsprechend Formel (24), und dass die zweite ringförmige Verbindung ausgewählt ist aus der Gruppe enthaltend Verbindungen entsprechend einer der Formeln (25), (26), (27), (28), (29), (30), (31), (32), (33), (34), (35), insbesondere eine der oben aufgeführten Verbindungen.

In einer Ausführungsvariante ist es denkbar, dass die erste ringförmige Verbindung eine Verbindung entsprechend Formel (25) ist, insbesondere eine der oben aufgeführten Verbindungen entsprechend Formel (25), und dass die zweite ringförmige Verbindung ausgewählt ist aus der Gruppe enthaltend Verbindungen entsprechend einer der Formeln (24), (26), (27), (28), (29), (30), (31), (32), (33), (34), (35), insbesondere eine der oben aufgeführten Verbindungen.

In einer Ausführungsvariante ist es denkbar, dass die erste ringförmige Verbindung eine Verbindung entsprechend Formel (26) ist, insbesondere eine der oben aufgeführten Verbindungen entsprechend Formel (26), und dass die zweite ringförmige Verbindung ausgewählt ist aus der Gruppe enthaltend Verbindungen entsprechend einer der Formeln (24), (25), (27), (28), (29), (30), (31), (32), (33), (34), (35), insbesondere eine der oben aufgeführten Verbindungen.

In einer Ausführungsvariante ist es denkbar, dass die erste ringförmige Verbindung eine Verbindung entsprechend Formel (27) ist, insbesondere eine der oben aufgeführten Verbindungen entsprechend Formel (27), und dass die zweite ringförmige Verbindung ausgewählt ist aus der Gruppe enthaltend Verbindungen entsprechend einer der Formeln (24), (25), (26), (28), (29), (30), (31), (32), (33), (34), (35), insbesondere eine der oben aufgeführten Verbindungen.

In einer Ausführungsvariante ist es denkbar, dass die erste ringförmige Verbindung eine Verbindung entsprechend Formel (28) ist, insbesondere eine der oben aufgeführten Verbindungen entsprechend Formel (28), und dass die zweite ringförmige Verbindung ausgewählt ist aus der Gruppe enthaltend Verbindungen entsprechend einer der Formeln (24), (25), (26), (27), (29), (30), (31), (32), (33), (34), (35), insbesondere eine der oben aufgeführten Verbindungen.

In einer Ausführungsvariante ist es denkbar, dass die erste ringförmige Verbindung eine Verbindung entsprechend Formel (29) ist, insbesondere eine der oben aufgeführten Verbindungen entsprechend Formel (29), und dass die zweite ringförmige Verbindung ausgewählt ist aus der Gruppe enthaltend Verbindungen entsprechend einer der Formeln (24), (25), (26), (27), (28), (30), (31), (32), (33), (34), (35), insbesondere eine der oben aufgeführten Verbindungen.

In einer Ausführungsvariante ist es denkbar, dass die erste ringförmige Verbindung eine Verbindung entsprechend Formel (30) ist, insbesondere eine der oben aufgeführten Verbindungen entsprechend Formel (30), und dass die zweite ringförmige Verbindung ausgewählt ist aus der Gruppe enthaltend Verbindungen entsprechend einer der Formeln (24), (25), (26), (27), (28), (29), (31), (32), (33), (34), (35), insbesondere eine der oben aufgeführten Verbindungen.

In einer Ausführungsvariante ist es denkbar, dass die erste ringförmige Verbindung eine Verbindung entsprechend Formel (31) ist, insbesondere eine der oben aufgeführten Verbindungen entsprechend Formel (31), und dass die zweite ringförmige Verbindung ausgewählt ist aus der Gruppe enthaltend Verbindungen entsprechend einer der Formeln (24), (25), (26), (27), (28), (29), (30), (32), (33), (34), (35), insbesondere eine der oben aufgeführten Verbindungen.

In einer Ausführungsvariante ist es denkbar, dass die erste ringförmige Verbindung eine Verbindung entsprechend Formel (32) ist, insbesondere eine der oben aufgeführten Verbindungen entsprechend Formel (32), und dass die zweite ringförmige Verbindung ausgewählt ist aus der Gruppe enthaltend Verbindungen entsprechend einer der Formeln (24), (25), (26), (27), (28), (29), (30), (31), (33), (34), (35), insbesondere eine der oben aufgeführten Verbindungen.

In einer Ausführungsvariante ist es denkbar, dass die erste ringförmige Verbindung eine Verbindung entsprechend Formel (33) ist, insbesondere eine der oben aufgeführten Verbindungen entsprechend Formel (33), und dass die zweite ringförmige Verbindung ausgewählt ist aus der Gruppe enthaltend Verbindungen entsprechend einer der Formeln (24), (25), (26), (27), (28), (29), (30), (31), (32), (34), (35), insbesondere eine der oben aufgeführten Verbindungen.

In einer Ausführungsvariante ist es denkbar, dass die erste ringförmige Verbindung eine Verbindung entsprechend Formel (34) ist, insbesondere eine der oben aufgeführten Verbindungen entsprechend Formel (34), und dass die zweite ringförmige Verbindung ausgewählt ist aus der Gruppe enthaltend Verbindungen entsprechend einer der Formeln (24), (25), (26), (27), (28), (29), (30), (31), (32), (33), (35), insbesondere eine der oben aufgeführten Verbindungen.

In einer Ausführungsvariante ist es denkbar, dass die erste ringförmige Verbindung eine Verbindung entsprechend Formel (35) ist, insbesondere eine der oben aufgeführten Verbindungen entsprechend Formel (35), und dass die zweite ringförmige Verbindung ausgewählt ist aus der Gruppe enthaltend Verbindungen entsprechend einer der Formeln (24), (25), (26), (27), (28), (29), (30), (31), (32), (33), (34), insbesondere eine der oben aufgeführten Verbindungen.

Sind beide ringförmige Verbindungen Pyrrol-Verbindungen und haben die ringförmigen Verbindungen unterschiedliche Reste R¹, so sind beispielsweise, aber nicht abschließend, folgende Ausführungsvarianten denkbar.

In einer Ausführungsvariante ist es denkbar, dass die erste ringförmige Verbindung eine Verbindung entsprechend Formel (1) ist, insbesondere eine der oben aufgeführten Verbindungen entsprechend Formel (1), und dass die zweite ringförmige Verbindung ausgewählt ist aus der Gruppe enthaltend Verbindungen entsprechend einer der Formeln (2), (3), (4), (5), (6), (7), (8), (9), (10), (11), (12), insbesondere eine der oben aufgeführten Verbindungen.

In einer Ausführungsvariante ist es denkbar, dass die erste ringförmige Verbindung eine Verbindung entsprechend Formel (2) ist, insbesondere eine der oben aufgeführten Verbindungen entsprechend Formel (2), und dass die zweite ringförmige Verbindung ausgewählt ist aus der Gruppe enthaltend Verbindungen entsprechend einer der Formeln (1), (3), (4), (5), (6), (7), (8), (9), (10), (11), (12), insbesondere eine der oben aufgeführten Verbindungen.

In einer Ausführungsvariante ist es denkbar, dass die erste ringförmige Verbindung eine Verbindung entsprechend Formel (3) ist, insbesondere eine der oben aufgeführten Verbindungen entsprechend Formel (3), und dass die zweite ringförmige Verbindung ausgewählt ist aus der Gruppe enthaltend Verbindungen entsprechend einer der Formeln (1), (2), (4), (5), (6), (7), (8), (9), (10), (11), (12), insbesondere eine der oben aufgeführten Verbindungen.

In einer Ausführungsvariante ist es denkbar, dass die erste ringförmige Verbindung eine Verbindung entsprechend Formel (4) ist, insbesondere eine der oben aufgeführten Verbindungen entsprechend Formel (4), und dass die zweite ringförmige Verbindung ausgewählt ist aus der Gruppe enthaltend Verbindungen entsprechend einer der Formeln (1), (2), (3), (5), (6), (7), (8), (9), (10), (11), (12), insbesondere eine der oben aufgeführten Verbindungen.

In einer Ausführungsvariante ist es denkbar, dass die erste ringförmige Verbindung eine Verbindung entsprechend Formel (5) ist, insbesondere eine der oben aufgeführten Verbindungen entsprechend Formel (5), und dass die zweite ringförmige Verbindung ausgewählt ist aus der Gruppe enthaltend Verbindungen entsprechend einer der Formeln (1), (2), (3), (4), (6), (7), (8), (9), (10), (11), (12), insbesondere eine der oben aufgeführten Verbindungen.

In einer Ausführungsvariante ist es denkbar, dass die erste ringförmige Verbindung eine Verbindung entsprechend Formel (6) ist, insbesondere eine der oben aufgeführten Verbindungen entsprechend Formel (6), und dass die zweite ringförmige Verbindung ausgewählt ist aus der Gruppe enthaltend Verbindungen entsprechend einer der Formeln (1), (2), (3), (4), (5), (7), (8), (9), (10), (11), (12), insbesondere eine der oben aufgeführten Verbindungen.

In einer Ausführungsvariante ist es denkbar, dass die erste ringförmige Verbindung eine Verbindung entsprechend Formel (7) ist, insbesondere eine der oben aufgeführten Verbindungen entsprechend Formel (7), und dass die zweite ringförmige Verbindung ausgewählt ist aus der Gruppe enthaltend Verbindungen entsprechend einer der Formeln (1), (2), (3), (4), (5), (6), (8), (9), (10), (11), (12), insbesondere eine der oben aufgeführten Verbindungen.

In einer Ausführungsvariante ist es denkbar, dass die erste ringförmige Verbindung eine Verbindung entsprechend Formel (8) ist, insbesondere eine der oben aufgeführten Verbindungen entsprechend Formel (8), und dass die zweite ringförmige Verbindung ausgewählt ist aus der Gruppe enthaltend Verbindungen entsprechend einer der Formeln (1), (2), (3), (4), (5), (6), (7), (9), (10), (11), (12), insbesondere eine der oben aufgeführten Verbindungen.

In einer Ausführungsvariante ist es denkbar, dass die erste ringförmige Verbindung eine Verbindung entsprechend Formel (9) ist, insbesondere eine der oben aufgeführten Verbindungen entsprechend Formel (9), und dass die zweite ringförmige Verbindung ausgewählt ist aus der Gruppe enthaltend Verbindungen entsprechend einer der Formeln (1), (2), (3), (4), (5), (6), (7), (8), (10), (11), (12), insbesondere eine der oben aufgeführten Verbindungen.

In einer Ausführungsvariante ist es denkbar, dass die erste ringförmige Verbindung eine Verbindung entsprechend Formel (10) ist, insbesondere eine der oben aufgeführten Verbindungen entsprechend Formel (10), und dass die zweite ringförmige Verbindung ausgewählt ist aus der Gruppe enthaltend Verbindungen entsprechend einer der Formeln (1), (2), (3), (4), (5), (6), (7), (8), (9), (11), (12), insbesondere eine der oben aufgeführten Verbindungen.

In einer Ausführungsvariante ist es denkbar, dass die erste ringförmige Verbindung eine Verbindung entsprechend Formel (11) ist, insbesondere eine der oben aufgeführten Verbindungen entsprechend Formel (11), und dass die zweite ringförmige Verbindung ausgewählt ist aus der Gruppe enthaltend Verbindungen entsprechend einer der Formeln (1), (2), (3), (4), (5), (6), (7), (8), (9), (10), (12), insbesondere eine der oben aufgeführten Verbindungen.

In einer Ausführungsvariante ist es denkbar, dass die erste ringförmige Verbindung eine Verbindung entsprechend Formel (12) ist, insbesondere eine der oben aufgeführten Verbindungen entsprechend Formel (12), und dass die zweite ringförmige Verbindung ausgewählt ist aus der Gruppe enthaltend Verbindungen entsprechend einer der Formeln (1), (2), (3), (4), (5), (6), (7), (8), (9), (10), (11), insbesondere eine der oben aufgeführten Verbindungen.

Ist die erste ringförmige Verbindung eine Pyrrolidin-Verbindung und die zweite ringförmige Verbindung eine Pyrrol-Verbindung oder umgekehrt, so sind beispielsweise, aber nicht abschließend, folgende Ausführungsvarianten denkbar.

In einer ersten Ausführungsvariante ist es denkbar, dass die erste ringförmige Verbindung eine Verbindung entsprechend Formel (24) ist, insbesondere eine der oben aufgeführten Verbindungen entsprechend Formel (24), und dass die zweite ringförmige Verbindung ausgewählt ist aus der Gruppe enthaltend Verbindungen entsprechend einer der Formeln (1), (2), (3), (4), (5), (6), (7), (8), (9), (10), (11), (12) insbesondere eine der oben aufgeführten Verbindungen.

In einer weiteren Ausführungsvariante ist es denkbar, dass die erste ringförmige Verbindung eine Verbindung entsprechend Formel (25) ist, insbesondere eine der oben aufgeführten Verbindungen entsprechend Formel (25), und dass die zweite ringförmige Verbindung ausgewählt ist aus der Gruppe enthaltend Verbindungen entsprechend einer der Formeln (1), (2), (3), (4), (5), (6), (7), (8), (9), (10), (11), (12) insbesondere eine der oben aufgeführten Verbindungen.

In einer weiteren Ausführungsvariante ist es denkbar, dass die erste ringförmige Verbindung eine Verbindung entsprechend Formel (26) ist, insbesondere eine der oben aufgeführten Verbindungen entsprechend Formel (26), und dass die zweite ringförmige Verbindung ausgewählt ist aus der Gruppe enthaltend Verbindungen entsprechend einer der Formeln (1), (2), (3), (4), (5), (6), (7), (8), (9), (10), (11), (12) insbesondere eine der oben aufgeführten Verbindungen.

In einer weiteren Ausführungsvariante ist es denkbar, dass die erste ringförmige Verbindung eine Verbindung entsprechend Formel (27) ist, insbesondere eine der oben aufgeführten Verbindungen entsprechend Formel (27), und dass die zweite ringförmige Verbindung ausgewählt ist aus der Gruppe enthaltend Verbindungen entsprechend einer der Formeln (1), (2), (3), (4), (5), (6), (7), (8), (9), (10), (11), (12) insbesondere eine der oben aufgeführten Verbindungen.

In einer weiteren Ausführungsvariante ist es denkbar, dass die erste ringförmige Verbindung eine Verbindung entsprechend Formel (28) ist, insbesondere eine der oben aufgeführten Verbindungen entsprechend Formel (28), und dass die zweite ringförmige Verbindung ausgewählt ist aus der Gruppe enthaltend Verbindungen entsprechend einer der Formeln (1), (2), (3), (4), (5), (6), (7), (8), (9), (10), (11), (12) insbesondere eine der oben aufgeführten Verbindungen.

In einer weiteren Ausführungsvariante ist es denkbar, dass die erste ringförmige Verbindung eine Verbindung entsprechend Formel (29) ist, insbesondere eine der oben aufgeführten Verbindungen entsprechend Formel (29), und dass die zweite ringförmige Verbindung ausgewählt ist aus der Gruppe enthaltend Verbindungen entsprechend einer der Formeln (1), (2), (3), (4), (5), (6), (7), (8), (9), (10), (11), (12) insbesondere eine der oben aufgeführten Verbindungen.

In einer weiteren Ausführungsvariante ist es denkbar, dass die erste ringförmige Verbindung eine Verbindung entsprechend Formel (24) ist, insbesondere eine der oben aufgeführten Verbindungen entsprechend Formel (24), und dass die zweite ringförmige Verbindung ausgewählt ist aus der Gruppe enthaltend Verbindungen entsprechend einer der Formeln (1), (2), (3), (4), (5), (6), (7), (8), (9), (10), (11), (12) insbesondere eine der oben aufgeführten Verbindungen.

In einer weiteren Ausführungsvariante ist es denkbar, dass die erste ringförmige Verbindung eine Verbindung entsprechend Formel (31) ist, insbesondere eine der oben aufgeführten Verbindungen entsprechend Formel (31), und dass die zweite ringförmige Verbindung ausgewählt ist aus der Gruppe enthaltend Verbindungen entsprechend einer der Formeln (1), (2), (3), (4), (5), (6), (7), (8), (9), (10), (11), (12) insbesondere eine der oben aufgeführten Verbindungen.

In einer weiteren Ausführungsvariante ist es denkbar, dass die erste ringförmige Verbindung eine Verbindung entsprechend Formel (32) ist, insbesondere eine der oben aufgeführten Verbindungen entsprechend Formel (32), und dass die zweite ringförmige Verbindung ausgewählt ist aus der Gruppe enthaltend Verbindungen entsprechend einer der Formeln (1), (2), (3), (4), (5), (6), (7), (8), (9), (10), (11), (12) insbesondere eine der oben aufgeführten Verbindungen.

In einer weiteren Ausführungsvariante ist es denkbar, dass die erste ringförmige Verbindung eine Verbindung entsprechend Formel (33) ist, insbesondere eine der oben aufgeführten Verbindungen entsprechend Formel (33), und dass die zweite ringförmige Verbindung ausgewählt ist aus der Gruppe enthaltend Verbindungen entsprechend einer der Formeln (1), (2), (3), (4), (5), (6), (7), (8), (9), (10), (11), (12) insbesondere eine der oben aufgeführten Verbindungen.

In einer weiteren Ausführungsvariante ist es denkbar, dass die erste ringförmige Verbindung eine Verbindung entsprechend Formel (34) ist, insbesondere eine der oben aufgeführten Verbindungen entsprechend Formel (34), und dass die zweite ringförmige Verbindung ausgewählt ist aus der Gruppe enthaltend Verbindungen entsprechend einer der Formeln (1), (2), (3), (4), (5), (6), (7), (8), (9), (10), (11), (12) insbesondere eine der oben aufgeführten Verbindungen.

In einer weiteren Ausführungsvariante ist es denkbar, dass die erste ringförmige Verbindung eine Verbindung entsprechend Formel (35) ist, insbesondere eine der oben aufgeführten Verbindungen entsprechend Formel (35), und dass die zweite ringförmige Verbindung ausgewählt ist aus der Gruppe enthaltend Verbindungen entsprechend einer der Formeln (1), (2), (3), (4), (5), (6), (7), (8), (9), (10), (11), (12) insbesondere eine der oben aufgeführten Verbindungen.

In einer weiteren Ausführungsvariante ist es denkbar, dass die erste ringförmige Verbindung eine Verbindung entsprechend Formel (1) ist, insbesondere eine der oben aufgeführten Verbindungen entsprechend Formel (1), und dass die zweite ringförmige Verbindung ausgewählt ist aus der Gruppe enthaltend Verbindungen entsprechend einer der Formeln (24), (25), (26), (27), (28), (29), (30), (31), (32), (33), (34), (35) insbesondere eine der oben aufgeführten Verbindungen.

In einer weiteren Ausführungsvariante ist es denkbar, dass die erste ringförmige Verbindung eine Verbindung entsprechend Formel (2) ist, insbesondere eine der oben aufgeführten Verbindungen entsprechend Formel (2), und dass die zweite ringförmige Verbindung ausgewählt ist aus der Gruppe enthaltend Verbindungen entsprechend einer der Formeln (24), (25), (26), (27), (28), (29), (30), (31), (32), (33), (34), (35) insbesondere eine der oben aufgeführten Verbindungen.

In einer weiteren Ausführungsvariante ist es denkbar, dass die erste ringförmige Verbindung eine Verbindung entsprechend Formel (3) ist, insbesondere eine der oben aufgeführten Verbindungen entsprechend Formel (3), und dass die zweite ringförmige Verbindung ausgewählt ist aus der Gruppe enthaltend Verbindungen entsprechend einer der Formeln (24), (25), (26), (27), (28), (29), (30), (31), (32), (33), (34), (35) insbesondere eine der oben aufgeführten Verbindungen.

In einer weiteren Ausführungsvariante ist es denkbar, dass die erste ringförmige Verbindung eine Verbindung entsprechend Formel (4) ist, insbesondere eine der oben aufgeführten Verbindungen entsprechend Formel (4), und dass die zweite ringförmige Verbindung ausgewählt ist aus der Gruppe enthaltend Verbindungen entsprechend einer der Formeln (24), (25), (26), (27), (28), (29), (30), (31), (32), (33), (34), (35) insbesondere eine der oben aufgeführten Verbindungen.

In einer weiteren Ausführungsvariante ist es denkbar, dass die erste ringförmige Verbindung eine Verbindung entsprechend Formel (5) ist, insbesondere eine der oben aufgeführten Verbindungen entsprechend Formel (5), und dass die zweite ringförmige Verbindung ausgewählt ist aus der Gruppe enthaltend Verbindungen entsprechend einer der Formeln (24), (25), (26), (27), (28), (29), (30), (31), (32), (33), (34), (35) insbesondere eine der oben aufgeführten Verbindungen.

In einer weiteren Ausführungsvariante ist es denkbar, dass die erste ringförmige Verbindung eine Verbindung entsprechend Formel (6) ist, insbesondere eine der oben aufgeführten Verbindungen entsprechend Formel (6), und dass die zweite ringförmige Verbindung ausgewählt ist aus der Gruppe enthaltend Verbindungen entsprechend einer der Formeln (24), (25), (26), (27), (28), (29), (30), (31), (32), (33), (34), (35) insbesondere eine der oben aufgeführten Verbindungen.

In einer weiteren Ausführungsvariante ist es denkbar, dass die erste ringförmige Verbindung eine Verbindung entsprechend Formel (7) ist, insbesondere eine der oben aufgeführten Verbindungen entsprechend Formel (7), und dass die zweite ringförmige Verbindung ausgewählt ist aus der Gruppe enthaltend Verbindungen entsprechend einer der Formeln (24), (25), (26), (27), (28), (29), (30), (31), (32), (33), (34), (35) insbesondere eine der oben aufgeführten Verbindungen.

In einer weiteren Ausführungsvariante ist es denkbar, dass die erste ringförmige Verbindung eine Verbindung entsprechend Formel (8) ist, insbesondere eine der oben aufgeführten Verbindungen entsprechend Formel (8), und dass die zweite ringförmige Verbindung ausgewählt ist aus der Gruppe enthaltend Verbindungen entsprechend einer der Formeln (24), (25), (26), (27), (28), (29), (30), (31), (32), (33), (34), (35) insbesondere eine der oben aufgeführten Verbindungen.

In einer weiteren Ausführungsvariante ist es denkbar, dass die erste ringförmige Verbindung eine Verbindung entsprechend Formel (9) ist, insbesondere eine der oben aufgeführten Verbindungen entsprechend Formel (9), und dass die zweite ringförmige Verbindung ausgewählt ist aus der Gruppe enthaltend Verbindungen entsprechend einer der Formeln (24), (25), (26), (27), (28), (29), (30), (31), (32), (33), (34), (35) insbesondere eine der oben aufgeführten Verbindungen.

In einer weiteren Ausführungsvariante ist es denkbar, dass die erste ringförmige Verbindung eine Verbindung entsprechend Formel (10) ist, insbesondere eine der oben aufgeführten Verbindungen entsprechend Formel (10), und dass die zweite ringförmige Verbindung ausgewählt ist aus der Gruppe enthaltend Verbindungen entsprechend einer der Formeln (24), (25), (26), (27), (28), (29), (30), (31), (32), (33), (34), (35) insbesondere eine der oben aufgeführten Verbindungen.

In einer weiteren Ausführungsvariante ist es denkbar, dass die erste ringförmige Verbindung eine Verbindung entsprechend Formel (11) ist, insbesondere eine der oben aufgeführten Verbindungen entsprechend Formel (11), und dass die zweite ringförmige Verbindung ausgewählt ist aus der Gruppe enthaltend Verbindungen entsprechend einer der Formeln (24), (25), (26), (27), (28), (29), (30), (31), (32), (33), (34), (35) insbesondere eine der oben aufgeführten Verbindungen.

In einer weiteren Ausführungsvariante ist es denkbar, dass die erste ringförmige Verbindung eine Verbindung entsprechend Formel (12) ist, insbesondere eine der oben aufgeführten Verbindungen entsprechend Formel (12), und dass die zweite ringförmige Verbindung ausgewählt ist aus der Gruppe enthaltend Verbindungen entsprechend einer der Formeln (24), (25), (26), (27), (28), (29), (30), (31), (32), (33), (34), (35) insbesondere eine der oben aufgeführten Verbindungen.

In einer weiteren Variante ist es vorgesehen, dass eine der ringförmigen Verbindungen eine Ringverbindung mit einer Ringstruktur, die sechs Ringatome aufweist, ist und dass die andere ringförmige Verbindung eine Ringverbindung mit einer Ringstruktur, die fünf Ringatome aufweist ist. Die erste ringförmige Verbindung kann insofern eine Pyrrolidinium-Verbindungen entsprechend Formel (VIII) oder eine Pyrrolium-Verbindungen entsprechend Formel (IX) sein, während die zweite ringförmige Verbindung eine Piperidinium-Verbindung entsprechend Formel (VII) oder eine Pyridinum-Verbindung entsprechend Formel (VI) ist.

Mit anderen Worten, bei einem Elektrolyt, der eine erste ringförmige Verbindung entsprechend Formel (I), eine zweite ringförmige Verbindung entsprechend Formel (I), wenigstens 5 Gewichtsprozent Wasser und optional eine oder mehrere weitere Komponenten wie oben beschrieben aufweist, kann die erste ringförmige Verbindung eine Pyrrolidin-Verbindung entsprechend Formel (VIII) sein und die zweite ringförmige Verbindung eine Pyridin-Verbindung entsprechend Formel (VI) sein.

Man erkennt insofern Folgendes: Ist die erste ringförmige Verbindung eine Pyrrrolidin-Verbindung und die zweite ringförmige Verbindung eine Pyridin-Verbindung oder umgekehrt, so sind beispielsweise, aber nicht abschließend, folgende Ausführungsvarianten denkbar.

In einer ersten Ausführungsvariante ist es denkbar, dass die erste ringförmige Verbindung eine Verbindung entsprechend Formel (24) ist, insbesondere eine der oben aufgeführten Verbindungen entsprechend Formel (24), und dass die zweite ringförmige Verbindung ausgewählt ist aus der Gruppe enthaltend Verbindungen entsprechend einer der Formeln (70), (71), (72), (73), (74), (75), (76), (77), (78), (79), (80), (81) insbesondere eine der oben aufgeführten Verbindungen.

In einer weitere Ausführungsvariante ist es denkbar, dass die erste ringförmige Verbindung eine Verbindung entsprechend Formel (25) ist, insbesondere eine der oben aufgeführten Verbindungen entsprechend Formel (25), und dass die zweite ringförmige Verbindung ausgewählt ist aus der Gruppe enthaltend Verbindungen entsprechend einer der Formeln (70), (71), (72), (73), (74), (75), (76), (77), (78), (79), (80), (81) insbesondere eine der oben aufgeführten Verbindungen.

In einer weitere Ausführungsvariante ist es denkbar, dass die erste ringförmige Verbindung eine Verbindung entsprechend Formel (26) ist, insbesondere eine der oben aufgeführten Verbindungen entsprechend Formel (26), und dass die zweite ringförmige Verbindung ausgewählt ist aus der Gruppe enthaltend Verbindungen entsprechend einer der Formeln (70), (71), (72), (73), (74), (75), (76), (77), (78), (79), (80), (81) insbesondere eine der oben aufgeführten Verbindungen.

In einer weitere Ausführungsvariante ist es denkbar, dass die erste ringförmige Verbindung eine Verbindung entsprechend Formel (27) ist, insbesondere eine der oben aufgeführten Verbindungen entsprechend Formel (27), und dass die zweite ringförmige Verbindung ausgewählt ist aus der Gruppe enthaltend Verbindungen entsprechend einer der Formeln (70), (71), (72), (73), (74), (75), (76), (77), (78), (79), (80), (81) insbesondere eine der oben aufgeführten Verbindungen.

In einer weitere Ausführungsvariante ist es denkbar, dass die erste ringförmige Verbindung eine Verbindung entsprechend Formel (28) ist, insbesondere eine der oben aufgeführten Verbindungen entsprechend Formel (28), und dass die zweite ringförmige Verbindung ausgewählt ist aus der Gruppe enthaltend Verbindungen entsprechend einer der Formeln (70), (71), (72), (73), (74), (75), (76), (77), (78), (79), (80), (81) insbesondere eine der oben aufgeführten Verbindungen.

In einer weitere Ausführungsvariante ist es denkbar, dass die erste ringförmige Verbindung eine Verbindung entsprechend Formel (29) ist, insbesondere eine der oben aufgeführten Verbindungen entsprechend Formel (29), und dass die zweite ringförmige Verbindung ausgewählt ist aus der Gruppe enthaltend Verbindungen entsprechend einer der Formeln (70), (71), (72), (73), (74), (75), (76), (77), (78), (79), (80), (81) insbesondere eine der oben aufgeführten Verbindungen.

In einer weitere Ausführungsvariante ist es denkbar, dass die erste ringförmige Verbindung eine Verbindung entsprechend Formel (30) ist, insbesondere eine der oben aufgeführten Verbindungen entsprechend Formel (30), und dass die zweite ringförmige Verbindung ausgewählt ist aus der Gruppe enthaltend Verbindungen entsprechend einer der Formeln (70), (71), (72), (73), (74), (75), (76), (77), (78), (79), (80), (81) insbesondere eine der oben aufgeführten Verbindungen.

In einer weitere Ausführungsvariante ist es denkbar, dass die erste ringförmige Verbindung eine Verbindung entsprechend Formel (31) ist, insbesondere eine der oben aufgeführten Verbindungen entsprechend Formel (31), und dass die zweite ringförmige Verbindung ausgewählt ist aus der Gruppe enthaltend Verbindungen entsprechend einer der Formeln (70), (71), (72), (73), (74), (75), (76), (77), (78), (79), (80), (81) insbesondere eine der oben aufgeführten Verbindungen.

In einer weitere Ausführungsvariante ist es denkbar, dass die erste ringförmige Verbindung eine Verbindung entsprechend Formel (32) ist, insbesondere eine der oben aufgeführten Verbindungen entsprechend Formel (32), und dass die zweite ringförmige Verbindung ausgewählt ist aus der Gruppe enthaltend Verbindungen entsprechend einer der Formeln (70), (71), (72), (73), (74), (75), (76), (77), (78), (79), (80), (81) insbesondere eine der oben aufgeführten Verbindungen.

In einer weitere Ausführungsvariante ist es denkbar, dass die erste ringförmige Verbindung eine Verbindung entsprechend Formel (33) ist, insbesondere eine der oben aufgeführten Verbindungen entsprechend Formel (33), und dass die zweite ringförmige Verbindung ausgewählt ist aus der Gruppe enthaltend Verbindungen entsprechend einer der Formeln (70), (71), (72), (73), (74), (75), (76), (77), (78), (79), (80), (81) insbesondere eine der oben aufgeführten Verbindungen.

In einer weitere Ausführungsvariante ist es denkbar, dass die erste ringförmige Verbindung eine Verbindung entsprechend Formel (34) ist, insbesondere eine der oben aufgeführten Verbindungen entsprechend Formel (34), und dass die zweite ringförmige Verbindung ausgewählt ist aus der Gruppe enthaltend Verbindungen entsprechend einer der Formeln (70), (71), (72), (73), (74), (75), (76), (77), (78), (79), (80), (81) insbesondere eine der oben aufgeführten Verbindungen.

In einer weitere Ausführungsvariante ist es denkbar, dass die erste ringförmige Verbindung eine Verbindung entsprechend Formel (35) ist, insbesondere eine der oben aufgeführten Verbindungen entsprechend Formel (35), und dass die zweite ringförmige Verbindung ausgewählt ist aus der Gruppe enthaltend Verbindungen entsprechend einer der Formeln (70), (71), (72), (73), (74), (75), (76), (77), (78), (79), (80), (81) insbesondere eine der oben aufgeführten Verbindungen.

In einer weitere Ausführungsvariante ist es denkbar, dass die erste ringförmige Verbindung eine Verbindung entsprechend Formel (70) ist, insbesondere eine der oben aufgeführten Verbindungen entsprechend Formel (70), und dass die zweite ringförmige Verbindung ausgewählt ist aus der Gruppe enthaltend Verbindungen entsprechend einer der Formeln (24), (25), (26), (27), (28), (29), (30), (31), (32), (33), (34), (35) insbesondere eine der oben aufgeführten Verbindungen.

In einer weitere Ausführungsvariante ist es denkbar, dass die erste ringförmige Verbindung eine Verbindung entsprechend Formel (71) ist, insbesondere eine der oben aufgeführten Verbindungen entsprechend Formel (71), und dass die zweite ringförmige Verbindung ausgewählt ist aus der Gruppe enthaltend Verbindungen entsprechend einer der Formeln (24), (25), (26), (27), (28), (29), (30), (31), (32), (33), (34), (35) insbesondere eine der oben aufgeführten Verbindungen.

In einer weitere Ausführungsvariante ist es denkbar, dass die erste ringförmige Verbindung eine Verbindung entsprechend Formel (72) ist, insbesondere eine der oben aufgeführten Verbindungen entsprechend Formel (72), und dass die zweite ringförmige Verbindung ausgewählt ist aus der Gruppe enthaltend Verbindungen entsprechend einer der Formeln (24), (25), (26), (27), (28), (29), (30), (31), (32), (33), (34), (35) insbesondere eine der oben aufgeführten Verbindungen.

In einer weitere Ausführungsvariante ist es denkbar, dass die erste ringförmige Verbindung eine Verbindung entsprechend Formel (73) ist, insbesondere eine der oben aufgeführten Verbindungen entsprechend Formel (73), und dass die zweite ringförmige Verbindung ausgewählt ist aus der Gruppe enthaltend Verbindungen entsprechend einer der Formeln (24), (25), (26), (27), (28), (29), (30), (31), (32), (33), (34), (35) insbesondere eine der oben aufgeführten Verbindungen.

In einer weitere Ausführungsvariante ist es denkbar, dass die erste ringförmige Verbindung eine Verbindung entsprechend Formel (74) ist, insbesondere eine der oben aufgeführten Verbindungen entsprechend Formel (74), und dass die zweite ringförmige Verbindung ausgewählt ist aus der Gruppe enthaltend Verbindungen entsprechend einer der Formeln (24), (25), (26), (27), (28), (29), (30), (31), (32), (33), (34), (35) insbesondere eine der oben aufgeführten Verbindungen.

In einer weitere Ausführungsvariante ist es denkbar, dass die erste ringförmige Verbindung eine Verbindung entsprechend Formel (75) ist, insbesondere eine der oben aufgeführten Verbindungen entsprechend Formel (75), und dass die zweite ringförmige Verbindung ausgewählt ist aus der Gruppe enthaltend Verbindungen entsprechend einer der Formeln (24), (25), (26), (27), (28), (29), (30), (31), (32), (33), (34), (35) insbesondere eine der oben aufgeführten Verbindungen.

In einer weitere Ausführungsvariante ist es denkbar, dass die erste ringförmige Verbindung eine Verbindung entsprechend Formel (76) ist, insbesondere eine der oben aufgeführten Verbindungen entsprechend Formel (76), und dass die zweite ringförmige Verbindung ausgewählt ist aus der Gruppe enthaltend Verbindungen entsprechend einer der Formeln (24), (25), (26), (27), (28), (29), (30), (31), (32), (33), (34), (35) insbesondere eine der oben aufgeführten Verbindungen.

In einer weitere Ausführungsvariante ist es denkbar, dass die erste ringförmige Verbindung eine Verbindung entsprechend Formel (77) ist, insbesondere eine der oben aufgeführten Verbindungen entsprechend Formel (77), und dass die zweite ringförmige Verbindung ausgewählt ist aus der Gruppe enthaltend Verbindungen entsprechend einer der Formeln (24), (25), (26), (27), (28), (29), (30), (31), (32), (33), (34), (35) insbesondere eine der oben aufgeführten Verbindungen.

In einer weitere Ausführungsvariante ist es denkbar, dass die erste ringförmige Verbindung eine Verbindung entsprechend Formel (78) ist, insbesondere eine der oben aufgeführten Verbindungen entsprechend Formel (78), und dass die zweite ringförmige Verbindung ausgewählt ist aus der Gruppe enthaltend Verbindungen entsprechend einer der Formeln (24), (25), (26), (27), (28), (29), (30), (31), (32), (33), (34), (35) insbesondere eine der oben aufgeführten Verbindungen.

In einer weitere Ausführungsvariante ist es denkbar, dass die erste ringförmige Verbindung eine Verbindung entsprechend Formel (79) ist, insbesondere eine der oben aufgeführten Verbindungen entsprechend Formel (79), und dass die zweite ringförmige Verbindung ausgewählt ist aus der Gruppe enthaltend Verbindungen entsprechend einer der Formeln (24), (25), (26), (27), (28), (29), (30), (31), (32), (33), (34), (35) insbesondere eine der oben aufgeführten Verbindungen.

In einer weitere Ausführungsvariante ist es denkbar, dass die erste ringförmige Verbindung eine Verbindung entsprechend Formel (80) ist, insbesondere eine der oben aufgeführten Verbindungen entsprechend Formel (80), und dass die zweite ringförmige Verbindung ausgewählt ist aus der Gruppe enthaltend Verbindungen entsprechend einer der Formeln (24), (25), (26), (27), (28), (29), (30), (31), (32), (33), (34), (35) insbesondere eine der oben aufgeführten Verbindungen.

In einer weitere Ausführungsvariante ist es denkbar, dass die erste ringförmige Verbindung eine Verbindung entsprechend Formel (81) ist, insbesondere eine der oben aufgeführten Verbindungen entsprechend Formel (81), und dass die zweite ringförmige Verbindung ausgewählt ist aus der Gruppe enthaltend Verbindungen entsprechend einer der Formeln (24), (25), (26), (27), (28), (29), (30), (31), (32), (33), (34), (35) insbesondere eine der oben aufgeführten Verbindungen.

Mit anderen Worten, bei einem Elektrolyt, der eine erste ringförmige Verbindung entsprechend Formel (I), eine zweite ringförmige Verbindung entsprechend Formel (I), wenigstens 5 Gewichtsprozent Wasser und optional eine oder mehrere weitere Komponenten wie oben beschrieben aufweist, kann die erste ringförmige Verbindung eine Pyrrolidin-Verbindung entsprechend Formel (VIII) sein und die zweite ringförmige Verbindung eine Piperidin-Verbindung entsprechend Formel (VII) sein.

Man erkennt insofern Folgendes: Ist die erste ringförmige Verbindung eine Pyrrrolidin-Verbindung und die zweite ringförmige Verbindung eine Piperidin-Verbindung oder umgekehrt, so sind beispielsweise, aber nicht abschließend, folgende Ausführungsvarianten denkbar.

In einer ersten Ausführungsvariante ist es denkbar, dass die erste ringförmige Verbindung eine Verbindung entsprechend Formel (24) ist, insbesondere eine der oben aufgeführten Verbindungen entsprechend Formel (24), und dass die zweite ringförmige Verbindung ausgewählt ist aus der Gruppe enthaltend Verbindungen entsprechend einer der Formeln (47), (48), (49), (50), (51), (52), (53), (54), (55), (56), (57), (58) insbesondere eine der oben aufgeführten Verbindungen.

In einer weiteren Ausführungsvariante ist es denkbar, dass die erste ringförmige Verbindung eine Verbindung entsprechend Formel (25) ist, insbesondere eine der oben aufgeführten Verbindungen entsprechend Formel (25), und dass die zweite ringförmige Verbindung ausgewählt ist aus der Gruppe enthaltend Verbindungen entsprechend einer der Formeln (47), (48), (49), (50), (51), (52), (53), (54), (55), (56), (57), (58) insbesondere eine der oben aufgeführten Verbindungen.

In einer weiteren Ausführungsvariante ist es denkbar, dass die erste ringförmige Verbindung eine Verbindung entsprechend Formel (26) ist, insbesondere eine der oben aufgeführten Verbindungen entsprechend Formel (26), und dass die zweite ringförmige Verbindung ausgewählt ist aus der Gruppe enthaltend Verbindungen entsprechend einer der Formeln (47), (48), (49), (50), (51), (52), (53), (54), (55), (56), (57), (58) insbesondere eine der oben aufgeführten Verbindungen.

In einer weiteren Ausführungsvariante ist es denkbar, dass die erste ringförmige Verbindung eine Verbindung entsprechend Formel (27) ist, insbesondere eine der oben aufgeführten Verbindungen entsprechend Formel (27), und dass die zweite ringförmige Verbindung ausgewählt ist aus der Gruppe enthaltend Verbindungen entsprechend einer der Formeln (47), (48), (49), (50), (51), (52), (53), (54), (55), (56), (57), (58) insbesondere eine der oben aufgeführten Verbindungen.

In einer weiteren Ausführungsvariante ist es denkbar, dass die erste ringförmige Verbindung eine Verbindung entsprechend Formel (28) ist, insbesondere eine der oben aufgeführten Verbindungen entsprechend Formel (28), und dass die zweite ringförmige Verbindung ausgewählt ist aus der Gruppe enthaltend Verbindungen entsprechend einer der Formeln (47), (48), (49), (50), (51), (52), (53), (54), (55), (56), (57), (58) insbesondere eine der oben aufgeführten Verbindungen.

In einer weiteren Ausführungsvariante ist es denkbar, dass die erste ringförmige Verbindung eine Verbindung entsprechend Formel (29) ist, insbesondere eine der oben aufgeführten Verbindungen entsprechend Formel (29), und dass die zweite ringförmige Verbindung ausgewählt ist aus der Gruppe enthaltend Verbindungen entsprechend einer der Formeln (47), (48), (49), (50), (51), (52), (53), (54), (55), (56), (57), (58) insbesondere eine der oben aufgeführten Verbindungen.

In einer weiteren Ausführungsvariante ist es denkbar, dass die erste ringförmige Verbindung eine Verbindung entsprechend Formel (30) ist, insbesondere eine der oben aufgeführten Verbindungen entsprechend Formel (30), und dass die zweite ringförmige Verbindung ausgewählt ist aus der Gruppe enthaltend Verbindungen entsprechend einer der Formeln (47), (48), (49), (50), (51), (52), (53), (54), (55), (56), (57), (58) insbesondere eine der oben aufgeführten Verbindungen.

In einer weiteren Ausführungsvariante ist es denkbar, dass die erste ringförmige Verbindung eine Verbindung entsprechend Formel (31) ist, insbesondere eine der oben aufgeführten Verbindungen entsprechend Formel (31), und dass die zweite ringförmige Verbindung ausgewählt ist aus der Gruppe enthaltend Verbindungen entsprechend einer der Formeln (47), (48), (49), (50), (51), (52), (53), (54), (55), (56), (57), (58) insbesondere eine der oben aufgeführten Verbindungen.

In einer weiteren Ausführungsvariante ist es denkbar, dass die erste ringförmige Verbindung eine Verbindung entsprechend Formel (32) ist, insbesondere eine der oben aufgeführten Verbindungen entsprechend Formel (32), und dass die zweite ringförmige Verbindung ausgewählt ist aus der Gruppe enthaltend Verbindungen entsprechend einer der Formeln (47), (48), (49), (50), (51), (52), (53), (54), (55), (56), (57), (58) insbesondere eine der oben aufgeführten Verbindungen.

In einer weiteren Ausführungsvariante ist es denkbar, dass die erste ringförmige Verbindung eine Verbindung entsprechend Formel (33) ist, insbesondere eine der oben aufgeführten Verbindungen entsprechend Formel (33), und dass die zweite ringförmige Verbindung ausgewählt ist aus der Gruppe enthaltend Verbindungen entsprechend einer der Formeln (47), (48), (49), (50), (51), (52), (53), (54), (55), (56), (57), (58) insbesondere eine der oben aufgeführten Verbindungen.

In einer weiteren Ausführungsvariante ist es denkbar, dass die erste ringförmige Verbindung eine Verbindung entsprechend Formel (34) ist, insbesondere eine der oben aufgeführten Verbindungen entsprechend Formel (34), und dass die zweite ringförmige Verbindung ausgewählt ist aus der Gruppe enthaltend Verbindungen entsprechend einer der Formeln (47), (48), (49), (50), (51), (52), (53), (54), (55), (56), (57), (58) insbesondere eine der oben aufgeführten Verbindungen.

In einer weiteren Ausführungsvariante ist es denkbar, dass die erste ringförmige Verbindung eine Verbindung entsprechend Formel (35) ist, insbesondere eine der oben aufgeführten Verbindungen entsprechend Formel (35), und dass die zweite ringförmige Verbindung ausgewählt ist aus der Gruppe enthaltend Verbindungen entsprechend einer der Formeln (47), (48), (49), (50), (51), (52), (53), (54), (55), (56), (57), (58) insbesondere eine der oben aufgeführten Verbindungen.

In einer weiteren Ausführungsvariante ist es denkbar, dass die erste ringförmige Verbindung eine Verbindung entsprechend Formel (47) ist, insbesondere eine der oben aufgeführten Verbindungen entsprechend Formel (47), und dass die zweite ringförmige Verbindung ausgewählt ist aus der Gruppe enthaltend Verbindungen entsprechend einer der Formeln (24), (25), (26), (27), (28), (29), (30), (31), (32), (33), (34), (35) insbesondere eine der oben aufgeführten Verbindungen.

In einer weiteren Ausführungsvariante ist es denkbar, dass die erste ringförmige Verbindung eine Verbindung entsprechend Formel (48) ist, insbesondere eine der oben aufgeführten Verbindungen entsprechend Formel (48), und dass die zweite ringförmige Verbindung ausgewählt ist aus der Gruppe enthaltend Verbindungen entsprechend einer der Formeln (24), (25), (26), (27), (28), (29), (30), (31), (32), (33), (34), (35) insbesondere eine der oben aufgeführten Verbindungen.

In einer weiteren Ausführungsvariante ist es denkbar, dass die erste ringförmige Verbindung eine Verbindung entsprechend Formel (49) ist, insbesondere eine der oben aufgeführten Verbindungen entsprechend Formel (49), und dass die zweite ringförmige Verbindung ausgewählt ist aus der Gruppe enthaltend Verbindungen entsprechend einer der Formeln (24), (25), (26), (27), (28), (29), (30), (31), (32), (33), (34), (35) insbesondere eine der oben aufgeführten Verbindungen.

In einer weiteren Ausführungsvariante ist es denkbar, dass die erste ringförmige Verbindung eine Verbindung entsprechend Formel (50) ist, insbesondere eine der oben aufgeführten Verbindungen entsprechend Formel (50), und dass die zweite ringförmige Verbindung ausgewählt ist aus der Gruppe enthaltend Verbindungen entsprechend einer der Formeln (24), (25), (26), (27), (28), (29), (30), (31), (32), (33), (34), (35) insbesondere eine der oben aufgeführten Verbindungen.

In einer weiteren Ausführungsvariante ist es denkbar, dass die erste ringförmige Verbindung eine Verbindung entsprechend Formel (51) ist, insbesondere eine der oben aufgeführten Verbindungen entsprechend Formel (51), und dass die zweite ringförmige Verbindung ausgewählt ist aus der Gruppe enthaltend Verbindungen entsprechend einer der Formeln (24), (25), (26), (27), (28), (29), (30), (31), (32), (33), (34), (35) insbesondere eine der oben aufgeführten Verbindungen.

In einer weiteren Ausführungsvariante ist es denkbar, dass die erste ringförmige Verbindung eine Verbindung entsprechend Formel (52) ist, insbesondere eine der oben aufgeführten Verbindungen entsprechend Formel (52), und dass die zweite ringförmige Verbindung ausgewählt ist aus der Gruppe enthaltend Verbindungen entsprechend einer der Formeln (24), (25), (26), (27), (28), (29), (30), (31), (32), (33), (34), (35) insbesondere eine der oben aufgeführten Verbindungen.

In einer weiteren Ausführungsvariante ist es denkbar, dass die erste ringförmige Verbindung eine Verbindung entsprechend Formel (53) ist, insbesondere eine der oben aufgeführten Verbindungen entsprechend Formel (53), und dass die zweite ringförmige Verbindung ausgewählt ist aus der Gruppe enthaltend Verbindungen entsprechend einer der Formeln (24), (25), (26), (27), (28), (29), (30), (31), (32), (33), (34), (35) insbesondere eine der oben aufgeführten Verbindungen.

In einer weiteren Ausführungsvariante ist es denkbar, dass die erste ringförmige Verbindung eine Verbindung entsprechend Formel (54) ist, insbesondere eine der oben aufgeführten Verbindungen entsprechend Formel (54), und dass die zweite ringförmige Verbindung ausgewählt ist aus der Gruppe enthaltend Verbindungen entsprechend einer der Formeln (24), (25), (26), (27), (28), (29), (30), (31), (32), (33), (34), (35) insbesondere eine der oben aufgeführten Verbindungen.

In einer weiteren Ausführungsvariante ist es denkbar, dass die erste ringförmige Verbindung eine Verbindung entsprechend Formel (55) ist, insbesondere eine der oben aufgeführten Verbindungen entsprechend Formel (55), und dass die zweite ringförmige Verbindung ausgewählt ist aus der Gruppe enthaltend Verbindungen entsprechend einer der Formeln (24), (25), (26), (27), (28), (29), (30), (31), (32), (33), (34), (35) insbesondere eine der oben aufgeführten Verbindungen.

In einer weiteren Ausführungsvariante ist es denkbar, dass die erste ringförmige Verbindung eine Verbindung entsprechend Formel (56) ist, insbesondere eine der oben aufgeführten Verbindungen entsprechend Formel (56), und dass die zweite ringförmige Verbindung ausgewählt ist aus der Gruppe enthaltend Verbindungen entsprechend einer der Formeln (24), (25), (26), (27), (28), (29), (30), (31), (32), (33), (34), (35) insbesondere eine der oben aufgeführten Verbindungen.

In einer weiteren Ausführungsvariante ist es denkbar, dass die erste ringförmige Verbindung eine Verbindung entsprechend Formel (57) ist, insbesondere eine der oben aufgeführten Verbindungen entsprechend Formel (57), und dass die zweite ringförmige Verbindung ausgewählt ist aus der Gruppe enthaltend Verbindungen entsprechend einer der Formeln (24), (25), (26), (27), (28), (29), (30), (31), (32), (33), (34), (35) insbesondere eine der oben aufgeführten Verbindungen.

In einer weiteren Ausführungsvariante ist es denkbar, dass die erste ringförmige Verbindung eine Verbindung entsprechend Formel (58) ist, insbesondere eine der oben aufgeführten Verbindungen entsprechend Formel (58), und dass die zweite ringförmige Verbindung ausgewählt ist aus der Gruppe enthaltend Verbindungen entsprechend einer der Formeln (24), (25), (26), (27), (28), (29), (30), (31), (32), (33), (34), (35) insbesondere eine der oben aufgeführten Verbindungen.

Mit anderen Worten, bei einem Elektrolyt, der eine erste ringförmige Verbindung entsprechend Formel (I), eine zweite ringförmige Verbindung entsprechend Formel (I), wenigstens 5 Gewichtsprozent Wasser und optional eine oder mehrere weitere Komponenten wie oben beschrieben aufweist, kann die erste ringförmige Verbindung eine Pyrrol-Verbindung entsprechend Formel (IX) sein und die zweite ringförmige Verbindung eine Pyridin-Verbindung entsprechend Formel (VI) sein.

Man erkennt insofern Folgendes: Ist die erste ringförmige Verbindung eine Pyrrol-Verbindung und die zweite ringförmige Verbindung eine Pyridin-Verbindung oder umgekehrt, so sind beispielsweise, aber nicht abschließend, folgende Ausführungsvarianten denkbar.

In einer ersten Ausführungsvariante ist es denkbar, dass die erste ringförmige Verbindung eine Verbindung entsprechend Formel (1) ist, insbesondere eine der oben aufgeführten Verbindungen entsprechend Formel (1), und dass die zweite ringförmige Verbindung ausgewählt ist aus der Gruppe enthaltend Verbindungen entsprechend einer der Formeln (70), (71), (72), (73), (74), (75), (76), (77), (78), (79), (80), (81) insbesondere eine der oben aufgeführten Verbindungen.

In einer weiteren Ausführungsvariante ist es denkbar, dass die erste ringförmige Verbindung eine Verbindung entsprechend Formel (2) ist, insbesondere eine der oben aufgeführten Verbindungen entsprechend Formel (2), und dass die zweite ringförmige Verbindung ausgewählt ist aus der Gruppe enthaltend Verbindungen entsprechend einer der Formeln (70), (71), (72), (73), (74), (75), (76), (77), (78), (79), (80), (81) insbesondere eine der oben aufgeführten Verbindungen.

In einer weiteren Ausführungsvariante ist es denkbar, dass die erste ringförmige Verbindung eine Verbindung entsprechend Formel (3) ist, insbesondere eine der oben aufgeführten Verbindungen entsprechend Formel (3), und dass die zweite ringförmige Verbindung ausgewählt ist aus der Gruppe enthaltend Verbindungen entsprechend einer der Formeln (70), (71), (72), (73), (74), (75), (76), (77), (78), (79), (80), (81) insbesondere eine der oben aufgeführten Verbindungen.

In einer weiteren Ausführungsvariante ist es denkbar, dass die erste ringförmige Verbindung eine Verbindung entsprechend Formel (4) ist, insbesondere eine der oben aufgeführten Verbindungen entsprechend Formel (4), und dass die zweite ringförmige Verbindung ausgewählt ist aus der Gruppe enthaltend Verbindungen entsprechend einer der Formeln (70), (71), (72), (73), (74), (75), (76), (77), (78), (79), (80), (81) insbesondere eine der oben aufgeführten Verbindungen.

In einer weiteren Ausführungsvariante ist es denkbar, dass die erste ringförmige Verbindung eine Verbindung entsprechend Formel (5) ist, insbesondere eine der oben aufgeführten Verbindungen entsprechend Formel (5), und dass die zweite ringförmige Verbindung ausgewählt ist aus der Gruppe enthaltend Verbindungen entsprechend einer der Formeln (70), (71), (72), (73), (74), (75), (76), (77), (78), (79), (80), (81) insbesondere eine der oben aufgeführten Verbindungen.

In einer weiteren Ausführungsvariante ist es denkbar, dass die erste ringförmige Verbindung eine Verbindung entsprechend Formel (6) ist, insbesondere eine der oben aufgeführten Verbindungen entsprechend Formel (6), und dass die zweite ringförmige Verbindung ausgewählt ist aus der Gruppe enthaltend Verbindungen entsprechend einer der Formeln (70), (71), (72), (73), (74), (75), (76), (77), (78), (79), (80), (81) insbesondere eine der oben aufgeführten Verbindungen.

In einer weiteren Ausführungsvariante ist es denkbar, dass die erste ringförmige Verbindung eine Verbindung entsprechend Formel (7) ist, insbesondere eine der oben aufgeführten Verbindungen entsprechend Formel (7), und dass die zweite ringförmige Verbindung ausgewählt ist aus der Gruppe enthaltend Verbindungen entsprechend einer der Formeln (70), (71), (72), (73), (74), (75), (76), (77), (78), (79), (80), (81) insbesondere eine der oben aufgeführten Verbindungen.

In einer weiteren Ausführungsvariante ist es denkbar, dass die erste ringförmige Verbindung eine Verbindung entsprechend Formel (8) ist, insbesondere eine der oben aufgeführten Verbindungen entsprechend Formel (8), und dass die zweite ringförmige Verbindung ausgewählt ist aus der Gruppe enthaltend Verbindungen entsprechend einer der Formeln (70), (71), (72), (73), (74), (75), (76), (77), (78), (79), (80), (81) insbesondere eine der oben aufgeführten Verbindungen.

In einer weiteren Ausführungsvariante ist es denkbar, dass die erste ringförmige Verbindung eine Verbindung entsprechend Formel (9) ist, insbesondere eine der oben aufgeführten Verbindungen entsprechend Formel (9), und dass die zweite ringförmige Verbindung ausgewählt ist aus der Gruppe enthaltend Verbindungen entsprechend einer der Formeln (70), (71), (72), (73), (74), (75), (76), (77), (78), (79), (80), (81) insbesondere eine der oben aufgeführten Verbindungen.

In einer weiteren Ausführungsvariante ist es denkbar, dass die erste ringförmige Verbindung eine Verbindung entsprechend Formel (10) ist, insbesondere eine der oben aufgeführten Verbindungen entsprechend Formel (10), und dass die zweite ringförmige Verbindung ausgewählt ist aus der Gruppe enthaltend Verbindungen entsprechend einer der Formeln (70), (71), (72), (73), (74), (75), (76), (77), (78), (79), (80), (81) insbesondere eine der oben aufgeführten Verbindungen.

In einer weiteren Ausführungsvariante ist es denkbar, dass die erste ringförmige Verbindung eine Verbindung entsprechend Formel (11) ist, insbesondere eine der oben aufgeführten Verbindungen entsprechend Formel (11), und dass die zweite ringförmige Verbindung ausgewählt ist aus der Gruppe enthaltend Verbindungen entsprechend einer der Formeln (70), (71), (72), (73), (74), (75), (76), (77), (78), (79), (80), (81) insbesondere eine der oben aufgeführten Verbindungen.

In einer weiteren Ausführungsvariante ist es denkbar, dass die erste ringförmige Verbindung eine Verbindung entsprechend Formel (12) ist, insbesondere eine der oben aufgeführten Verbindungen entsprechend Formel (12), und dass die zweite ringförmige Verbindung ausgewählt ist aus der Gruppe enthaltend Verbindungen entsprechend einer der Formeln (70), (71), (72), (73), (74), (75), (76), (77), (78), (79), (80), (81) insbesondere eine der oben aufgeführten Verbindungen.

In einer weiteren Ausführungsvariante ist es denkbar, dass die erste ringförmige Verbindung eine Verbindung entsprechend Formel (70) ist, insbesondere eine der oben aufgeführten Verbindungen entsprechend Formel (70), und dass die zweite ringförmige Verbindung ausgewählt ist aus der Gruppe enthaltend Verbindungen entsprechend einer der Formeln (1), (2), (3), (4), (5), (6), (7), (8), (9), (10), (11), (12) insbesondere eine der oben aufgeführten Verbindungen.

In einer weiteren Ausführungsvariante ist es denkbar, dass die erste ringförmige Verbindung eine Verbindung entsprechend Formel (71) ist, insbesondere eine der oben aufgeführten Verbindungen entsprechend Formel (71), und dass die zweite ringförmige Verbindung ausgewählt ist aus der Gruppe enthaltend Verbindungen entsprechend einer der Formeln (1), (2), (3), (4), (5), (6), (7), (8), (9), (10), (11), (12) insbesondere eine der oben aufgeführten Verbindungen.

In einer weiteren Ausführungsvariante ist es denkbar, dass die erste ringförmige Verbindung eine Verbindung entsprechend Formel (72) ist, insbesondere eine der oben aufgeführten Verbindungen entsprechend Formel (72), und dass die zweite ringförmige Verbindung ausgewählt ist aus der Gruppe enthaltend Verbindungen entsprechend einer der Formeln (1), (2), (3), (4), (5), (6), (7), (8), (9), (10), (11), (12) insbesondere eine der oben aufgeführten Verbindungen.

In einer weiteren Ausführungsvariante ist es denkbar, dass die erste ringförmige Verbindung eine Verbindung entsprechend Formel (73) ist, insbesondere eine der oben aufgeführten Verbindungen entsprechend Formel (73), und dass die zweite ringförmige Verbindung ausgewählt ist aus der Gruppe enthaltend Verbindungen entsprechend einer der Formeln (1), (2), (3), (4), (5), (6), (7), (8), (9), (10), (11), (12) insbesondere eine der oben aufgeführten Verbindungen.

In einer weiteren Ausführungsvariante ist es denkbar, dass die erste ringförmige Verbindung eine Verbindung entsprechend Formel (74) ist, insbesondere eine der oben aufgeführten Verbindungen entsprechend Formel (74), und dass die zweite ringförmige Verbindung ausgewählt ist aus der Gruppe enthaltend Verbindungen entsprechend einer der Formeln (1), (2), (3), (4), (5), (6), (7), (8), (9), (10), (11), (12) insbesondere eine der oben aufgeführten Verbindungen.

In einer weiteren Ausführungsvariante ist es denkbar, dass die erste ringförmige Verbindung eine Verbindung entsprechend Formel (75) ist, insbesondere eine der oben aufgeführten Verbindungen entsprechend Formel (75), und dass die zweite ringförmige Verbindung ausgewählt ist aus der Gruppe enthaltend Verbindungen entsprechend einer der Formeln (1), (2), (3), (4), (5), (6), (7), (8), (9), (10), (11), (12) insbesondere eine der oben aufgeführten Verbindungen.

In einer weiteren Ausführungsvariante ist es denkbar, dass die erste ringförmige Verbindung eine Verbindung entsprechend Formel (76) ist, insbesondere eine der oben aufgeführten Verbindungen entsprechend Formel (76), und dass die zweite ringförmige Verbindung ausgewählt ist aus der Gruppe enthaltend Verbindungen entsprechend einer der Formeln (1), (2), (3), (4), (5), (6), (7), (8), (9), (10), (11), (12) insbesondere eine der oben aufgeführten Verbindungen.

In einer weiteren Ausführungsvariante ist es denkbar, dass die erste ringförmige Verbindung eine Verbindung entsprechend Formel (77) ist, insbesondere eine der oben aufgeführten Verbindungen entsprechend Formel (77), und dass die zweite ringförmige Verbindung ausgewählt ist aus der Gruppe enthaltend Verbindungen entsprechend einer der Formeln (1), (2), (3), (4), (5), (6), (7), (8), (9), (10), (11), (12) insbesondere eine der oben aufgeführten Verbindungen.

In einer weiteren Ausführungsvariante ist es denkbar, dass die erste ringförmige Verbindung eine Verbindung entsprechend Formel (78) ist, insbesondere eine der oben aufgeführten Verbindungen entsprechend Formel (78), und dass die zweite ringförmige Verbindung ausgewählt ist aus der Gruppe enthaltend Verbindungen entsprechend einer der Formeln (1), (2), (3), (4), (5), (6), (7), (8), (9), (10), (11), (12) insbesondere eine der oben aufgeführten Verbindungen.

In einer weiteren Ausführungsvariante ist es denkbar, dass die erste ringförmige Verbindung eine Verbindung entsprechend Formel (79) ist, insbesondere eine der oben aufgeführten Verbindungen entsprechend Formel (79), und dass die zweite ringförmige Verbindung ausgewählt ist aus der Gruppe enthaltend Verbindungen entsprechend einer der Formeln (1), (2), (3), (4), (5), (6), (7), (8), (9), (10), (11), (12) insbesondere eine der oben aufgeführten Verbindungen.

In einer weiteren Ausführungsvariante ist es denkbar, dass die erste ringförmige Verbindung eine Verbindung entsprechend Formel (80) ist, insbesondere eine der oben aufgeführten Verbindungen entsprechend Formel (80), und dass die zweite ringförmige Verbindung ausgewählt ist aus der Gruppe enthaltend Verbindungen entsprechend einer der Formeln (1), (2), (3), (4), (5), (6), (7), (8), (9), (10), (11), (12) insbesondere eine der oben aufgeführten Verbindungen.

In einer weiteren Ausführungsvariante ist es denkbar, dass die erste ringförmige Verbindung eine Verbindung entsprechend Formel (81) ist, insbesondere eine der oben aufgeführten Verbindungen entsprechend Formel (81), und dass die zweite ringförmige Verbindung ausgewählt ist aus der Gruppe enthaltend Verbindungen entsprechend einer der Formeln (1), (2), (3), (4), (5), (6), (7), (8), (9), (10), (11), (12) insbesondere eine der oben aufgeführten Verbindungen.

Mit anderen Worten, bei einem Elektrolyt, der eine erste ringförmige Verbindung entsprechend Formel (I), eine zweite ringförmige Verbindung entsprechend Formel (I), wenigstens 5 Gewichtsprozent Wasser und optional eine oder mehrere weitere Komponenten wie oben beschrieben aufweist, kann die erste ringförmige Verbindung eine Pyrrol-Verbindung entsprechend Formel (IX) sein und die zweite ringförmige Verbindung eine Piperidin-Verbindung entsprechend Formel (VII) sein.

Man erkennt insofern Folgendes: Ist die erste ringförmige Verbindung eine Pyrrol-Verbindung und die zweite ringförmige Verbindung eine Piperidin-Verbindung oder umgekehrt, so sind beispielsweise, aber nicht abschließend, folgende Ausführungsvarianten denkbar.

In einer ersten Ausführungsvariante ist es denkbar, dass die erste ringförmige Verbindung eine Verbindung entsprechend Formel (1) ist, insbesondere eine der oben aufgeführten Verbindungen entsprechend Formel (1), und dass die zweite ringförmige Verbindung ausgewählt ist aus der Gruppe enthaltend Verbindungen entsprechend einer der Formeln (47), (48), (49), (50), (51), (52), (53), (54), (55), (56), (57), (58) insbesondere eine der oben aufgeführten Verbindungen.

In einer weiteren Ausführungsvariante ist es denkbar, dass die erste ringförmige Verbindung eine Verbindung entsprechend Formel (2) ist, insbesondere eine der oben aufgeführten Verbindungen entsprechend Formel (2), und dass die zweite ringförmige Verbindung ausgewählt ist aus der Gruppe enthaltend Verbindungen entsprechend einer der Formeln (47), (48), (49), (50), (51), (52), (53), (54), (55), (56), (57), (58) insbesondere eine der oben aufgeführten Verbindungen.

In einer weiteren Ausführungsvariante ist es denkbar, dass die erste ringförmige Verbindung eine Verbindung entsprechend Formel (3) ist, insbesondere eine der oben aufgeführten Verbindungen entsprechend Formel (3), und dass die zweite ringförmige Verbindung ausgewählt ist aus der Gruppe enthaltend Verbindungen entsprechend einer der Formeln (47), (48), (49), (50), (51), (52), (53), (54), (55), (56), (57), (58) insbesondere eine der oben aufgeführten Verbindungen.

In einer weiteren Ausführungsvariante ist es denkbar, dass die erste ringförmige Verbindung eine Verbindung entsprechend Formel (4) ist, insbesondere eine der oben aufgeführten Verbindungen entsprechend Formel (4), und dass die zweite ringförmige Verbindung ausgewählt ist aus der Gruppe enthaltend Verbindungen entsprechend einer der Formeln (47), (48), (49), (50), (51), (52), (53), (54), (55), (56), (57), (58) insbesondere eine der oben aufgeführten Verbindungen.

In einer weiteren Ausführungsvariante ist es denkbar, dass die erste ringförmige Verbindung eine Verbindung entsprechend Formel (5) ist, insbesondere eine der oben aufgeführten Verbindungen entsprechend Formel (5), und dass die zweite ringförmige Verbindung ausgewählt ist aus der Gruppe enthaltend Verbindungen entsprechend einer der Formeln (47), (48), (49), (50), (51), (52), (53), (54), (55), (56), (57), (58) insbesondere eine der oben aufgeführten Verbindungen.

In einer weiteren Ausführungsvariante ist es denkbar, dass die erste ringförmige Verbindung eine Verbindung entsprechend Formel (6) ist, insbesondere eine der oben aufgeführten Verbindungen entsprechend Formel (6), und dass die zweite ringförmige Verbindung ausgewählt ist aus der Gruppe enthaltend Verbindungen entsprechend einer der Formeln (47), (48), (49), (50), (51), (52), (53), (54), (55), (56), (57), (58) insbesondere eine der oben aufgeführten Verbindungen.

In einer weiteren Ausführungsvariante ist es denkbar, dass die erste ringförmige Verbindung eine Verbindung entsprechend Formel (7) ist, insbesondere eine der oben aufgeführten Verbindungen entsprechend Formel (7), und dass die zweite ringförmige Verbindung ausgewählt ist aus der Gruppe enthaltend Verbindungen entsprechend einer der Formeln (47), (48), (49), (50), (51), (52), (53), (54), (55), (56), (57), (58) insbesondere eine der oben aufgeführten Verbindungen.

In einer weiteren Ausführungsvariante ist es denkbar, dass die erste ringförmige Verbindung eine Verbindung entsprechend Formel (8) ist, insbesondere eine der oben aufgeführten Verbindungen entsprechend Formel (8), und dass die zweite ringförmige Verbindung ausgewählt ist aus der Gruppe enthaltend Verbindungen entsprechend einer der Formeln (47), (48), (49), (50), (51), (52), (53), (54), (55), (56), (57), (58) insbesondere eine der oben aufgeführten Verbindungen.

In einer weiteren Ausführungsvariante ist es denkbar, dass die erste ringförmige Verbindung eine Verbindung entsprechend Formel (9) ist, insbesondere eine der oben aufgeführten Verbindungen entsprechend Formel (9), und dass die zweite ringförmige Verbindung ausgewählt ist aus der Gruppe enthaltend Verbindungen entsprechend einer der Formeln (47), (48), (49), (50), (51), (52), (53), (54), (55), (56), (57), (58) insbesondere eine der oben aufgeführten Verbindungen.

In einer weiteren Ausführungsvariante ist es denkbar, dass die erste ringförmige Verbindung eine Verbindung entsprechend Formel (10) ist, insbesondere eine der oben aufgeführten Verbindungen entsprechend Formel (10), und dass die zweite ringförmige Verbindung ausgewählt ist aus der Gruppe enthaltend Verbindungen entsprechend einer der Formeln (47), (48), (49), (50), (51), (52), (53), (54), (55), (56), (57), (58) insbesondere eine der oben aufgeführten Verbindungen.

In einer weiteren Ausführungsvariante ist es denkbar, dass die erste ringförmige Verbindung eine Verbindung entsprechend Formel (11) ist, insbesondere eine der oben aufgeführten Verbindungen entsprechend Formel (11), und dass die zweite ringförmige Verbindung ausgewählt ist aus der Gruppe enthaltend Verbindungen entsprechend einer der Formeln (47), (48), (49), (50), (51), (52), (53), (54), (55), (56), (57), (58) insbesondere eine der oben aufgeführten Verbindungen.

In einer weiteren Ausführungsvariante ist es denkbar, dass die erste ringförmige Verbindung eine Verbindung entsprechend Formel (12) ist, insbesondere eine der oben aufgeführten Verbindungen entsprechend Formel (12), und dass die zweite ringförmige Verbindung ausgewählt ist aus der Gruppe enthaltend Verbindungen entsprechend einer der Formeln (47), (48), (49), (50), (51), (52), (53), (54), (55), (56), (57), (58) insbesondere eine der oben aufgeführten Verbindungen.

In einer weiteren Ausführungsvariante ist es denkbar, dass die erste ringförmige Verbindung eine Verbindung entsprechend Formel (47) ist, insbesondere eine der oben aufgeführten Verbindungen entsprechend Formel (47), und dass die zweite ringförmige Verbindung ausgewählt ist aus der Gruppe enthaltend Verbindungen entsprechend einer der Formeln (1), (2), (3), (4), (5), (6), (7), (8), (9), (10), (11), (12) insbesondere eine der oben aufgeführten Verbindungen.

In einer weiteren Ausführungsvariante ist es denkbar, dass die erste ringförmige Verbindung eine Verbindung entsprechend Formel (48) ist, insbesondere eine der oben aufgeführten Verbindungen entsprechend Formel (48), und dass die zweite ringförmige Verbindung ausgewählt ist aus der Gruppe enthaltend Verbindungen entsprechend einer der Formeln (1), (2), (3), (4), (5), (6), (7), (8), (9), (10), (11), (12) insbesondere eine der oben aufgeführten Verbindungen.

In einer weiteren Ausführungsvariante ist es denkbar, dass die erste ringförmige Verbindung eine Verbindung entsprechend Formel (49) ist, insbesondere eine der oben aufgeführten Verbindungen entsprechend Formel (49), und dass die zweite ringförmige Verbindung ausgewählt ist aus der Gruppe enthaltend Verbindungen entsprechend einer der Formeln (1), (2), (3), (4), (5), (6), (7), (8), (9), (10), (11), (12) insbesondere eine der oben aufgeführten Verbindungen.

In einer weiteren Ausführungsvariante ist es denkbar, dass die erste ringförmige Verbindung eine Verbindung entsprechend Formel (50) ist, insbesondere eine der oben aufgeführten Verbindungen entsprechend Formel (50), und dass die zweite ringförmige Verbindung ausgewählt ist aus der Gruppe enthaltend Verbindungen entsprechend einer der Formeln (1), (2), (3), (4), (5), (6), (7), (8), (9), (10), (11), (12) insbesondere eine der oben aufgeführten Verbindungen.

In einer weiteren Ausführungsvariante ist es denkbar, dass die erste ringförmige Verbindung eine Verbindung entsprechend Formel (51) ist, insbesondere eine der oben aufgeführten Verbindungen entsprechend Formel (51), und dass die zweite ringförmige Verbindung ausgewählt ist aus der Gruppe enthaltend Verbindungen entsprechend einer der Formeln (1), (2), (3), (4), (5), (6), (7), (8), (9), (10), (11), (12) insbesondere eine der oben aufgeführten Verbindungen.

In einer weiteren Ausführungsvariante ist es denkbar, dass die erste ringförmige Verbindung eine Verbindung entsprechend Formel (52) ist, insbesondere eine der oben aufgeführten Verbindungen entsprechend Formel (52), und dass die zweite ringförmige Verbindung ausgewählt ist aus der Gruppe enthaltend Verbindungen entsprechend einer der Formeln (1), (2), (3), (4), (5), (6), (7), (8), (9), (10), (11), (12) insbesondere eine der oben aufgeführten Verbindungen.

In einer weiteren Ausführungsvariante ist es denkbar, dass die erste ringförmige Verbindung eine Verbindung entsprechend Formel (53) ist, insbesondere eine der oben aufgeführten Verbindungen entsprechend Formel (53), und dass die zweite ringförmige Verbindung ausgewählt ist aus der Gruppe enthaltend Verbindungen entsprechend einer der Formeln (1), (2), (3), (4), (5), (6), (7), (8), (9), (10), (11), (12) insbesondere eine der oben aufgeführten Verbindungen.

In einer weiteren Ausführungsvariante ist es denkbar, dass die erste ringförmige Verbindung eine Verbindung entsprechend Formel (54) ist, insbesondere eine der oben aufgeführten Verbindungen entsprechend Formel (54), und dass die zweite ringförmige Verbindung ausgewählt ist aus der Gruppe enthaltend Verbindungen entsprechend einer der Formeln (1), (2), (3), (4), (5), (6), (7), (8), (9), (10), (11), (12) insbesondere eine der oben aufgeführten Verbindungen.

In einer weiteren Ausführungsvariante ist es denkbar, dass die erste ringförmige Verbindung eine Verbindung entsprechend Formel (55) ist, insbesondere eine der oben aufgeführten Verbindungen entsprechend Formel (55), und dass die zweite ringförmige Verbindung ausgewählt ist aus der Gruppe enthaltend Verbindungen entsprechend einer der Formeln (1), (2), (3), (4), (5), (6), (7), (8), (9), (10), (11), (12) insbesondere eine der oben aufgeführten Verbindungen.

In einer weiteren Ausführungsvariante ist es denkbar, dass die erste ringförmige Verbindung eine Verbindung entsprechend Formel (56) ist, insbesondere eine der oben aufgeführten Verbindungen entsprechend Formel (56), und dass die zweite ringförmige Verbindung ausgewählt ist aus der Gruppe enthaltend Verbindungen entsprechend einer der Formeln (1), (2), (3), (4), (5), (6), (7), (8), (9), (10), (11), (12) insbesondere eine der oben aufgeführten Verbindungen.

In einer weiteren Ausführungsvariante ist es denkbar, dass die erste ringförmige Verbindung eine Verbindung entsprechend Formel (57) ist, insbesondere eine der oben aufgeführten Verbindungen entsprechend Formel (57), und dass die zweite ringförmige Verbindung ausgewählt ist aus der Gruppe enthaltend Verbindungen entsprechend einer der Formeln (1), (2), (3), (4), (5), (6), (7), (8), (9), (10), (11), (12) insbesondere eine der oben aufgeführten Verbindungen.

In einer weiteren Ausführungsvariante ist es denkbar, dass die erste ringförmige Verbindung eine Verbindung entsprechend Formel (58) ist, insbesondere eine der oben aufgeführten Verbindungen entsprechend Formel (58), und dass die zweite ringförmige Verbindung ausgewählt ist aus der Gruppe enthaltend Verbindungen entsprechend einer der Formeln (1), (2), (3), (4), (5), (6), (7), (8), (9), (10), (11), (12) insbesondere eine der oben aufgeführten Verbindungen.

Unabhängig von der Auswahl der ringförmigen Verbindungen entsprechend Formel (I) hat es sich gezeigt, dass es vorteilhaft ist, wenn die erste ringförmige Verbindung und die zweite ringförmige Verbindung in einem Verhältnis von 1:1 bis 9:1 zueinander vorliegen.

Es versteht sich von selbst, dass das Verhältnis zwischen der ersten und zweiten ringförmigen Verbindung dabei nicht auf die exakten Werte beschränkt ist, sondern innerhalb gewisser Toleranzgrenzen liegt. So ist denkbar, dass die erste ringförmige Verbindung und die zweite ringförmige Verbindung im Verhältnis von 1:1 bis 1:9 oder in einem der nachfolgend aufgeführten Bereiche jeweils innerhalb einer Toleranzgrenze von ±5% bis ±10% vorliegen.

Beispielsweise ist es denkbar, dass die erste ringförmige Verbindung und die zweite ringförmige Verbindung in einem Verhältnis von 1:1 bis 1:9, 1:1,5 bis 1:9, 1:2 bis 1:9, 1:2,5 bis 1:9, 1:3 bis 1:9, 1:3,5 bis 1:9, 1:4 bis 1:9, 1:4,5 bis 1:9, 1:5 bis 1:9, 1:5,5 bis 1:9, 1:6 bis 1:9, 1:6,5 bis 1:9, 1:7: bis 1:9, 1:7,5 bis 1:9, 1:8 bis 1:9, oder 1:8,5 bis 1:9 vorliegen.

Selbstverständlich ist auch denkbar dass die erste ringförmige Verbindung und die zweite ringförmige Verbindung in einem Verhältnis von 1:1 bis 1:8,5, 1:1,5 bis 1:8,5, 1:2 bis 1: 8,5, 1:2,5 bis 1: 8,5, 1:3 bis 1: 8,5, 1:3,5 bis 1: 8,5, 1:4 bis 1: 8,5, 1:4,5 bis 1: 8,5, 1:5 bis 1:8,5, 1:5,5 bis 1:8,5, 1:6 bis 1:8,5, 1:6,5 bis 1:8,5, 1:7: bis 1:8,5, 1:7,5 bis 1:8,5, 1:8 bis 1:8,5, 1:1 bis 1:8, 1:1,5 bis 1:8, 1:2 bis 1:8, 1:2,5 bis 1:8, 1:3 bis 1:8, 1:3,5 bis 1:8, 1:4 bis 1:8, 1:4,5 bis 1:8, 1:5 bis 1:8, 1:5,5 bis 1:8, 1:6 bis 1:8, 1:6,5 bis 1:8, 1:7: bis 1:8, 1:7,5 bis 1:8, 1:1 bis 1:7,5, 1:1,5 bis 1:7,5, 1:2 bis 1:7,5, 1:2,5 bis 1:7,5, 1:3 bis 1:7,5, 1:3,5 bis 1:7,5, 1:4 bis 1:7,5, 1:4,5 bis 1:7,5, 1:5 bis 1:7,5, 1:5,5 bis 1:7,5, 1:6 bis 1:7,5, 1:6,5 bis 1:7,5, 1:7: bis 1:7,5, 1:1 bis 1:7, 1:1,5 bis 1:7, 1:2 bis 1:7, 1:2,5 bis 1:7, 1:3 bis 1:7, 1:3,5 bis 1:7, 1:4 bis 1:7, 1:4,5 bis 1:7, 1:5 bis 1:7, 1:5,5 bis 1:7, 1:6 bis 1:7, 1:6,5 bis 1:7, 1:1 bis 1:6,5, 1:1,5 bis 1:6,5, 1:2 bis 1:6,5, 1:2,5 bis 1:6,5, 1:3 bis 1:6,5, 1:3,5 bis 1:6,5, 1:4 bis 1:6,5, 1:4,5 bis 1:6,5, 1:5 bis 1:6,5, 1:5,5 bis 1:6,5, 1:6 bis 1:6,5, 1:1 bis 1:6, 1:1,5 bis 1:6, 1:2 bis 1:6, 1:2,5 bis 1:6, 1:3 bis 1:6, 1:3,5 bis 1:6, 1:4 bis 1:6, 1:4,5 bis 1:6, 1:5 bis 1:6, 1:5,5 bis 1:6, 1:1 bis 1:5,5, 1:1,5 bis 1:5,5, 1:2 bis 1:5,5, 1:2,5 bis 1:5,5, 1:3 bis 1:5,5, 1:3,5 bis 1:5,5, 1:4 bis 1:5,5, 1:4,5 bis 1:5,5, 1:5 bis 1:5,5, 1:1 bis 1:5, 1:1,5 bis 1:5, 1:2 bis 1:5, 1:2,5 bis 1:5, 1:3 bis 1:5, 1:3,5 bis 1:5, 1:4 bis 1:5, 1:4,5 bis 1:5, 1:1 bis 1:4,5, 1:1,5 bis 1:4,5, 1:2 bis 1:4,5, 1:2,5 bis 1:4,5, 1:3 bis 1:4,5, 1:3,5 bis 1:4,5, 1:4 bis 1:4,5, 1:1 bis 1:4, 1:1,5 bis 1:4, 1:2 bis 1:4, 1:2,5 bis 1:4, 1:3 bis 1:4, 1:3,5 bis 1:4, 1:1 bis 1:3,5, 1:1,5 bis 1:3,5, 1:2 bis 1:3,5, 1:2,5 bis 1:3,5, 1:3 bis 1:3,5, 1:1 bis 1:3, 1:1,5 bis 1:3, 1:2 bis 1:3, 1:2,5 bis 1:3, 1:1 bis 1:2,5, 1:1,5 bis 1:2,5, 1:2 bis 1:2,5, 1:1 bis 1:2, 1:1,5 bis 1:2 vorliegen.

In einer weiterführenden Ausführungsvariante ist es auch vorstellbar, dass der Elektrolyt mehr als zwei verschiedene ringförmige Verbindungen aufweist. Der Elektrolyt kann beispielsweise drei, vier oder fünf voneinander verschiedene ringförmige Verbindungen aufweisen. Dabei kann jede der ringförmigen Verbindungen aus den oben beschriebenen Verbindungen ausgewählt sein. Günstig ist es dabei, wenn R¹ H oder CH3 ist und wenn Y ausgewählt ist aus Cl, F oder CH3SO3. Bevorzugt ist es auch, wenn die Ringstruktur eine Pyridin oder Piperidin-Struktur ist. Denkbar ist auch hier, dass die ringförmigen Verbindungen entweder den gleichen Rest R¹ aufweisen oder unterschiedliche Reste R¹. Auch ist - wie bei einem Elektrolyten mit zwei ringförmigen Verbindungen - sowohl vorstellbar, dass die ringförmigen Verbindungen das gleiche Anion Y aufweisen. Alternativ ist auch hier denkbar, dass die ringförmigen Verbindungen unterschiedliche Anionen Y aufweisen. Im Übrigen gilt auch für einen Elektrolyten mit mehr als zwei ringförmigen Verbindungen und einem elektrochemischen Gassensor mit einem solchen Elektrolyten explizit das bereits oben ausgeführte, insbesondere in Bezug auf den möglichen Wasseranteil, die Wahl der Elektroden und alle weiteren oben beschriebenen Merkmale. Beispielsweise ist es auch hier denkbar, dass alle ringförmigen Verbindungen zu gleichen Teilen vorliegen.

Man erkennt weiter, dass es besonders günstig ist, wenn das Halogenid, das Y bildet, ausgewählt ist aus Cl, Br, F oder I, bevorzugt aus Cl oder F. Der Cyanid-Rest, der Y bilden kann, ist in einem bevorzugten Ausführungsbeispiel CN. Ein Beispiel für ein erfindungsgemäßes Alkylsulfonat, welches Y bilden kann ist Methylsulfonat. Ein Beispiel für ein halogeniertes Alkylsulfonat ist Triflat. Man erkennt insofern, dass es günstig ist wenn Y ausgewählt ist aus Cl, Br, F, I, CN, CH₃SO₃. Besonders bevorzugt ist es dabei wenn Y ausgewählt ist aus Cl, F, MeSO₃.

Enthält der Elektrolyt mehr als eine ringförmige Verbindung entsprechend der Formel (I), so ist denkbar, dass Y für alle ringförmige Verbindungen des Elektrolyten gleich ist. Alternativ ist auch vorstellbar, dass Y für beide ringförmigen Verbindungen des Elektrolyten unterschiedlich ist.

In jedem Fall hat es sich als vorteilhaft herausgestellt, wenn der Elektrolyt zwischen wenigstens 5 und höchsten 50 Gewichtsprozent H₂O, zwischen wenigstens 5 und höchstens 40 Gewichtsprozent, zwischen wenigstens 5 und höchstens 30 Gewichtsprozent H₂O enthält oder zwischen wenigstens 5 und höchstens 20 Gewichtsprozent H₂O enthält. Im Sinne der vorliegenden Anmeldung schließen dabei die Formulierungen wenigstens und höchstens jeweils den Wert auf den sie sich beziehen ein. Unter wenigstens 5 Gewichtsprozent wird mithin 5 Gewichtsprozent oder mehr verstanden. Unter höchstens 50 Gewichtsprozent wird mithin 50 Gewichtsprozent oder weniger verstanden. Darüber hinaus versteht es sich von selbst, dass die angegebenen Werte rein exemplarisch sind und in gewissen Grenzen, beispielsweise ±0,25, ±0,5 oder ±1 Gewichtsprozent, schwanken können. In diesem Sinne wird im Folgenden insofern bspw. die Schreibweise "20±0,25 Gewichtsprozent" verstanden als "20 Gewichtsprozent mit einer Schwankung von 0,25 Gewichtsprozent nach oben oder nach unten".

Insofern erkennt man, dass es vorteilhaft ist, wenn der Elektrolyt wenigstens 5±0,25 Gewichtsprozent Wasser, wenigstens 5,5±0,25 Gewichtsprozent Wasser, wenigstens 6±0,25 Gewichtsprozent Wasser, wenigstens 6,5±0,25 Gewichtsprozent Wasser, wenigstens 7±0,25 Gewichtsprozent Wasser, wenigstens 7,5±0,25 Gewichtsprozent Wasser, wenigstens 8±0,25 Gewichtsprozent Wasser, wenigstens 8,5±0,25 Gewichtsprozent Wasser, wenigstens 9±0,25 Gewichtsprozent Wasser, wenigstens 9,5±0,25 Gewichtsprozent Wasser oder wenigstens 10±0,25 Gewichtsprozent Wasser enthält.

Man erkennt weiter, dass es vorteilhaft ist, wenn der Elektrolyt höchstens 50±1 Gewichtsprozent Wasser, höchstens 47,5±1 Gewichtsprozent Wasser, höchstens 45±1 Gewichtsprozent Wasser, höchstens 42,5±1 Gewichtsprozent Wasser, höchstens 40±1 Gewichtsprozent Wasser, höchstens 37,5±1 Gewichtsprozent Wasser, höchstens 35±1 Gewichtsprozent Wasser, höchstens 32,5±1 Gewichtsprozent Wasser, höchstens 30±1 Gewichtsprozent Wasser, höchstens 27,5±1 Gewichtsprozent Wasser, höchstens 25±1 Gewichtsprozent Wasser, höchstens 22,5±1 Gewichtsprozent Wasser oder höchstens 20±1 Gewichtsprozent Wasser enthält.

Insgesamt ist es mithin günstig, wenn der Elektrolyt wenigstens 5±0,25 Gewichtsprozent Wasser und höchstens 50±1 Gewichtsprozent Wasser, wenigstens 5±0,25 Gewichtsprozent Wasser und höchstens 47,5±1 Gewichtsprozent Wasser, wenigstens 5±0,25 Gewichtsprozent Wasser und höchstens 45±1 Gewichtsprozent Wasser, wenigstens 5±0,25 Gewichtsprozent Wasser und höchstens 45±1 Gewichtsprozent Wasser, wenigstens 5±0,25 Gewichtsprozent Wasser und höchstens 40±1 Gewichtsprozent Wasser, wenigstens 5±0,25 Gewichtsprozent Wasser und höchstens 37,5±1 Gewichtsprozent Wasser, wenigstens 5±0,25 Gewichtsprozent Wasser und höchstens 35±1 Gewichtsprozent Wasser, wenigstens 5±0,25 Gewichtsprozent Wasser und höchstens 35±1 Gewichtsprozent Wasser, wenigstens 5±0,25 Gewichtsprozent Wasser und höchstens 30±1 Gewichtsprozent Wasser, wenigstens 5±0,25 Gewichtsprozent Wasser und höchstens 27,5±1 Gewichtsprozent Wasser, wenigstens 5±0,25 Gewichtsprozent Wasser und höchstens 25±1 Gewichtsprozent Wasser, wenigstens 5±0,25 Gewichtsprozent Wasser und höchstens 25±1 Gewichtsprozent Wasser oder wenigstens 5±0,25 Gewichtsprozent Wasser und höchstens 20±1 Gewichtsprozent Wasser, wenigstens 5,5±0,25 Gewichtsprozent Wasser und höchstens 50±1 Gewichtsprozent Wasser, wenigstens 5,5±0,25 Gewichtsprozent Wasser und höchstens 47,5±1 Gewichtsprozent Wasser, wenigstens 5,5±0,25 Gewichtsprozent Wasser und höchstens 45±1 Gewichtsprozent Wasser, wenigstens 5,5±0,25 Gewichtsprozent Wasser und höchstens 45,5±1 Gewichtsprozent Wasser, wenigstens 5,5±0,25 Gewichtsprozent Wasser und höchstens 40±1 Gewichtsprozent Wasser, wenigstens 5,5±0,25 Gewichtsprozent Wasser und höchstens 37,5±1 Gewichtsprozent Wasser, wenigstens 5,5±0,25 Gewichtsprozent Wasser und höchstens 35±1 Gewichtsprozent Wasser, wenigstens 5,5±0,25 Gewichtsprozent Wasser und höchstens 35,5±1 Gewichtsprozent Wasser, wenigstens 5,5±0,25 Gewichtsprozent Wasser und höchstens 30±1 Gewichtsprozent Wasser, wenigstens 5,5±0,25 Gewichtsprozent Wasser und höchstens 27,5±1 Gewichtsprozent Wasser, wenigstens 5,5±0,25 Gewichtsprozent Wasser und höchstens 25±1 Gewichtsprozent Wasser, wenigstens 5,5±0,25 Gewichtsprozent Wasser und höchstens 25,5±1 Gewichtsprozent Wasser oder wenigstens 5,5±0,25 Gewichtsprozent Wasser und höchstens 20±1 Gewichtsprozent Wasser, wenigstens 6±0,25 Gewichtsprozent Wasser und höchstens 50±1 Gewichtsprozent Wasser, wenigstens 6±0,25 Gewichtsprozent Wasser und höchstens 47,5±1 Gewichtsprozent Wasser, wenigstens 6±0,25 Gewichtsprozent Wasser und höchstens 45±1 Gewichtsprozent Wasser, wenigstens 6±0,25 Gewichtsprozent Wasser und höchstens 46±1 Gewichtsprozent Wasser, wenigstens 6±0,25 Gewichtsprozent Wasser und höchstens 40±1 Gewichtsprozent Wasser, wenigstens 6±0,25 Gewichtsprozent Wasser und höchstens 37,5±1 Gewichtsprozent Wasser, wenigstens 6±0,25 Gewichtsprozent Wasser und höchstens 35±1 Gewichtsprozent Wasser, wenigstens 6±0,25 Gewichtsprozent Wasser und höchstens 36±1 Gewichtsprozent Wasser, wenigstens 6±0,25 Gewichtsprozent Wasser und höchstens 30±1 Gewichtsprozent Wasser, wenigstens 6±0,25 Gewichtsprozent Wasser und höchstens 27,5±1 Gewichtsprozent Wasser, wenigstens 6±0,25 Gewichtsprozent Wasser und höchstens 25±1 Gewichtsprozent Wasser, wenigstens 6±0,25 Gewichtsprozent Wasser und höchstens 26±1 Gewichtsprozent Wasser oder wenigstens 6±0,25 Gewichtsprozent Wasser und höchstens 20±1 Gewichtsprozent Wasser, wenigstens 6,5±0,25 Gewichtsprozent Wasser und höchstens 50±1 Gewichtsprozent Wasser, wenigstens 6,5±0,25 Gewichtsprozent Wasser und höchstens 47,5±1 Gewichtsprozent Wasser, wenigstens 6,5±0,25 Gewichtsprozent Wasser und höchstens 45±1 Gewichtsprozent Wasser, wenigstens 6,5±0,25 Gewichtsprozent Wasser und höchstens 46,5±1 Gewichtsprozent Wasser, wenigstens 6,5±0,25 Gewichtsprozent Wasser und höchstens 40±1 Gewichtsprozent Wasser, wenigstens 6,5±0,25 Gewichtsprozent Wasser und höchstens 37,5±1 Gewichtsprozent Wasser, wenigstens 6,5±0,25 Gewichtsprozent Wasser und höchstens 35±1 Gewichtsprozent Wasser, wenigstens 6,5±0,25 Gewichtsprozent Wasser und höchstens 36,5±1 Gewichtsprozent Wasser, wenigstens 6,5±0,25 Gewichtsprozent Wasser und höchstens 30±1 Gewichtsprozent Wasser, wenigstens 6,5±0,25 Gewichtsprozent Wasser und höchstens 27,5±1 Gewichtsprozent Wasser, wenigstens 6,5±0,25 Gewichtsprozent Wasser und höchstens 25±1 Gewichtsprozent Wasser, wenigstens 6,5±0,25 Gewichtsprozent Wasser und höchstens 26,5±1 Gewichtsprozent Wasser oder wenigstens 6,5±0,25 Gewichtsprozent Wasser und höchstens 20±1 Gewichtsprozent Wasser, wenigstens 7±0,25 Gewichtsprozent Wasser und höchstens 50±1 Gewichtsprozent Wasser, wenigstens 7±0,25 Gewichtsprozent Wasser und höchstens 47,5±1 Gewichtsprozent Wasser, wenigstens 7±0,25 Gewichtsprozent Wasser und höchstens 45±1 Gewichtsprozent Wasser, wenigstens 7±0,25 Gewichtsprozent Wasser und höchstens 47±1 Gewichtsprozent Wasser, wenigstens 7±0,25 Gewichtsprozent Wasser und höchstens 40±1 Gewichtsprozent Wasser, wenigstens 7±0,25 Gewichtsprozent Wasser und höchstens 37,5±1 Gewichtsprozent Wasser, wenigstens 7±0,25 Gewichtsprozent Wasser und höchstens 35±1 Gewichtsprozent Wasser, wenigstens 7±0,25 Gewichtsprozent Wasser und höchstens 37±1 Gewichtsprozent Wasser, wenigstens 7±0,25 Gewichtsprozent Wasser und höchstens 30±1 Gewichtsprozent Wasser, wenigstens 7±0,25 Gewichtsprozent Wasser und höchstens 27,5±1 Gewichtsprozent Wasser, wenigstens 7±0,25 Gewichtsprozent Wasser und höchstens 25±1 Gewichtsprozent Wasser, wenigstens 7±0,25 Gewichtsprozent Wasser und höchstens 27±1 Gewichtsprozent Wasser oder wenigstens 7±0,25 Gewichtsprozent Wasser und höchstens 20±1 Gewichtsprozent Wasser, wenigstens 7,5±0,25 Gewichtsprozent Wasser und höchstens 50±1 Gewichtsprozent Wasser, wenigstens 7,5±0,25 Gewichtsprozent Wasser und höchstens 47,5±1 Gewichtsprozent Wasser, wenigstens 7,5±0,25 Gewichtsprozent Wasser und höchstens 45±1 Gewichtsprozent Wasser, wenigstens 7,5±0,25 Gewichtsprozent Wasser und höchstens 47,5±1 Gewichtsprozent Wasser, wenigstens 7,5±0,25 Gewichtsprozent Wasser und höchstens 40±1 Gewichtsprozent Wasser, wenigstens 7,5±0,25 Gewichtsprozent Wasser und höchstens 37,5±1 Gewichtsprozent Wasser, wenigstens 7,5±0,25 Gewichtsprozent Wasser und höchstens 35±1 Gewichtsprozent Wasser, wenigstens 7,5±0,25 Gewichtsprozent Wasser und höchstens 37,5±1 Gewichtsprozent Wasser, wenigstens 7,5±0,25 Gewichtsprozent Wasser und höchstens 30±1 Gewichtsprozent Wasser, wenigstens 7,5±0,25 Gewichtsprozent Wasser und höchstens 27,5±1 Gewichtsprozent Wasser, wenigstens 7,5±0,25 Gewichtsprozent Wasser und höchstens 25±1 Gewichtsprozent Wasser, wenigstens 7,5±0,25 Gewichtsprozent Wasser und höchstens 27,5±1 Gewichtsprozent Wasser oder wenigstens 7,5±0,25 Gewichtsprozent Wasser und höchstens 20±1 Gewichtsprozent Wasser, wenigstens 8±0,25 Gewichtsprozent Wasser und höchstens 50±1 Gewichtsprozent Wasser, wenigstens 8±0,25 Gewichtsprozent Wasser und höchstens 47,5±1 Gewichtsprozent Wasser, wenigstens 8±0,25 Gewichtsprozent Wasser und höchstens 45±1 Gewichtsprozent Wasser, wenigstens 8±0,25 Gewichtsprozent Wasser und höchstens 48±1 Gewichtsprozent Wasser, wenigstens 8±0,25 Gewichtsprozent Wasser und höchstens 40±1 Gewichtsprozent Wasser, wenigstens 8±0,25 Gewichtsprozent Wasser und höchstens 37,5±1 Gewichtsprozent Wasser, wenigstens 8±0,25 Gewichtsprozent Wasser und höchstens 35±1 Gewichtsprozent Wasser, wenigstens 8±0,25 Gewichtsprozent Wasser und höchstens 38±1 Gewichtsprozent Wasser, wenigstens 8±0,25 Gewichtsprozent Wasser und höchstens 30±1 Gewichtsprozent Wasser, wenigstens 8±0,25 Gewichtsprozent Wasser und höchstens 27,5±1 Gewichtsprozent Wasser, wenigstens 8±0,25 Gewichtsprozent Wasser und höchstens 25±1 Gewichtsprozent Wasser, wenigstens 8±0,25 Gewichtsprozent Wasser und höchstens 28±1 Gewichtsprozent Wasser oder wenigstens 8±0,25 Gewichtsprozent Wasser und höchstens 20±1 Gewichtsprozent Wasser, wenigstens 8,5±0,25 Gewichtsprozent Wasser und höchstens 50±1 Gewichtsprozent Wasser, wenigstens 8,5±0,25 Gewichtsprozent Wasser und höchstens 47,5±1 Gewichtsprozent Wasser, wenigstens 8,5±0,25 Gewichtsprozent Wasser und höchstens 45±1 Gewichtsprozent Wasser, wenigstens 8,5±0,25 Gewichtsprozent Wasser und höchstens 48,5±1 Gewichtsprozent Wasser, wenigstens 8,5±0,25 Gewichtsprozent Wasser und höchstens 40±1 Gewichtsprozent Wasser, wenigstens 8,5±0,25 Gewichtsprozent Wasser und höchstens 37,5±1 Gewichtsprozent Wasser, wenigstens 8,5±0,25 Gewichtsprozent Wasser und höchstens 35±1 Gewichtsprozent Wasser, wenigstens 8,5±0,25 Gewichtsprozent Wasser und höchstens 38,5±1 Gewichtsprozent Wasser, wenigstens 8,5±0,25 Gewichtsprozent Wasser und höchstens 30±1 Gewichtsprozent Wasser, wenigstens 8,5±0,25 Gewichtsprozent Wasser und höchstens 27,5±1 Gewichtsprozent Wasser, wenigstens 8,5±0,25 Gewichtsprozent Wasser und höchstens 25±1 Gewichtsprozent Wasser, wenigstens 8,5±0,25 Gewichtsprozent Wasser und höchstens 28,5±1 Gewichtsprozent Wasser oder wenigstens 8,5±0,25 Gewichtsprozent Wasser und höchstens 20±1 Gewichtsprozent Wasser, wenigstens 9±0,25 Gewichtsprozent Wasser und höchstens 50±1 Gewichtsprozent Wasser, wenigstens 9±0,25 Gewichtsprozent Wasser und höchstens 47,5±1 Gewichtsprozent Wasser, wenigstens 9±0,25 Gewichtsprozent Wasser und höchstens 45±1 Gewichtsprozent Wasser, wenigstens 9±0,25 Gewichtsprozent Wasser und höchstens 49±1 Gewichtsprozent Wasser, wenigstens 9±0,25 Gewichtsprozent Wasser und höchstens 40±1 Gewichtsprozent Wasser, wenigstens 9±0,25 Gewichtsprozent Wasser und höchstens 37,5±1 Gewichtsprozent Wasser, wenigstens 9±0,25 Gewichtsprozent Wasser und höchstens 35±1 Gewichtsprozent Wasser, wenigstens 9±0,25 Gewichtsprozent Wasser und höchstens 39±1 Gewichtsprozent Wasser, wenigstens 9±0,25 Gewichtsprozent Wasser und höchstens 30±1 Gewichtsprozent Wasser, wenigstens 9±0,25 Gewichtsprozent Wasser und höchstens 27,5±1 Gewichtsprozent Wasser, wenigstens 9±0,25 Gewichtsprozent Wasser und höchstens 25±1 Gewichtsprozent Wasser, wenigstens 9±0,25 Gewichtsprozent Wasser und höchstens 29±1 Gewichtsprozent Wasser oder wenigstens 9±0,25 Gewichtsprozent Wasser und höchstens 20±1 Gewichtsprozent Wasser, wenigstens 9,5±0,25 Gewichtsprozent Wasser und höchstens 50±1 Gewichtsprozent Wasser, wenigstens 9,5±0,25 Gewichtsprozent Wasser und höchstens 47,5±1 Gewichtsprozent Wasser, wenigstens 9,5±0,25 Gewichtsprozent Wasser und höchstens 45±1 Gewichtsprozent Wasser, wenigstens 9,5±0,25 Gewichtsprozent Wasser und höchstens 49,5±1 Gewichtsprozent Wasser, wenigstens 9,5±0,25 Gewichtsprozent Wasser und höchstens 40±1 Gewichtsprozent Wasser, wenigstens 9,5±0,25 Gewichtsprozent Wasser und höchstens 37,5±1 Gewichtsprozent Wasser, wenigstens 9,5±0,25 Gewichtsprozent Wasser und höchstens 35±1 Gewichtsprozent Wasser, wenigstens 9,5±0,25 Gewichtsprozent Wasser und höchstens 39,5±1 Gewichtsprozent Wasser, wenigstens 9,5±0,25 Gewichtsprozent Wasser und höchstens 30±1 Gewichtsprozent Wasser, wenigstens 9,5±0,25 Gewichtsprozent Wasser und höchstens 27,5±1 Gewichtsprozent Wasser, wenigstens 9,5±0,25 Gewichtsprozent Wasser und höchstens 25±1 Gewichtsprozent Wasser, wenigstens 9,5±0,25 Gewichtsprozent Wasser und höchstens 29,5±1 Gewichtsprozent Wasser oder wenigstens 9,5±0,25 Gewichtsprozent Wasser und höchstens 20±1 Gewichtsprozent Wasser, wenigstens 10±0,25 Gewichtsprozent Wasser und höchstens 50±1 Gewichtsprozent Wasser, wenigstens 10±0,25 Gewichtsprozent Wasser und höchstens 47,5±1 Gewichtsprozent Wasser, wenigstens 10±0,25 Gewichtsprozent Wasser und höchstens 45±1 Gewichtsprozent Wasser, wenigstens 10±0,25 Gewichtsprozent Wasser und höchstens 410±1 Gewichtsprozent Wasser, wenigstens 10±0,25 Gewichtsprozent Wasser und höchstens 40±1 Gewichtsprozent Wasser, wenigstens 10±0,25 Gewichtsprozent Wasser und höchstens 37,5±1 Gewichtsprozent Wasser, wenigstens 10±0,25 Gewichtsprozent Wasser und höchstens 35±1 Gewichtsprozent Wasser, wenigstens 10±0,25 Gewichtsprozent Wasser und höchstens 310±1 Gewichtsprozent Wasser, wenigstens 10±0,25 Gewichtsprozent Wasser und höchstens 30±1 Gewichtsprozent Wasser, wenigstens 10±0,25 Gewichtsprozent Wasser und höchstens 27,5±1 Gewichtsprozent Wasser, wenigstens 10±0,25 Gewichtsprozent Wasser und höchstens 25±1 Gewichtsprozent Wasser, wenigstens 10±0,25 Gewichtsprozent Wasser und höchstens 210±1 Gewichtsprozent Wasser oder wenigstens 10±0,25 Gewichtsprozent Wasser und höchstens 20±1 Gewichtsprozent Wasser enthält.

In einem weiteren Aspekt betrifft die Erfindung eine Elektrochemischen Gassensor mit einem wie oben beschriebenen Elektrolyten. Mit anderen Worten, die Erfindung betrifft einen elektrochemischen Gassensor mit einer ersten Elektrode, wenigstens einer zweiten Elektrode und mit einem Elektrolyten entsprechend dem oben Ausgeführten.

Dabei kann es, unabhängig von der Wahl der ringförmigen Verbindungen ist es in einem weiteren Ausführungsbeispiel von Vorteil sein, wenn die erste und/oder die zweite Elektrode aus einem Edelmetall oder einer Edelmetall-Legierung besteht. Beispielsweise ist vorstellbar, dass Ir, Pt, Pd oder eine Legierung mit einem dieser Stoffe als Elektrodenmaterial verwendet werden kann. Ein erfindungsgemäßer Gassensor mit einer solchen Elektrode als Messelektrode kann insbesondere zum Nachweis von SO₂, NO₂, NO, O₃ und/oder H₂S verwendet werden.

In einem weiteren Ausführungsbeispiel ist es denkbar, dass die erste und/oder die zweite Elektrode aus einem Kohlenstoffmaterial besteht. Insbesondere stellt sich, überraschenderweise, die Verwendung von CNT, DLC oder Graphen-Elektroden als vorteilhaft heraus. In jedem Fall ist es möglich durch die Wahl des Elektrodenmaterials aus der Gruppe Ir, Pt, Pd, CNT, DLC oder Graphen-Elektroden gemeinsam mit dem hierin beschriebenen erfindungsgemäßen Elektrolyten eine deutlich verbesserte Empfindlichkeit und ein deutlich reduziertes Rauschen des Messsignales zu erreichen. Wobei der Gassensor mit dem hierin beschriebenen Elektrolyten auch bei Verwendung einer Kohlenstoffelektrode zum Nachweis von SO₂, NO₂, O₃ und/oder H₂S verwendet werden kann.

Weitere Merkmale, Einzelheiten und Vorteile der Erfindung ergeben sich aus dem Wortlaut der Ansprüche, sowie aus der folgenden Beschreibung der Zeichnungen, sowie aus den nachfolgend beschriebenen Ausführungsbeispielen. Es versteht sich von selbst, dass diese nur exemplarisch dazu dienen, die Ausführung der Erfindung zu erläutern und dass die Erfindung entsprechend den Ansprüchen keineswegs auf die hier gezeigten Beispiele beschränkt ist. Es zeigen:
- Fig. 1: Schematische Darstellung eines erfindungsgemäßen Elektrochemischen Gassensors.
- Fig. 2: Messkurve zum Beispiel 1, wobei auf der x-Achse die Zeit und auf der y-Achse die gemessene Signalstärke in µA dargestellt ist. Zum Zeitpunkt t1 wurde begonnen, den Sensor mit dem Messgas zu begasen. Zum Zeitpunkt t2 wurde die Begasung beendet.
- Fig. 3: Messkurve zum Beispiel 2, wobei auf der x-Achse die Zeit und auf der y-Achse die gemessene Signalstärke in µA dargestellt ist. Zum Zeitpunkt t1 wurde begonnen, den Sensor mit dem Messgas zu begasen. Zum Zeitpunkt t2 wurde die Begasung beendet.
- Fig. 4: Messkurve zum Beispiel 3, wobei auf der x-Achse die Zeit und auf der y-Achse die gemessene Signalstärke in µA dargestellt ist. Zum Zeitpunkt t1 wurde begonnen, den Sensor mit dem Messgas zu begasen. Zum Zeitpunkt t2 wurde die Begasung beendet.
- Fig. 5a bis 5d: Die Figuren 5a, 5b, 5c und 5d zeigen die Messkurven zum Beispiel 4, wobei Figur 5a die Unempfindlichkeit des Sensors gegenüber Temperaturschwankungen, Figur 5b die Konstanz der Sensorqualität bei extrem niedrigen Temperaturen, Figur 5c die Linearität des Sensors gegenüber steigender Analytkonzentration und Figur 5d das grundsätzliche Messignal des Sensors zeigt.
- Fig. 6: Messkurve zum Beispiel 5, wobei auf der x-Achse die Zeit und auf der y-Achse die gemessene Signalstärke in µA dargestellt ist. Zum Zeitpunkt t1 wurde begonnen, den Sensor mit dem Messgas zu begasen. Zum Zeitpunkt t2 wurde die Begasung beendet.
- Fig. 7: Messkurve zum Beispiel 6, wobei auf der x-Achse die Zeit und auf der y-Achse die gemessene Signalstärke in µA dargestellt ist. Zum Zeitpunkt t1 wurde begonnen, den Sensor mit dem Messgas zu begasen. Zum Zeitpunkt t2 wurde die Begasung beendet.
- Fig.8: Messkurve zum Beispiel 7, wobei auf der x-Achse die Zeit und auf der y-Achse die gemessene Signalstärke in µA dargestellt ist. Zum Zeitpunkt t1 wurde begonnen, den Sensor mit dem Messgas zu begasen. Zum Zeitpunkt t2 wurde die Begasung beendet.
- Fig. 9: Messkurve zum Beispiel 8, wobei auf der x-Achse die Zeit und auf der y-Achse die gemessene Signalstärke in µA dargestellt ist. Zum Zeitpunkt t1 wurde begonnen, den Sensor mit dem Messgas zu begasen. Zum Zeitpunkt t2 wurde die Begasung beendet.
- Fig. 10: Messkurve zum Beispiel 9, wobei auf der x-Achse die Zeit und auf der y-Achse die gemessene Signalstärke in µA dargestellt ist. Zum Zeitpunkt t1 wurde begonnen, den Sensor mit dem Messgas zu begasen. Zum Zeitpunkt t2 wurde die Begasung beendet.
- Fig. 11: Messkurve zum Beispiel 10, wobei auf der x-Achse die Zeit und auf der y-Achse die gemessene Signalstärke in µA dargestellt ist. Zum Zeitpunkt t1 wurde begonnen, den Sensor mit dem Messgas zu begasen. Zum Zeitpunkt t2 wurde die Begasung beendet.
- Fig. 12: Messkurve zum Beispiel 11, wobei auf der x-Achse die Zeit und auf der y-Achse die gemessene Signalstärke in µA dargestellt ist. Zum Zeitpunkt t1 wurde begonnen, den Sensor mit dem Messgas zu begasen. Zum Zeitpunkt t2 wurde die Begasung beendet.
- Fig. 13: Messkurve zum Beispiel 12, wobei auf der x-Achse die Zeit und auf der y-Achse die gemessene Signalstärke in µA dargestellt ist. Zum Zeitpunkt t1 wurde begonnen, den Sensor mit dem Messgas zu begasen. Zum Zeitpunkt t2 wurde die Begasung beendet.
- Fig. 14: Messkurve zum Beispiel 13, wobei auf der x-Achse die Zeit und auf der y-Achse die gemessene Signalstärke in µA dargestellt ist. Zum Zeitpunkt t1 wurde begonnen, den Sensor mit dem Messgas zu begasen. Zum Zeitpunkt t2 wurde die Begasung beendet.
- Fig. 15: Messkurve zum Beispiel 14, wobei auf der x-Achse die Zeit und auf der y-Achse die gemessene Signalstärke in µA dargestellt ist. Zum Zeitpunkt t1 wurde begonnen, den Sensor mit dem Messgas zu begasen. Zum Zeitpunkt t2 wurde die Begasung beendet.
- Fig. 16: Messkurve zum Beispiel 15, wobei auf der x-Achse die Zeit und auf der y-Achse die gemessene Signalstärke in µA dargestellt ist. Zum Zeitpunkt t1 wurde begonnen, den Sensor mit dem Messgas zu begasen. Zum Zeitpunkt t2 wurde die Begasung beendet.
- Fig. 17: Messkurve zum Beispiel 16, wobei auf der x-Achse die Zeit und auf der y-Achse die gemessene Signalstärke in µA dargestellt ist. Zum Zeitpunkt t1 wurde begonnen, den Sensor mit dem Messgas zu begasen. Zum Zeitpunkt t2 wurde die Begasung beendet.
- Fig. 18: Messkurve zum Beispiel 17, wobei auf der x-Achse die Zeit und auf der y-Achse die gemessene Signalstärke in µA dargestellt ist. Zum Zeitpunkt t1 wurde begonnen, den Sensor mit dem Messgas zu begasen. Zum Zeitpunkt t2 wurde die Begasung beendet.
- Fig. 19: Messkurve zum Beispiel 18, wobei auf der x-Achse die Zeit und auf der y-Achse die gemessene Signalstärke in µA dargestellt ist. Zu den Zeitpunkten t1 und t3 wurde jeweils begonnen, den Sensor mit dem Messgas zu begasen. Zu den Zeitpunkten t2 und t4 wurde die Begasung jeweils wieder beendet.
- Fig. 20: Messkurve zum Beispiel 19, wobei auf der x-Achse die Zeit und auf der y-Achse die gemessene Signalstärke in µA dargestellt ist. Zum Zeitpunkt t1 wurde begonnen, den Sensor mit dem Messgas zu begasen. Zum Zeitpunkt t2 wurde die Begasung beendet.
- Fig. 21: Messkurve zum Beispiel 20, wobei auf der x-Achse die Zeit und auf der y-Achse die gemessene Signalstärke in µA dargestellt ist. Zum Zeitpunkt t1 wurde begonnen, den Sensor mit dem Messgas zu begasen. Zum Zeitpunkt t2 wurde die Begasung beendet.
- Fig. 22: Messkurve zum Beispiel 21, wobei auf der x-Achse die Zeit und auf der y-Achse die gemessene Signalstärke in µA dargestellt ist. Zum Zeitpunkt t1 wurde begonnen, den Sensor mit dem Messgas zu begasen. Zum Zeitpunkt t2 wurde die Begasung beendet.
- Fig. 23: Messkurve zum Beispiel 22, wobei auf der x-Achse die Zeit und auf der y-Achse die gemessene Signalstärke in µA dargestellt ist. Zum Zeitpunkt t1 wurde begonnen, den Sensor mit dem Messgas zu begasen. Zum Zeitpunkt t2 wurde die Begasung beendet.
- Fig. 24: Messkurve zum Beispiel 23, wobei auf der x-Achse die Zeit und auf der y-Achse die gemessene Signalstärke in µA dargestellt ist. Zum Zeitpunkt t1 wurde begonnen, den Sensor mit dem Messgas zu begasen. Zum Zeitpunkt t2 wurde die Begasung beendet.
- Fig. 25: Messkurve zum Beispiel 24, wobei auf der x-Achse die Zeit und auf der y-Achse die gemessene Signalstärke in µA dargestellt ist. Zum Zeitpunkt t1 wurde begonnen, den Sensor mit dem Messgas zu begasen. Zum Zeitpunkt t2 wurde die Begasung beendet.
- Fig. 26: Messkurve zum Beispiel 25, wobei auf der x-Achse die Zeit und auf der y-Achse die gemessene Signalstärke in µA dargestellt ist. Zum Zeitpunkt t1 wurde begonnen, den Sensor mit dem Messgas zu begasen. Zum Zeitpunkt t2 wurde die Begasung beendet.
- Fig. 27: Messkurve zum Beispiel 26, wobei auf der x-Achse die Zeit und auf der y-Achse die gemessene Signalstärke in µA dargestellt ist. In den Zeitpunkten t1, t3, t5, t7, t9 und t11 wurde jeweils begonnen, den Sensor mit dem Messgas zu begasen. In den Zeitpunkten t2, t4, t6, t8, t10 und t12 wurde die Begasung jeweils beendet.
- Fig. 28: Messkurven zum Beispiel 27, wobei auf der x-Achse jeweils die Zeit und auf der y-Achse jeweils die gemessene Signalstärke in µA dargestellt ist. Zum Zeitpunkt t1 wurde begonnen, den Sensor mit dem Messgas - hier 4ppm SO₂ (A)- zu begasen. Zum Zeitpunkt t2 wurde die Begasung mit 8ppm SO₂ fortgesetzt (B). Bei t3 wurde die Gaskonzentration auf 2ppm (C) erniedrigt und nach t4 die Begasung beendet.
- Fig. 29: Messkurve zum Beispiel 28, wobei auf der x-Achse jeweils die Zeit und auf der y-Achse jeweils die gemessene Signalstärke in µA dargestellt ist. Zum Zeitpunkt t1 wurde begonnen, den Sensor mit dem Messgas zu begasen. Zum Zeitpunkt t2 wurde die Begasung mit einer höhren Konzentration des Messgases fortgesetzt, im Zeitpunkt t3 wieder erniedrigt und im Zeitpunkt t4 beendet
- Fig. 30: Messkurven zum Beispiel 29, wobei auf der x-Achse jeweils die Zeit und auf der y-Achse jeweils die gemessene Signalstärke in µA dargestellt ist. Zum Zeitpunkt t1 wurde begonnen, den Sensor mit dem Messgas zu begasen. Zum Zeitpunkt t2 wurde die Begasung mit einer höhren Konzentration des Messgases fortgesetzt, im Zeitpunkt t3 wieder erniedrigt und im Zeitpunkt t4 wurde die Begasung beendet.

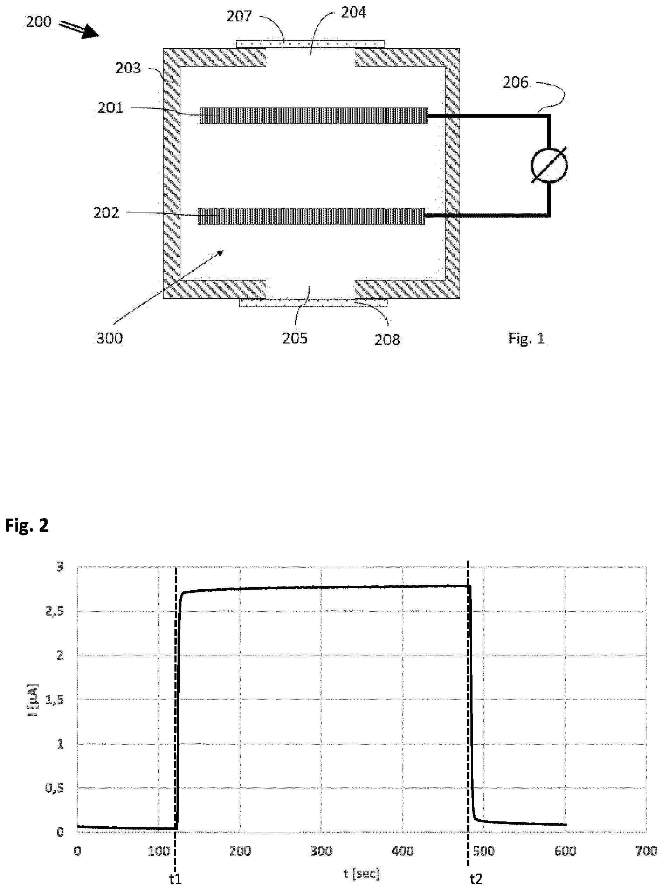

Fig. 1 zeigt eine schematische Darstellung eines elektrochemischen Gassensors 200. Der Gassensor 200 hat ein Gehäuse 203, in welchem eine erste Elektrode 201 und eine zweite Elektrode 202 angeordnet sind. Die Elektroden 201, 202 sind elektrisch leitend über eine elektrische Verbindung 206 miteinander verbunden. Das Gehäuse 203 hat einen Gaseinlass 204 und einen Gasauslass 205. Durch den Gaseinlass 204 kann das zu untersuchende Gas in den Gassensor 200 hineinströmen. Durch den Gasauslass 205 kann ein Druckausgleich erfolgen und es können auftretende gasförmige Reaktionsprodukte wieder aus dem Gassensor 200 austreten. Es sind vielfältige Abwandlungen dieses Aufbaus vorstellbar. So erkennt man, dass in dem gezeigten Beispiel vor dem Gaseinlass 204 und dem Gasauslass 205 jeweils eine gasdurchlässige Membran 207, 208 angeordnet ist. Diese können in einer alternativen Ausführungsform auch fehlen. In einer weiteren, alternativen (nicht dargestellten) Ausführungsform ist auch vorstellbar, dass der Gassensor 200 mehr als zwei Elektroden aufweist.

Bei allen Ausführungsformen ist jedoch, wie in Fig. 1 dargestellt, in dem Gehäuse 203 ein Elektrolyt 300 angeordnet. Der Elektrolyt 300 besteht aus einem Gemisch, welches eine erste ringförmige Verbindung und wenigstens 5 Gewichtsprozent Wasser enthält; wobei sowohl die erste ringförmige Verbindung als auch die zweite ringförmige Verbindung der Formel (I)

[(A)R¹R²]⁺[Y]⁻

entspricht;
- wobei (A) eine Ringstruktur ist, ausgewählt aus der Gruppe Pyridinium, Piperidinium, Pyrrolidinium, Pyrrolium;
- wobei der Rest R¹ am Stickstoffatom der Ringstruktur A angeordnet ist;
- wobei R¹ ein Kohlenwasserstoffrest ist mit 1, 2, 3, 4, 5, 6, 7, 8, 9, 10, 11 oder 12 C-Atomen;
- wobei R² ein Wasserstoffatom oder ein Kohlenwasserstoffrest mit 1, 2, 3 oder 4 C-Atomen ist;
- und wobei Y ausgewählt ist aus der Gruppe enthaltend Halogenid, Cyanid, Alkylsulfonat, halogeniertes Alkylsulfonat, Acetat.

Insbesondere ist der Elektrolyt 300 ein Elektrolyt entsprechend einem der nachfolgend beschriebenen Beispiele. Es versteht sich jedoch von selbst, dass die vorliegende Erfindung nicht auf diese Beispiele beschränkt ist, sondern dass diese nur zur Erläuterung dienen. Selbstverständlich sind auch weitere nicht explizit hier aufgeführte Ausführungsformen, die dem Wortlaut der Ansprüche entsprechen von der Erfindung umfasst.

### Beispiel 1

In Beispiel 1 wurde ein elektrochemischer Gassensor, der wenigstens eine erste Elektrode, eine zweite Elektrode und einen Elektrolyten aufwies, für einen definierten Zeitraum mit 10 ppm H₂S begast. Fig. 2 zeigt die dabei erhaltene Messkurve.

Der verwendete Elektrolyt enthält als erste ringförmige Verbindung 1-Butylpyridiniumchlorid und als zweite ringförmige Verbindung 1-Hexylpyridiniumchlorid. Des Weiteren enthält der Elektrolyt Tris als Puffersubstanz. Die Puffersubstanz liegt in einer 0,5 molaren Konzentration vor. Außerdem enthält der Elektrolyt 30 Gewichtsprozent Wasser.

Das Beispiel zeigt also einen Elektrolyten und das Messverhalten eines elektrochemischen Gassensors mit einem Elektrolyten, der eine erste ringförmige Verbindung entsprechend Formel (VI), genauer entsprechend Formel (73) und Formel (84), eine zweite ringförmige Verbindung entsprechend Formel (VI), genauer entsprechend Formel (75) und Formel (86), Tris als Puffer in 0,5 molarer Konzentration, sowie 30% Wasser enthält. Für die erste ringförmige Verbindung entsprechend Formel (I) ist R¹ mithin ein Butylrest, also ein Kohlenwasserstoff mit 4 C-Atomen, nämlich C₄H₇, für die zweite ringförmige Verbindung entsprechend Formel (I) ist R1 mithin ein Hexylrest, also ein Kohlenwasserstoff mit 6 C-Atomen, nämlich C₆H₁₁. R² ist für beide ringförmigen Verbindungen ein Wasserstoff. A ist in beiden Fällen ein Pyridin-Ring und Y ist in beiden Fällen Chlorid.

In der gezeigten Abbildung in Fig. 2 ist auf der x-Achse die Zeit in Sekunden und auf der y-Achse die gemessene Signalstärke in µA dargestellt. Zum Zeitpunkt t1 wurde die Begasung mit H₂S begonnen. Zum Zeitpunkt t2 wurde die Begasung wieder beendet.

Man erkennt, dass der Gassensor auf die Präsenz des Analyten, in diesem Fall H₂S, reagiert, indem direkt nach dem Start der Begasung im Zeitpunkt t1 das Signal von 0 µA auf etwa 0,6 µA bis 0,7 µA ansteigt und mit dem Ende der Begasung im Zeitpunkt t2 wieder auf 0 µA zurückgeht.

### Beispiel 2

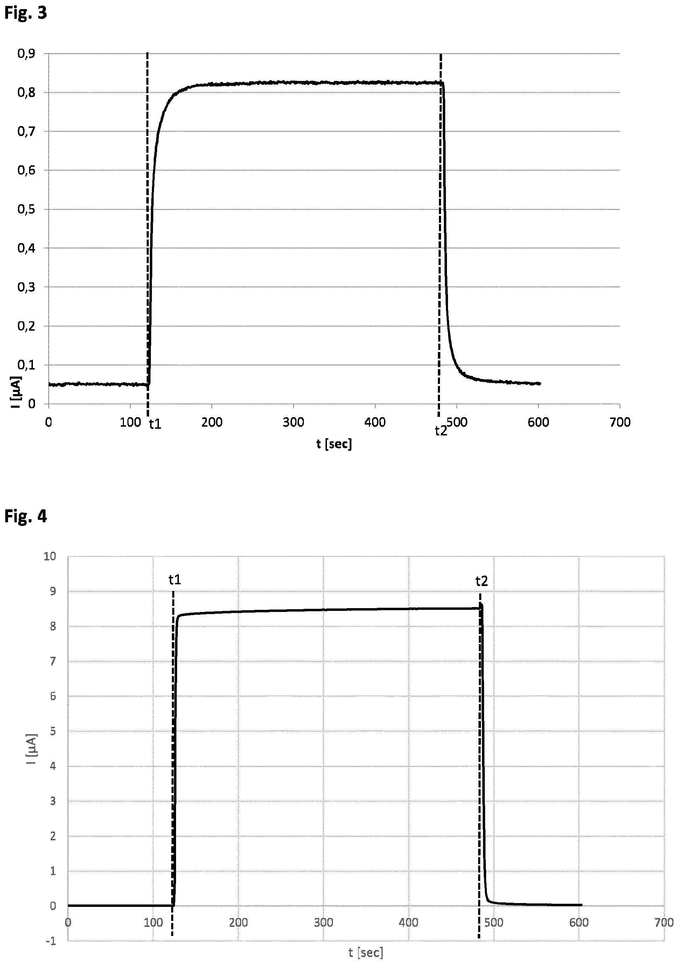

In Beispiel 2 wurde ein elektrochemischer Gassensor, der wenigstens eine erste Elektrode, eine zweite Elektrode und einen Elektrolyten aufwies, für einen definierten Zeitraum mit 5 ppm SO₂ begast. Fig. 3 zeigt die dabei erhaltene Messkurve.

Der verwendete Elektrolyt enthält als erste ringförmige Verbindung 1-Butylpyridiniumchlorid und als zweite ringförmige Verbindung 1-Hexylpyridiniumchlorid. Des Weiteren enthält der Elektrolyt Tris als Puffersubstanz. Die Puffersubstanz liegt in einer 0,5 molaren Konzentration vor. Außerdem enthält der Elektrolyt 30 Gewichtsprozent Wasser. Für die erste ringförmige Verbindung entsprechend Formel (I) ist R¹ mithin ein Butylrest, also ein Kohlenwasserstoff mit 4 C-Atomen, nämlich C₄H₇, für die zweite ringförmige Verbindung entsprechend Formel (I) ist R1 mithin ein Hexylrest, also ein Kohlenwasserstoff mit 6 C-Atomen, nämlich C₆H₁₁. R² ist für beide ringförmigen Verbindungen ein Wasserstoff. A ist in beiden Fällen ein Pyridin-Ring und Y ist in beiden Fällen Chlorid.

Das Beispiel zeigt also einen Elektrolyten und das Messverhalten eines elektrochemischen Gassensors mit einem Elektrolyten, der eine erste ringförmige Verbindung entsprechend Formel (VI), genauer entsprechend Formel (73) und Formel (84), eine zweite ringförmige Verbindung entsprechend Formel (VI), genauer entsprechend Formel (75) und Formel (86), Tris als Puffer in 0,5 molarer Konzentration, sowie 30% Wasser enthält.

In der gezeigten Abbildung in Fig. 3 ist auf der x-Achse die Zeit (t) in Sekunden und auf der y-Achse die gemessene Signalstärke (I) in µA dargestellt. Zum Zeitpunkt t1 wurde die Begasung mit SO₂ begonnen. Zum Zeitpunkt t2 wurde die Begasung wieder beendet.

Man erkennt, dass der Gassensor auf die Präsenz des Analyten, in diesem Fall SO₂, reagiert, indem direkt nach dem Start der Begasung im Zeitpunkt t1 das Signal von 0 µA auf etwas über 2,5 µA ansteigt und mit dem Ende der Begasung im Zeitpunkt t2 wieder auf 0 µA zurückgeht.

### Beispiel 3

In Beispiel 3 wurde ein elektrochemischer Gassensor, der wenigstens eine erste Elektrode, eine zweite Elektrode und einen Elektrolyten aufwies, für einen definierten Zeitraum mit 20 ppm H₂S begast. Fig. 4 zeigt die dabei erhaltene Messkurve.

Der verwendete Elektrolyt enthält als erste ringförmige Verbindung 1-Butylpyridiniumchlorid und als zweite ringförmige Verbindung 1-Hexylpyridiniumchlorid. Des Weiteren enthält der Elektrolyt Kaliumacetat als Zuschlag. Die erste ringförmige Verbindung, die zweite ringförmige Verbindung und der Zuschlag liegen im Verhältnis 1:1:1 vor. Außerdem enthält der Elektrolyt 20 Gewichtsprozent Wasser. Für die erste ringförmige Verbindung entsprechend Formel (I) ist R¹ mithin ein Butylrest, also ein Kohlenwasserstoff mit 4 C-Atomen, nämlich C₄H₇, für die zweite ringförmige Verbindung entsprechend Formel (I) ist R1 mithin ein Hexylrest, also ein Kohlenwasserstoff mit 6 C-Atomen, nämlich C₆H₁₁. R² ist für beide ringförmigen Verbindungen ein Wasserstoff. A ist in beiden Fällen ein Pyridin-Ring und Y ist in beiden Fällen Chlorid.

Das Beispiel zeigt also einen Elektrolyten und das Messverhalten eines elektrochemischen Gassensors mit einem Elektrolyten, der eine erste ringförmige Verbindung entsprechend Formel (VI), genauer entsprechend Formel (73) und Formel (84), eine zweite ringförmige Verbindung entsprechend Formel (VI), genauer entsprechend Formel (75) und Formel (86), Kaliumacetat als Zuschlag, sowie 20% Wasser enthält.

In der gezeigten Abbildung in Fig. 4 ist auf der x-Achse die Zeit (t) in Sekunden und auf der y-Achse die gemessene Signalstärke (I) in µA dargestellt. Zum Zeitpunkt t1 wurde die Begasung mit SO₂ begonnen. Zum Zeitpunkt t2 wurde die Begasung wieder beendet.

Man erkennt, dass der Gassensor auf die Präsenz des Analyten, in diesem Fall H₂S, reagiert, indem direkt nach dem Start der Begasung im Zeitpunkt t1 das Signal von 0 µA auf etwa 8,5 µA ansteigt und mit dem Ende der Begasung im Zeitpunkt t2 wieder auf 0 µA zurückgeht.

### Beispiel 4

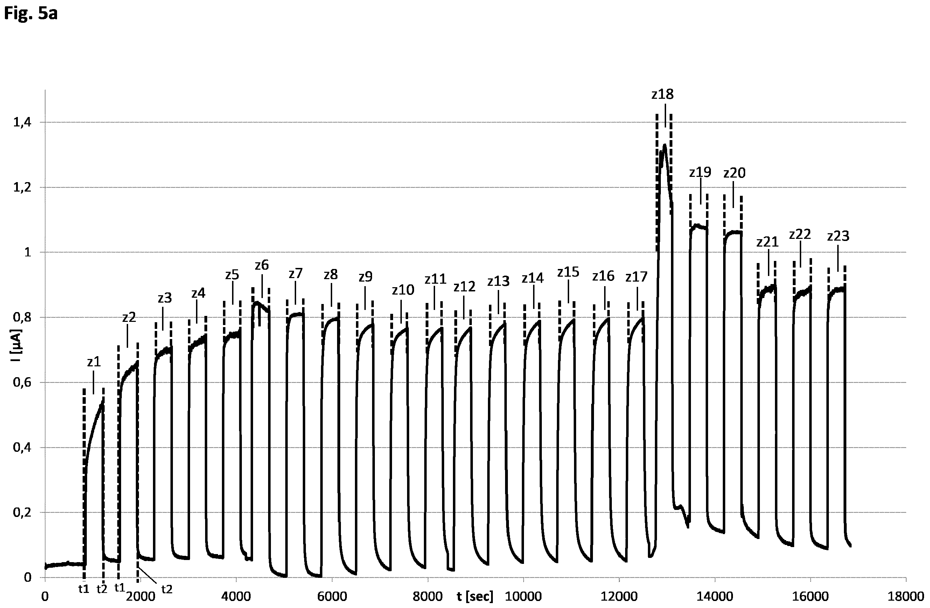

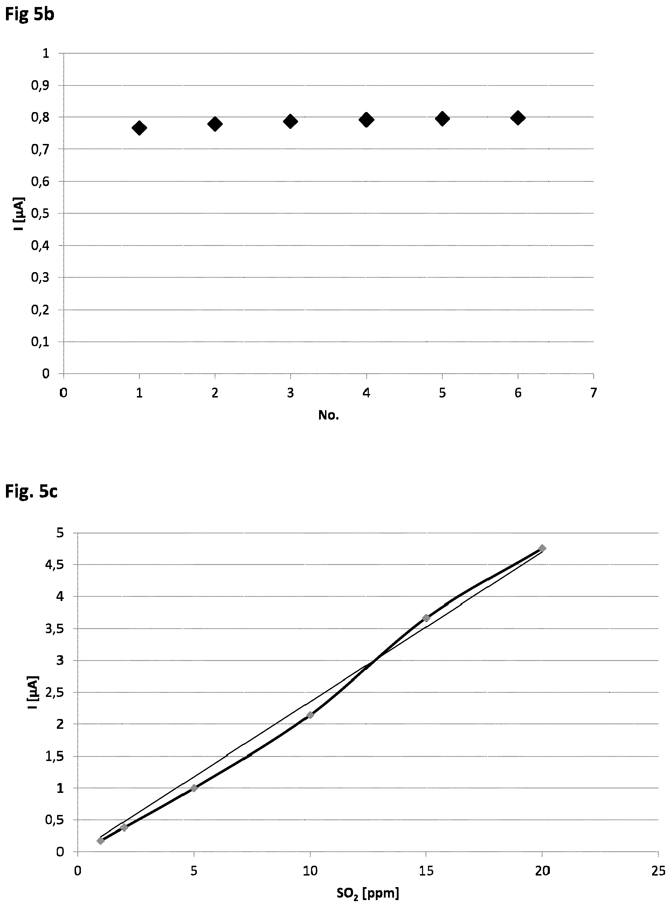

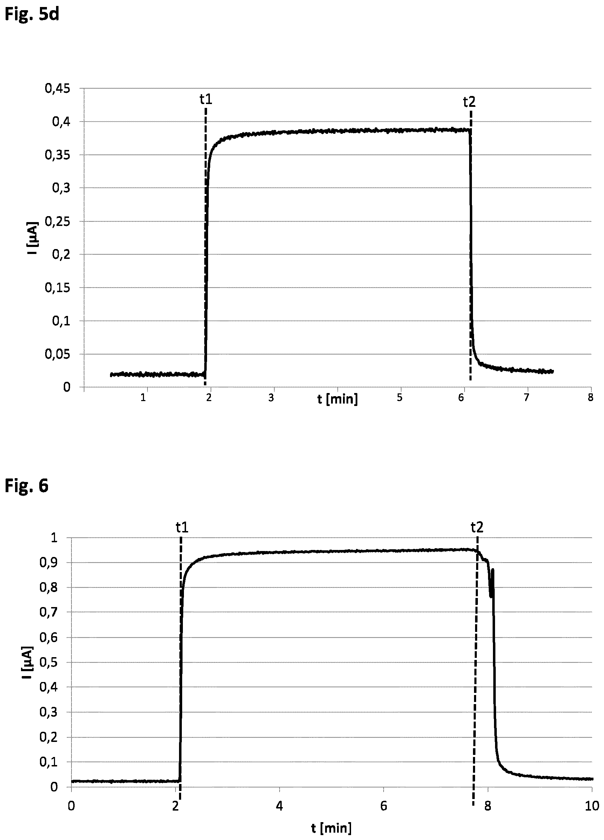

In Beispiel 4 wurde ein besonders vorteilhafter elektrochemischer Gassensor, der wenigstens eine erste Elektrode, eine zweite Elektrode und einen Elektrolyten aufwies, für einen definierten Zeitraum einmal mit 2 ppm SO₂ und einmal mit 5 ppm SO₂ begast. Die Figuren 5a, 5b, 5c, und 5d zeigen die bei der Untersuchung dieses Sensors erhaltenen Messkurve.

Der verwendete Elektrolyt enthält als erste ringförmige Verbindung 1-Butylpyridiniumchlorid und als zweite ringförmige Verbindung 1-Hexylpyridiniumchlorid in einem molaren Verhältnis von 1:1. Des Weiteren enthält der Elektrolyt Triethanolamin (TEA) als Puffer in einer 0,5 molaren Konzentration. Außerdem enthält der Elektrolyt 30 Gewichtsprozent Wasser. Für die erste ringförmige Verbindung entsprechend Formel (I) ist R¹ mithin ein Butylrest, also ein Kohlenwasserstoff mit 4 C-Atomen, nämlich C₄H₇, für die zweite ringförmige Verbindung entsprechend Formel (I) ist R1 mithin ein Hexylrest, also ein Kohlenwasserstoff mit 6 C-Atomen, nämlich C₆H₁₁. R² ist für beide ringförmigen Verbindungen ein Wasserstoff. A ist in beiden Fällen ein Pyridin-Ring und Y ist in beiden Fällen Chlorid.

Das Beispiel zeigt also einen Elektrolyten und das Messverhalten eines elektrochemischen Gassensors mit einem Elektrolyten, der eine erste ringförmige Verbindung entsprechend Formel (VI), genauer entsprechend Formel (73) und Formel (84), eine zweite ringförmige Verbindung entsprechend Formel (VI), genauer entsprechend Formel (75) und Formel (86), TEA als Puffer, sowie 30% Wasser enthält.

In der gezeigten Abbildung in Fig. 5a ist auf der x-Achse die Zeit (t) in Sekunden und auf der y-Achse die gemessene Signalstärke (I) in µA dargestellt. Der Sensor wurde dabei über mehrere Messzyklen unter verschiedenen Temperaturbedingungen beobachtet. Ein Messzyklus beginnt dabei jeweils im Zeitpunkt t1 und endet jeweils im Zeitpunkt t2. Dabei wurde zum Zeitpunkt t1 jeweils die Begasung mit SO₂ begonnen. Zum Zeitpunkt t2 wurde die Begasung wieder beendet. Zwischen dem Endzeitpunkt t2 und dem nächsten Startzeitpunkt t1 lag jeweils eine kurze Ruhepause, in der der Sensor weiterlief, jedoch keine Analytbegasung stattfand. In den ersten fünf Zyklen z1, z2, z3, z4 und z5 wurde der Sensor zunächst bei Raumtemperatur betrieben. Danach wurde die Umgebungstemperatur im Verlauf der nächsten sechs Zyklen z6, z7, z8, z9, z10, z11 auf -40°C abgesenkt. Für die darauf folgenden sechs Zyklen z12, z13, z14, z15, z16 wurde der Sensor dann bei - 40°C betrieben. Im Anschluss wurde die Umgebungstemperatur von -40°C um insgesamt 70K auf 30°C erhöht und der Sensor wurde für die letzten sechs Zyklen z18, z19, z20, z21, z22, z23 bei 30°C Umgebungstemperatur betrieben. Man erkennt, dass der Sensor trotz der starken Schwankungen der Umgebungstemperatur in Gegenwart des Analyten ein nahezu konstantes Messsignal zwischen 0,6 µA und 1 µA liefert. Der Sensor ist mithin auch unter stark schwanken Klimabedingungen zuverlässig.

Dies wird auch durch die in Abbildung in Fig. 5b dargestellten Ergebnisse bestätigt. Hier ist auf der x-Achse die laufende Nummer der Messung und auf der y-Achse die gemessene Signalstärke in µA dargestellt. Die laufende Nummer des Messung entspricht dabei den in Fig. 5a dargestellten Messzyklen, wobei die laufende Nummer 1 dem Messzyklus z1, die laufende Nummer 2 dem Messzyklus z2, die laufende Nummer 3 dem Messzyklus z3 und so fort entspricht. Insgesamt wurden sechs aufeinander folgende Messungen bei einer Umgebungstemperatur von -40°C durchgeführt. Man erkennt, dass das vom Sensor gelieferte Messsignal dabei konstant in einem sehr engen Bereich zwischen 0,7 µA und 0,8 µA lag.

Der als Signal ausgegebene Messstrom hängt dabei linear mit der Konzentration des Analyten zusammen, wie man in Fig. 5c erkennt. Hier ist auf der x-Achse die Konzentration des Analyten (SO₂ in ppm) und auf der y-Achse die gemessene Signalstärke in µA dargestellt. Die Kurve K zeigt dabei die Regressionsgerade der Mittelwerte aller insgesamt durchgeführten Messungen bei den Konzentrationen 1ppm, 2ppm, 5ppm, 10ppm, 15 ppm und 20 ppm. Man erkennt, dass das Messsignal linear mit der Konzentration ansteigt.

In der gezeigten Abbildung in Fig. 5d ist auf der x-Achse die Zeit in Sekunden und auf der y-Achse die gemessene Signalstärke in µA dargestellt. Zum Zeitpunkt t1 wurde die Begasung mit 2ppm SO₂ begonnen. Zum Zeitpunkt t2 wurde die Begasung wieder beendet. Man erkennt, dass der Gassensor auf die Präsenz des Analyten, in diesem Fall SO₂, reagiert, indem direkt nach dem Start der Begasung im Zeitpunkt t1 das Signal von 0 µA auf etwa 0,4 µA ansteigt und mit dem Ende der Begasung im Zeitpunkt t2 wieder auf 0 µA zurückgeht.

### Beispiel 5

In Beispiel 5 wurde ein elektrochemischer Gassensor, der wenigstens eine erste Elektrode, eine zweite Elektrode und einen Elektrolyten aufwies, für einen definierten Zeitraum mit 5 ppm SO₂ begast. Fig. 6 zeigt die dabei erhaltene Messkurve.

Der verwendete Elektrolyt enthält als ringförmige Verbindung 1-Butylpyridiniumchlorid. Des Weiteren enthält der Elektrolyt Tris als Puffersubstanz. Die Puffersubstanz liegt in einer 0,5 molaren Konzentration vor. Außerdem enthält der Elektrolyt 30 Gewichtsprozent Wasser. Für die ringförmige Verbindung entsprechend Formel (I) ist R¹ mithin ein Butylrest, also ein Kohlenwasserstoff mit 4 C-Atomen, nämlich C₄H₇. R² ist ein Wasserstoff, A ein Pyridin-Ring und Y ist Chlorid.

Das Beispiel zeigt also einen Elektrolyten und das Messverhalten eines elektrochemischen Gassensors mit einem Elektrolyten, der eine erste ringförmige Verbindung entsprechend Formel (VI), genauer entsprechend Formel (73) und Formel (84), eine weitere Komponente, nämlich Tris als Puffer in 0,5 molarer Konzentration, sowie 30% Wasser enthält.

In der gezeigten Abbildung in Fig. 6 ist auf der x-Achse die Zeit (t) in Minuten und auf der y-Achse die gemessene Signalstärke (I) in µA dargestellt. Zum Zeitpunkt t1 wurde die Begasung mit 5ppm SO₂ begonnen. Zum Zeitpunkt t2 wurde die Begasung wieder beendet.

Man erkennt, dass der Gassensor auf die Präsenz des Analyten, in diesem Fall SO₂, reagiert, indem direkt nach dem Start der Begasung im Zeitpunkt t1 das Signal von 0 µA auf etwa 0,9 µA ansteigt und mit dem Ende der Begasung im Zeitpunkt t2 wieder auf 0 µA zurückgeht.

### Beispiel 6

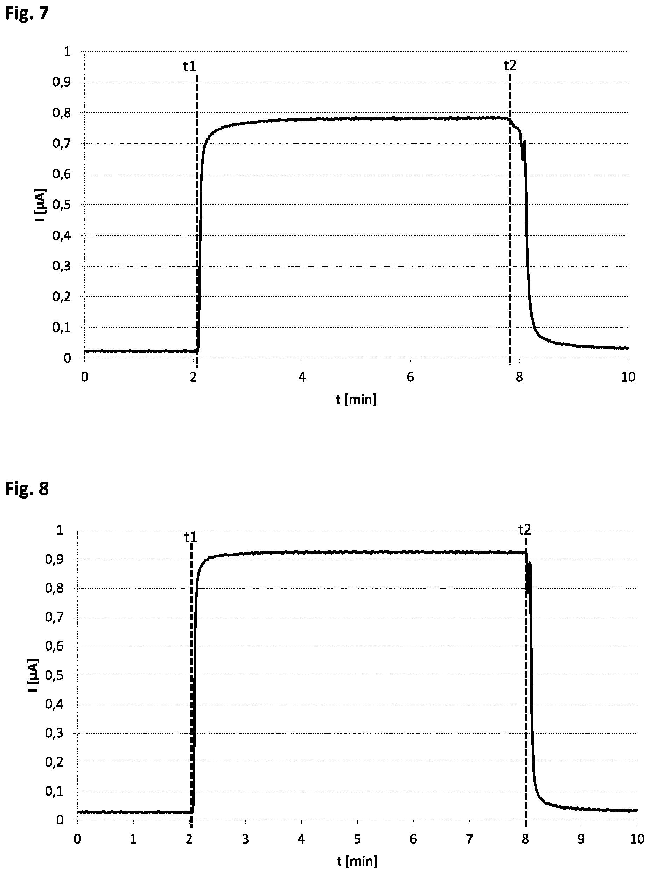

In Beispiel 6 wurde ein elektrochemischer Gassensor, der wenigstens eine erste Elektrode, eine zweite Elektrode und einen Elektrolyten aufwies, für einen definierten Zeitraum mit 5 ppm SO₂ begast. Fig. 7 zeigt die dabei erhaltene Messkurve.

Der verwendete Elektrolyt enthält als ringförmige Verbindung 1-Hexylpyridiniumchlorid. Des Weiteren enthält der Elektrolyt Tris als Puffersubstanz. Die Puffersubstanz liegt in einer 0,5 molaren Konzentration vor. Außerdem enthält der Elektrolyt 30 Gewichtsprozent Wasser. Für die ringförmige Verbindung entsprechend Formel (I) ist R¹ mithin ein Hexylrest, also ein Kohlenwasserstoff mit 6 C-Atomen, nämlich C₆H₁₁. R² ist ein Wasserstoff, A ein Pyridin-Ring und Y ist Chlorid.

Das Beispiel zeigt also einen Elektrolyten und das Messverhalten eines elektrochemischen Gassensors mit einem Elektrolyten, der eine erste ringförmige Verbindung entsprechend Formel (VI), genauer entsprechend Formel (75) und Formel (86), eine weitere Komponente, nämlich Tris als Puffer in 0,5 molarer Konzentration, sowie 30% Wasser enthält.

In der gezeigten Abbildung in Fig. 7 ist auf der x-Achse die Zeit (t) in Minuten und auf der y-Achse die gemessene Signalstärke (I) in µA dargestellt. Zum Zeitpunkt t1 wurde die Begasung mit 5ppm SO₂ begonnen. Zum Zeitpunkt t2 wurde die Begasung wieder beendet.

Man erkennt, dass der Gassensor auf die Präsenz des Analyten, in diesem Fall SO₂, reagiert, indem direkt nach dem Start der Begasung im Zeitpunkt t1 das Signal von 0 µA auf etwa 0,8 µA ansteigt und mit dem Ende der Begasung im Zeitpunkt t2 wieder auf 0 µA zurückgeht.

### Beispiel 7

In Beispiel 7 wurde ein elektrochemischer Gassensor, der wenigstens eine erste Elektrode, eine zweite Elektrode und einen Elektrolyten aufwies, für einen definierten Zeitraum mit 5 ppm SO₂ begast. Fig. 8 zeigt die dabei erhaltene Messkurve.

Der verwendete Elektrolyt enthält als ringförmige Verbindung 1-Butylpyridiniummethansulfonat. Des Weiteren enthält der Elektrolyt Tris als Puffersubstanz. Die Puffersubstanz liegt in einer 0,5 molaren Konzentration vor. Außerdem enthält der Elektrolyt 30 Gewichtsprozent Wasser.

Das Beispiel zeigt also einen Elektrolyten und das Messverhalten eines elektrochemischen Gassensors mit einem Elektrolyten, der eine erste ringförmige Verbindung entsprechend Formel (VI), genauer entsprechend Formel (73) und Formel (84), eine weitere Komponente, nämlich Tris als Puffer in 0,5 molarer Konzentration, sowie 30% Wasser enthält. Für die ringförmige Verbindung entsprechend Formel (I) ist R¹ mithin ein Butylrest, also ein Kohlenwasserstoff mit 4 C-Atomen, nämlich C₄H₇. R² ist ein Wasserstoff, A ein Pyridin-Ring und Y ist Methansulfonat.

In der gezeigten Abbildung in Fig. 8 ist auf der x-Achse die Zeit (t) in Minuten und auf der y-Achse die gemessene Signalstärke (I) in µA dargestellt. Zum Zeitpunkt t1 wurde die Begasung mit 5ppm SO₂ begonnen. Zum Zeitpunkt t2 wurde die Begasung wieder beendet.

Man erkennt, dass der Gassensor auf die Präsenz des Analyten, in diesem Fall SO₂, reagiert, indem direkt nach dem Start der Begasung im Zeitpunkt t1 das Signal von 0 µA auf etwa 0,9 µA ansteigt und mit dem Ende der Begasung im Zeitpunkt t2 wieder auf 0 µA zurückgeht.

### Beispiel 8

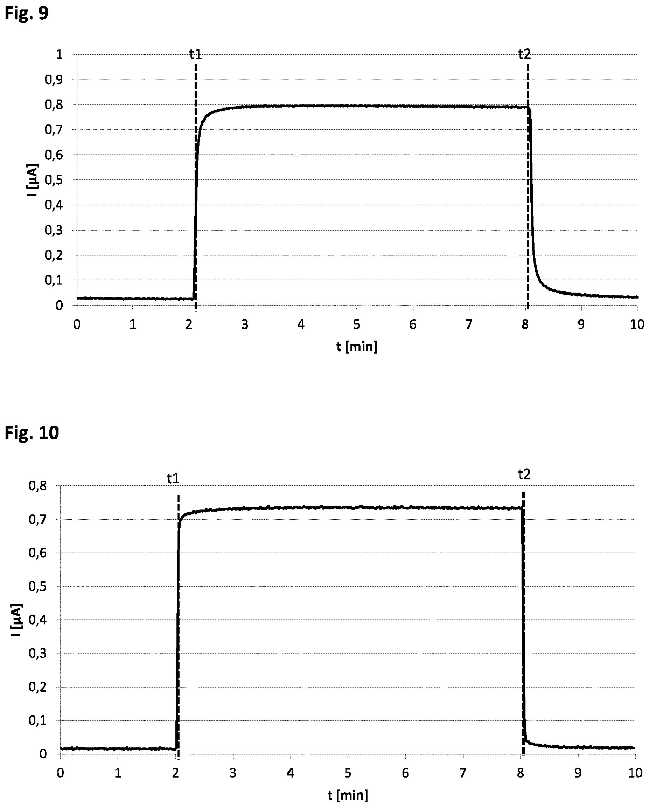

In Beispiel 8 wurde ein elektrochemischer Gassensor, der wenigstens eine erste Elektrode, eine zweite Elektrode und einen Elektrolyten aufwies, für einen definierten Zeitraum mit 5 ppm SO₂ begast. Fig. 9 zeigt die dabei erhaltene Messkurve.

Der verwendete Elektrolyt enthält als ringförmige Verbindung 1-Hexylpyridiniummethansulfonat. Des Weiteren enthält der Elektrolyt Tris als Puffersubstanz. Die Puffersubstanz liegt in einer 0,5 molaren Konzentration vor. Außerdem enthält der Elektrolyt 30 Gewichtsprozent Wasser. Für die ringförmige Verbindung entsprechend Formel (I) ist R¹ mithin ein Hexylrest, also ein Kohlenwasserstoff mit 6 C-Atomen, nämlich C₆H₁₁. R² ist ein Wasserstoff, A ein Pyridin-Ring und Y ist Methansulfonat.

Das Beispiel zeigt also einen Elektrolyten und das Messverhalten eines elektrochemischen Gassensors mit einem Elektrolyten, der eine erste ringförmige Verbindung entsprechend Formel (VI), genauer entsprechend Formel (75) und Formel (86), eine weitere Komponente, nämlich Tris als Puffer in 0,5 molarer Konzentration, sowie 30% Wasser enthält.

In der gezeigten Abbildung in Fig. 9 ist auf der x-Achse die Zeit (t) in Minuten und auf der y-Achse die gemessene Signalstärke (I) in µA dargestellt. Zum Zeitpunkt t1 wurde die Begasung mit 5ppm SO₂ begonnen. Zum Zeitpunkt t2 wurde die Begasung wieder beendet.

Man erkennt, dass der Gassensor auf die Präsenz des Analyten, in diesem Fall SO₂, reagiert, indem direkt nach dem Start der Begasung im Zeitpunkt t1 das Signal von 0 µA auf etwa 0,8 µA ansteigt und mit dem Ende der Begasung im Zeitpunkt t2 wieder auf 0 µA zurückgeht.

### Beispiel 9

In Beispiel 9 wurde ein elektrochemischer Gassensor, der wenigstens eine erste Elektrode, eine zweite Elektrode und einen Elektrolyten aufwies, für einen definierten Zeitraum mit 5 ppm SO₂ begast. Fig. 10 zeigt die dabei erhaltene Messkurve.

Der verwendete Elektrolyt enthält als erste ringförmige Verbindung 1-Butylpyridiniummethansulfonat und als zweite ringförmige Verbindung 1-Hexylpyridiniummethansulfonat. Des Weiteren enthält der Elektrolyt Tris als Puffersubstanz. Die Puffersubstanz liegt in einer 0,5 molaren Konzentration vor. Die erste und die zweite ringförmige Verbindung liegen im Verhältnis 1:1 zueinander vor. Außerdem enthält der Elektrolyt 30 Gewichtsprozent Wasser. Für die erste ringförmige Verbindung entsprechend Formel (I) ist R¹ mithin ein Butylrest, also ein Kohlenwasserstoff mit 4 C-Atomen, nämlich C₄H₇, für die zweite ringförmige Verbindung entsprechend Formel (I) ist R1 mithin ein Hexylrest, also ein Kohlenwasserstoff mit 6 C-Atomen, nämlich C₆H₁₁. R² ist für beide ringförmigen Verbindungen ein Wasserstoff. A ist in beiden Fällen ein Pyridin-Ring und Y ist in beiden Fällen Methansulfonat.

Das Beispiel zeigt also einen Elektrolyten und das Messverhalten eines elektrochemischen Gassensors mit einem Elektrolyten, der eine erste ringförmige Verbindung entsprechend Formel (VI), genauer entsprechend Formel (73) und Formel (84), eine zweite ringförmige Verbindung entsprechend Formel (VI), genauer entsprechend Formel (75) und Formel (86), Tris als Puffer in 0,5 molarer Konzentration, sowie 30% Wasser enthält.

In der gezeigten Abbildung in Fig. 10 ist auf der x-Achse die Zeit (t) in Minuten und auf der y-Achse die gemessene Signalstärke (I) in µA dargestellt. Zum Zeitpunkt t1 wurde die Begasung mit SO₂ begonnen. Zum Zeitpunkt t2 wurde die Begasung wieder beendet.

Man erkennt, dass der Gassensor auf die Präsenz des Analyten, in diesem Fall SO₂, reagiert, indem direkt nach dem Start der Begasung im Zeitpunkt t1 das Signal von 0 µA auf etwa 0,7 µA ansteigt und mit dem Ende der Begasung im Zeitpunkt t2 wieder auf 0 µA zurückgeht.

### Beispiel 10

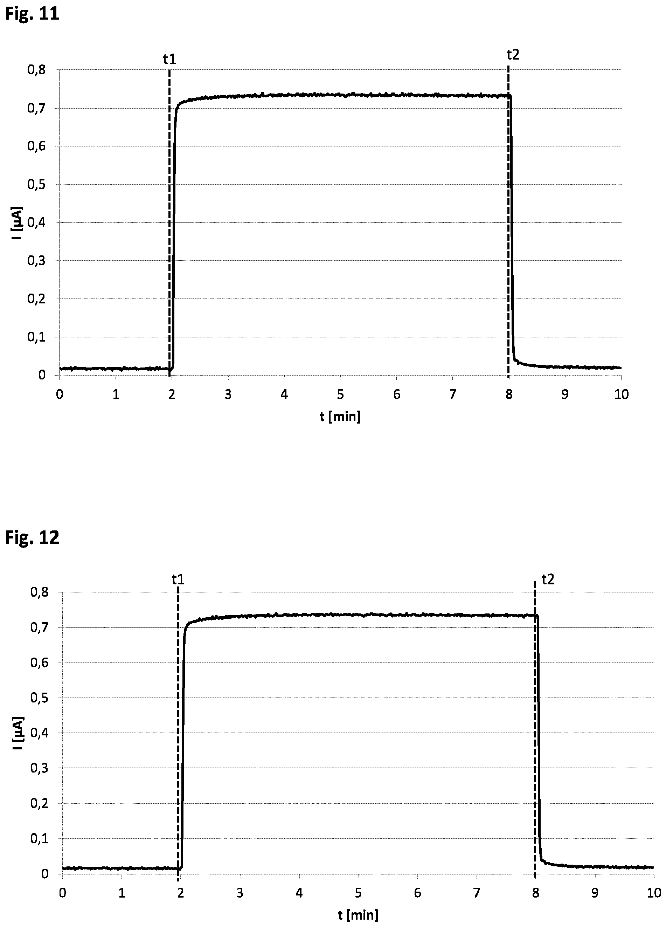

In Beispiel 10 wurde ein elektrochemischer Gassensor, der wenigstens eine erste Elektrode, eine zweite Elektrode und einen Elektrolyten aufwies, für einen definierten Zeitraum mit 5 ppm SO₂ begast. Fig. 11 zeigt die dabei erhaltene Messkurve.

Der verwendete Elektrolyt enthält als erste ringförmige Verbindung 1-Butylpyridiniumchlorid und als zweite ringförmige Verbindung 1-Butylpyridiniummethansulfonat. Des Weiteren enthält der Elektrolyt Tris als Puffersubstanz. Die Puffersubstanz liegt in einer 0,5 molaren Konzentration vor. Die erste und die zweite ringförmige Verbindung liegen im Verhältnis 1:1 zueinander vor. Außerdem enthält der Elektrolyt 20 Gewichtsprozent Wasser. Sowohl für die erste ringförmige Verbindung entsprechend Formel (I) als auch für die zweite ringförmige Verbindung entsprechend Formel (I) ist R¹ mithin ein Butylrest, also ein Kohlenwasserstoff mit 4 C-Atomen, nämlich C₄H₇. R² ist für beide ringförmigen Verbindungen ein Wasserstoff. A ist in beiden Fällen ein Pyridin-Ring. Für die erste ringförmige Verbindung entsprechend Formel (I) ist Y Chlorid, für die zweite ringförmige Verbindung entsprechend Forme (I) ist Y Methansulfonat.

Das Beispiel zeigt also einen Elektrolyten und das Messverhalten eines elektrochemischen Gassensors mit einem Elektrolyten, der eine erste ringförmige Verbindung entsprechend Formel (VI), genauer entsprechend Formel (73) und Formel (84), eine zweite ringförmige Verbindung entsprechend Formel (VI), genauer entsprechend Formel (73) und Formel (84), Tris als Puffer in 0,5 molarer Konzentration, sowie 20% Wasser enthält.

In der gezeigten Abbildung in Fig. 11 ist auf der x-Achse die Zeit (t) in Sekunden und auf der y-Achse die gemessene Signalstärke (I) in µA dargestellt. Zum Zeitpunkt t1 wurde die Begasung mit SO₂ begonnen. Zum Zeitpunkt t2 wurde die Begasung wieder beendet.

Man erkennt, dass der Gassensor auf die Präsenz des Analyten, in diesem Fall SO₂, reagiert, indem direkt nach dem Start der Begasung im Zeitpunkt t1 das Signal von 0 µA auf etwa 0,7µA ansteigt und mit dem Ende der Begasung im Zeitpunkt t2 wieder auf 0 µA zurückgeht.

### Beispiel 11

In Beispiel 11 wurde ein elektrochemischer Gassensor, der wenigstens eine erste Elektrode, eine zweite Elektrode und einen Elektrolyten aufwies, für einen definierten Zeitraum mit 5 ppm SO₂ begast. Fig. 12 zeigt die dabei erhaltene Messkurve.

Der verwendete Elektrolyt enthält als erste ringförmige Verbindung 1-Hexylpyridiniumchlorid und als zweite ringförmige Verbindung 1-Hexylpyridiniummethansulfonat. Des Weiteren enthält der Elektrolyt Tris als Puffersubstanz. Die Puffersubstanz liegt in einer 0,5 molaren Konzentration vor. Die erste und die zweite ringförmige Verbindung liegen im Verhältnis 1:1 zueinander vor. Außerdem enthält der Elektrolyt 30 Gewichtsprozent Wasser. Sowohl für die erste als auch für die zweite ringförmige Verbindung entsprechend Formel (I) ist R¹ mithin ein Butylrest, also ein Kohlenwasserstoff mit 6 C-Atomen, nämlich C₆H₁₁. R² ist für beide ringförmigen Verbindungen ein Wasserstoff. A ist in beiden Fällen ein Pyridin-Ring. Y ist für die erste ringförmige Verbindung entsprechend Formel (I) Chlorid, für die zweite ringförmige Verbindung entsprechend Formel (I) Methansulfonat.

Das Beispiel zeigt also einen Elektrolyten und das Messverhalten eines elektrochemischen Gassensors mit einem Elektrolyten, der eine erste ringförmige Verbindung entsprechend Formel (VI), genauer entsprechend Formel (75) und Formel (86), eine zweite ringförmige Verbindung entsprechend Formel (VI), genauer entsprechend Formel (75) und Formel (86), Tris als Puffer in 0,5 molarer Konzentration, sowie 30% Wasser enthält.

In der gezeigten Abbildung in Fig. 12 ist auf der x-Achse die Zeit (t) in Minuten und auf der y-Achse die gemessene Signalstärke (I) in µA dargestellt. Zum Zeitpunkt t1 wurde die Begasung mit SO₂ begonnen. Zum Zeitpunkt t2 wurde die Begasung wieder beendet.

Man erkennt, dass der Gassensor auf die Präsenz des Analyten, in diesem Fall SO₂, reagiert, indem direkt nach dem Start der Begasung im Zeitpunkt t1 das Signal von 0 µA auf etwa 0,6 µA ansteigt und mit dem Ende der Begasung im Zeitpunkt t2 wieder auf 0 µA zurückgeht.

### Beispiel 12

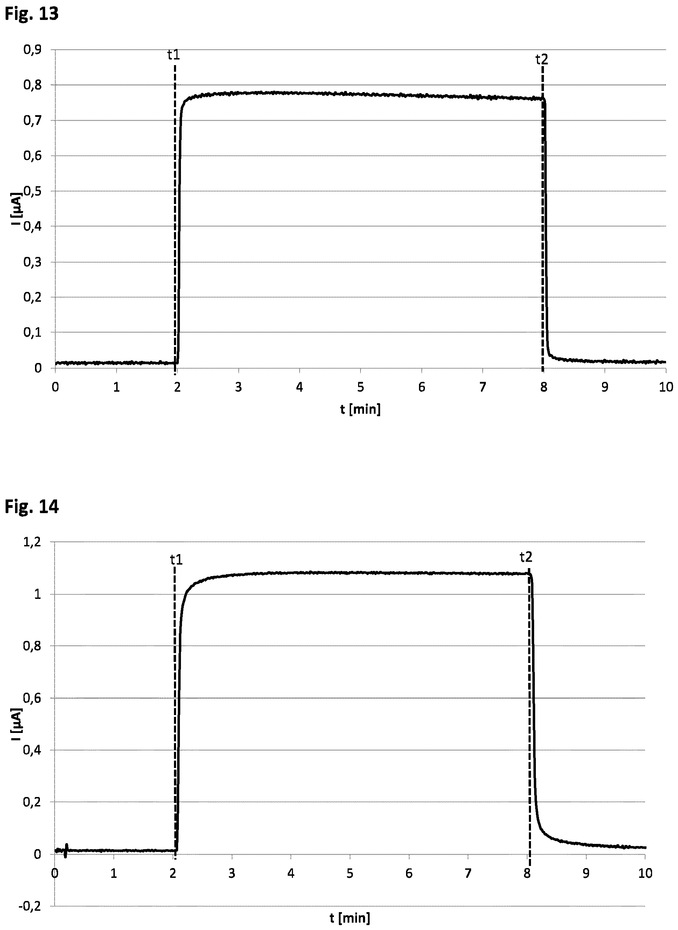

In Beispiel 12 wurde ein elektrochemischer Gassensor, der wenigstens eine erste Elektrode, eine zweite Elektrode und einen Elektrolyten aufwies, für einen definierten Zeitraum mit 5 ppm SO₂ begast. Fig. 13 zeigt die dabei erhaltene Messkurve.

Der verwendete Elektrolyt enthält als erste ringförmige Verbindung 1-Hexylpyridiniumchlorid und als zweite ringförmige Verbindung 1-Butylpyridiniummethansulfonat. Des Weiteren enthält der Elektrolyt Tris als Puffersubstanz. Die Puffersubstanz liegt in einer 0,5 molaren Konzentration vor. Die erste und die zweite ringförmige Verbindung liegen im Verhältnis 1:1 zueinander vor. Außerdem enthält der Elektrolyt 30 Gewichtsprozent Wasser. Für die erste ringförmige Verbindung entsprechend Formel (I) ist R¹ mithin ein Hexylrest, also ein Kohlenwasserstoff mit 6 C-Atomen, nämlich C₆H₁₁, für die zweite ringförmige Verbindung entsprechend Formel (I) ist R1 mithin ein Butylrest, also ein Kohlenwasserstoff mit 4 C-Atomen, nämlich C₄H₇. R² ist für beide ringförmigen Verbindungen ein Wasserstoff. A ist in beiden Fällen ein Pyridin-Ring. Für die erste ringförmige Verbindung entsprechend Formel (I) ist Y Chlorid, für die zweite Methansulfonat.

Das Beispiel zeigt also einen Elektrolyten und das Messverhalten eines elektrochemischen Gassensors mit einem Elektrolyten, der eine erste ringförmige Verbindung entsprechend Formel (VI), genauer entsprechend Formel (75) und Formel (86), eine zweite ringförmige Verbindung entsprechend Formel (VI), genauer entsprechend Formel (73) und Formel (84), Tris als Puffer in 0,5 molarer Konzentration, sowie 30% Wasser enthält.

In der gezeigten Abbildung in Fig. 13 ist auf der x-Achse die Zeit (t) in Minuten und auf der y-Achse die gemessene Signalstärke (I) in µA dargestellt. Zum Zeitpunkt t1 wurde die Begasung mit SO₂ begonnen. Zum Zeitpunkt t2 wurde die Begasung wieder beendet.

Man erkennt, dass der Gassensor auf die Präsenz des Analyten, in diesem Fall SO₂, reagiert, indem direkt nach dem Start der Begasung im Zeitpunkt t1 das Signal von 0 µA auf etwa 0,8 µA ansteigt und mit dem Ende der Begasung im Zeitpunkt t2 wieder auf 0 µA zurückgeht.

### Beispiel 13

In Beispiel 13 wurde ein elektrochemischer Gassensor, der wenigstens eine erste Elektrode, eine zweite Elektrode und einen Elektrolyten aufwies, in für einen definierten Zeitraum mit 5 ppm SO₂ begast. Fig. 14 zeigt die dabei erhaltene Messkurve.

Der verwendete Elektrolyt enthält als erste ringförmige Verbindung 1-Hexylpyridiniumchlorid und als zweite ringförmige Verbindung 1-Butylpyridiniumchlorid. Des Weiteren enthält der Elektrolyt BisTris als Puffersubstanz. Die Puffersubstanz liegt in einer 0,5 molaren Konzentration vor. Die erste und die zweite ringförmige Verbindung liegen im Verhältnis 1:1 zueinander vor. Außerdem enthält der Elektrolyt 20 Gewichtsprozent Wasser. Für die erste ringförmige Verbindung entsprechend Formel (I) ist R¹ mithin ein Hexylrest, also ein Kohlenwasserstoff mit 6 C-Atomen, nämlich C₆H₁₁, für die zweite ringförmige Verbindung entsprechend Formel (I) ist R1 mithin ein Butylrest, also ein Kohlenwasserstoff mit 4 C-Atomen, nämlich C₄H₇. R² ist für beide ringförmigen Verbindungen ein Wasserstoff. A ist in beiden Fällen ein Pyridin-Ring, Y Chlorid.

Das Beispiel zeigt also einen Elektrolyten und das Messverhalten eines elektrochemischen Gassensors mit einem Elektrolyten, der eine erste ringförmige Verbindung entsprechend Formel (VI), genauer entsprechend Formel (75) und Formel (86), eine zweite ringförmige Verbindung entsprechend Formel (VI), genauer entsprechend Formel (73) und Formel (84), BisTris als Puffer in 0,5 molarer Konzentration, sowie 20% Wasser enthält.

In der gezeigten Abbildung in Fig. 14 ist auf der x-Achse die Zeit (t) in Minuten und auf der y-Achse die gemessene Signalstärke (I) in µA dargestellt. Zum Zeitpunkt t1 wurde die Begasung mit SO₂ begonnen. Zum Zeitpunkt t2 wurde die Begasung wieder beendet.

Man erkennt, dass der Gassensor auf die Präsenz des Analyten, in diesem Fall SO₂, reagiert, indem direkt nach dem Start der Begasung im Zeitpunkt t1 das Signal von 0 µA auf etwa 1 µA ansteigt und mit dem Ende der Begasung im Zeitpunkt t2 wieder auf 0 µA zurückgeht.

### Beispiel 14

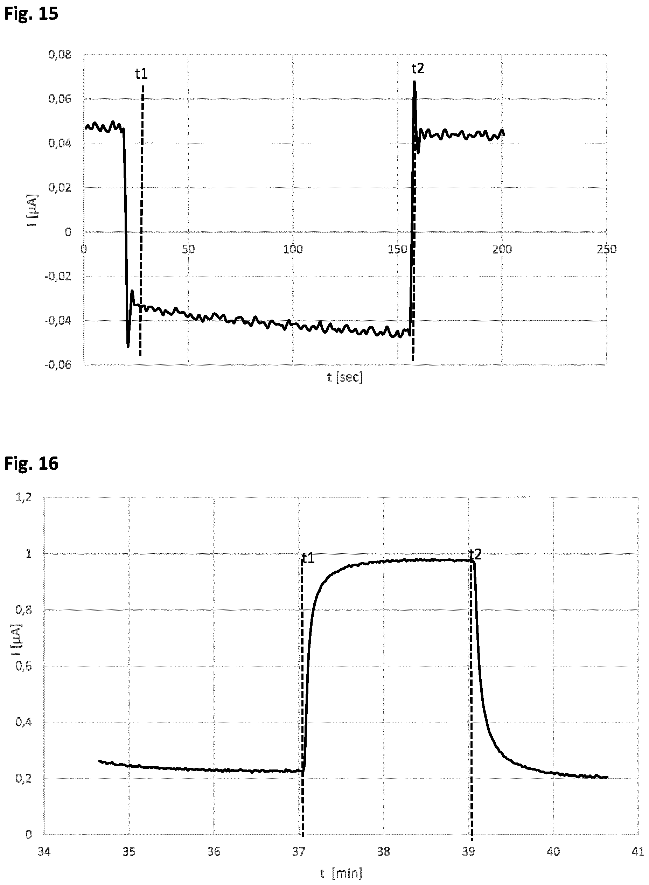

In Beispiel 14 wurde ein elektrochemischer Gassensor, der wenigstens eine erste Elektrode, eine zweite Elektrode und einen Elektrolyten aufwies, in für einen definierten Zeitraum mit 5 ppm NO₂ begast. Fig. 15 zeigt die dabei erhaltene Messkurve.

Der verwendete Elektrolyt enthält als erste ringförmige Verbindung 1-Hexylpyridiniumchlorid und als zweite ringförmige Verbindung 1-Butylpyridiniumchlorid. Des Weiteren enthält der Elektrolyt BisTris als Puffersubstanz. Die Puffersubstanz liegt in einer 0,5 molaren Konzentration vor. Die erste und die zweite ringförmige Verbindung liegen im Verhältnis 1:1 zueinander vor. Außerdem enthält der Elektrolyt 20 Gewichtsprozent Wasser. Für die erste ringförmige Verbindung entsprechend Formel (I) ist R¹ mithin ein Hexylrest, also ein Kohlenwasserstoff mit 6 C-Atomen, nämlich C₆H₁₁, für die zweite ringförmige Verbindung entsprechend Formel (I) ist R1 mithin ein Butylrest, also ein Kohlenwasserstoff mit 4 C-Atomen, nämlich C₄H₇. R² ist für beide ringförmigen Verbindungen ein Wasserstoff. A ist in beiden Fällen ein Pyridin-Ring, Y Chlorid.

Das Beispiel zeigt also einen Elektrolyten und das Messverhalten eines elektrochemischen Gassensors mit einem Elektrolyten, der eine erste ringförmige Verbindung entsprechend Formel (VI), genauer entsprechend Formel (75) und Formel (86), eine zweite ringförmige Verbindung entsprechend Formel (VI), genauer entsprechend Formel (73) und Formel (84), BisTris als Puffer in 0,5 molarer Konzentration, sowie 20% Wasser enthält.

In der gezeigten Abbildung in Fig. 15 ist auf der x-Achse die Zeit (t) in Sekunden und auf der y-Achse die gemessene Signalstärke (I) in µA dargestellt. Zum Zeitpunkt t1 wurde die Begasung mit NO₂ begonnen. Zum Zeitpunkt t2 wurde die Begasung wieder beendet.

Man erkennt, dass der Gassensor auf die Präsenz des Analyten, in diesem Fall NO₂, reagiert, indem direkt nach dem Start der Begasung im Zeitpunkt t1 das Signal von etwa +0,01 µA auf etwa -0,04 µA abfällt und mit dem Ende der Begasung im Zeitpunkt t2 wieder auf +0,01 µA zurückgeht.

### Beispiel 15

In Beispiel 15 wurde ein elektrochemischer Gassensor, der wenigstens eine erste Elektrode, eine zweite Elektrode und einen Elektrolyten aufwies, in für einen definierten Zeitraum mit 5 ppm SO₂ begast. Fig. 16 zeigt die dabei erhaltene Messkurve.

Der verwendete Elektrolyt enthält als erste ringförmige Verbindung 1-Hexylpyridiniumchlorid und als zweite ringförmige Verbindung 1-Butylpyridiniumchlorid. Des Weiteren enthält der Elektrolyt EGTA als Puffersubstanz. Die Puffersubstanz liegt in einer 0,25 molaren Konzentration vor. Die erste und die zweite ringförmige Verbindung liegen im Verhältnis 1:1 zueinander vor. Außerdem enthält der Elektrolyt 23 Gewichtsprozent Wasser. Für die erste ringförmige Verbindung entsprechend Formel (I) ist R¹ mithin ein Hexylrest, also ein Kohlenwasserstoff mit 6 C-Atomen, nämlich C₆H₁₁, für die zweite ringförmige Verbindung entsprechend Formel (I) ist R1 mithin ein Butylrest, also ein Kohlenwasserstoff mit 4 C-Atomen, nämlich C₄H₇. R² ist für beide ringförmigen Verbindungen ein Wasserstoff. A ist in beiden Fällen ein Pyridin-Ring, Y Chlorid.

Das Beispiel zeigt also einen Elektrolyten und das Messverhalten eines elektrochemischen Gassensors mit einem Elektrolyten, der eine erste ringförmige Verbindung entsprechend Formel (VI), genauer entsprechend Formel (75) und Formel (86), eine zweite ringförmige Verbindung entsprechend Formel (VI), genauer entsprechend Formel (73) und Formel (84), EGTA als Puffer in 0,25 molarer Konzentration, sowie 23% Wasser enthält.

In der gezeigten Abbildung in Fig. 16 ist auf der x-Achse die Zeit (t) in Minuten und auf der y-Achse die gemessene Signalstärke (I) in µA dargestellt. Zum Zeitpunkt t1 wurde die Begasung mit SO₂ begonnen. Zum Zeitpunkt t2 wurde die Begasung wieder beendet.

Man erkennt, dass der Gassensor auf die Präsenz des Analyten, in diesem Fall SO₂, reagiert, indem direkt nach dem Start der Begasung im Zeitpunkt t1 das Signal von 0,2 µA auf etwa 1 µA ansteigt und mit dem Ende der Begasung im Zeitpunkt t2 wieder auf 0,2 µA zurückgeht.

### Beispiel 16

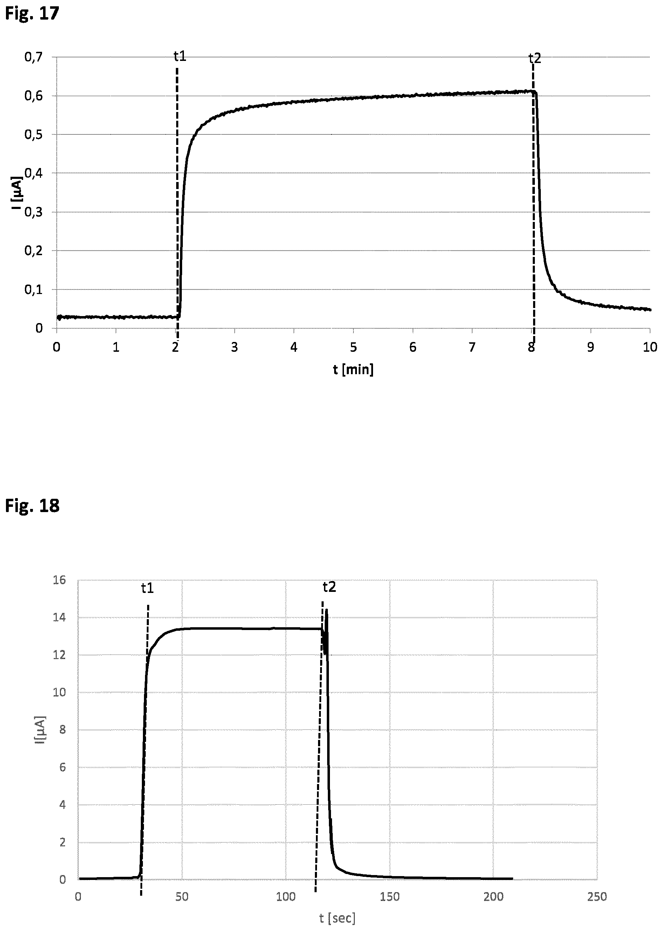

In Beispiel 16 wurde ein elektrochemischer Gassensor, der wenigstens eine erste Elektrode, eine zweite Elektrode und einen Elektrolyten aufwies, in für einen definierten Zeitraum mit 5 ppm SO₂ begast. Fig. 17 zeigt die dabei erhaltene Messkurve.

Der verwendete Elektrolyt enthält als erste ringförmige Verbindung 1-Hexylpyridiniumchlorid und als zweite ringförmige Verbindung 1-Butylpyridiniumchlorid. Des Weiteren enthält der Elektrolyt ADA (N-(2-Acetamido)-iminodiessigsäure) als Puffersubstanz. Die Puffersubstanz liegt in einer 0,5 molaren Konzentration vor. Die erste und die zweite ringförmige Verbindung liegen im Verhältnis 1:1 zueinander vor. Außerdem enthält der Elektrolyt 30 Gewichtsprozent Wasser. Für die erste ringförmige Verbindung entsprechend Formel (I) ist R¹ mithin ein Hexylrest, also ein Kohlenwasserstoff mit 6 C-Atomen, nämlich C₆H₁₁, für die zweite ringförmige Verbindung entsprechend Formel (I) ist R1 mithin ein Butylrest, also ein Kohlenwasserstoff mit 4 C-Atomen, nämlich C₄H₇. R² ist für beide ringförmigen Verbindungen ein Wasserstoff. A ist in beiden Fällen ein Pyridin-Ring, Y Chlorid.

Das Beispiel zeigt also einen Elektrolyten und das Messverhalten eines elektrochemischen Gassensors mit einem Elektrolyten, der eine erste ringförmige Verbindung entsprechend Formel (VI), genauer entsprechend Formel (75) und Formel (86), eine zweite ringförmige Verbindung entsprechend Formel (VI), genauer entsprechend Formel (73) und Formel (84), ADA als Puffer in 0,5 molarer Konzentration, sowie 30% Wasser enthält.

In der gezeigten Abbildung in Fig. 17 ist auf der x-Achse die Zeit (t) in Minuten und auf der y-Achse die gemessene Signalstärke (I) in µA dargestellt. Zum Zeitpunkt t1 wurde die Begasung mit SO₂ begonnen. Zum Zeitpunkt t2 wurde die Begasung wieder beendet.

Man erkennt, dass der Gassensor auf die Präsenz des Analyten, in diesem Fall SO₂, reagiert, indem direkt nach dem Start der Begasung im Zeitpunkt t1 das Signal von 0 µA auf etwa 0,6 µA ansteigt und mit dem Ende der Begasung im Zeitpunkt t2 wieder auf 0 µA zurückgeht.

### Beispiel 17

In Beispiel 17 wurde ein elektrochemischer Gassensor, der wenigstens eine erste Elektrode, eine zweite Elektrode und einen Elektrolyten aufwies, in für einen definierten Zeitraum mit 20 ppm SO₂ begast. Fig. 18 zeigt die dabei erhaltene Messkurve.

Der verwendete Elektrolyt enthält als erste ringförmige Verbindung 1-Methyl-1-propylpiperidiniumchlorid und als zweite ringförmige Verbindung 1-Butyl-4-methylpyridiniumchlorid. Des Weiteren enthält der Elektrolyt Kaliumhydrogenphthalat als Puffersubstanz, sowie CuCl₂ als Mediator (Reaktionsvermittler). Die Puffersubstanz liegt in einer 0,37 molaren Konzentration vor, der Reaktionsvermittler in einer 0,37 molaren Konzentration. Die erste und die zweite ringförmige Verbindung liegen im Verhältnis 1:1 zueinander vor. Außerdem enthält der Elektrolyt 20 Gewichtsprozent Wasser. Für die erste ringförmige Verbindung entsprechend Formel (I) ist R¹ mithin ein Methylrest, also ein Kohlenwasserstoff mit 1 C-Atomen, nämlich CH₃, für die zweite ringförmige Verbindung entsprechend Formel (I) ist R1 mithin ein Butylrest, also ein Kohlenwasserstoff mit 4 C-Atomen, nämlich C₄H₇. R² ist für die erste ringförmige Verbindung ein Propylrest, also eine Kohlenwasserstoffrest mit 3 C-Atomen, für die zweite ringförmigen Verbindungen ein Methylrest, also ein Kohlenwasserstoffrest mit einem C-Atom. A ist für die erste ringförmige Verbindung ein Piperidinring, für die zweite ringförmige Verbindung ein Pyridinring, Y in beiden Fällen Chlorid.

Das Beispiel zeigt also einen Elektrolyten und das Messverhalten eines elektrochemischen Gassensors mit einem Elektrolyten, der eine erste ringförmige Verbindung entsprechend Formel (VII), genauer entsprechend Formel (47), eine zweite ringförmige Verbindung entsprechend Formel (VI), genauer entsprechend Formel (73) und Formel (84), Kaliumhydrogenphtalat als Puffer, CuCl₂ als Reaktionsvermittler, sowie 20% Wasser enthält.

In der gezeigten Abbildung in Fig. 18 ist auf der x-Achse die Zeit (t) in Minuten und auf der y-Achse die gemessene Signalstärke (I) in µA dargestellt. Der Sensor wurde über zwei Zyklen von jeweils ca. 1,5 Minuten begast. In den Zeitpunkten t1 und t3 wurde jeweils mit der Begasung begonnen. In den Zeitpunkten t2 und t4 wurde die Begasung jeweils wieder eingestellt.

Man erkennt, dass der Gassensor auf die Präsenz des Analyten, in diesem Fall SO₂, reagiert, indem direkt nach dem Start der Begasung in allen drei Zyklen das Signal von 0 µA auf etwa 16 µA ansteigt und mit dem Ende der Begasung wieder auf 0 µA zurückgeht.

### Beispiel 18

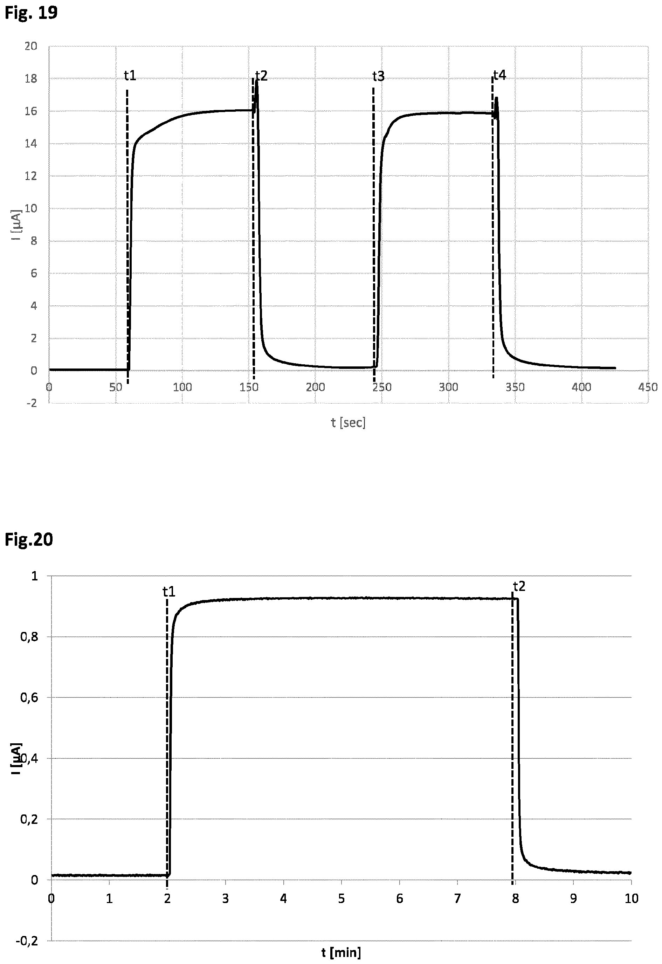

In Beispiel 18 wurde ein elektrochemischer Gassensor, der wenigstens eine erste Elektrode, eine zweite Elektrode und einen Elektrolyten aufwies, in für einen definierten Zeitraum mit 20 ppm SO₂ begast. Fig. 19 zeigt die dabei erhaltene Messkurve.

Der verwendete Elektrolyt enthält als erste ringförmige Verbindung 1-Methyl-1-propylpiperidiniumchlorid und als zweite ringförmige Verbindung 1-Butyl-3-methylpyridiniumchlorid. Des Weiteren enthält der Elektrolyt Kaliumhydrogenphthalat als Puffersubstanz, sowie CuCl₂ als Mediator (Reaktionsvermittler). Die Puffersubstanz liegt in einer 0,37 molaren Konzentration vor, der Reaktionsvermittler in einer 0,37 molaren Konzentration. Die erste und die zweite ringförmige Verbindung liegen im Verhältnis 1:1 zueinander vor. Außerdem enthält der Elektrolyt 20 Gewichtsprozent Wasser. Für die erste ringförmige Verbindung entsprechend Formel (I) ist R¹ mithin ein Methylrest, also ein Kohlenwasserstoff mit 1 C-Atomen, nämlich CH₃, für die zweite ringförmige Verbindung entsprechend Formel (I) ist R1 mithin ein Butylrest, also ein Kohlenwasserstoff mit 4 C-Atomen, nämlich C₄H₇. R² ist für die erste ringförmige Verbindung ein Propylrest, also eine Kohlenwasserstoffrest mit 3 C-Atomen, für die zweite ringförmigen Verbindungen ein Methylrest, also ein Kohlenwasserstoffrest mit einem C-Atom. A ist für die erste ringförmige Verbindung ein Piperidinring, für die zweite ringförmige Verbindung ein Pyridinring, Y in beiden Fällen Chlorid.

Das Beispiel zeigt also einen Elektrolyten und das Messverhalten eines elektrochemischen Gassensors mit einem Elektrolyten, der eine erste ringförmige Verbindung entsprechend Formel (VII), genauer entsprechend Formel (47), eine zweite ringförmige Verbindung entsprechend Formel (VI), genauer entsprechend Formel (73) und Formel (84), Kaliumhydrogenphtalat als Puffer, CuCl₂ als Reaktionsvermittler, sowie 20% Wasser enthält.

In der gezeigten Abbildung in Fig. 19 ist auf der x-Achse die Zeit (t) in Sekunden und auf der y-Achse die gemessene Signalstärke (I) in µA dargestellt. Der Sensor wurde über zwei Zyklen von jeweils ca. 1,5 Minuten begast. In den Zeitpunkten t1 und t3 wurde jeweils mit der Begasung begonnen. In den Zeitpunkten t2 und t4 wurde die Begasung jeweils wieder eingestellt.

Man erkennt, dass der Gassensor auf die Präsenz des Analyten, in diesem Fall SO₂, reagiert, indem direkt nach dem Start der Begasung in allen drei Zyklen das Signal von 0 µA auf etwa 16 µA ansteigt und mit dem Ende der Begasung wieder auf 0 µA zurückgeht.

### Beispiel 19

In Beispiel 19 wurde ein elektrochemischer Gassensor, der wenigstens eine erste Elektrode, eine zweite Elektrode und einen Elektrolyten aufwies, in für einen definierten Zeitraum mit 5 ppm SO₂ begast. Fig. 20 zeigt die dabei erhaltene Messkurve.

Der verwendete Elektrolyt enthält als ringförmige Verbindung 1-Butylpyridiniumchlorid und als zweite ringförmige Verbindung 1-Hexylpyridiniummethansulfonat. Des Weiteren enthält der Elektrolyt BisTris als Puffersubstanz, sowie Ethylenglycol (1,2,3-Propantriol) als Zuschlag. Die Puffersubstanz liegt in einer 0,5 molaren Konzentration vor, der Zuschlag in einer 0,16 molaren Konzentration. Außerdem enthält der Elektrolyt 30 Gewichtsprozent Wasser. Für die ringförmige Verbindung entsprechend Formel (I) ist R¹ mithin ein Butylrest, also ein Kohlenwasserstoff mit 4 C-Atomen, nämlich C₄H₇, R² ist ein Wasserstoffatom, A ein Pyridinring und Y Chlorid, für die zweite ringförmige Verbindung entsprechend Formel (I) ist R1 mithin ein Hexylrest, also ein Kohlenwasserstoff mit 6 C-Atomen, nämlich C₆H₁₃. R² ist für beide ringförmigen Verbindungen ein Wasserstoff. A ist in beiden Fällen ein Pyridin-Ring, Y Methansulfonat..

Das Beispiel zeigt also einen Elektrolyten und das Messverhalten eines elektrochemischen Gassensors mit einem Elektrolyten, der eine ringförmige Verbindung entsprechend Formel (VI), genauer entsprechend Formel (73) und Formel (84), BisTris als Puffer, Ethylenglycol als Zuschlag, sowie 30% Wasser enthält.

In der gezeigten Abbildung in Fig. 20 ist auf der x-Achse die Zeit (t) in Minuten und auf der y-Achse die gemessene Signalstärke (I) in µA dargestellt. Der Sensor wurde über einen Zeitraum von ca. 6 Minuten begast. Im Zeitpunkten t1 wurde jeweils mit der Begasung begonnen. Im Zeitpunkten t2 wurde die Begasung wieder eingestellt.

Man erkennt, dass der Gassensor auf die Präsenz des Analyten, in diesem Fall SO₂, reagiert, indem direkt nach dem Start der Begasung im Zeitpunkt t1 das Signal von 0 µA auf etwa 0,9 µA ansteigt und mit dem Ende der Begasung wieder auf 0 µA zurückgeht.

### Beispiel 20

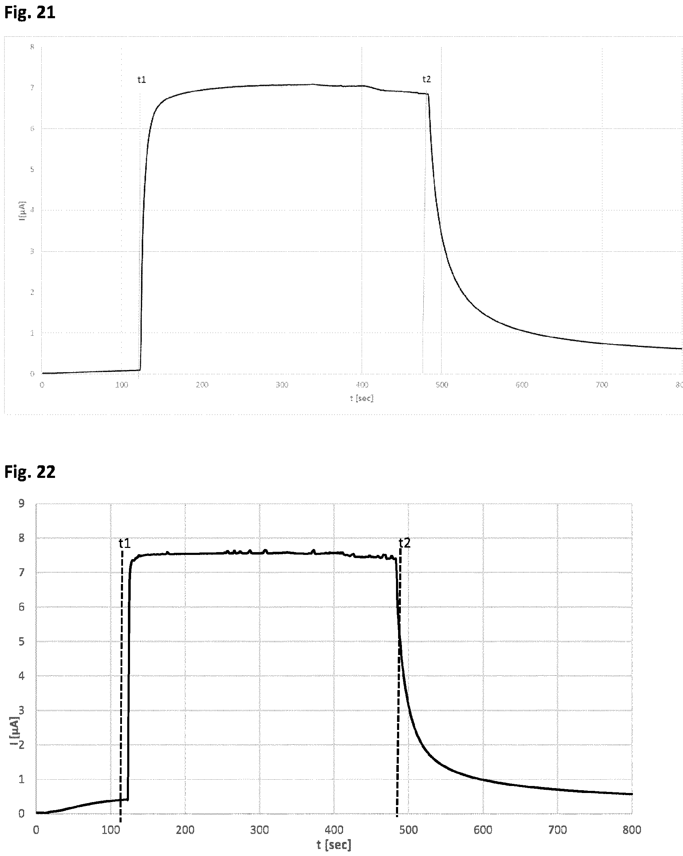

In Beispiel 20 wurde ein elektrochemischer Gassensor, der wenigstens eine erste Elektrode, eine zweite Elektrode und einen Elektrolyten aufwies, in für einen definierten Zeitraum mit 20 ppm SO₂ begast. Fig. 21 zeigt die dabei erhaltene Messkurve.

Der verwendete Elektrolyt enthält als erste ringförmige Verbindung 1-Methyl-1-propylpiperidiniumchlorid und als zweite ringförmige Verbindung 1-Butyl-2-methylpyridiniumchlorid. Des Weiteren enthält der Elektrolyt Kaliumhydrogenphthalat als Puffersubstanz, sowie CuCl₂ als Mediator (Reaktionsvermittler). Die Puffersubstanz liegt in einer 0,5 molaren Konzentration vor, der Reaktionsvermittler in einer 0,1 molaren Konzentration. Die erste und die zweite ringförmige Verbindung liegen im Verhältnis 1:1 zueinander vor. Außerdem enthält der Elektrolyt 30Gewichtsprozent Wasser. Für die erste ringförmige Verbindung entsprechend Formel (I) ist R¹ mithin ein Methylrest, also ein Kohlenwasserstoff mit 1 C-Atomen, nämlich CH₃, für die zweite ringförmige Verbindung entsprechend Formel (I) ist R1 mithin ein Butylrest, also ein Kohlenwasserstoff mit 4 C-Atomen, nämlich C₄H₇. R² ist für die erste ringförmige Verbindung ein Propylrest, also eine Kohlenwasserstoffrest mit 3 C-Atomen, für die zweite ringförmigen Verbindungen ein Methylrest, also ein Kohlenwasserstoffrest mit einem C-Atom. A ist für die erste ringförmige Verbindung ein Piperidinring, für die zweite ringförmige Verbindung ein Pyridinring, Y in beiden Fällen Chlorid.

Das Beispiel zeigt also einen Elektrolyten und das Messverhalten eines elektrochemischen Gassensors mit einem Elektrolyten, der eine erste ringförmige Verbindung entsprechend Formel (VII), genauer entsprechend Formel (47), eine zweite ringförmige Verbindung entsprechend Formel (VI), genauer entsprechend Formel (73) und Formel (84), Kaliumhydrogenphtalat als Puffer, CuCl₂ als Reaktionsvermittler, sowie 30% Wasser enthält.

In der gezeigten Abbildung in Fig. 21 ist auf der x-Achse die Zeit (t) in Sekunden und auf der y-Achse die gemessene Signalstärke (I) in µA dargestellt. Im Zeitpunkten t1 wurde mit der Begasung begonnen. Im den Zeitpunkten t2 wurde die Begasung jeweils wieder eingestellt.

Man erkennt, dass der Gassensor auf die Präsenz des Analyten, in diesem Fall SO₂, reagiert, indem das Signal direkt nach dem Start der Begasung von 0 µA auf etwa 5 µA ansteigt und unmittelbar mit dem Ende der Begasung wieder auf 0,5 µA abfällt.

### Beispiel 21

In Beispiel 21 wurde ein elektrochemischer Gassensor, der wenigstens eine erste Elektrode, eine zweite Elektrode und einen Elektrolyten aufwies, in für einen definierten Zeitraum mit 20 ppm SO₂ begast. Fig. 22 zeigt die dabei erhaltene Messkurve.

Der verwendete Elektrolyt enthält als erste ringförmige Verbindung 1-Methyl-1-propylpiperidiniumchlorid und als zweite ringförmige Verbindung 1-Butyl-3-methylpyridiniumchlorid. Des Weiteren enthält der Elektrolyt Kaliumhydrogenphthalat als Puffersubstanz, sowie CuCl₂ als Mediator (Reaktionsvermittler). Die Puffersubstanz liegt in einer 0,5 molaren Konzentration vor, der Reaktionsvermittler in einer 0,05 molaren Konzentration. Die erste und die zweite ringförmige Verbindung liegen im Verhältnis 1:9 zueinander vor. Außerdem enthält der Elektrolyt 22 Gewichtsprozent Wasser. Für die erste ringförmige Verbindung entsprechend Formel (I) ist R¹ mithin ein Methylrest, also ein Kohlenwasserstoff mit 1 C-Atomen, nämlich CH₃, für die zweite ringförmige Verbindung entsprechend Formel (I) ist R1 mithin ein Butylrest, also ein Kohlenwasserstoff mit 4 C-Atomen, nämlich C₄H₇. R² ist für die erste ringförmige Verbindung ein Propylrest, also eine Kohlenwasserstoffrest mit 3 C-Atomen, für die zweite ringförmigen Verbindungen ein Methylrest, also ein Kohlenwasserstoffrest mit einem C-Atom. A ist für die erste ringförmige Verbindung ein Piperidinring, für die zweite ringförmige Verbindung ein Pyridinring, Y in beiden Fällen Chlorid.

Das Beispiel zeigt also einen Elektrolyten und das Messverhalten eines elektrochemischen Gassensors mit einem Elektrolyten, der eine erste ringförmige Verbindung entsprechend Formel (VII), genauer entsprechend Formel (47), eine zweite ringförmige Verbindung entsprechend Formel (VI), genauer entsprechend Formel (73) und Formel (84), Kaliumhydrogenphtalat als Puffer, CuCl₂ als Reaktionsvermittler, sowie 22% Wasser enthält.

In der gezeigten Abbildung in Fig. 22 ist auf der x-Achse die Zeit (t) in Sekunden und auf der y-Achse die gemessene Signalstärke (I) in µA dargestellt. Im Zeitpunkten t1 wurde mit der Begasung begonnen. Im Zeitpunkten t2 wurde die Begasung wieder eingestellt.

Man erkennt, dass der Gassensor auf die Präsenz des Analyten, in diesem Fall SO₂, reagiert, indem direkt nach dem Start der Begasung in allen drei Zyklen das Signal von 0 µA auf etwa 7,5 µA ansteigt und unmittelbar mit dem Ende der Begasung wieder abfällt.

### Beispiel 22

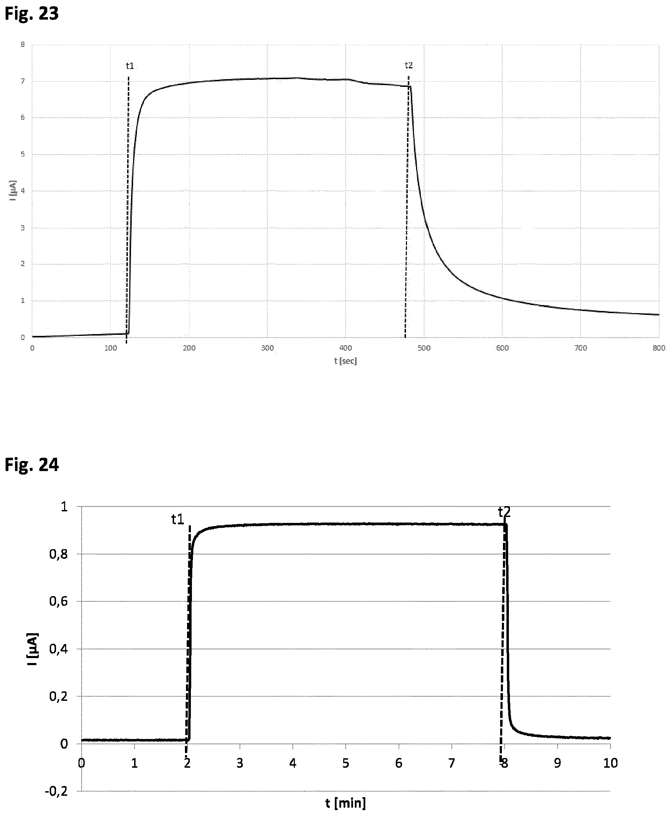

In Beispiel 22 wurde ein elektrochemischer Gassensor, der wenigstens eine erste Elektrode, eine zweite Elektrode und einen Elektrolyten aufwies, in für einen definierten Zeitraum mit 20 ppm SO₂ begast. Fig. 23 zeigt die dabei erhaltene Messkurve.

Der verwendete Elektrolyt enthält als erste ringförmige Verbindung 1-Methyl-1-propylpiperidiniumchlorid und als zweite ringförmige Verbindung 1-Butyl-2-methylpyridiniumchlorid. Des Weiteren enthält der Elektrolyt Kaliumhydrogenphthalat als Puffersubstanz, sowie CuCl₂ als Mediator (Reaktionsvermittler). Die Puffersubstanz liegt in einer 0,5 molaren Konzentration vor, der Reaktionsvermittler in einer 0,1 molaren Konzentration. Die erste und die zweite ringförmige Verbindung liegen im Verhältnis 1:1 zueinander vor. Außerdem enthält der Elektrolyt 22 Gewichtsprozent Wasser. Für die erste ringförmige Verbindung entsprechend Formel (I) ist R¹ mithin ein Methylrest, also ein Kohlenwasserstoff mit 1 C-Atomen, nämlich CH₃, für die zweite ringförmige Verbindung entsprechend Formel (I) ist R1 mithin ein Butylrest, also ein Kohlenwasserstoff mit 4 C-Atomen, nämlich C₄H₇. R² ist für die erste ringförmige Verbindung ein Propylrest, also eine Kohlenwasserstoffrest mit 3 C-Atomen, für die zweite ringförmigen Verbindungen ein Methylrest, also ein Kohlenwasserstoffrest mit einem C-Atom. A ist für die erste ringförmige Verbindung ein Piperidinring, für die zweite ringförmige Verbindung ein Pyridinring, Y in beiden Fällen Chlorid.

Das Beispiel zeigt also einen Elektrolyten und das Messverhalten eines elektrochemischen Gassensors mit einem Elektrolyten, der eine erste ringförmige Verbindung entsprechend Formel (VII), genauer entsprechend Formel (47), eine zweite ringförmige Verbindung entsprechend Formel (VI), genauer entsprechend Formel (73) und Formel (84), Kaliumhydrogenphtalat als Puffer, CuCl₂ als Reaktionsvermittler, sowie 22% Wasser enthält.

In der gezeigten Abbildung in Fig. 23 ist auf der x-Achse die Zeit (t) in Sekunden und auf der y-Achse die gemessene Signalstärke (I) in µA dargestellt. Im Zeitpunkten t1 wurde mit der Begasung begonnen. Im den Zeitpunkten t2 wurde die Begasung jeweils wieder eingestellt.

Man erkennt, dass der Gassensor auf die Präsenz des Analyten, in diesem Fall SO₂, reagiert, indem das Signal direkt nach dem Start der Begasung von 0 µA auf etwa 7 µA ansteigt und unmittelbar mit dem Ende der Begasung wieder auf 0,5 µA abfällt.

### Beispiel 23

In Beispiel 23 wurde ein elektrochemischer Gassensor, der wenigstens eine erste Elektrode, eine zweite Elektrode und einen Elektrolyten aufwies, für einen definierten Zeitraum mit 5 ppm SO₂ begast. Fig. 24 zeigt die dabei erhaltene Messkurve.

Der verwendete Elektrolyt enthält als erste ringförmige Verbindung 1-Hexylpyridiniummethansulfonat und als zweite ringförmige Verbindung 1-Butylpyridiniumchlorid. Des Weiteren enthält der Elektrolyt BisTris als Puffersubstanz, sowie 1% Ethylenglycol als Zuschlag. Die Puffersubstanz liegt in einer 0,5 molaren Konzentration vor. Die erste und die zweite ringförmige Verbindung liegen im Verhältnis 1:1 zueinander vor. Außerdem enthält der Elektrolyt 23 Gewichtsprozent Wasser. Für die erste ringförmige Verbindung entsprechend Formel (I) ist R¹ mithin ein Hexylrest, also ein Kohlenwasserstoff mit 6 C-Atomen, nämlich C₆H₁₁, für die zweite ringförmige Verbindung entsprechend Formel (I) ist R1 mithin ein Butylrest, also ein Kohlenwasserstoff mit 4 C-Atomen, nämlich C₄H₇. R² ist für beide ringförmigen Verbindungen ein Wasserstoff. A ist in beiden Fällen ein Pyridin-Ring, Y ist für die erste ringförmige Verbindung Methansulfonat, für die zweite ringförmige Verbindung Chlorid.

Das Beispiel zeigt also einen Elektrolyten und das Messverhalten eines elektrochemischen Gassensors mit einem Elektrolyten, der eine erste ringförmige Verbindung entsprechend Formel (VI), genauer entsprechend Formel (75) und Formel (86), eine zweite ringförmige Verbindung entsprechend Formel (VI), genauer entsprechend Formel (73) und Formel (84), BisTris als Puffer in 0,5 molarer Konzentration, Ethylenglycol als Zuschlag, sowie 23% Wasser enthält.

In der gezeigten Abbildung in Fig. 24 ist auf der x-Achse die Zeit (t) in Minuten und auf der y-Achse die gemessene Signalstärke (I) in µA dargestellt. Zum Zeitpunkt t1 wurde die Begasung mit SO₂ begonnen. Zum Zeitpunkt t2 wurde die Begasung wieder beendet.

Man erkennt, dass der Gassensor auf die Präsenz des Analyten, in diesem Fall SO₂, reagiert, indem direkt nach dem Start der Begasung im Zeitpunkt t1 das Signal von etwa 0 µA auf etwa 0,9 µA ansteigt und mit dem Ende der Begasung im Zeitpunkt t2 wieder auf den Ausgangswert abfällt.

### Beispiel 24

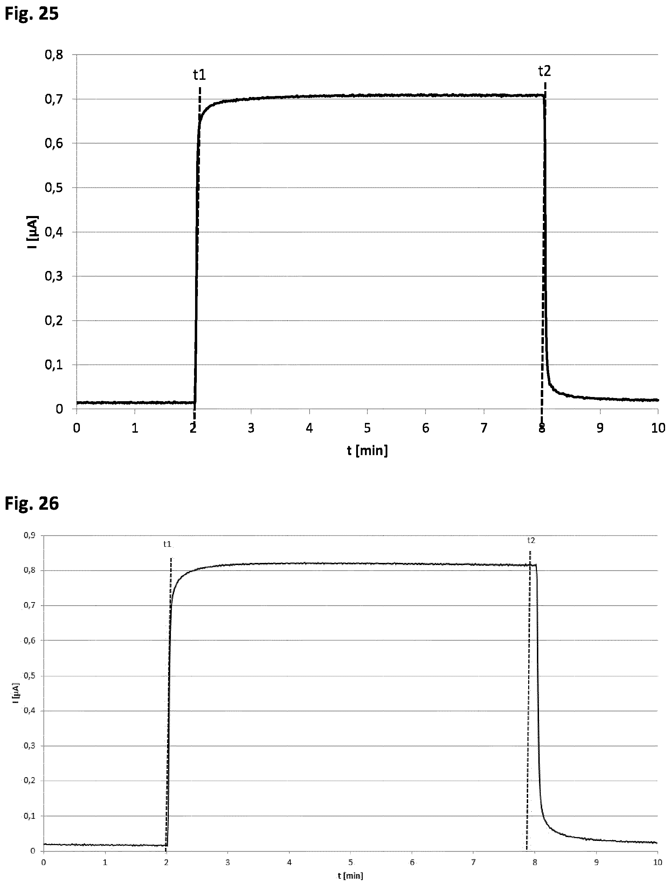

In Beispiel 24 wurde ein elektrochemischer Gassensor, der wenigstens eine erste Elektrode, eine zweite Elektrode und einen Elektrolyten aufwies, für einen definierten Zeitraum mit 5 ppm SO₂ begast. Fig. 25 zeigt die dabei erhaltene Messkurve.

Der verwendete Elektrolyt enthält als ringförmige Verbindung 1-Hexylpyridiniumchlorid. Des Weiteren enthält der Elektrolyt BisTris als Puffersubstanz und Ethylenglycol als Zuschlag. Die Puffersubstanz liegt in einer 0,5 molaren Konzentration vor, der Zuschlag in einer Konzentration von 1 Gewichtsprozent. Außerdem enthält der Elektrolyt 28 Gewichtsprozent Wasser.

Das Beispiel zeigt also einen Elektrolyten und das Messverhalten eines elektrochemischen Gassensors mit einem Elektrolyten, der eine ringförmige Verbindung entsprechend Formel (VI), genauer entsprechend Formel (73) und Formel (84), eine weitere Komponente, nämlich Tris als Puffer in 0,5 molarer Konzentration, sowie 28% Wasser enthält. Für die ringförmige Verbindung entsprechend Formel (I) ist R¹ mithin ein Butylrest, also ein Kohlenwasserstoff mit 4 C-Atomen, nämlich C₄H₇. R² ist ein Wasserstoff, A ein Pyridin-Ring und Y ist Chlorid.

In der gezeigten Abbildung in Fig. 25 ist auf der x-Achse die Zeit (t) in Minuten und auf der y-Achse die gemessene Signalstärke (I) in µA dargestellt. Zum Zeitpunkt t1 wurde die Begasung mit 5ppm SO₂ begonnen. Zum Zeitpunkt t2 wurde die Begasung wieder beendet.

Man erkennt, dass der Gassensor auf die Präsenz des Analyten, in diesem Fall SO₂, reagiert, indem direkt nach dem Start der Begasung im Zeitpunkt t1 das Signal von 0 µA auf etwa 0,7 µA ansteigt und mit dem Ende der Begasung im Zeitpunkt t2 wieder auf 0 µA zurückgeht.

### Beispiel 25

In Beispiel 25 wurde ein elektrochemischer Gassensor, der wenigstens eine erste Elektrode, eine zweite Elektrode und einen Elektrolyten aufwies, für einen definierten Zeitraum mit 5 ppm SO₂ begast. Fig. 26 zeigt die dabei erhaltene Messkurve.

Der verwendete Elektrolyt enthält als ringförmige Verbindung 1-Hexylpyridiniummethynsulfonat. Des Weiteren enthält der Elektrolyt BisTris als Puffersubstanz und 1,2,3-Propantriol (Glycerin) als Zuschlag. Die Puffersubstanz liegt in einer 0,5 molaren Konzentration vor, der Zuschlag in einer Konzentration von 1 Gewichtsprozent. Außerdem enthält der Elektrolyt 28 Gewichtsprozent Wasser.

Das Beispiel zeigt also einen Elektrolyten und das Messverhalten eines elektrochemischen Gassensors mit einem Elektrolyten, der eine ringförmige Verbindung entsprechend Formel (VI), genauer entsprechend Formel (75) und Formel (86), eine weitere Komponente, nämlich Tris als Puffer in 0,5 molarer Konzentration, sowie 28% Wasser enthält. Für die ringförmige Verbindung entsprechend Formel (I) ist R¹ mithin ein Hexylrest, also ein Kohlenwasserstoff mit 6 C-Atomen, nämlich C₆H₁₁. R² ist ein Wasserstoff, A ein Pyridin-Ring und Y ist Methansulfonat.

In der gezeigten Abbildung in Fig. 26 ist auf der x-Achse die Zeit (t) in Minuten und auf der y-Achse die gemessene Signalstärke (I) in µA dargestellt. Zum Zeitpunkt t1 wurde die Begasung mit 5ppm SO₂ begonnen. Zum Zeitpunkt t2 wurde die Begasung wieder beendet.

Man erkennt, dass der Gassensor auf die Präsenz des Analyten, in diesem Fall SO₂, reagiert, indem direkt nach dem Start der Begasung im Zeitpunkt t1 das Signal von 0 µA auf etwa 0,8 µA ansteigt und mit dem Ende der Begasung im Zeitpunkt t2 wieder auf 0 µA zurückgeht.

### Beispiel 26

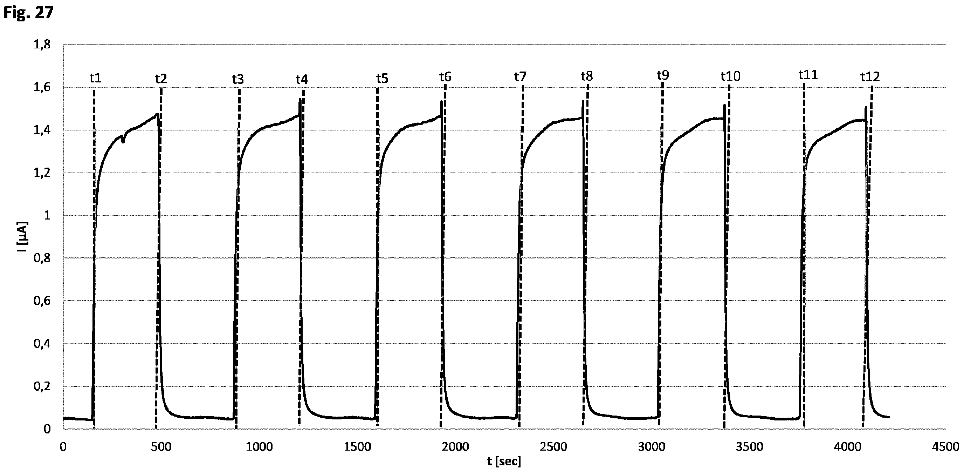

In Beispiel 26 wurde ein elektrochemischer Gassensor, der wenigstens eine erste Elektrode, eine zweite Elektrode und einen Elektrolyten aufwies, in für einen definierten Zeitraum mit 1 ppm SO₂ begast. Fig. 27 zeigt die dabei erhaltene Messkurve.

Der verwendete Elektrolyt enthält als erste ringförmige Verbindung 1-Methyl-1-propylpiperidiniumchlorid und als zweite ringförmige Verbindung 1-Butyl-3-methylpyridiniumchlorid. Des Weiteren enthält der Elektrolyt Kaliumhydrogenphthalat als Puffersubstanz, sowie CuCl₂ als Mediator (Reaktionsvermittler). Die Puffersubstanz liegt in einer 0,5 molaren Konzentration vor, der Reaktionsvermittler in einer 0,25 molaren Konzentration. Die erste und die zweite ringförmige Verbindung liegen im Verhältnis 1:9 zueinander vor. Außerdem enthält der Elektrolyt 14 Gewichtsprozent Wasser. Für die erste ringförmige Verbindung entsprechend Formel (I) ist R¹ mithin ein Methylrest, also ein Kohlenwasserstoff mit 1 C-Atomen, nämlich CH₃, für die zweite ringförmige Verbindung entsprechend Formel (I) ist R1 mithin ein Butylrest, also ein Kohlenwasserstoff mit 4 C-Atomen, nämlich C₄H₇. R² ist für die erste ringförmige Verbindung ein Propylrest, also eine Kohlenwasserstoffrest mit 3 C-Atomen, für die zweite ringförmigen Verbindungen ein Methylrest, also ein Kohlenwasserstoffrest mit einem C-Atom. A ist für die erste ringförmige Verbindung ein Piperidinring, für die zweite ringförmige Verbindung ein Pyridinring, Y für die erste ringförmige Verbindung Chlorid und für die zweite ringförmige Verbindung Methansulfonat.

Das Beispiel zeigt also einen Elektrolyten und das Messverhalten eines elektrochemischen Gassensors mit einem Elektrolyten, der eine erste ringförmige Verbindung entsprechend Formel (VII), genauer entsprechend Formel (47), eine zweite ringförmige Verbindung entsprechend Formel (VI), genauer entsprechend Formel (73) und Formel (84), Kaliumhydrogenphtalat als Puffer, CuCl₂ als Reaktionsvermittler, sowie 14% Wasser enthält.

In der gezeigten Abbildung in Fig. 27 ist auf der x-Achse die Zeit (t) in Sekunden und auf der y-Achse die gemessene Signalstärke (I) in µA dargestellt. Der Sensor wurde über insgesamt sechs Zyklen mit jeweils 1 ppm SO₂ begast. Im Zeitpunkten t1, t3, t5, t6, t7, t9 und t11 wurde jeweils mit der Begasung begonnen. Im den Zeitpunkten t2, t4, t6, t8, t10 und t12 wurde die Begasung jeweils wieder eingestellt.

Man erkennt, dass der Gassensor auf die Präsenz des Analyten, in diesem Fall SO₂, reagiert, indem das Signal direkt nach dem Start der Begasung von 0 µA bis zu einem Ausschlag von ca. 1,4 µA ansteigt und unmittelbar mit dem Ende der Begasung wieder auf 0 µA abfällt.

### Beispiel 27

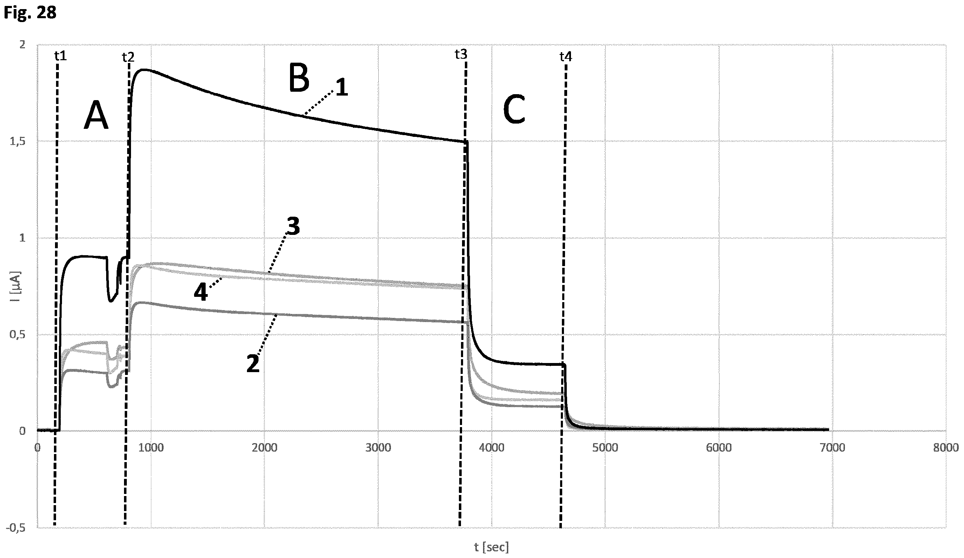

In Beispiel 27 wurden vier elektrochemischef Gassensoren, die jeweils wenigstens eine erste Elektrode, eine zweite Elektrode und einen Elektrolyten aufwies, für jeweils definierte Zeiträume mit unterschiedlichen Konzentrationen von SO₂, nämlich mit 4 ppm, mit 8 ppm und mit 2ppm SO₂, begast. Die Figur 28 zeigt die dabei erhaltenen Messkurven.

Der verwendete Elektrolyt enthält als ringförmige Verbindung 1-Butylpyridiniumchlorid und zwar ohne Methyl-Substitution (Kurve 1) und mit Methylsubstitution in ortho-Stellung (Kurve 2), metha-Stellung (Kurve 3) und para-Stellung (Kurve 4) zur Butlgruppe. Des Weiteren enthält der Elektrolyt 30 Gewichtsprozent Wasser.

Das Beispiel zeigt also einen Elektrolyten und das Messverhalten eines elektrochemischen Gassensors mit einem Elektrolyten, der eine erste ringförmige Verbindung entsprechend Formel (VII) sowie 30% Wasser enthält.

In der gezeigten Abbildung in Fig. 28 ist auf der x-Achse die Zeit (t) in Sekunden und auf der y-Achse die gemessene Signalstärke (I) in µA dargestellt. Zum Zeitpunkt t1 wurde die Begasung mit 4 ppm SO₂ begonnen. Zum Zeitpunkt t2 wurde die Begasung mit 8ppm SO₂ fortgesetzt, bei t3 die SO₂ Konzentration auf 2ppm gesenkt und bei t4 ganz beendet.

Man erkennt, dass alle vier Varianten des Gassensors auf eine Präsenz des Analyten, hier SO₂, reagieren. Änderungen der Analytkonzentrationen werden weitgehend linear angezeigt. Zwischen den Methylsubstituierten Elektrolyten besteht ein gradueller, zum unsubstituierten Ring ein signifikanter Unterschied in der Sensorempfindlichkeit.

### Beispiel 28

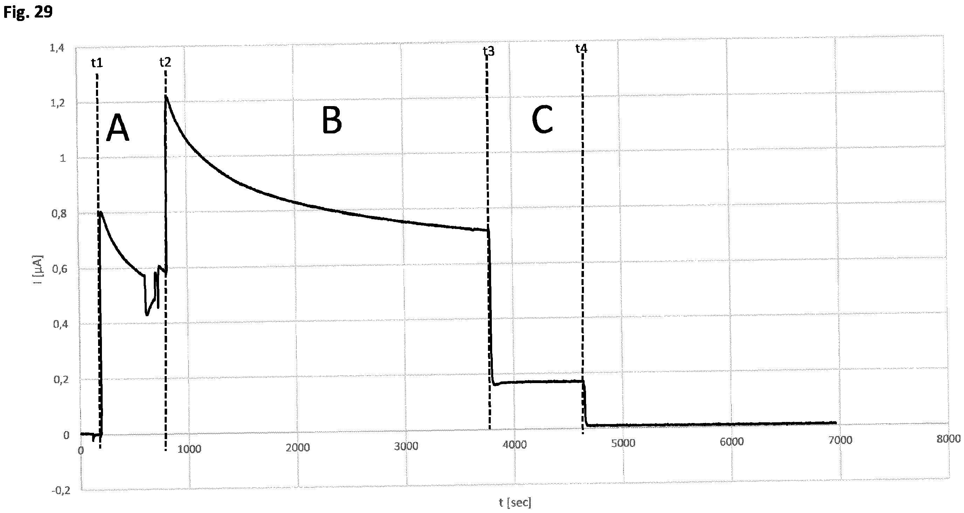

In Beispiel 28 wurde ein elektrochemischer Gassensor, der wenigstens eine erste Elektrode, eine zweite Elektrode und einen Elektrolyten aufwies, für jeweils definierte Zeiträume mit unterschiedlichen Konzentrationen von SO₂, nämlich mit 4 ppm, mit 8 ppm und mit 2ppm SO₂, begast. Figur 29 zeigt die dabei erhaltenen Messkurven.

Der verwendete Elektrolyt enthält als ringförmige Verbindung 1-Methyl-1-propylpiperiniumchlorid. Des Weiteren enthält der Elektrolyt Kaliumhydrogenphthalat als Puffer in einer Konzentration von 0,5mol/l sowie 30 Gewichtsprozent Wasser. Für die ringförmige Verbindung entsprechend Formel (I) ist R¹ mithin ein Methylrest, also ein Kohlenwasserstoff mit 1 C-Atomen, nämlich C₁H₃. R² ist ein Propylrest, also ein Kohlenwasserstoff mit 3 C-Atomen. A ist ein Piperidin-Ring, Y ist Chlorid.

Das Beispiel zeigt also einen Elektrolyten und das Messverhalten eines elektrochemischen Gassensors mit einem Elektrolyten, der eine erste ringförmige Verbindung entsprechend Formel (VII), genauer entsprechend Formel (47), sowie eine Puffersubstanz und 30% Wasser enthält.

In der gezeigten Abbildung in Fig. 29 ist auf der x-Achse die Zeit (t) in Sekunden und auf der y-Achse die gemessene Signalstärke (I) in µA dargestellt.

Man erkennt, dass der Gassensor auf die Präsenz des Analyten, in diesem Fall SO₂, reagiert, indem direkt nach dem Start der Begasung im Zeitpunkt t1 mit 4ppm SO₂ das Signal eine deutliche Änderung der Stromstärke in µA aufweist und mit der Erhöhung der Gaskonzentration im Zeitpunkt t2 auf 8ppm SO₂ annähernd eine Verdopplung des Signals eintritt. Bei Absenkung der Gaskonzentration auf 2ppm am Zeitpunkt t3 folgt auch hier das Sensorsignal der Gaskonzentrationsänderung und mit dem Ende der Begasung zu t4 geht es wieder auf den Ausgangswert zurück.

### Beispiel 29

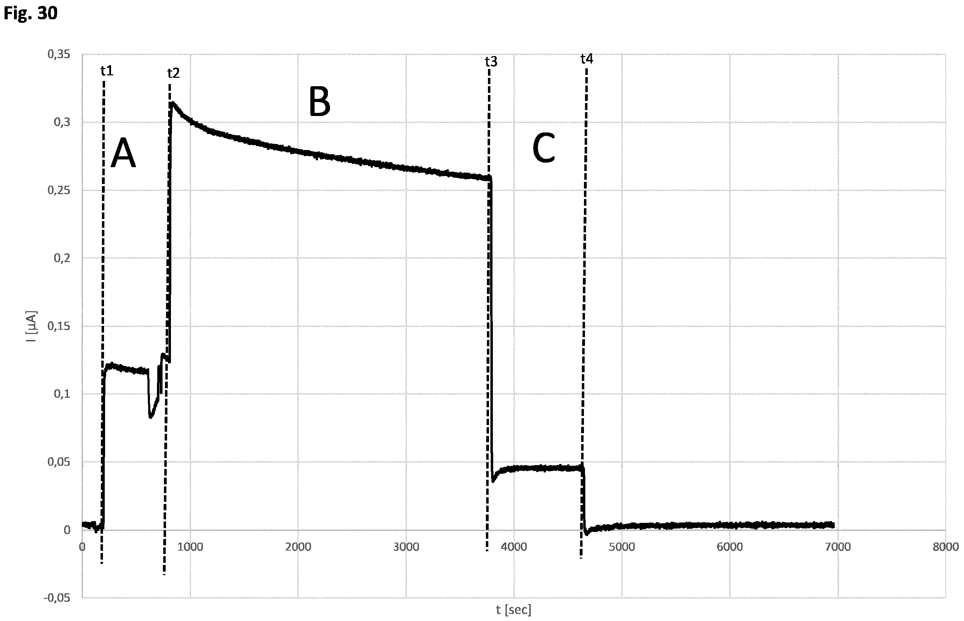

In Beispiel 29 wurde ein elektrochemischer Gassensor, der wenigstens eine erste Elektrode, eine zweite Elektrode und einen Elektrolyten aufwies, für jeweils definierte Zeiträume mit unterschiedlichen Konzentrationen von SO₂, nämlich mit 4 ppm, mit 8 ppm und mit 2ppm SO₂, begast. Die Figur 30 zeigt die dabei erhaltene Messkurve.

Der verwendete Elektrolyt enthält als ringförmige Verbindung N-Hexylpyridiniumchlorid. Des Weiteren enthält der Elektrolyt Kaliumhydrogenphthalat als Puffer in einer Konzentration von 0,5mol/l sowie 30 Gewichtsprozent Wasser. Für die ringförmige Verbindung entsprechend Formel (I) ist R¹ mithin ein Hexylrest, also ein Kohlenwasserstoff mit 6 C-Atomen, nämlich C₆H₁₁. R² ist ein Wasserstoffatom. A ist ein Pyridin-Ring, Y ist Chlorid.

Das Beispiel zeigt also einen Elektrolyten und das Messverhalten eines elektrochemischen Gassensors mit einem Elektrolyten, der eine ringförmige Verbindung entsprechend Formel (VI), genauer entsprechend Formel (75) und entsprechend Formel (86), sowie eine Puffersubstanz und 30% Wasser enthält.

In der gezeigten Abbildung in Fig. 30 ist auf der x-Achse die Zeit in Sekunden und auf der y-Achse die gemessene Signalstärke in µA dargestellt. Zum Zeitpunkt t1 wurde die Begasung mit 4 ppm SO₂ begonnen. Zum Zeitpunkt t2 wurde die Begasungskonzentration auf 8 ppm SO₂ erhöht, zum Zeitpunkt t3 auf 2 ppm gesenkt und zum Zeitpunkt t4 wieder beendet.

Man erkennt, dass der Gassensor auf die Präsenz des Analyten, in diesem Fall SO₂, reagiert, indem direkt nach dem Start der Begasung im Zeitpunkt t1 mit 4ppm SO₂ das Signal eine deutliche Änderung der Stromstärke in µA aufweist und mit der Erhöhung der Gaskonzentration im Zeitpunkt t2 auf 8ppm SO₂ annähernd eine Verdopplung des Signals eintritt. Bei Absenkung der Gaskonzentration auf 2ppm am Zeitpunkt t3 folgt auch hier das Sensorsignal der Gaskonzentrationsänderung und mit dem Ende der Begasung zu t4 geht es wieder auf den Ausgangswert zurück.

Die Erfindung ist nicht auf eine der vorbeschriebenen Ausführungsformen beschränkt, sondern in vielfältiger Weise abwandelbar.

Sämtliche aus den Ansprüchen, der Beschreibung und den Zeichnungen hervorgehenden Merkmale und Vorteile, einschließlich konstruktiver Einzelheiten, räumlicher Anordnungen und Verfahrensschritten, können sowohl für sich als auch in den verschiedensten Kombinationen erfindungswesentlich sein.

### Bezugszeichenliste

- 200: Gassensor
- 201: Elektrode
- 202: Elektrode
- 203: Gehäuse
- 204: Gaseinlass
- 205: Gasauslass
- 206: Verbindung
- 207: gasdurchlässige Membran
- 208: gasdurchlässige Membran

- 300: Elektrolyt

- t: Zeit
- I: Signalstärke

- t1, ..., t12: Zeitpunkt
- z1, ..., z20: Zyklus

## Patentansprüche

1. Elektrolyt für einen elektrochemischen Gassensor, wobei der Elektrolyt wenigstens eine ringförmige Verbindung und wenigstens 5 Gewichtsprozent Wasser enthält, wobei die ringförmige Verbindung der Formel (I)
[(A)R¹R²]+[Y]⁻
entspricht;
• wobei (A) eine Ringstruktur ist, ausgewählt aus der Gruppe Pyridinium, Piperidinium, Pyrrolidinium, Pyrrolium;
• wobei der Rest R¹ am Stickstoffatom der Ringstruktur A angeordnet ist;
• wobei R¹ ein Kohlenwasserstoffrest ist mit 1, 2, 3, 4, 5, 6, 7, 8, 9, 10, 11 oder 12 C-Atomen;
• wobei R² ein Wasserstoffatom oder ein Kohlenwasserstoffrest mit 1, 2, 3 oder 4 C-Atomen ist;
• und wobei Y ausgewählt ist aus der Gruppe enthaltend Halogenid, Cyanid, Alkylsulfonat, halogeniertes Alkylsulfonat, Acetat.

2. Elektrolyt nach Anspruch 1, **dadurch gekennzeichnet, dass** der Elektrolyt wenigstens eine weitere Komponente aufweist.

3. Elektrolyt nach Anspruch 2, **dadurch gekennzeichnet, dass** die weitere Komponente eine Puffersubstanz, ein Reaktionsvermittler, ein Zuschlag oder ein Gemisch aus zwei oder mehr dieser Komponenten ist.

4. Elektrolyt nach Anspruch 3, **dadurch gekennzeichnet, dass** die Puffersubstanz ausgewählt ist aus der Gruppe enthaltend EDTA, EGTA, TEA, Bis-Tris, ADA, Tris, Kaliumhydrogenphthalat, Pyridiniumformiat, MOPS, HEPES, CHES, TRICIN, PIPES oder Carbonat.

5. Elektrolyt nach Anspruch 3, **dadurch gekennzeichnet, dass** der Reaktionsvermittler ausgewählt ist aus der Gruppe CuCl₂, CoCl₃, Chinon, Chinonderivate, MnCl₂.

6. Elektrolyt nach Anspruch 3, **dadurch gekennzeichnet, dass** der Zuschlag ausgewählt ist aus Methansulfonsäure, Kaliumacetat, EMIM-Acetat, Pyridinum-Acetat, BaCl₂, CaCl₂, EuCl₂.

7. Elektrolyt nach einem der vorhergehenden Ansprüche, **dadurch gekennzeichnet, dass** der Elektrolyt wenigstens eine zweite ringförmige Verbindung aufweist, wobei auch die zweite ringförmige Verbindung der Formel (I)
[(A)R¹R²]⁺[Y]⁻
entspricht;
• wobei (A) eine Ringstruktur ist, ausgewählt aus der Gruppe Pyridinium, Piperidinium, Pyrrolidinium, Pyrrolium;
• wobei der Rest R¹ am Stickstoffatom der Ringstruktur A angeordnet ist;
• R¹ ein Kohlenwasserstoffrest ist mit 1, 2, 3, 4, 5, 6, 7, 8, 9, 10, 11 oder 12 C-Atomen;
• wobei R² ein Wasserstoffatom oder ein Kohlenwasserstoffrest mit 1, 2, 3 oder 4 C-Atomen ist;
• und wobei Y ausgewählt ist aus der Gruppe enthaltend Halogenid, Cyanid, Alkylsulfonat, halogeniertes Alkylsulfonat, Acetat;
und wobei die zweite ringförmige Verbindung eine andere Verbindung ist als die erste ringförmige Verbindung.

8. Elektrolyt nach einem der vorhergehenden Ansprüche **dadurch gekennzeichnet, dass** R¹ ausgewählt ist aus CH₃, C₂H₅, C₃H₇, C₄H₉, C₅H₁₁, C₆H₁₃, C₇H₁₅, C₈H₁₇, C₉H₁₉, C₁₀H₂₁, C₁₁H₂₃, C₁₂H₂₅.

9. Elektrolyt nach einem der vorhergehenden Ansprüche, **dadurch gekennzeichnet, dass** R² ausgewählt ist aus H, CH₃, C₂H₅, C₃H₇, C₄H₉.

10. Elektrolyt nach einem der vorhergehenden Ansprüche 7 bis 9, **dadurch gekennzeichnet, dass** die erste ringförmige Verbindung eine Pyridin-Verbindung entsprechend Formel (VI) ist und dass die zweite ringförmige Verbindung eine Piperidin-Verbindung entsprechend Formel (VII) ist.

11. Elektrolyt nach einem der Ansprüche 7 bis 9, **dadurch gekennzeichnet, dass** sowohl die erste ringförmige Verbindung als auch die zweite ringförmige Verbindung eine Pyridin-Verbindung entsprechend Formel (VI) ist.

12. Elektrolyt nach einem der Ansprüche 7 bis 9, **dadurch gekennzeichnet, dass** sowohl die erste ringförmige Verbindung als auch die zweite ringförmige Verbindung eine Piperidin-Verbindung entsprechend Formel (VII) ist.

13. Elektrolyt nach einem der Ansprüche 7 bis 9, **dadurch gekennzeichnet, dass** die erste ringförmige Verbindung eine Pyrrolidin-Verbindung entsprechend Formel (VIII) ist und dass die zweite ringförmige Verbindung eine Pyrrol-Verbindung entsprechend Formel (IX) ist.

14. Elektrolyt nach einem der Ansprüche 7 bis 9, **dadurch gekennzeichnet, dass** sowohl die erste als auch die zweite ringförmige Verbindung eine Pyrrolidin-Verbindung entsprechend Formel (VIII) ist.

15. Elektrolyt nach einem der Ansprüche 7 bis 9, **dadurch gekennzeichnet, dass** sowohl die erste als auch die zweite ringförmige Verbindung eine Pyrrol-Verbindung entsprechend Form (IX) ist.

16. Elektrolyt nach einem der Ansprüche 7 bis 9, **dadurch gekennzeichnet, dass** die erste ringförmige Verbindung eine Pyrrolidin-Verbindung entsprechend Formel (VII) ist und dass die zweite ringförmige Verbindung eine Pyridin-Verbindung entsprechend Formel (VI) ist.

17. Elektrolyt nach einem der Ansprüche 7 bis 9, **dadurch gekennzeichnet, dass** die erste ringförmige Verbindung eine Pyrrolidin-Verbindung entsprechend Formel (VIII) ist und dass die zweite ringförmige Verbindung eine Piperidin-Verbindung entsprechend Formel (VII) ist.

18. Elektrolyt nach einem der Ansprüche 7 bis 9, **dadurch gekennzeichnet, dass** die erste ringförmige Verbindung eine Pyrrol-Verbindung entsprechend Formel (IX) ist und dass die zweite ringförmige Verbindung eine Pyridin-Verbindung entsprechend Formel (VI) ist.

19. Elektrolyt nach einem der Ansprüche 7 bis 9, **dadurch gekennzeichnet, dass** die erste ringförmige Verbindung eine Pyrrol-Verbindung entsprechend Formel (IX) ist und dass die zweite ringförmige Verbindung eine Piperidin-Verbindung entsprechend Formel (VII) ist.

20. Elektrolyt nach einem der vorhergehenden Ansprüche, **dadurch gekennzeichnet, dass** die erste ringförmige Verbindung und die zweite ringförmige Verbindung im molaren Verhältnis 1:1 bis 1:9 zueinander vorliegen.

21. Elektrolyt nach einem der vorhergehenden Ansprüche, **dadurch gekennzeichnet, dass** Y ausgewählt ist aus Cl, Br, F, I, CN, CH₃SO₃, CF3SO3, CH3COO.

22. Elektrolyt nach einem der vorhergehenden Ansprüche, **dadurch gekennzeichnet, dass** der Elektrolyt zwischen wenigstens 5 und höchsten 50 Gewichtsprozent H₂O, zwischen wenigstens 5 und höchstens 40 Gewichtsprozent, zwischen wenigstens 5 und höchstens 30 Gewichtsprozent H₂O enthält oder zwischen wenigstens 5 und höchstens 20 Gewichtsprozent H₂O enthält.

23. Elektrochemischer Gassensor mit wenigstens einer ersten Elektrode, wenigstens einer zweiten Elektrode und mit einem Elektrolyten nach einem der Ansprüche 1 bis 22.

24. Verwendung eines elektrochemischen Gassensors entsprechend Anspruch 13 zum Nachweis von SO₂, NO₂, O₃ und/oder H₂S.
